(19) Europäisches Patentamt
European Patent Office
Office européen des brevets

(11) EP 3 247 770 B1

(12) **EUROPÄISCHE PATENTSCHRIFT**

(45) Veröffentlichungstag und Bekanntmachung des Hinweises auf die Patenterteilung:
**10.04.2019 Patentblatt 2019/15**

(21) Anmeldenummer: **16703725.8**

(22) Anmeldetag: **20.01.2016**

(51) Int Cl.:
***C09K 11/06*** *(2006.01)* ***H01L 51/50*** *(2006.01)*
***H01L 51/00*** *(2006.01)*

(86) Internationale Anmeldenummer:
**PCT/EP2016/051124**

(87) Internationale Veröffentlichungsnummer:
**WO 2016/116497 (28.07.2016 Gazette 2016/30)**

(54) **ORGANISCHE MOLEKÜLE, INSBESONDERE ZUR VERWENDUNG IN OPTOELEKTRONISCHEN BAUELEMENTEN**

ORGANIC MOLECULES, IN PARTICULAR, FOR USE IN OPTOELECTRONIC COMPONENTS

MOLÉCULES ORGANIQUES DESTINÉES EN PARTICULIER À ÊTRE UTILISÉES DANS DES COMPOSANTS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.01.2015 EP 15151870**
**20.05.2015 DE 102015107905**

(43) Veröffentlichungstag der Anmeldung:
**29.11.2017 Patentblatt 2017/48**

(73) Patentinhaber: **cynora GmbH**
**76646 Bruchsal (DE)**

(72) Erfinder:
- **AMBROSEK, David**
**10437 Berlin (DE)**
- **DANZ, Michael**
**76344 Eggenstein-Leopoldshafen (DE)**
- **FLÜGGE, Harald**
**76135 Karlsruhe (DE)**
- **FRIEDRICHS, Jana**
**76137 Karlsruhe (DE)**
- **GRAB, Tobias**
**76133 Karlsruhe (DE)**
- **JACOB, Andreas**
**30449 Hannover (DE)**
- **SEIFERMANN, Stefan**
**77815 Brühl (DE)**
- **VOLZ, Daniel**
**76137 Karlsruhe (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2014/061961 WO-A1-2014/146750**
**WO-A1-2016/003054 DE-A1-102010 045 405**

- **CHUN LIU ET AL: "Oxygen-promoted Pd/C-catalyzed Suzuki-Miyaura reaction of potassium aryltrifluoroborates", CHINESE CHEMICAL LETTERS., 1. Januar 2016 (2016-01-01), XP055270003, GB ISSN: 1001-8417, DOI: 10.1016/j.cclet.2015.12.022**
- **ZIYI GE ET AL: "Solution-processible Fluorinated Carbazole Derivative for Phosphorescent Organic Light-emitting Diodes", CHEMISTRY LETTERS, Bd. 37, Nr. 3, 1. Januar 2008 (2008-01-01) , Seiten 294-295, XP055217000, ISSN: 0366-7022, DOI: 10.1246/cl.2008.294**
- **PETER STROHRIEGL ET AL: "Novel host materials for blue phosphorescent OLEDs", PROCEEDINGS OF SPIE, Bd. 8829, 27. September 2013 (2013-09-27), Seite 882906, XP055216974, ISSN: 0277-786X, DOI: 10.1117/12.2023305**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 247 770 B1

## Beschreibung

Die Erfindung betrifft rein organische Moleküle und deren Verwendung in organischen lichtemittierenden Dioden (OLEDs) und in anderen optoelektronischen Bauelementen.

## Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermally activated delayed fluorescence oder auch singlet harvesting). Hierbei können beispielsweise Cu(I)-Komplexe verwendet werden, die aufgrund eines geringen Energieabstandes zwischen dem untersten Triplett-Zustand $T_1$ und dem darüberliegenden Singulett-Zustand $S_1$ ($\Delta E(S_1-T_1)$ Triplett-Exitonen thermisch in einen Singulett-Zustand rückbesetzen können. Neben der Verwendung von Übergangsmetallkomplexen können auch rein organische Moleküle diesen Effekt ausnutzen.

Einige solcher TADF-Materialien wurden bereits in ersten optoelektronischen Bauelementen eingesetzt. Die bisherigen Lösungen weisen jedoch Nachteile und Probleme auf: Die TADF-Materialien weisen in den optoelektronischen Bauelementen oftmals keine ausreichende Langzeitstabilität, keine ausreichende thermische oder keine ausreichende chemische Stabilität gegenüber Wasser und Sauerstoff auf. Außerdem sind nicht alle wichtigen Emissionsfarben verfügbar. Weiterhin sind einige TADF-Materialien nicht verdampfbar und dadurch für den Einsatz in kommerziellen optoelektronischen Bauteilen nicht geeignet. Auch weisen einige TADF-Materialien keine passenden Energielagen zu den weiteren im optoelektronischen Bauteil verwendeten Materialien (z.B. HOMO-Energien von TADF-Emittern von größer gleich - 5,9 eV) auf. Nicht mit allen TADF-Materialien lassen sich ausreichend hohe Effizienzen der optoelektronischen Bauelemente bei hohen Stromdichten bzw. hohe Leuchtdichten erreichen. Weiterhin sind die Synthesen einiger TADF-Materialien aufwendig.

Aus der WO 2016/003054 A1 sind lichtemittierende Bauelemente bekannt. Weiter sind aus der WO 2014/146750 A1 Verbindungen bekannt, die sich als Vorstufen für elektrolumineszierende Materialien eignen. Auch aus der DE 10 2010 045405 A1 sind Materialien für elektrolumineszierende Vorrichtungen bekannt. C. Liu et al. (Chinese Chemical Letters 2016, 27, 5, 631-634) beschreibt die Pd/C katalysierte Suzuki-Miyaura Reaktion von Kaliumaryltrifluoroboraten.

## Beschreibung

Die Erfindung betrifft in einem ersten Aspekt die Bereitstellung von organischen Molekülen, die eine Struktur der Formel 1 aufweisen oder eine Struktur der Formel 1 haben:

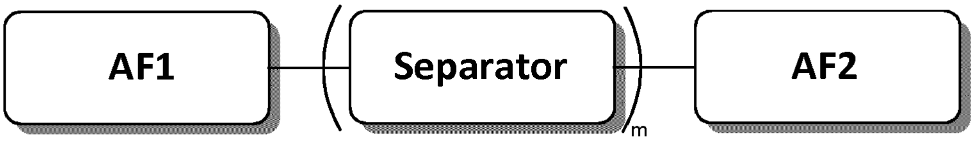


**Formel 1**

wobei gilt:

m ist 0 oder 1;
AF1 ist eine erste chemische Einheit, aufweisend ein konjugiertes System, insbesondere mindestens sechs in Konjugation stehende $\pi$-Elektronen (z. B. in Form mindestens eines aromatischen Systems);
AF2 ist eine zweite chemische Einheit, aufweisend ein konjugiertes System, insbesondere mindestens sechs in Konjugation stehende $\pi$-Elektronen (z. B. in Form mindestens eines aromatischen Systems;
AF1 $\neq$ AF2;
Separator ist eine chemische Einheit, die AF1 und AF2 verknüpft und deren elektronische Kommunikation über konjugierte Bindungen miteinander unterbricht;
und wobei der Separator an zwei voneinander beabstandeten Stellen an das konjugierte System von AF1 und von AF2 angebunden ist;
und wobei mindestens eine der beiden chemischen Einheiten AF1 oder AF2 substituiert ist durch eine fluorhaltige, elektronenziehende Gruppe FG, ausgewählt aus $CR^{**}_2F$, $CF_3$, $CF_2R^{**}$, $SF_5$, N-$(CF_3)_1$, N-$(CF_3)_2$, O-$CF_3$, S-$CF_3$, $CF_2CO_2R^*$ und einer Gruppe E*, die eine Aryl- oder Heteroarylgruppe mit 6 bis 18 aromatischen Ringatomen ist, die mit einem oder mehreren Resten $R^8$ substituiert sein kann, und die als Bestandteile des aromatischen Rings eine oder mehrere Gruppen V* enthält, wobei die Gruppen V* bei jedem Auftreten gleich oder verschieden gewählt sind aus =C(F)- und =C($CF_3$)-.

In einer Ausführungsform ist nur eine der beiden chemischen Einheiten AF1 oder AF2 substituiert durch eine fluorhaltige, elektronenziehende Gruppe FG.

In einer weiteren Ausführungsform ist die chemische Einheit AF1 substituiert durch eine fluorhaltige, elektronenziehende Gruppe FG und die chemische Einheit AF2 ist ausgewählt aus einer der in Tabelle 2 aufgeführten Strukturen, oder weist eine Struktur der Formel 3 auf.

R** ist bei jedem Auftreten gleich oder verschieden H, eine lineare Alkyl, Alkenyl-, Alkinylgruppe mit 3 bis 40 C-Atomen, oder eine lineare Alkenyl- oder Alkinylgruppe mit 2 bis 40 C-Atomen oder eine verzweigte oder cyclische Alkyl-, Alkenyl-, Alkinylgruppe mit 3 bis 40 C-Atomen, die optional partiell fluoriert oder perfluoriert ist;
und wobei gilt:

R* ist bei jedem Auftreten unabhängig voneinander ausgewählt aus der Gruppe bestehend aus H, Deuterium, Phenyl, Naphthyl, $N(R^2)_2$, -CN, -NC, -SCN, -$CF_3$, -$NO_2$, C(=O)OH, C(=O)$OR^3$, C(=O)$N(R^3)_2$, C(=O)$SR^3$, C(=S)$SR^3$, $Si(R^4)_3$, $B(OR^5)_2$, $B(N(R^6)_2)_2$, C(=O)$R^3$, P(=O)$(R^7)_2$, As(=O)$(R^7)_2$, P(=S)$(R^7)_2$, As(=S)$(R^7)_2$, S(=O)$R^3$, S=$NR^3$, S(=O)$NR^3$, $S(=O)_2NR^3$, $S(=O)_2R^3$, O-$S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C{=}CR^9$-, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-, -C=N-, -C(=O)O-, -C(=O)$N(R^3)$-, -P(=O)$(R^7)$-, -As(=O)$(R^7)$-, -P(=S)$(R^7)$, -As(=S)$(R^7)$-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R* auch miteinander ein mono- oder polycyclisches, aliphatisches, aromatisches und/oder benzoannelliertes Ringsystem bilden. In einer Ausführungsform ist das Ringsystem, dass gebildet werden kann, auf ein monozyklisches aliphatisches Ringsystem mit insgesamt fünf oder sechs Ringgliedern beschränkt.

$R^2$ ist bei jedem Auftreten unabhängig voneinander ausgewählt aus der Gruppe Ringatomen, das jeweils durch einen oder mehrere Reste $R^2$ substituiert

bestehend aus H, Deuterium, Phenyl, Naphthyl, $CF_3$, C(=O)OR$^3$, C(=O)N(R$^2$)$_2$, Si(R$^4$)$_3$, C(=O)R$^3$, P(=O)(R$^7$)$_2$, As(=O)(R$^7$)$_2$ P(=S)(R$^7$)$_2$, As(=S)(R$^7$)$_2$, S(=O)R$^3$, S(=O)$_2$R$^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R$^9$ substituiert sein kann, wobei eine oder mehrere benachbarte $CH_2$-Gruppen durch -R$^9$C=CR$^9$-, -C≡C-, bzw. eine benachbarte $CH_2$-Gruppe durch -Si(R$^4$)$_2$-,-Ge(R$^4$)$_2$-, -Sn(R$^4$)$_2$, -C(=O)-, -C(=S)-, -C(=Se)-, -C=N-, -C(=O)O-, -C(=O)N(R$^3$)-, -P(=O)(R$^7$)-,-As(=O)(R$^7$)-, -P(=S)(R$^7$)-, -As(=S)(R$^7$)-, -S(=O)-, -S(=O)$_2$-, -NR$^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste R$^9$ substituiert sein kann, oder eine Aryloxy- oder Heteroaryloxygruppe mit 5 bis 60 aromatischen Ringatomen, die durch einen oder mehrere Reste R$^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R$^9$ substituiert sein kann, oder eine Kombination dieser Systeme; dabei können zwei oder mehrere dieser Substituenten R$^2$ auch miteinander ein mono- oder polycyclisches, aliphatisches, aromatisches und/oder benzoannelliertes Ringsystem bilden;

R$^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten R$^3$ auch miteinander ein mono- oder polycyclisches, aliphatisches Ringsystem bilden;

R$^4$ ist bei jedem Auftreten unabhängig voneinander ausgewählt aus der Gruppe bestehend aus H, Deuterium, Phenyl, Naphthyl, N(R$^2$)$_2$, CN, $CF_3$, OH, C(=O)OR$^3$, C(=O)N(R$^3$)$_2$, C(=O)R$^3$, P(=O)(R$^7$)$_2$, As(=O)(R$^7$)$_2$, P(=S)(R$^7$)$_2$, As(=S)(R$^7$)$_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R$^9$ substituiert sein kann, wobei eine oder mehrere benachbarte $CH_2$-Gruppen durch -R$^9$C=CR$^9$-, -C≡C-, bzw. eine benachbarte $CH_2$-Gruppe durch -Si(R$^4$)$_2$-, -Ge(R$^4$)$_2$-, -Sn(R$^4$)$_2$-, -C(=O)-,-C(=S)-, -C(=Se)-, -C=N-, -C(=O)O-, -C(=O)N(R$^3$)-, -P(=O)(R$^7$)-, -As(=O)(R$^7$)-, -P(=S)(R$^7$),-As(=S)(R$^7$)-, -S(=O)-, -S(=O)$_2$-, -NR$^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste R$^8$ substituiert sein kann, oder eine Aryloxy- oder Heteroaryloxygruppe mit 5 bis 60 aromatischen Ringatomen, die durch einen oder mehrere Reste R$^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R$^9$ substituiert sein kann, oder eine Kombination dieser Systeme; dabei können zwei oder mehrere dieser Substituenten R$^4$ auch miteinander ein mono- oder polycyclisches, aliphatisches, aromatisches und/oder benzoannelliertes Ringsystem bilden;

R$^5$ ist bei jedem Auftreten unabhängig voneinander ausgewählt aus der Gruppe bestehend aus Phenyl, Naphthyl, $CF_3$, C(=O)R$^3$, P(=O)(R$^7$)$_2$, As(=O)(R$^7$)$_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R$^9$ substituiert sein kann, wobei eine oder mehrere benachbarte $CH_2$-Gruppen durch -R$^9$C=CR$^9$-, -C≡C-, bzw. eine benachbarte $CH_2$-Gruppe durch -Si(R$^4$)$_2$-, -Ge(R$^4$)$_2$-, -Sn(R$^4$)$_2$, -C(=O)-,-C(=S)-, -C(=Se)-, -C=N-, -C(=O)O-, -C(=O)N(R$^3$)-, -P(=O)(R$^7$)-, -As(=O)(R$^7$)-, -P(=S)(R$^7$)-,-As(=S)(R$^7$)-, -S(=O)-, -S(=O)$_2$-, -NR$^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste R$^9$ substituiert sein kann, oder eine Aryloxy- oder Heteroaryloxygruppe mit 5 bis 60 aromatischen Ringatomen, die durch einen oder mehrere Reste R$^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R$^9$ substituiert sein kann, oder eine Kombination dieser Systeme; dabei können zwei oder mehrere dieser Substituenten R$^5$ auch miteinander ein mono- oder polycyclisches, aliphatisches, aromatisches und/oder benzoannelliertes Ringsystem bilden;

R$^6$ ist bei jedem Auftreten unabhängig voneinander ausgewählt aus der Gruppe bestehend aus Phenyl, Naphthyl, $CF_3$, Si(R$^4$)$_3$, C(=O)R$^3$, P(=O)(R$^7$)$_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-,

Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch-$Si(R^4)_2$-, -$Ge(R^4)_2$-, -$Sn(R^4)_2$, -C(=O)-,-C(=S)-, -C(=Se)-, -C=N-, -C(=O)O-, -C(=O)N($R^3$)-, -P(=O)($R^7$)-, -As(=O)($R^7$)-, -P(=S)($R^7$),-As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, C(=O)$OR^3$, C(=O)$N(R^3)_2$, $Si(R^4)_3$, C(=O)$R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch -$Si(R^4)_2$-,-$Ge(R^4)_2$-, -$Sn(R^4)_2$, -C(=O)-, -C(=S)-, -C(=Se)-, -C=N-, -C(=O)O-, -C(=O)N($R^3$)-, -P(=O)($R^7$)-,-As(=O)($R^7$)-, -P(=S)($R^7$), -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, C(=O)$OR^3$, C(=O)$N(R^3)_2$, $Si(R^4)_3$, $B(OR^5)_2$, C(=O)$R^3$, P(=O)$(R^7)_2$, P(=S)$(R^7)_2$, As(=O)$(R^7)_2$, P(=S)$(R^7)_2$, S(=O)$R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-,-C=N-, -C(=O)O-, -C(=O)N($R^3$)-, -P(=O)($R^7$)-, -As(=O)($R^7$)-, -P(=S)($R^7$), -As(=S)($R^7$)-, -S(=O)-,-$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind insbesondere N, O, und S. Werden in der Beschreibung der Erfindung davon abweichende Definitionen angegeben, beispielsweise bezüglich der Zahl der aromatischen Ringatome oder der enthaltenen Heteroatome, so gelten diese abweichenden Definitionen.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind insbesondere ausgewählt aus, N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (insbesondere weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch Systeme wie 9,9'-Diarylfluoren, Triarylamin, Diarylether, Stilben etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen.

In einer Ausführungsform sind die chemischen Einheiten AF1 und AF2 derart über den Separator S miteinander verknüpft, dass die elektronische Kommunikation zwischen ihnen unterbrochen wird. Diese Unterbrechung ist durch eine Lokalisierung der Grenzorbitale HOMO und LUMO auf separaten Molekülteilen gekennzeichnet, sodass ein Charge-Transfer Übergang ermöglicht wird.

Die elektronische Kommunikation zwischen den zwei chemischen Einheiten AF1 und AF2 über konjugierte Bindungen mit einem optionalen Separator ist unterbrochen, wenn die Grenzorbitale HOMO und LUMO auf separaten Molekülteilen lokalisiert sind, sodass ein Charge-Transfer Übergang ermöglicht wird. Die Lokalisierung der Grenzorbitale HOMO oder LUMO wird dabei mithilfe der Dichtefunktionaltheorie (DFT) mit dem BP86-Funktional (Becke, A. D. Phys. Rev. A1988, 38, 3098-3100; Perdew, J. P. Phys. Rev. B1986, 33, 8822-8827) visualisiert: Aus der Einelektronen-Wellenfunktion wird die Einelektronendichte durch Quadrieren berechnet und über den Raum integriert, den der untersuchte Molekülteil einnimmt. Dieser Raum kann aus den Atomkoordinaten und den van-der-Waals-Radien der Atome bestimmt werden. Die resultierende Zahl entspricht dem Anteil des Orbitals auf dem Molekülteil. Eine mehrheitliche

Trennung der Grenzorbitale entspricht dabei einem Überlappungsparameter O im Bereich 0,1 bis 20%, um einen Charge-Transfer Übergang zu ermöglichen. Der Überlappungsparameter O zwischen der HOMO-Wellenfunktion $\phi_a$ und der der LUMO-Wellenfunktion $\phi_b$ ergibt sich aus dem Integral über den gesamten Raum über den jeweils kleineren Wert der quadrierten Wellenfunktion:

$$O = \int \rho \, \mathrm{d}\tau \text{ mit } \rho = \begin{cases} \varphi_a^2, & \text{wenn } |\varphi_a| < |\varphi_b| \\ \varphi_b^2, & \text{wenn } |\varphi_a| \geq |\varphi_b| \end{cases}$$

AF2 weist im Vergleich zu AF1 einen betragsmäßig niedrigeren HOMO-Zahlenwert auf (und damit auch entsprechend einen betragsmäßig niedrigeren LUMO-Zahlenwert) als AF2 ($|E_{HOMO}(AF2)| < |E_{HOMO}(AF1)|$ und $|E_{LUMO}(AF2)| < |E_{LUMO}(AF1)|$).

In einer Ausführungsform zeichnen sich die organischen Moleküle dadurch aus, dass

- die Differenz der Energie des HOMO der zweiten chemischen Einheit AF2 und der Energie des HOMO der ersten chemischen Einheit AF1 > 0,8 eV ist (Δ HOMO = HOMO(AF2)-HOMO(AF1) > 0,8 eV);
- die Differenz der Energie des LUMO der zweiten chemischen Einheit AF2 und der Energie des LUMO der ersten chemischen Einheit AF1 > 0,8 eV ist (Δ LUMO = LUMO(AF2)-LUMO(AF1) > 0,8 eV); und/oder
- die Differenz der Energie des LUMO der ersten chemischen Einheit AF1 und der Energie des HOMO der zweiten chemischen Einheit AF2 > 0,9 eV ist (Δ Gap = LUMO (AF1) - HOMO(AF2)> 0,9 eV).

Dabei werden die Energiewerte HOMO(AF1), HOMO(AF2), LUMO(AF1), LUMO(AF2) mithilfe der Dichtefunktionaltheorie (DFT) berechnet, wobei die Anknüpfungspositionen der ambifunktionalen Einheiten und der Separatoren mit einem Wasserstoffatom entsprechend ihrer chemischen Valenzen abgesättigt werden. Die angegebenen Grenzen beziehen sich auf Orbitalenergien in eV, die mit dem BP86-Funktional berechnet werden (Becke, A. D. Phys. Rev. A1988, 38, 3098-3100; Perdew, J. P. Phys. Rev. B1986, 33, 8822-8827).

In einer Ausführungsform ist mindestens eine AF mit oder $CF_3$ substituiert.

In einer Ausführungsform der Beschreibung umfasst die chemische Einheit AF1 eine Struktur der Formel 2-1 oder hat eine Struktur der Formel 2-1:

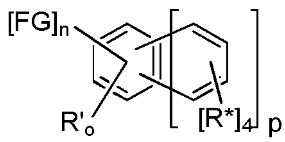

**Formel 2-1**

wobei für Formel 2-1 gilt:

n = 1 bis 5;
o = 1 bis 5;
n + o = 5;
p = 0 oder 1;
R' = Anknüpfungspunkt an den Separator, Anknüpfungspunkt an die andere chemische Einheit AF2 oder ein Rest R*; wobei genau ein R' einen Anknüpfungspunkt an den Separator oder an die andere chemische Einheit AF2 darstellt.

und wobei ansonsten die bei Formel 1 genannten Definitionen gelten.

In einer weiteren Ausführungsform der Beschreibung hat die chemische Einheit AF1 eine Struktur oder weist eine Struktur ausgewählt aus einer der folgenden Strukturen auf:

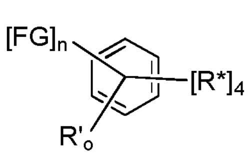

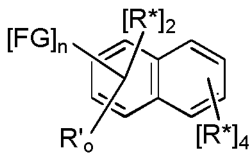

**Formel 2-1a** **Formel 2-1b**

und wobei die bei Formel 2-1 genannten Definitionen gelten.

In einer Ausführungsform der Erfindung hat die chemische Einheit AF1 eine Struktur der Formel 2-2 oder weist eine Struktur der Formel 2-2 auf

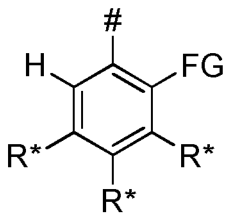

**Formel 2-2**

mit

# = Anknüpfungspunkt an den Separator oder Anknüpfungspunkt an die chemische Einheit AF2;

und wobei ansonsten die bei Formel 1 genannten Definitionen gelten.

In einer weiteren Ausführungsform hat die chemische Einheit AF1 eine Struktur oder weist eine Struktur auf ausgewählt aus einer folgenden Strukturen auf:

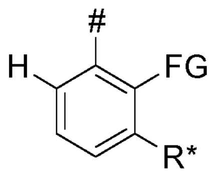

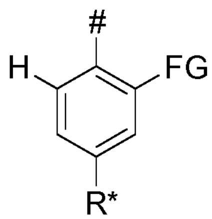

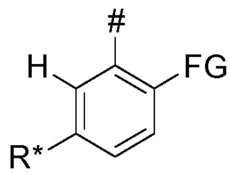

**Formel 2-2A** **Formel 2-2B** **Formel 2-2C**

mit

# = Anknüpfungspunkt an den Separator oder Anknüpfungspunkt an die chemische Einheit AF2;

und wobei ansonsten die bei Formel 1 genannten Definitionen gelten.

In einer Ausführungsform weist die chemische Einheit AF1 eine der in Tabelle 1 angegebenen Strukturen auf.

**Tabelle 1:** Beispiele für Fluor-haltige chemische Einheiten AF1, wobei der Anknüpfungspunkt an den Separator oder an die chemische Einheit AF2 mit Kleinbuchstaben a bis e bezeichnet sind

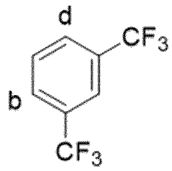
150

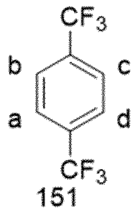
151

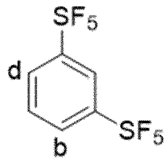
342

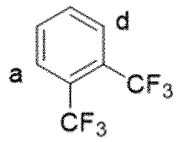
153

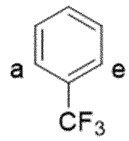
154

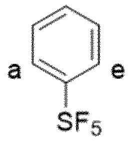
184

In einer Ausführungsform sind die chemischen Einheiten AF2 ausgewählt aus den in Tabelle 2 aufgeführten Verbindungen und werden wie in Formel 1 angegeben mit einer Fluor-haltigen AF1 kombiniert.

**Tabelle 2:** Auflistung der zweiten chemischen Einheiten AF2.

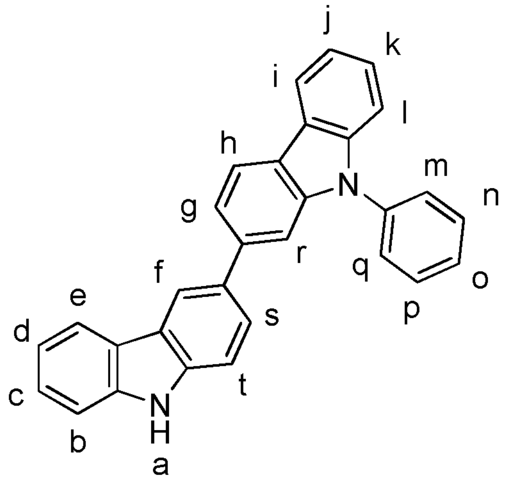

1

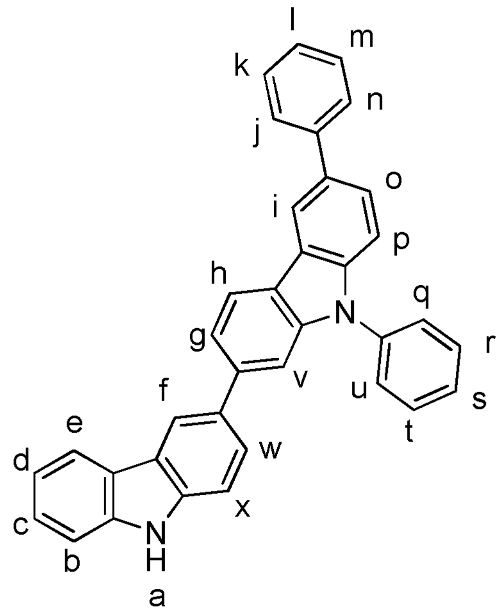

2

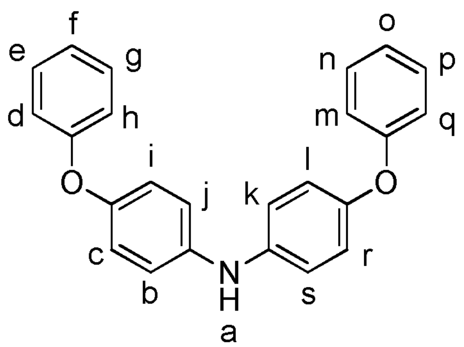

3

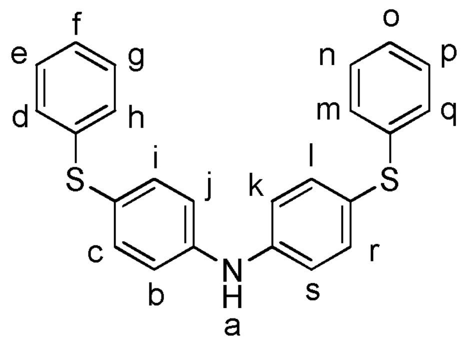

4

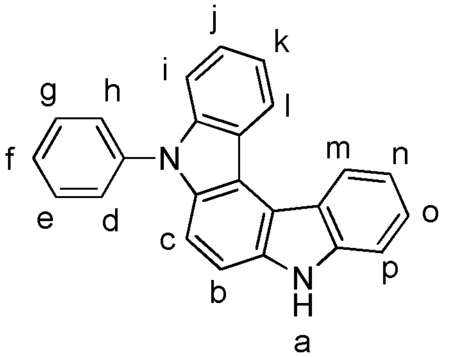

6

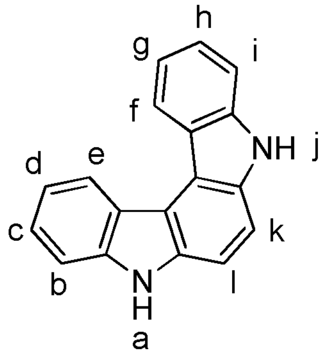

7

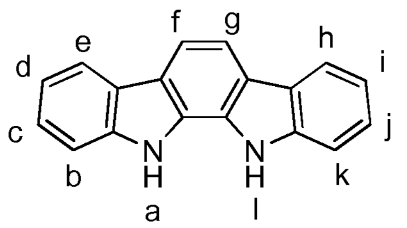

8

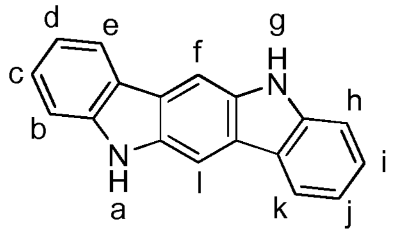

10

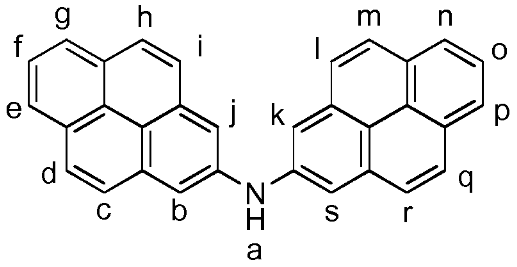

11

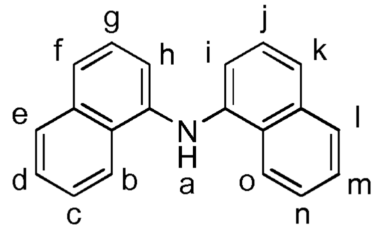

12

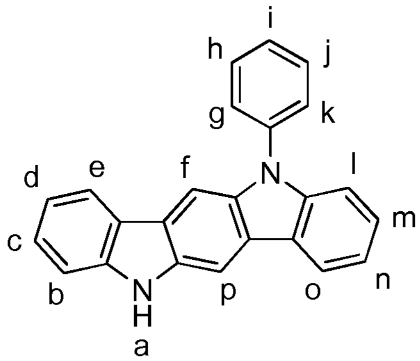

24

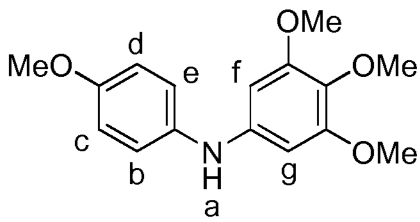

26

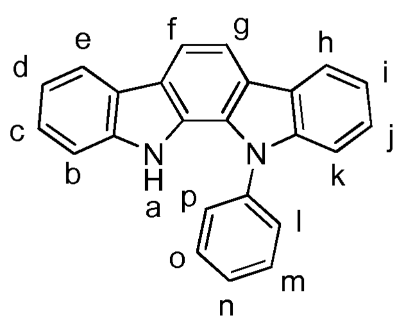

21

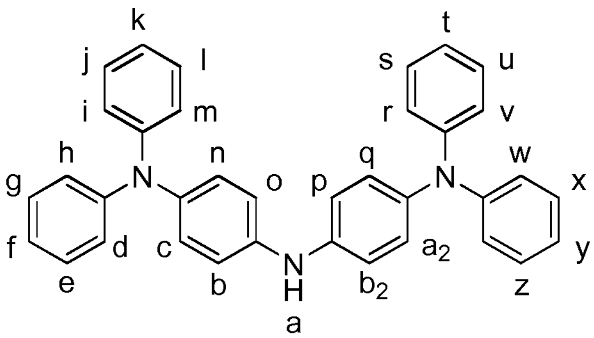

22

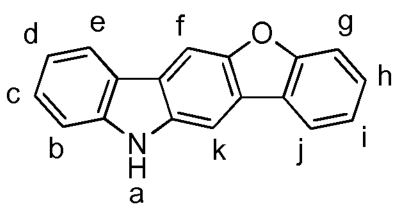

30

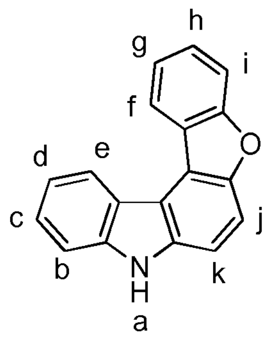

31

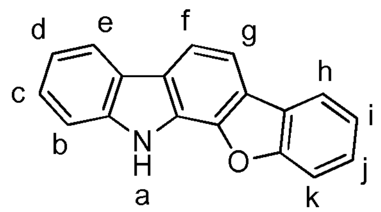

32

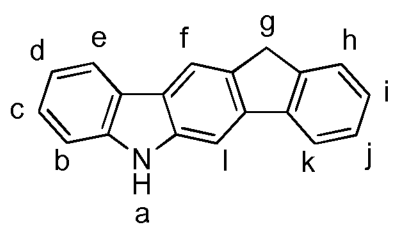

33

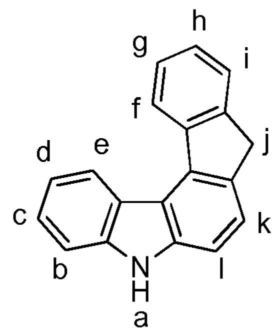

34

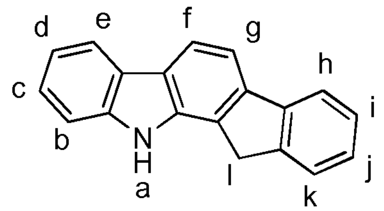

35

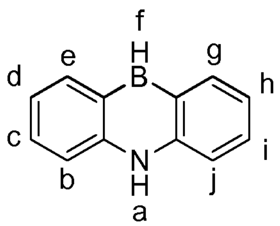

38

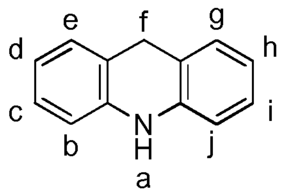

39

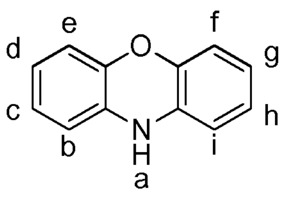

41

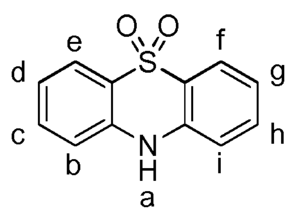

44

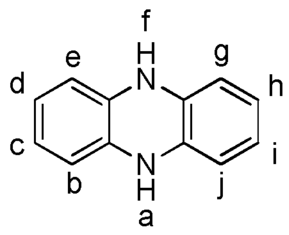

49

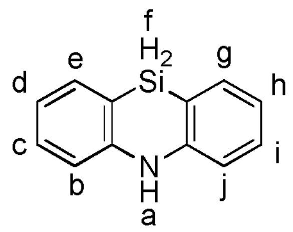

53

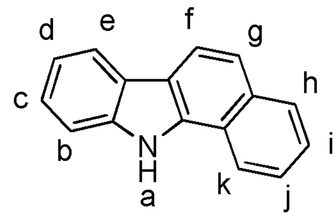

54

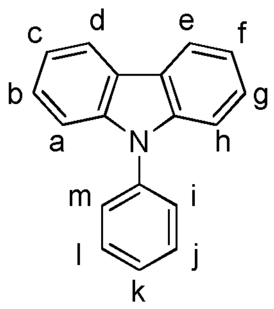

55

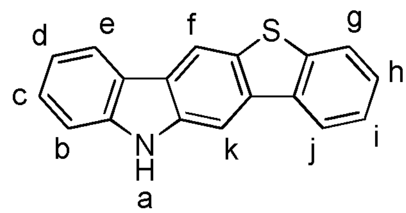

58

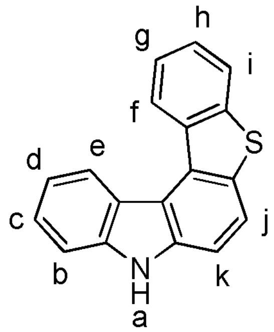

59

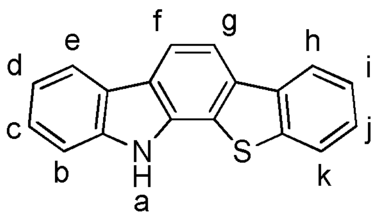

60

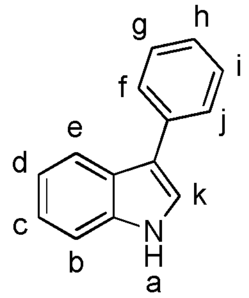

63

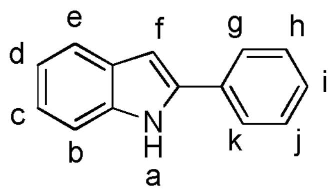

64

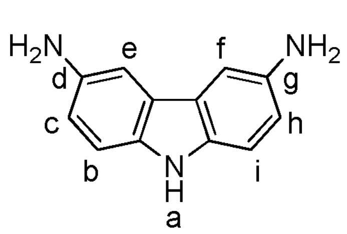

66

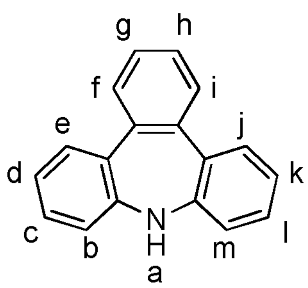

71

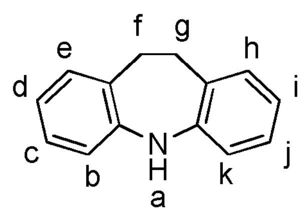

72

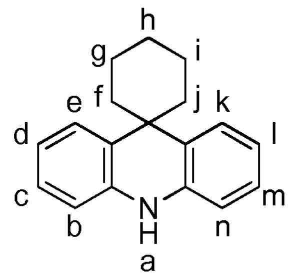

73

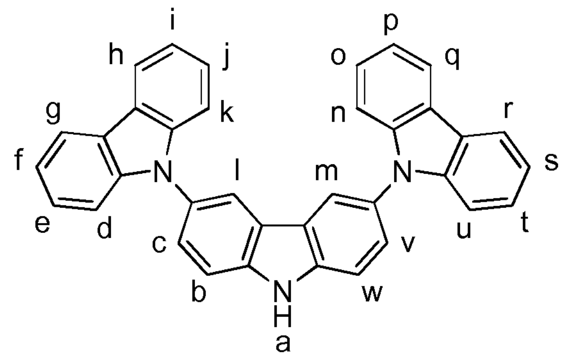

74

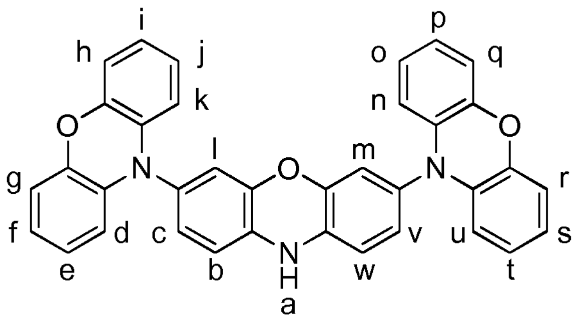

75

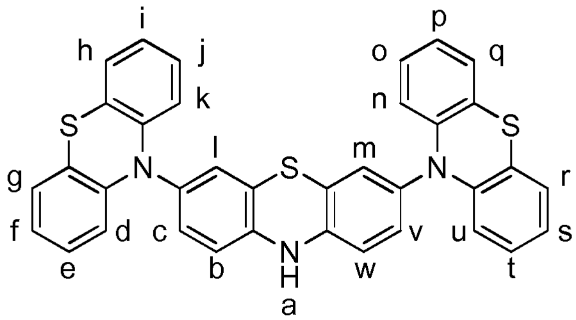

76

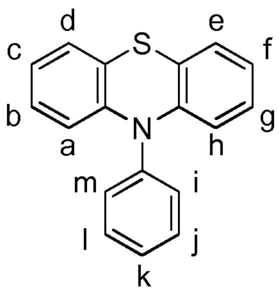

77

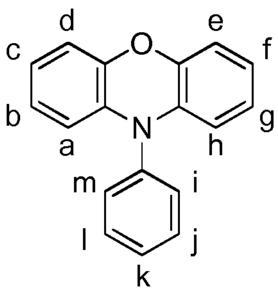

78

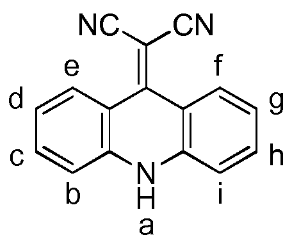

82

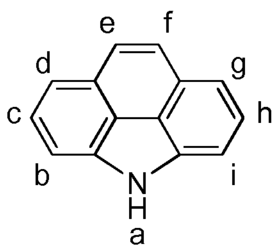

88

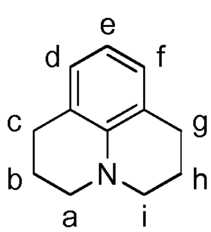

91

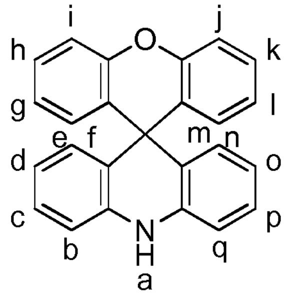

94

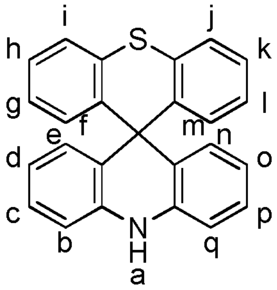

95

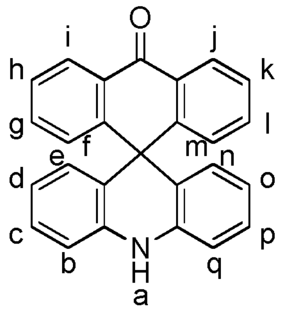

96

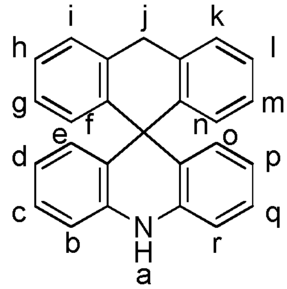

97

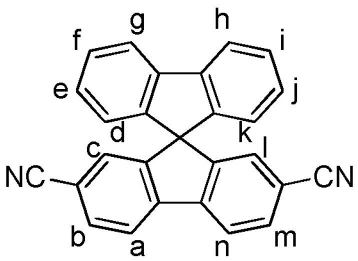

101

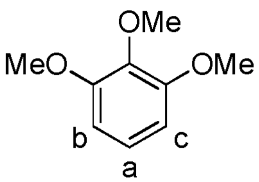

120

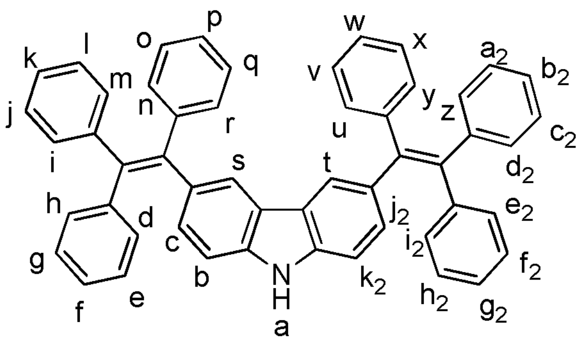

124

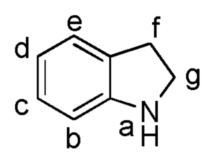

128

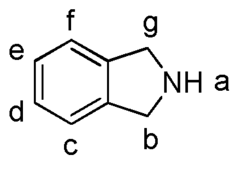

129

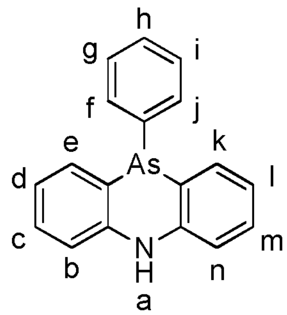

149

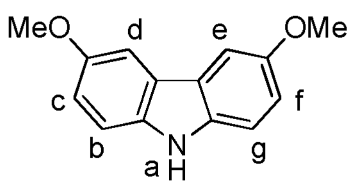

199

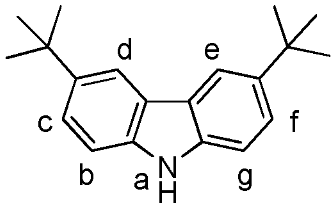

200

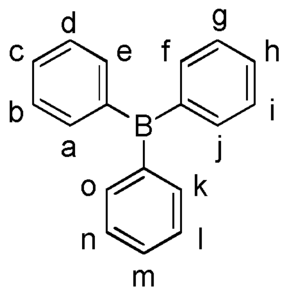

213

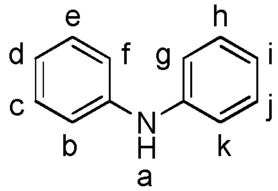

223

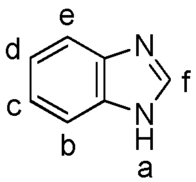

239

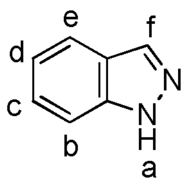

240

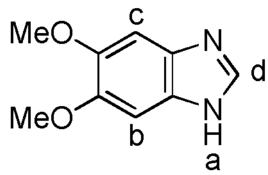

242

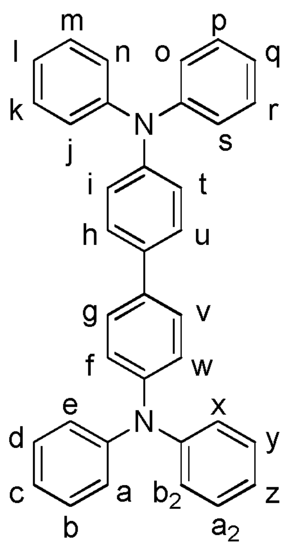

278

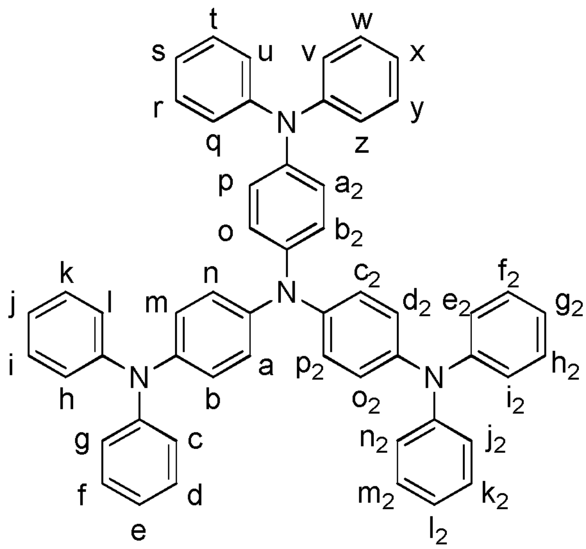

281

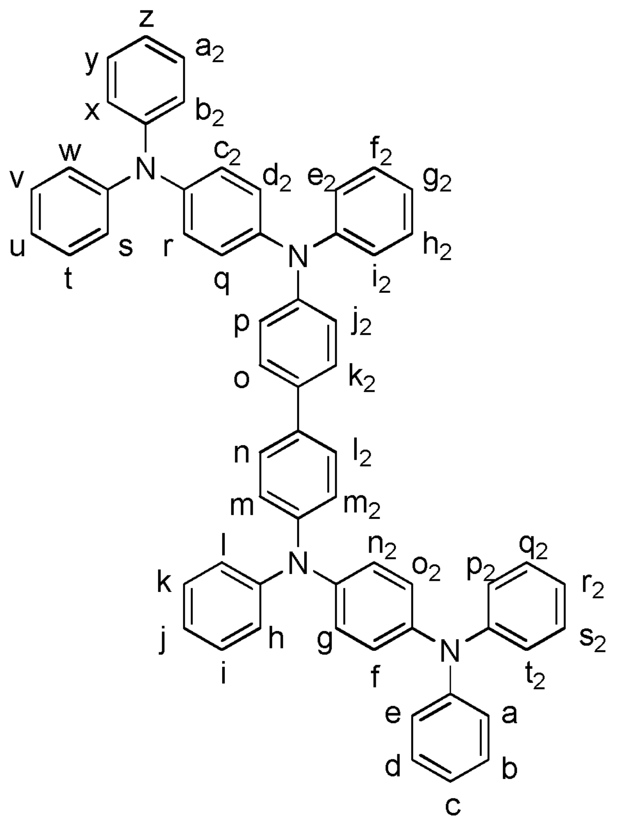

283

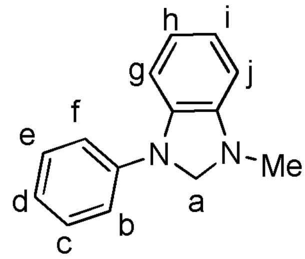

314

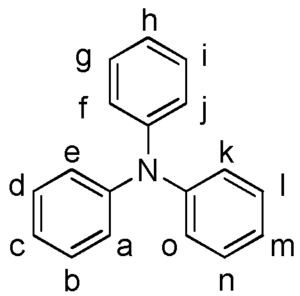

317

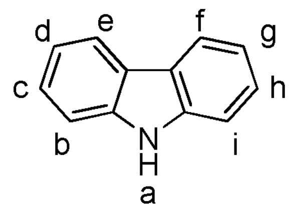

328

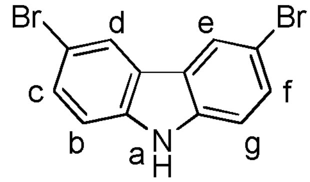

330

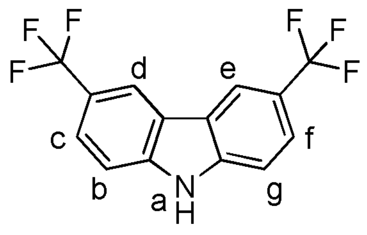

331

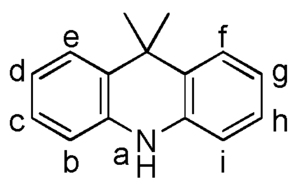

337

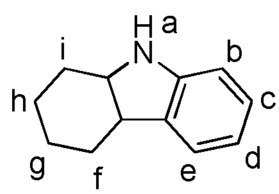

339

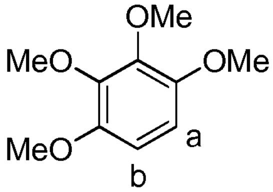

344

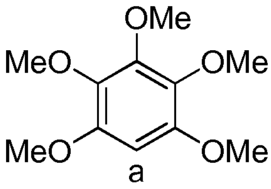

345

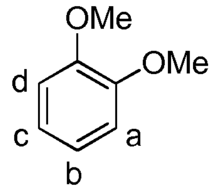

346

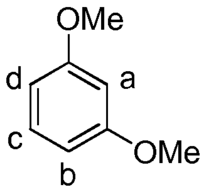

347

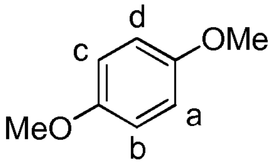

348

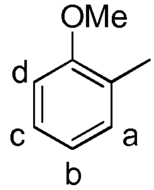

349

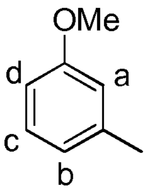

350

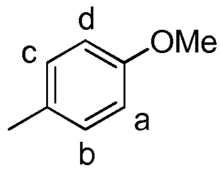

351

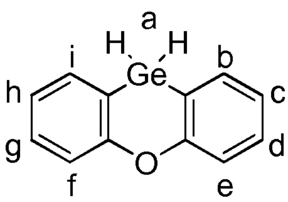

352

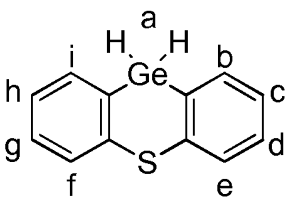

353

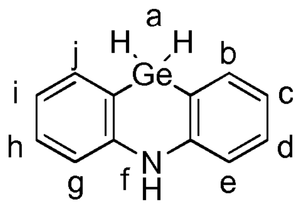

354

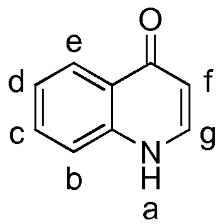

360

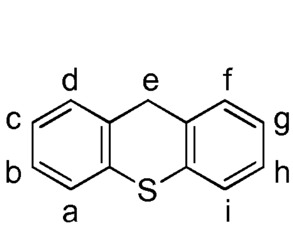

366

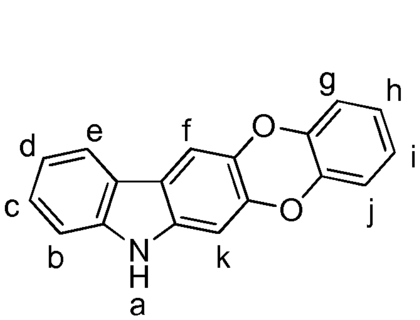

377

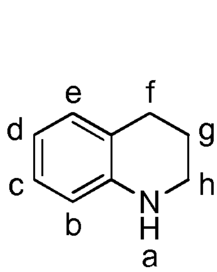

383

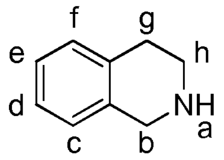

384

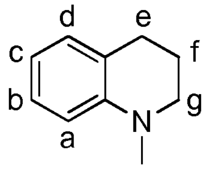

387

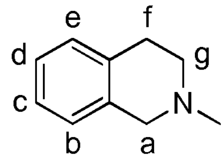

388

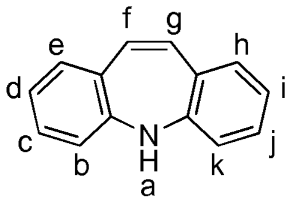

400

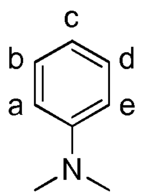

404

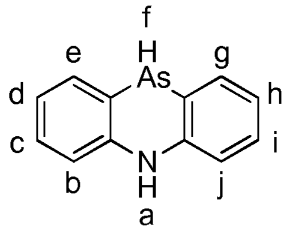

405

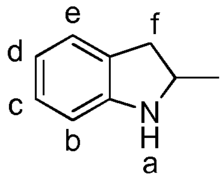

406

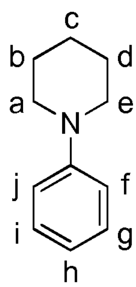

407

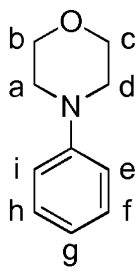

408

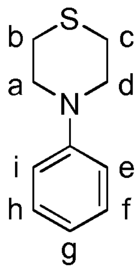

409

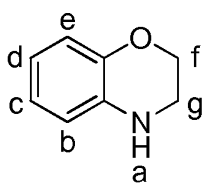

410

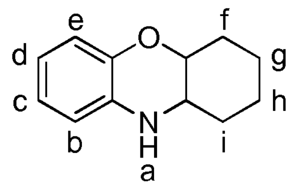

411

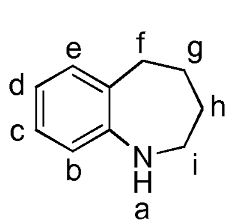

412

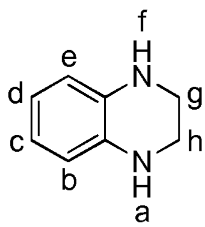

413

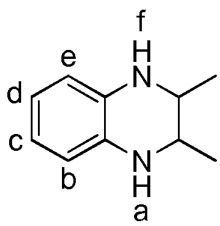

414

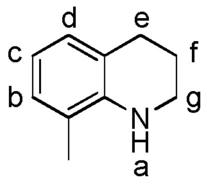

417

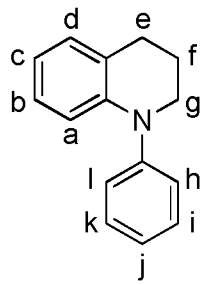

418

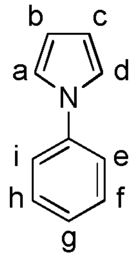

421

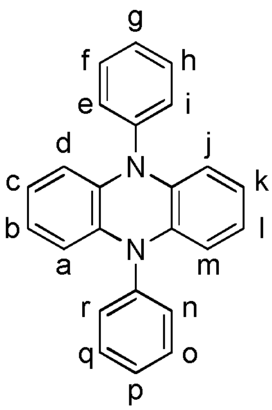

425

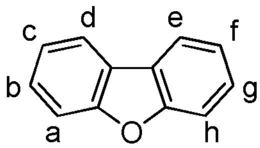

432

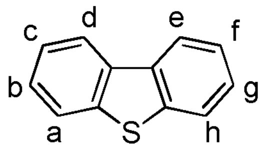

433

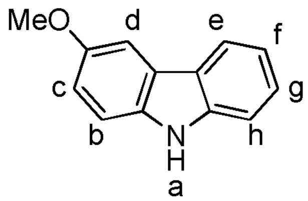

436

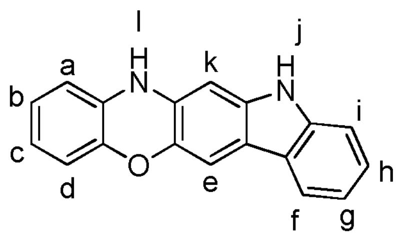

477

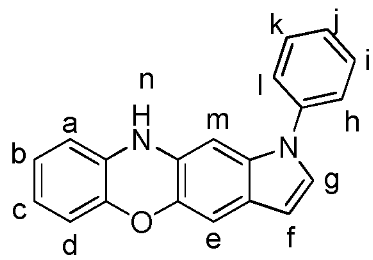

478

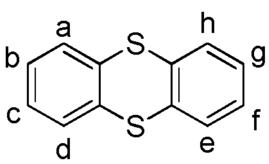

480

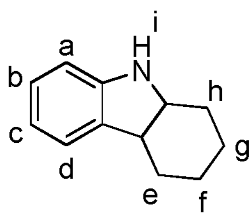

481

In Tabelle 2 sind mögliche Anknüpfungspositionen an den Separator oder die chemische Einheit AF1 mit Kleinbuchstaben a bis z bezeichnet.

In einer Ausführungsform der Erfindung wird AF1 gemäß Formel 19, einer eingeschränkten Form von Formel

2-1-a, verwendet.

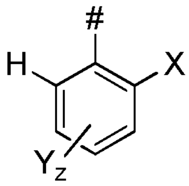

**Formel 19**

mit X gleich $CF_3$ und Y ausgewählt aus $CF_3$ oder CN,

wobei z = 1, 2 oder 3,

wobei z insbesondere 1 oder 2,

wobei # = Anknüpfungspunkt an den Separator oder Anknüpfungspunkt an die chemische Einheit AF2.

In einer Ausführungsform ist AF1 eine der hier aufgeführten Unterformeln von Formel 19:

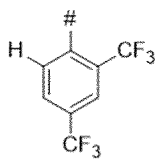
F19-2

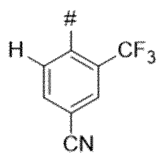
F19-3

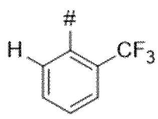
F19-4

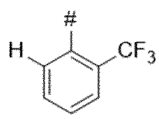
F19-5

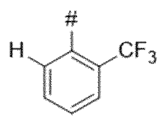
F19-6

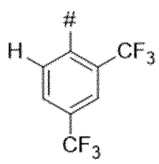
F19-8

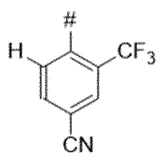
F19-9

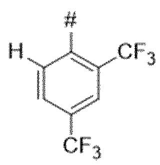
F19-14

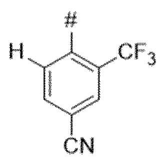
F19-15

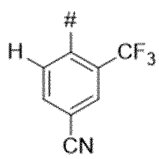
F19-16

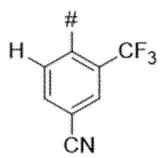
F19-17

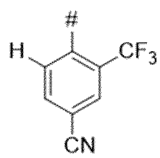
F19-18

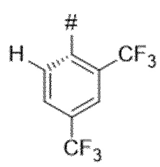
F19-20

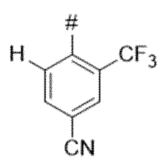
F19-21

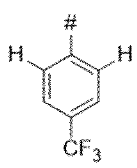
F19-22

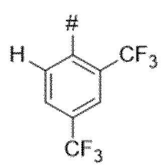
F19-23

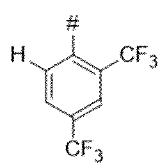
F19-24

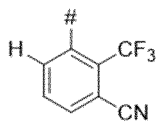

F19-26

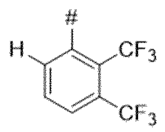

F19-27

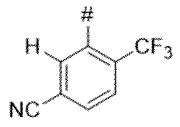

F19-29

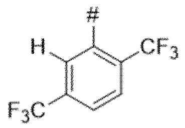

F19-30

wobei # = Anknüpfungspunkt an den Separator oder Anknüpfungspunkt an die chemische Einheit AF2. In einer Ausführungsform sind die chemischen Einheiten AF2 aus der Tabelle 3 ausgewählt und sind wie in Formel 1 angegeben mit einer Fluor-haltigen AF1 kombiniert.

**Tabelle 3:** Beispiele chemischer Einheiten AF2

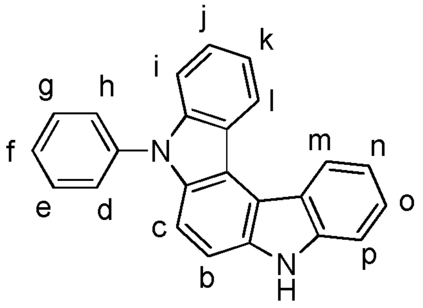

6

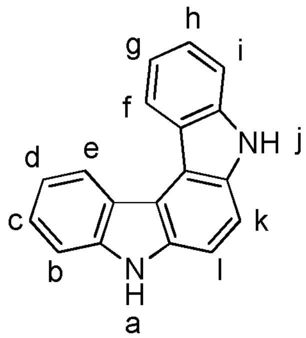

7

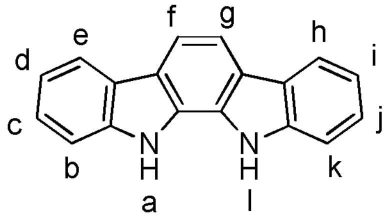

8

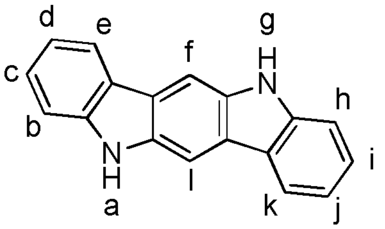

10

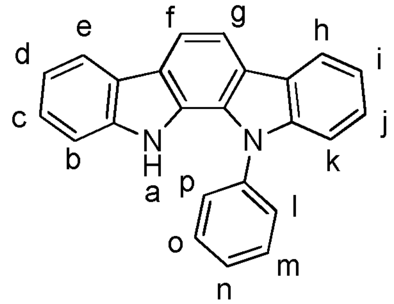

21

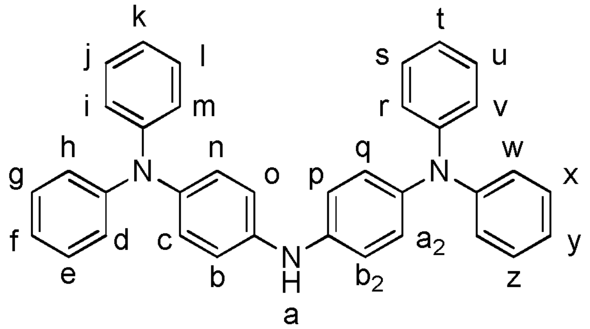

22

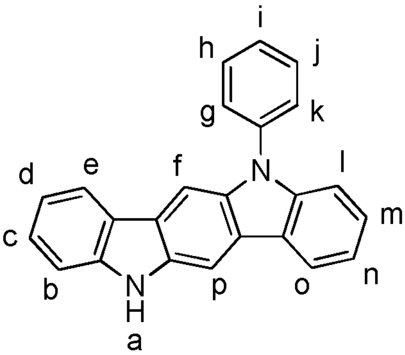

24

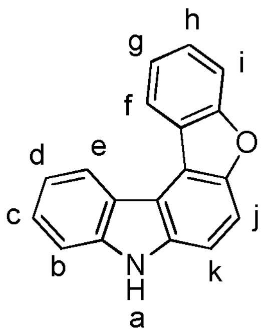
31

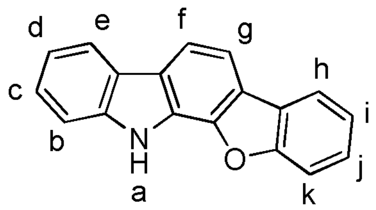
32

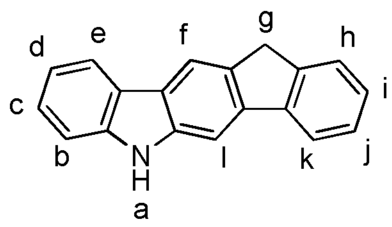
33

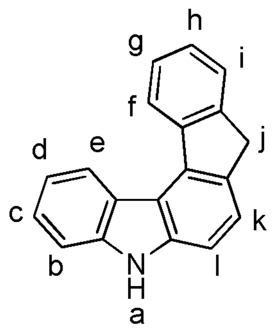
34

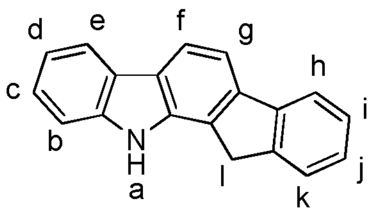
35

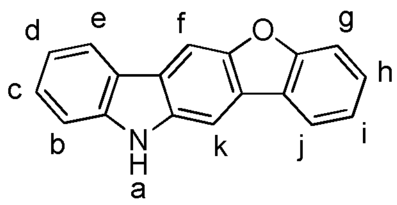
30

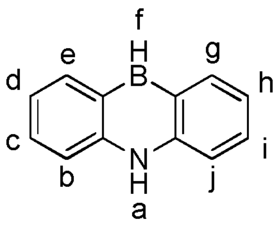
38

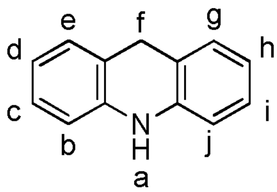
39

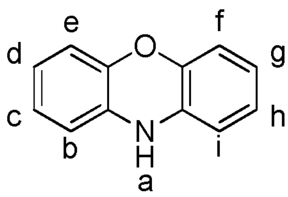

41

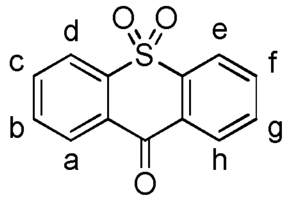

43

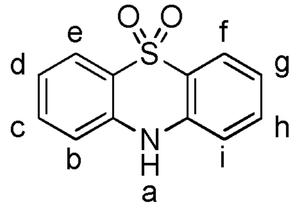

44

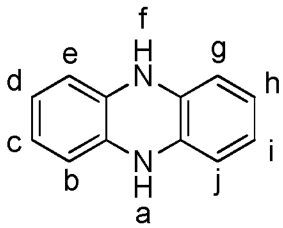

49

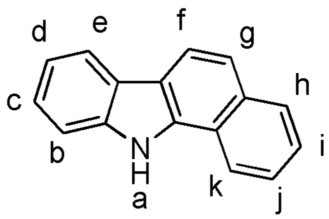

54

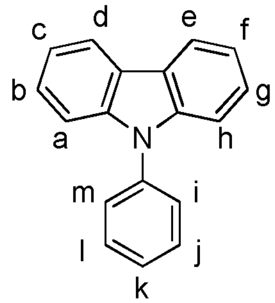

55

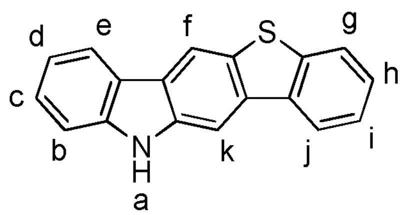

58

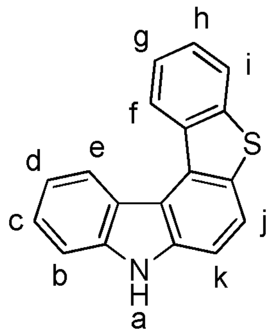

59

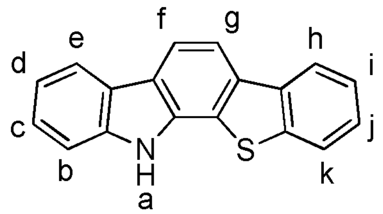

60

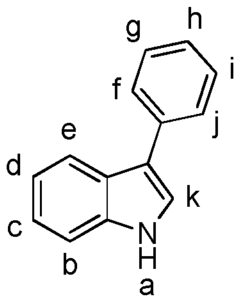

63

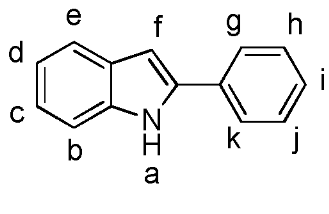

64

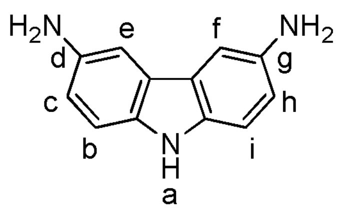

66

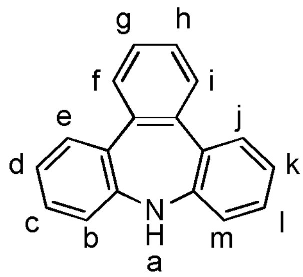

71

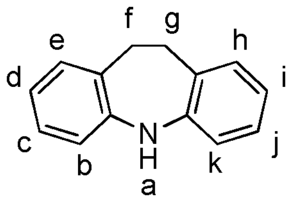

72

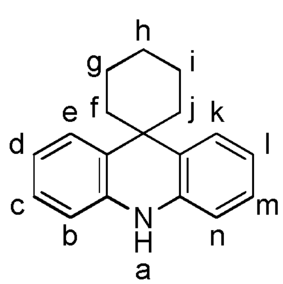

73

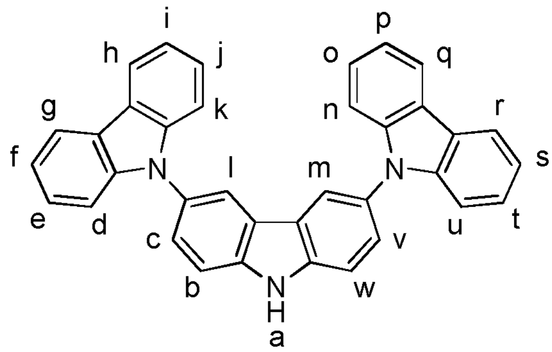

74

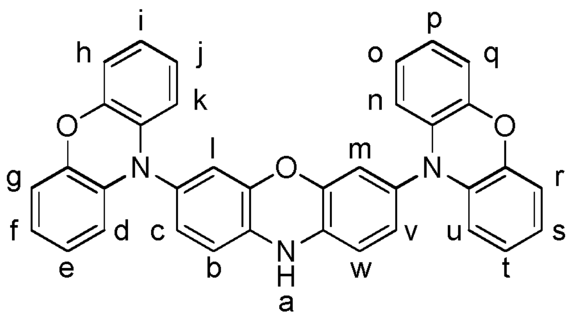

75

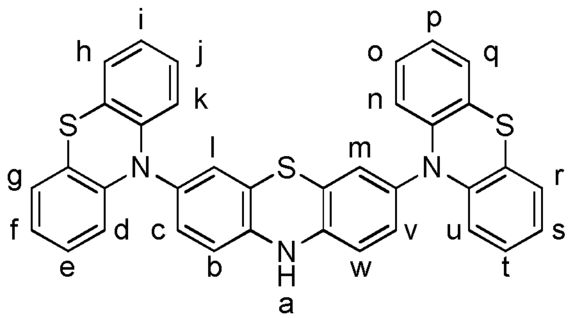

76

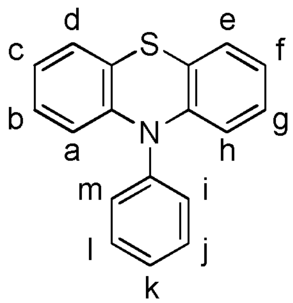

77

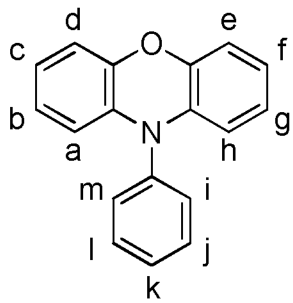

78

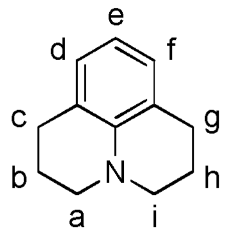

91

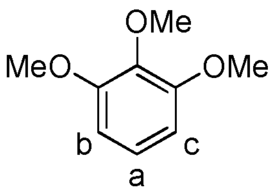

120

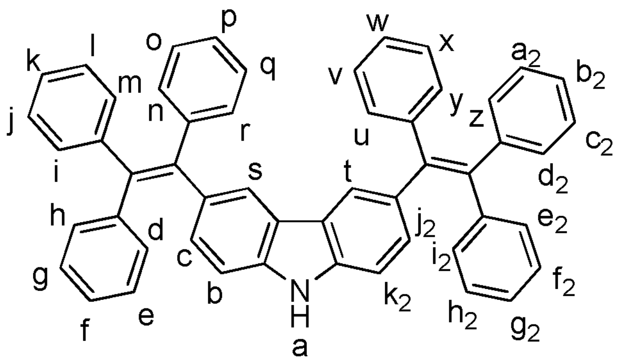

124

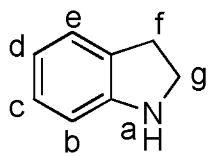

128

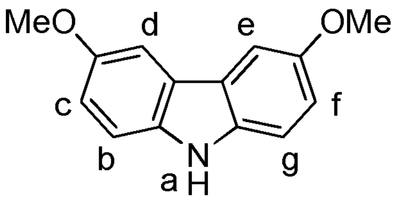

199

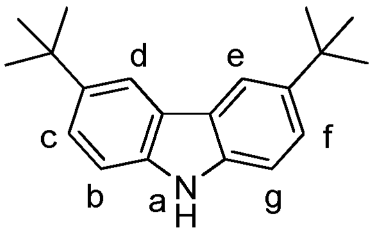

200

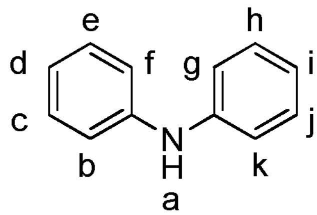

223

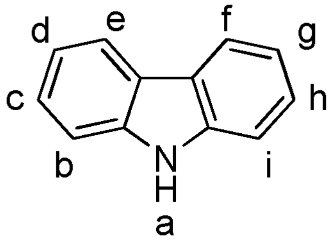

328

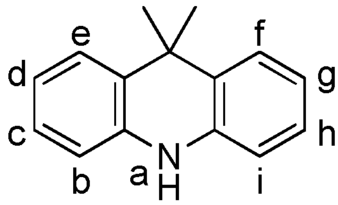

337

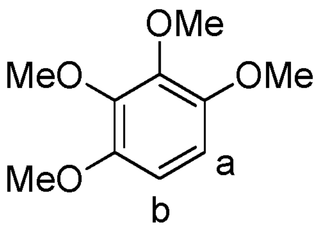

344

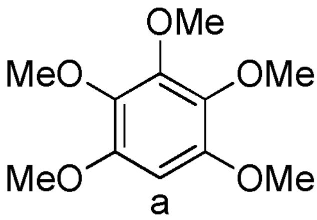

345

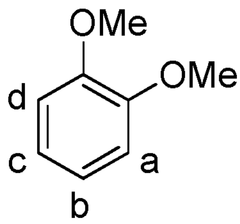

346

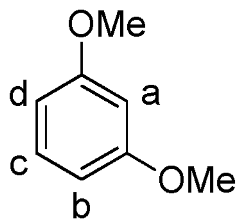

347

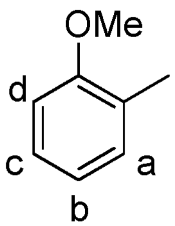

349

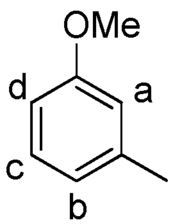

350

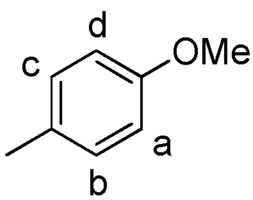

351

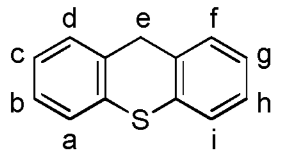
366

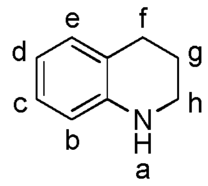
383

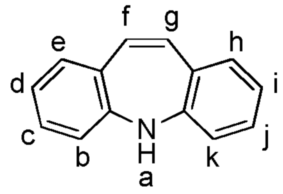
400

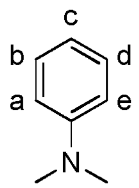
404

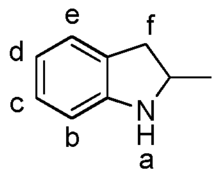
406

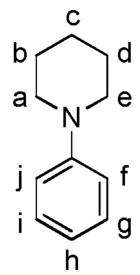
407

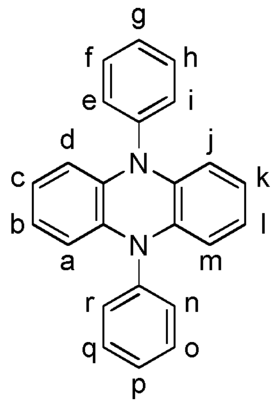
425

430

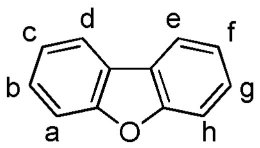
432

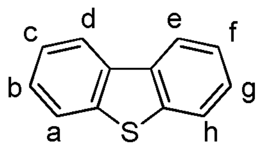
433

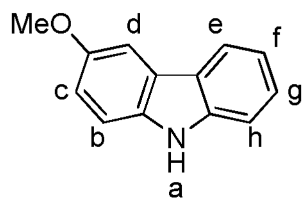
436

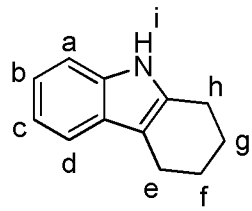
481

In Tabelle 3 sind mögliche Anknüpfungspositionen an den Separator oder die chemische Einheit AF1 mit Kleinbuchstaben a bis z bezeichnet.

In einer weiteren Ausführungsform weist die Einheit AF2 eine Struktur der Formel 3 auf oder hat eine Struktur der Formel 3

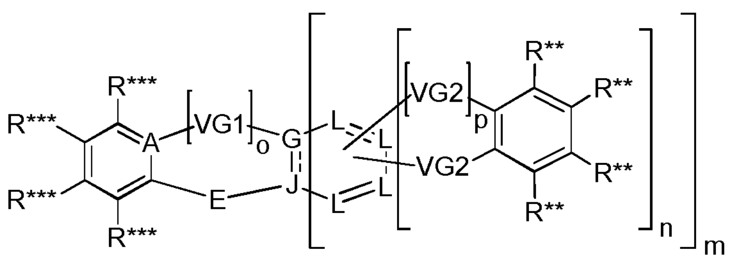

**Formel 3**

wobei gilt:

m ist 0 oder 1;

n ist 0 oder 1, wobei bei m = 0 immer auch n = 0 ist;

o ist 0 oder 1;

p ist 0 oder 1;

A ist CR*** wenn o = 0, ansonsten C;

VG1 = verbrückende Gruppe, ist ausgewählt aus der Gruppe bestehend aus

- NR**, $CR**_2$, O, S und einer C-C-Einfachbindung; oder
- NR**, $CR**_2$, O, S, einer C-C-Einfachbindung, BR**, AsR**, $SiR**_2$, $GeR**_2$,

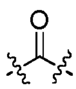, 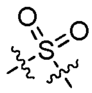, 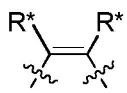, 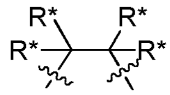, 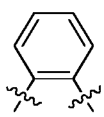, 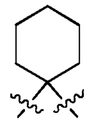, 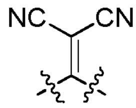, 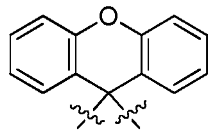,

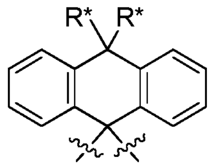, 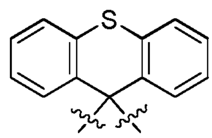, und 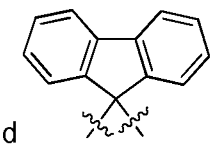,

wenn m = 1 und gleichzeitig n = 0;

VG2 = verbrückende Gruppe ist bei jedem Auftreten unabhängig voneinander ausgewählt aus der Gruppe bestehend aus $CR**_2$, NR**, O, S und einer C-C-Einfachbindung, wobei nicht zwei Einheiten VG2 gleichzeitig gleich einer C-C-Einfachbindung sind;

E ist NR**,

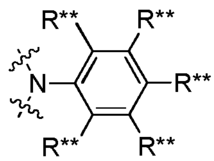,

O oder S;

G ist C wenn o = 1 und gleichzeitig m = 1; G ist CR** wenn o = 0 und gleichzeitig m = 1; G ist CR** oder $CR**_2$, wenn o = 1 und gleichzeitig m = 0; G ist R* wenn o = 0 und gleichzeitig m = 0; G ist CR**, $CR**_2$, N oder NR* wenn m = 0 und gleichzeitig VG1 eine C-C-Einfachbindung ist;

J ist C wenn m = 1; J ist CR**, $CR**_2$ oder NR** wenn m = 0;

L ist CR*** wenn n = 0; L ist CR** oder C (bei kovalenter Bindung zu VG2) wenn n = 1;

R*** ist R** oder ist ausgewählt aus den folgenden Einheiten, wobei maximal zwei der Reste R*** gleichzeitig gleich einer der folgenden Einheiten sind:

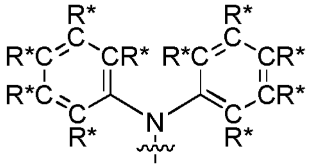
,
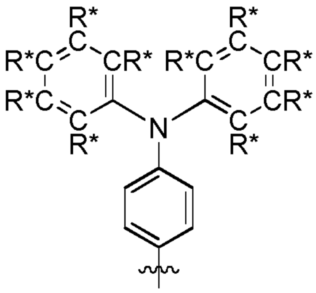
,
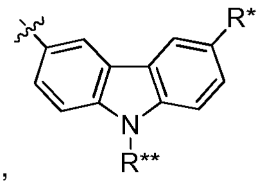
,
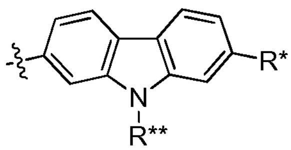

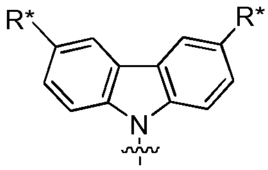
,
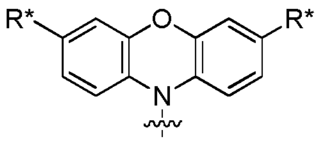
,
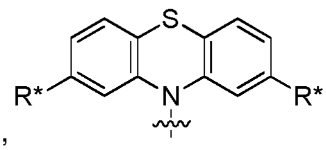
,

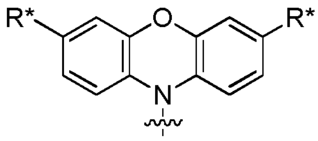
,
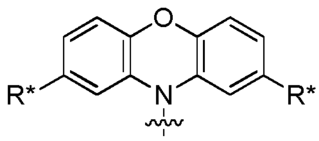
;

R** ist bei jedem Auftreten unabhängig voneinander ein Rest R* oder markiert eine Anknüpfungsstelle an einen Separator S oder die chemische Einheit AF1 wobei genau ein R** eine Anknüpfungsstelle an einen Separator S oder an die chemische Einheit AF1 ist.

R* ist wie bei Formel 1 definiert.

In einer weiteren Ausführungsform weist die Einheit AF2 eine Struktur der Formel 4 auf oder hat eine Struktur der Formel 4

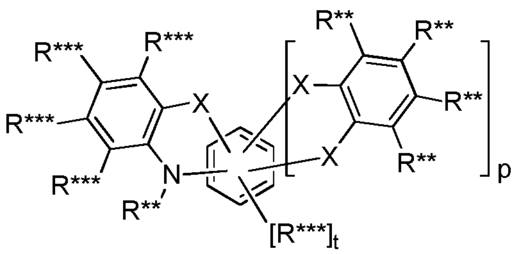

**Formel 4**

wobei in Formel 4 bedeutet:

p ist o oder 1;

t = 4 - 2p;

X ist $CR^{**}_2$, NR**, Sauerstoff, Schwefel oder eine direkte Bindung, wobei maximal zwei Platzhalter X gleichzeitig eine direkte Bindung sind, wobei diese nicht Bestandteil desselben Rings sind;

und im Übrigen die für Formel 3 gegebenen Definitionen gelten.

In einer weiteren Ausführungsform weist mindestens eine AF2 des organischen Moleküls eine Struktur der Formel 4A1-4A7 auf oder hat eine Struktur der Formel 4A1-4A7;

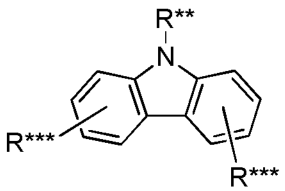

**Formel 4A1**

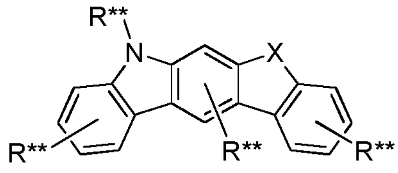

**Formel 4A2**

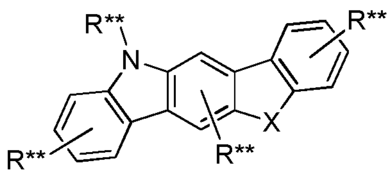

**Formel 4A3**

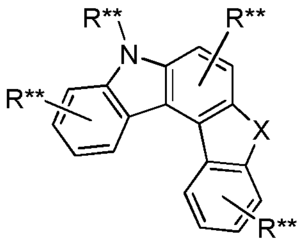

**Formel 4A4**

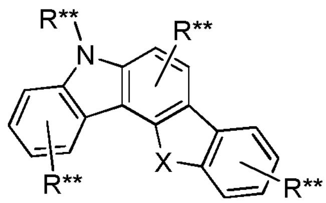

**Formel 4A5**

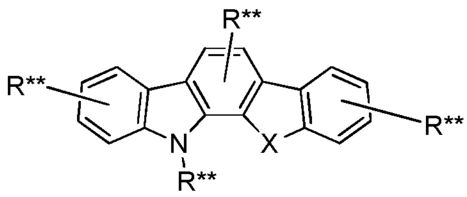

**Formel 4A6**

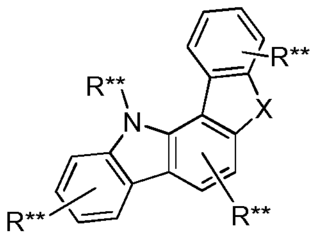

**Formel 4A7**

wobei in Formel 4A1-4A7 gilt:

X ist $C(R^{**})_2$, NR**, Sauerstoff oder Schwefel;

und im Übrigen die für Formel 3 gegebenen Definitionen gelten.

In einer weiteren Ausführungsform weisen die erfindungsgemäßen Moleküle einen Separator S der Formel 4S auf,

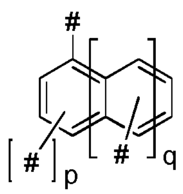

**Formel 4S**

wobei die Anknüpfungspositionen für die chemischen Einheiten AF1 und AF2 durch den Platzhalter # gekennzeichnet sind.

p = 0 oder 1,
q = 0 oder 1,
und wobei p ≠ q.

In einer Ausführungsform ist der Separator ausgewählt aus den in Tabelle 4 aufgeführten Strukturen:

**Tabelle 4:** Beispiele für Separatoren S. Die Anknüpfungsstellen für die chemischen Einheiten AF1 und AF2 sind mit # gekennzeichnet.

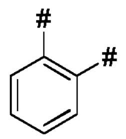
S-1

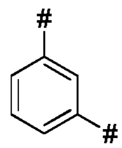
S-2

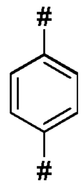
S-3

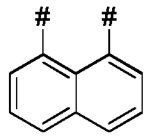
S-4

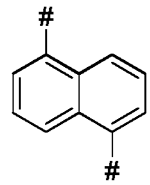
S-5

In einer Ausführungsform der Erfindung werden die Separatoren S-1, S-2 oder S-3 verwendet.

Durch Kombination der oben definierten Paare aus chemischen Einheiten AF1 und AF2 und der Festlegung der Verknüpfung durch einen Separator S ergeben sich entsprechend die beispielhaften erfindungsgemäßen Moleküle der Tabelle 5. Weitere organische Moleküle können durch Kombination der genannten Moleküleinheiten analog erhalten werden.

In einer weiteren Ausführungsform weist das organische Molekül eine Struktur der Formel 5 auf oder hat eine Struktur der Formel 5;

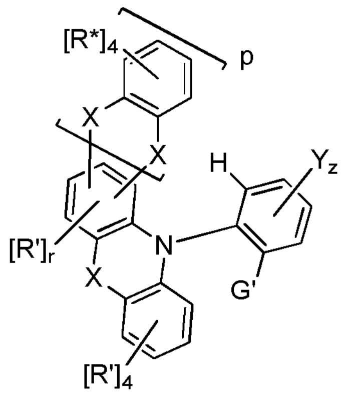

**Formel 5**

wobei in Formel 5 bedeutet:

p ist o oder 1;

X ist $CR^*_2$, NR*, Sauerstoff, Schwefel, eine direkte Bindung, wobei maximal zwei Platzhalter X gleichzeitig eine direkte Bindung sind, wobei diese nicht Bestandteil desselben Rings sind;

G' = $CF_3$;

Y = $CF_3$ oder CN;

z = 1 oder 2;

r = 4 - 2p

R' ist R* oder ist ausgewählt aus den folgenden Einheiten, wobei maximal zwei der Reste R' gleichzeitig gleich einer der folgenden Einheiten sind:

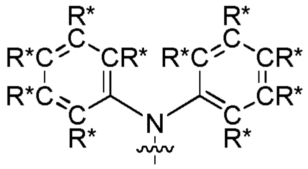, 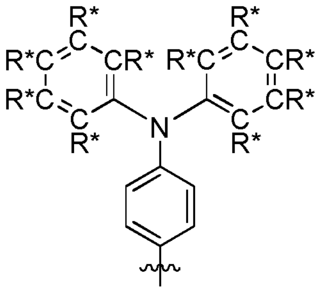, 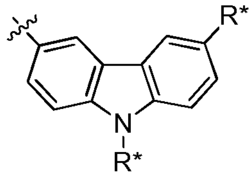, 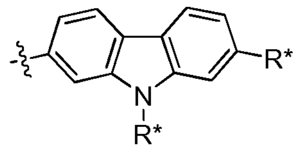,

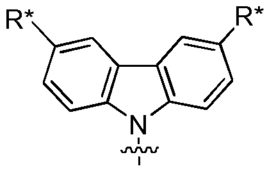, 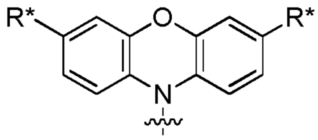, 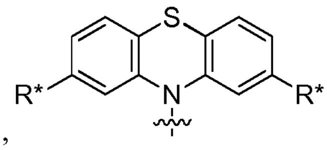,

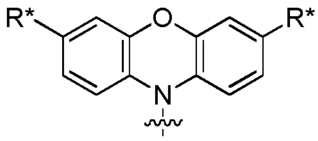, 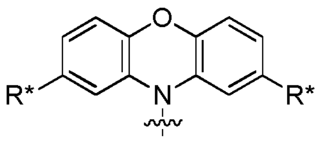;

und im Übrigen die für Formel 3 gegebenen Definitionen gelten.

In einer weiteren Ausführungsform weist das organische Molekül eine Struktur der Formel 6 auf oder hat eine Struktur der Formel 6;

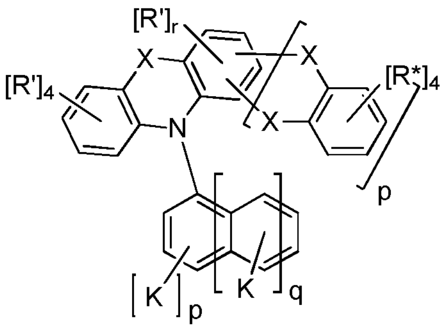 wobei K = 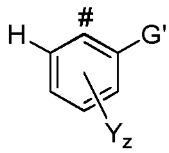

**Formel 6**

wobei in Formel 6 bedeutet:

p = 0 oder 1,

q = 0 oder 1,

und wobei $p \neq q$;

$r = 4 - 2p$;

X ist $CR^*_2$, $NR^*$, Sauerstoff, Schwefel, eine direkte Bindung, wobei maximal zwei Platzhalter X gleichzeitig eine direkte Bindung sind, wobei diese nicht Bestandteil desselben Rings sind;

$G' = CF_3$;

Y = $CF_3$ oder CN;

z = 1 oder 2;

# kennzeichnet die Position, über die die Einheit K angebunden ist;

R’ ist R* oder ist ausgewählt aus den folgenden Einheiten, wobei maximal zwei der Reste R’ gleichzeitig gleich einer der folgenden Einheiten sind:

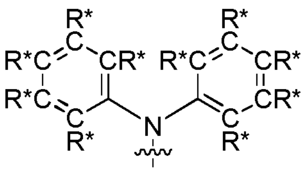 , 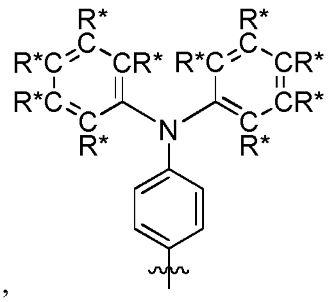 , 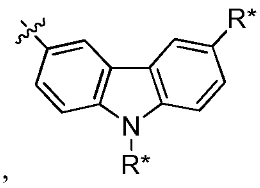 , 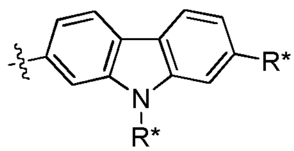 ,

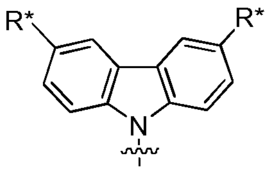 , 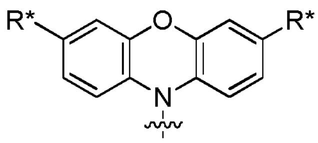 , 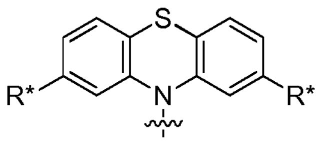 ,

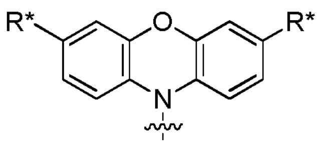 , 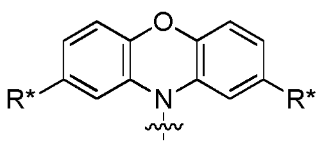 ;

und im Übrigen die für Formel 3 gegebenen Definitionen gelten.

**Tabelle 5:** Beispiele für erfindungsgemäße organische Moleküle (AF1-(S-)AF2) und Angabe der berechneten ΔE (S1-T1)-Werte. (nicht erfindungsgemäße Moleküle sind mit einem * markiert)

| **Molekülstruktur** | **ΔE(S1-T1)** |
|---|---|
| 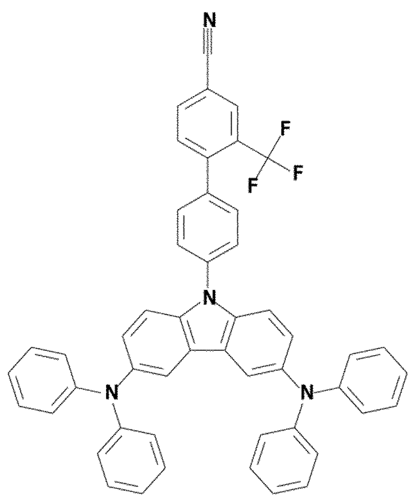 | 0.064eV |

(fortgesetzt)

| Molekülstruktur | ΔE(S1-T1) |
|---|---|
| 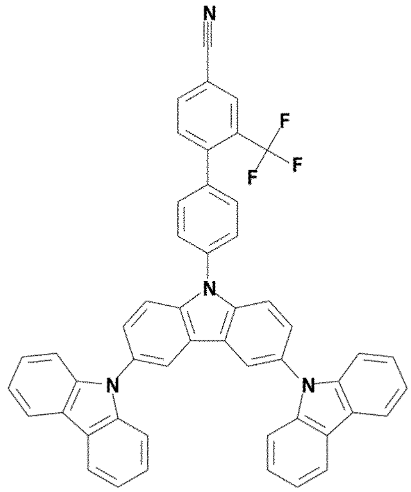 | 0.059eV |
| 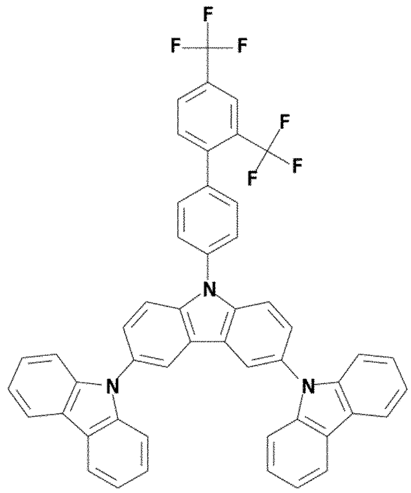 | 0.115eV |
| 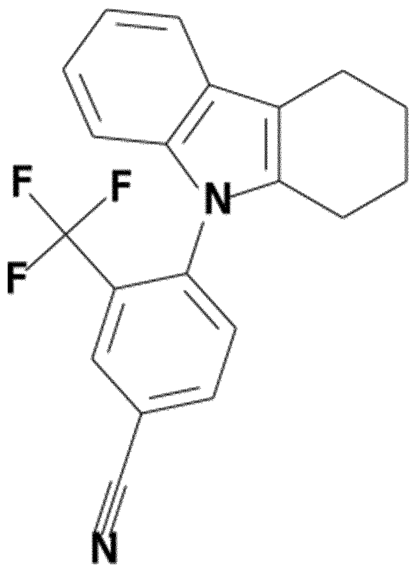 | 0.016eV |

(fortgesetzt)

| Molekülstruktur | ΔE(S1-T1) |
|---|---|
| 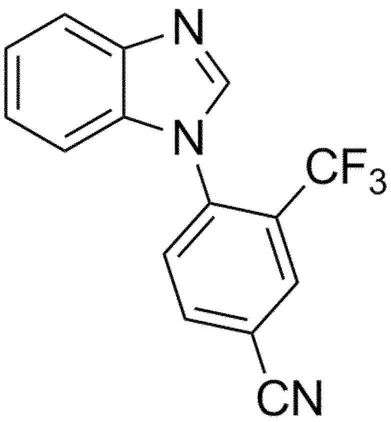 | 0.524eV |
| 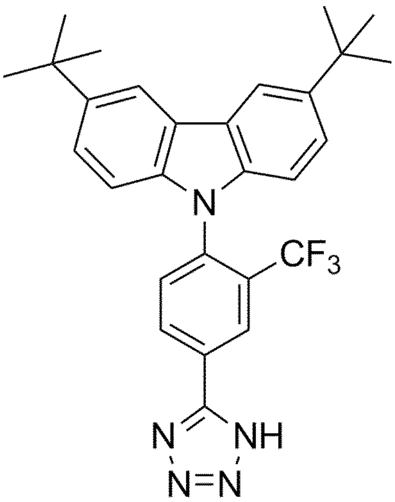 | 0.011eV |
| 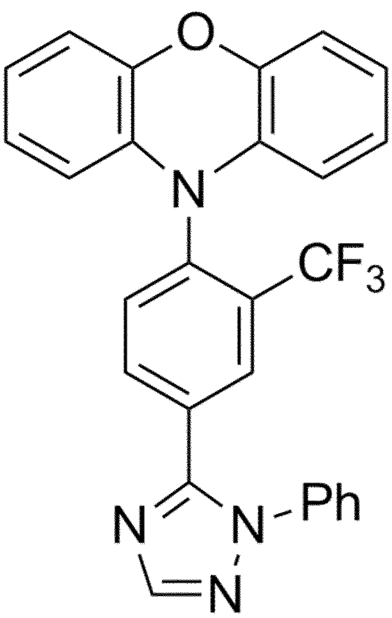 | 0.012eV |

(fortgesetzt)

| **Molekülstruktur** | **∆E(S1-T1)** |
|---|---|
| 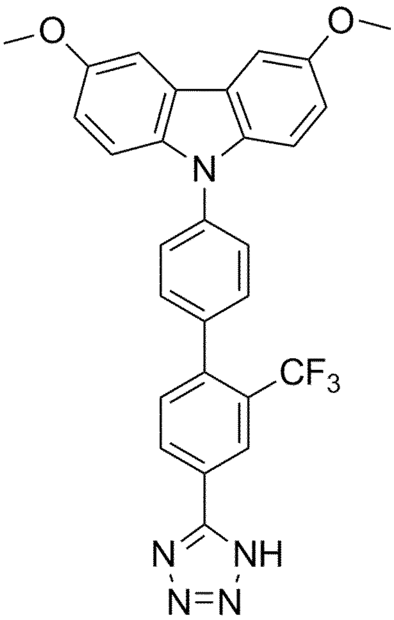 | 0.150eV |
| 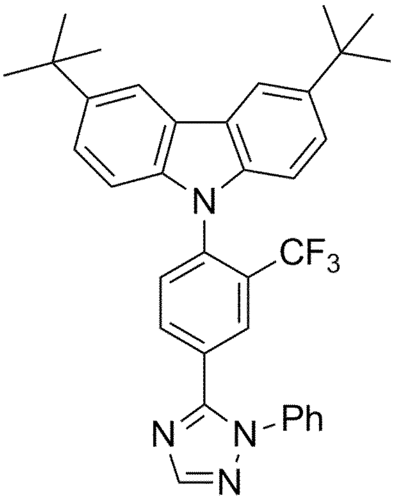 | 0.010eV |
| 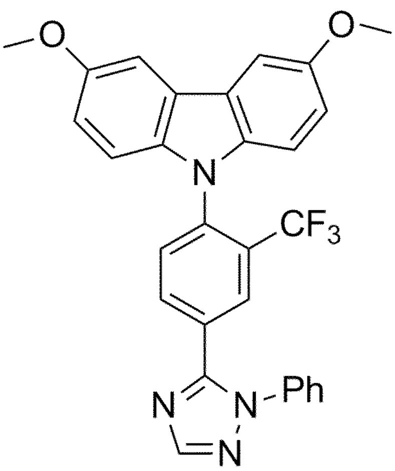 | 0.013eV |

(fortgesetzt)

| **Molekülstruktur** | **ΔE(S1-T1)** |
|---|---|
| 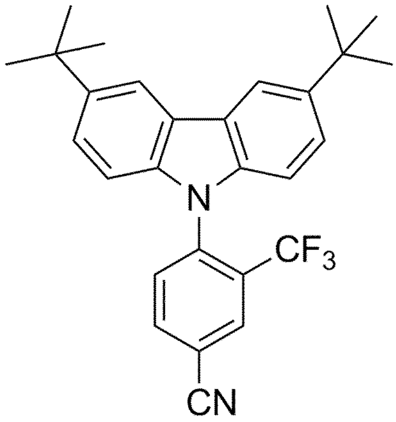 | 0.013eV |
| 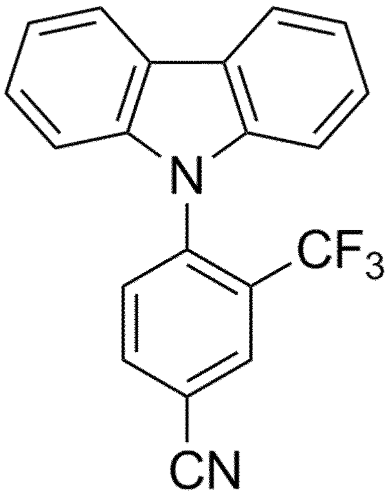 | 0.015eV |
| 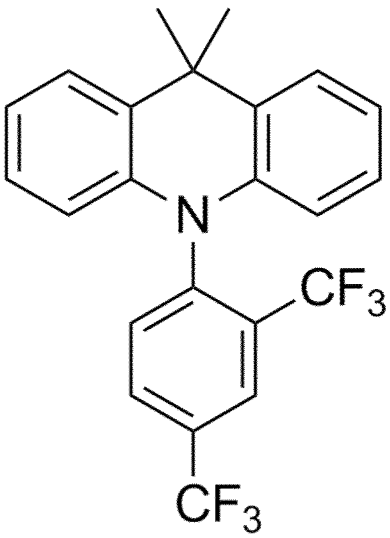 | 0.017eV |

(fortgesetzt)

| Molekülstruktur | ΔE(S1-T1) |
|---|---|
| 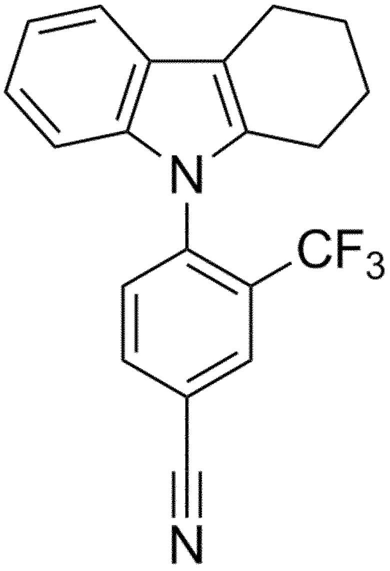 | 0.025eV |
| 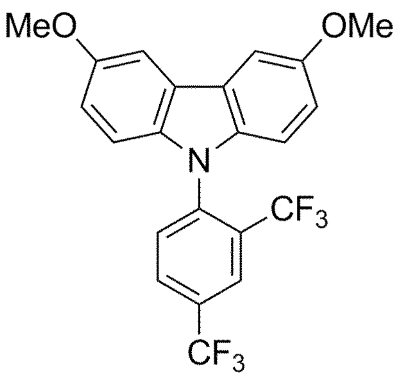 | 0.014eV |
| 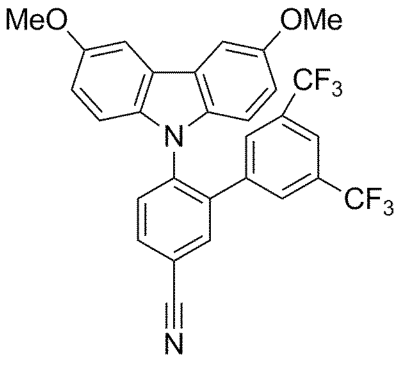 | 0.212eV |

(fortgesetzt)

| Molekülstruktur | ΔE(S1-T1) |
|---|---|
| 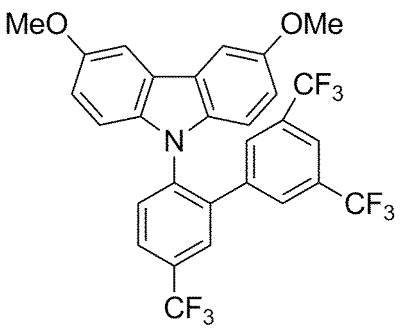 | 0.133eV |
| 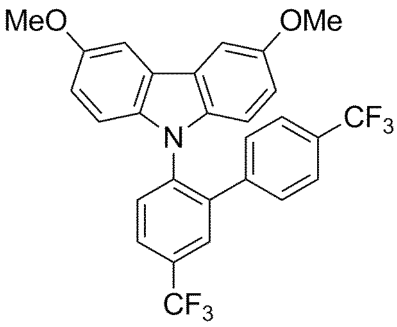 | 0.160eV |
| 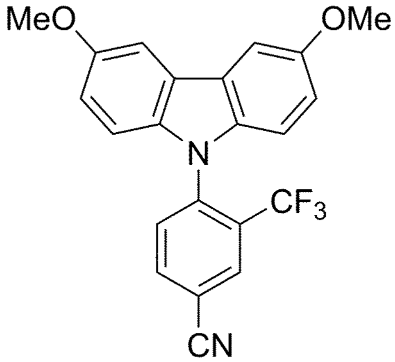 | 0.120eV |
| 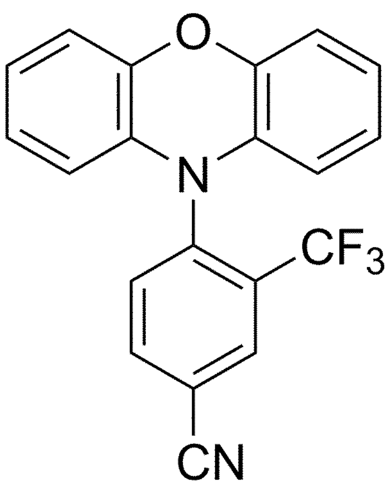 | 0.014eV |

(fortgesetzt)

| Molekülstruktur | ∆E(S1-T1) |
|---|---|
| 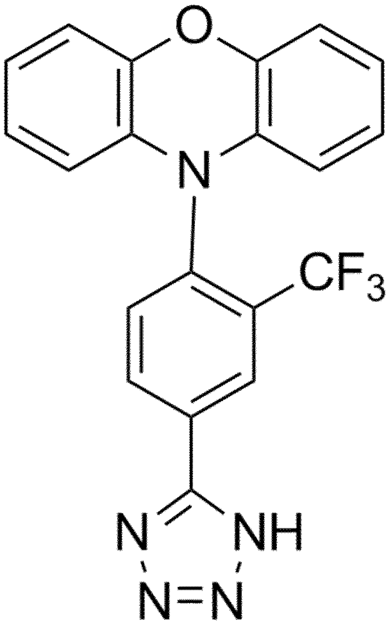 | 0.012eV |
| 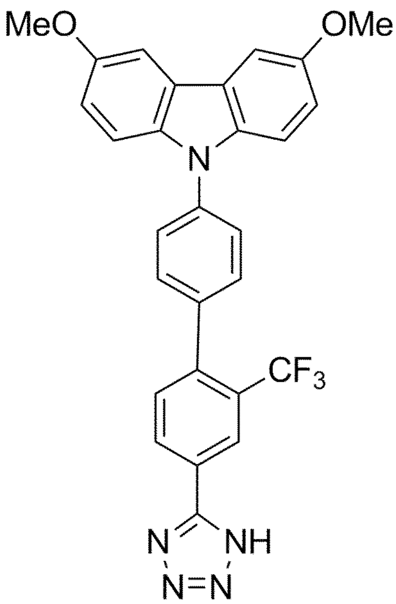 | 0.145eV |

(fortgesetzt)

| Molekülstruktur | ΔE(S1-T1) |
|---|---|
| 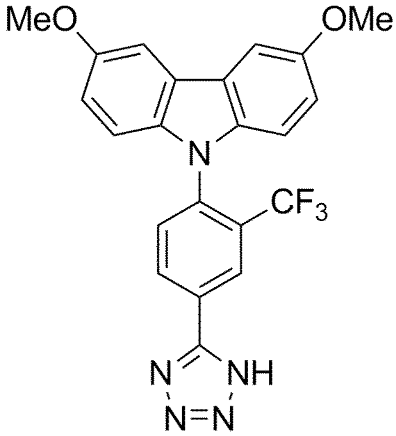 | 0.041eV |
| 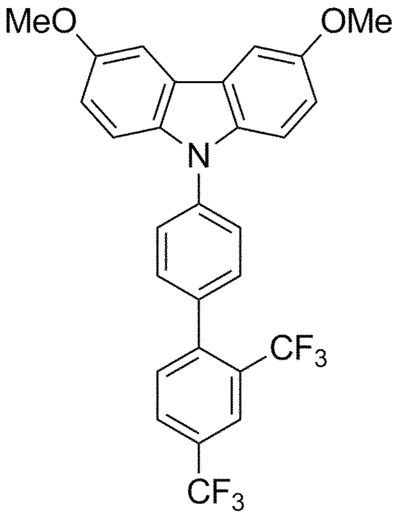 | 0.225eV |
| 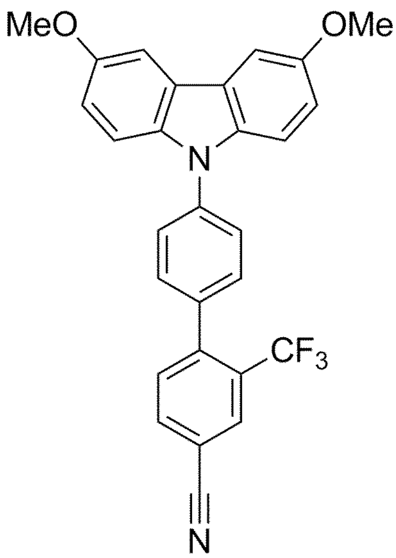 | 0.165eV |

(fortgesetzt)

| **Molekülstruktur** | **∆E(S1-T1)** |
|---|---|
| * 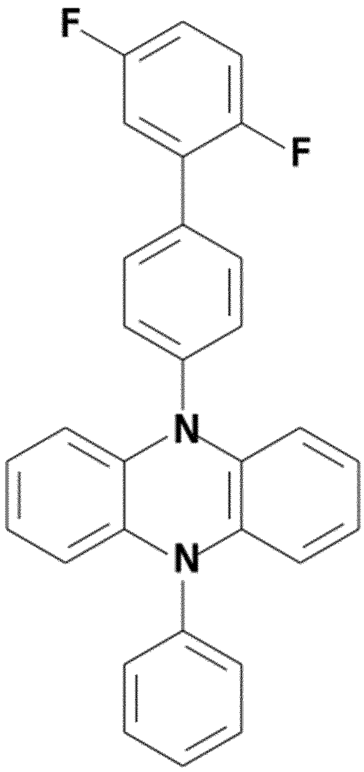 | 0.007eV |
| * 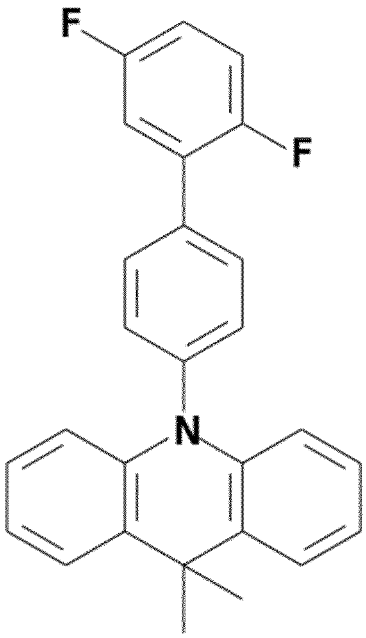 | 0.009eV |

(fortgesetzt)

| Molekülstruktur | ∆E(S1-T1) |
|---|---|
| 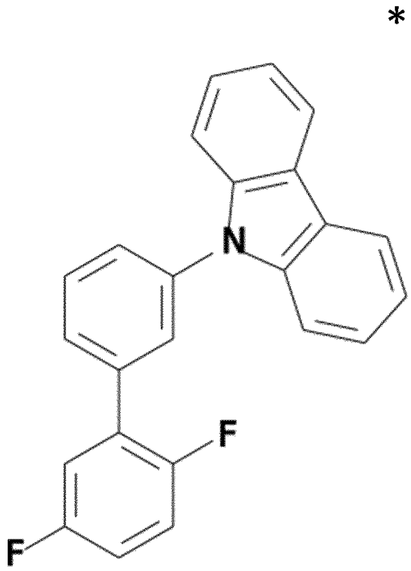 | 0.434eV |
| 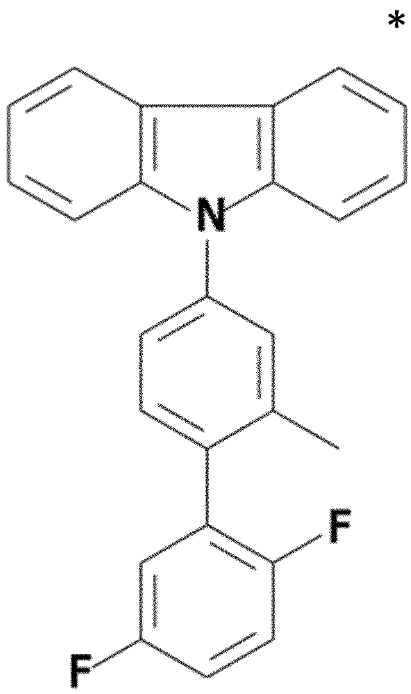 | 0.598eV |

(fortgesetzt)

| **Molekülstruktur** | **∆E(S1-T1)** |
|---|---|
| * 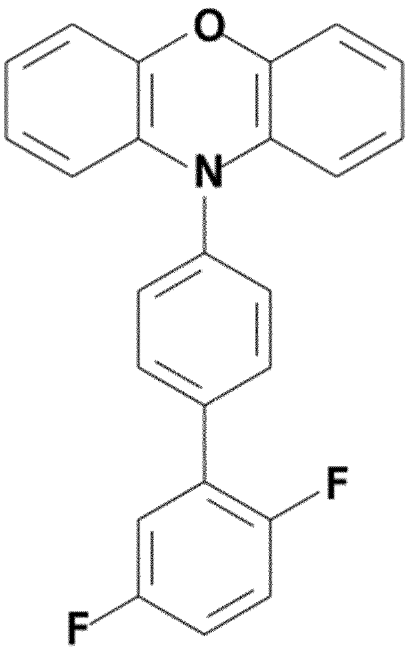 | 0.010eV |
| * 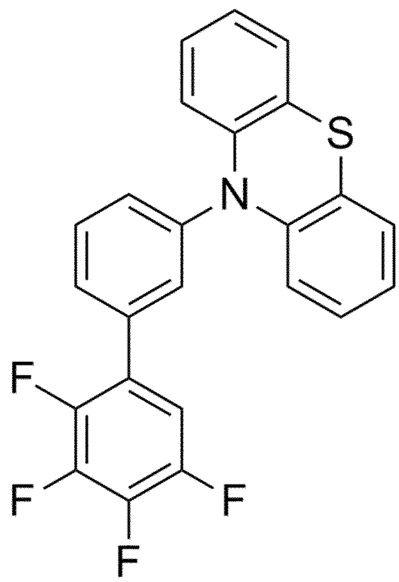 | 0.111eV |

(fortgesetzt)

| **Molekülstruktur** | **∆E(S1-T1)** |
|---|---|
| * 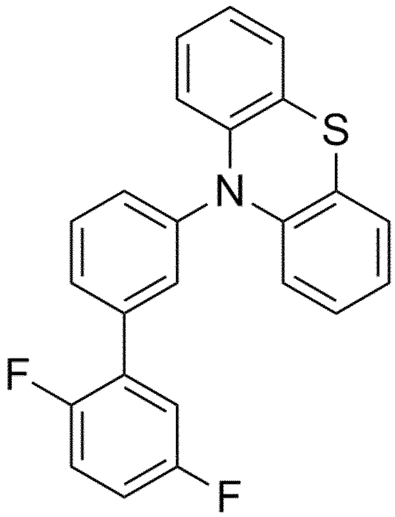 | 0.156eV |
| * 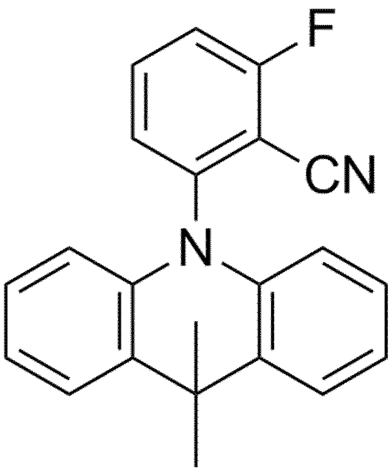 | 0.018eV |
| * 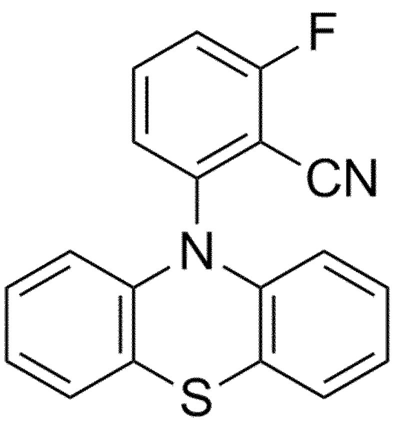 | 0.012eV |

Weitere Beispiele von erfindungsgemäßen Molekülen

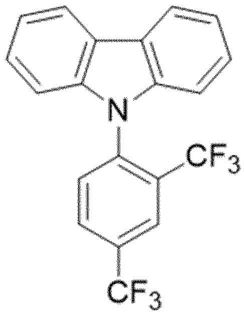
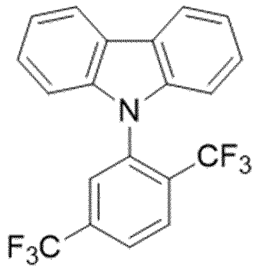
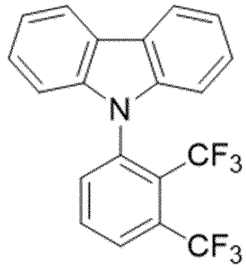
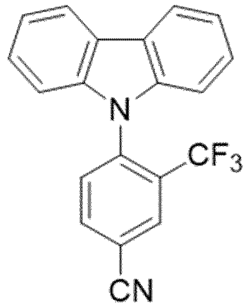

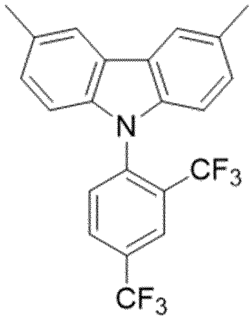
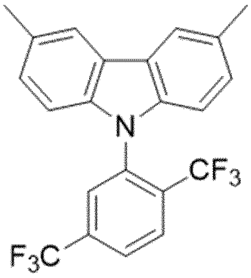
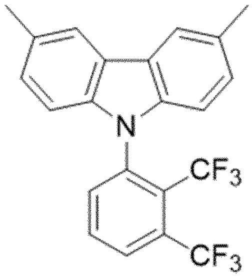
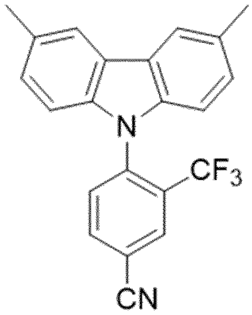

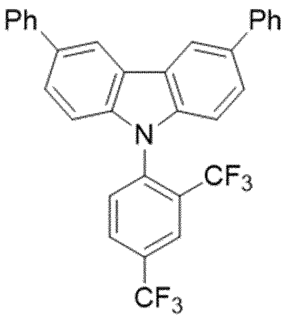
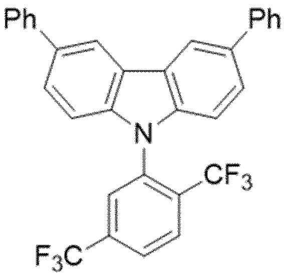
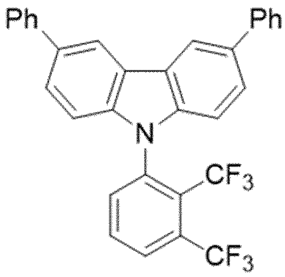
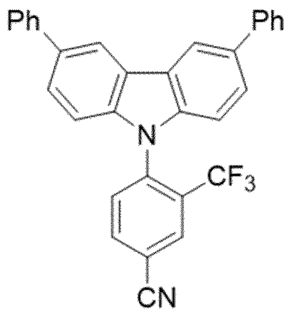

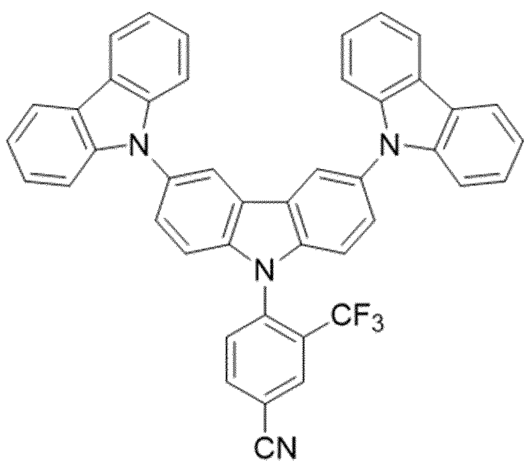
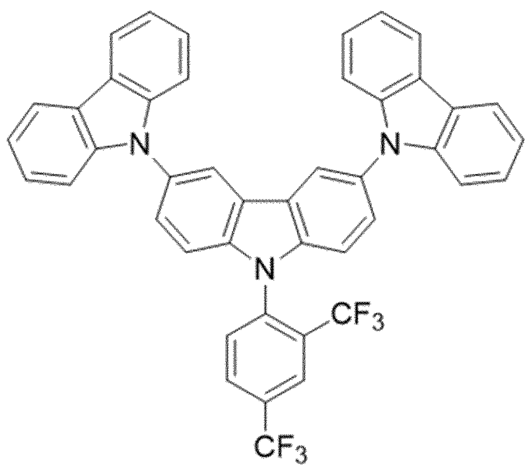

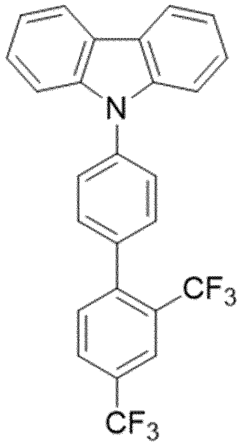

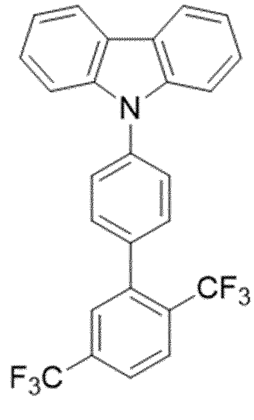

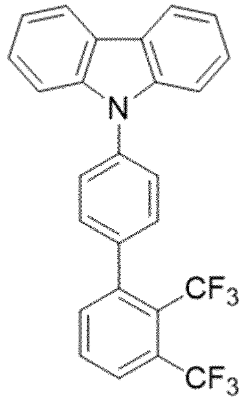

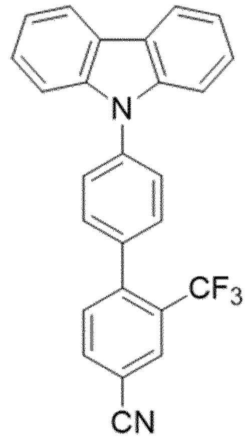

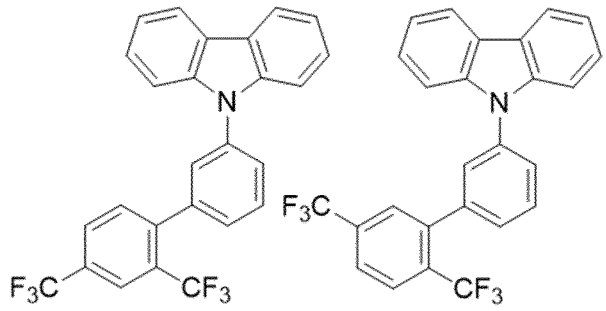

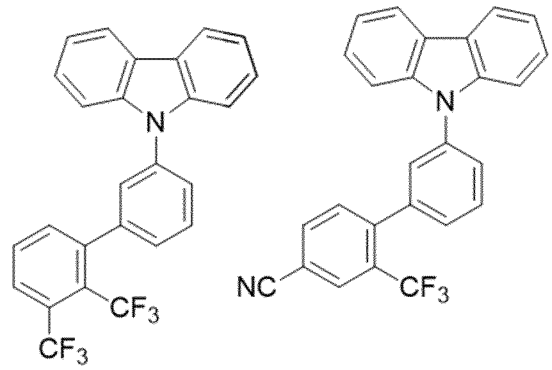

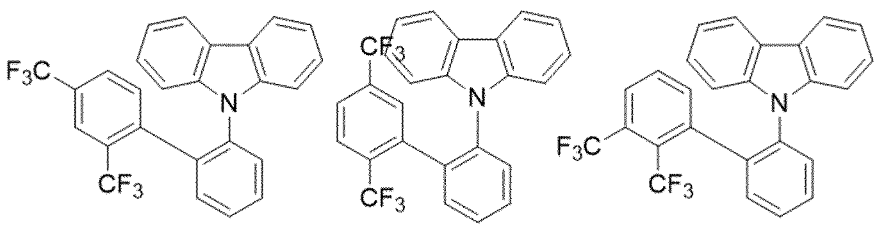

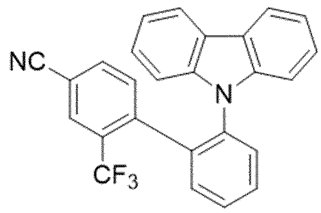

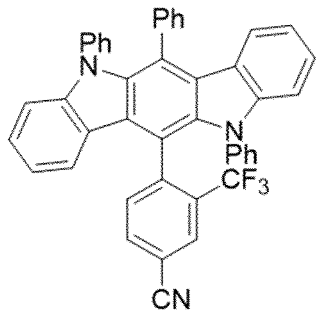

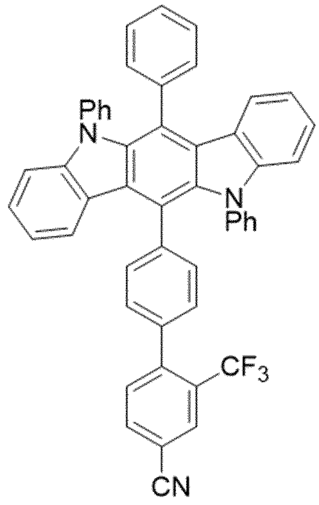

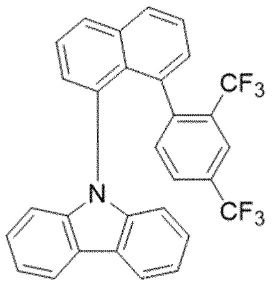

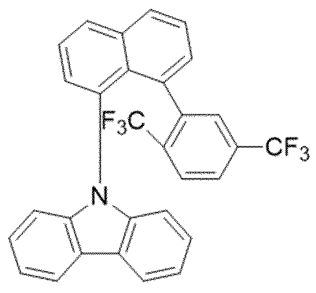

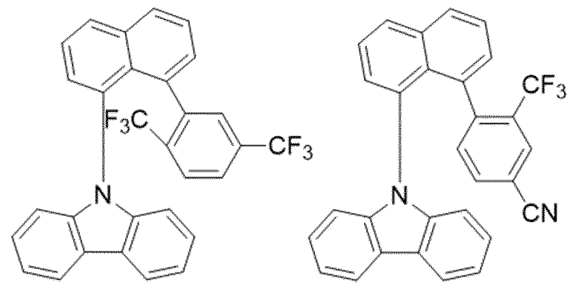

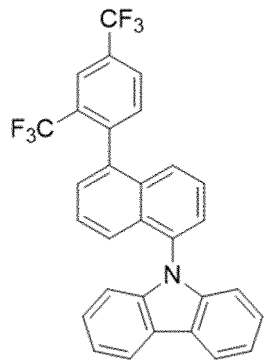

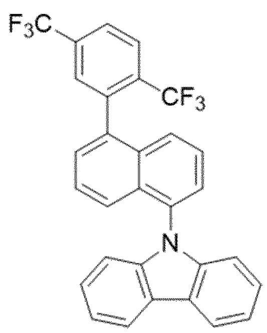

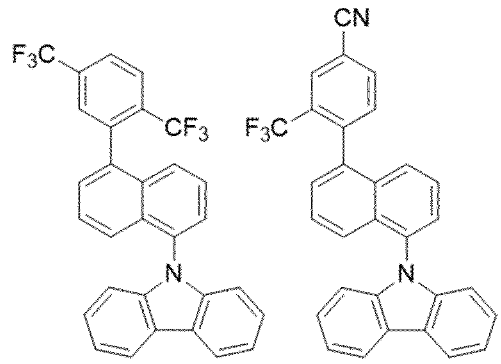

Beispiele weiterer erfindungsgemäßer organischer Moleküle, die durch Kombination der genannten Moleküleinheiten analog der obigen Beschreibung erhalten werden, sind in Tabelle 6 dargestellt. Die Benennung der Moleküle erfolgt nach dem Schema AF-S-AF, wobei die Benennung nach folgendem Schema erfolgte: Die Nummern rechts und links stehen für die chemischen Einheiten AF1 aus Tabelle 1 und AF2 aus Tabelle 2 oder aus Formel 3; "S" steht für einen Separator, der aus den Strukturen der Tabelle 4 auszuwählen ist, wobei die Anknüpfung der einzelnen Einheiten AF an einen Separator S über die in Tabelle 1 und Tabelle 2 mit Kleinbuchstaben definierten Positionen erfolgt.

Tabelle 6 beinhaltet gleichzeitig auch erfindungsgemäße Moleküle der Form AF1-AF2, die keinen Separator S enthalten, wobei die Einheiten AF1 und AF2 über die oben genannten Anknüpfungspositionen miteinander kovalent verbunden sind. Diese Moleküle ergeben sich aus Tabelle 6, indem die Spalte "S" vernachlässigt wird.

**Tabelle 6:** Erfindungsgemäße organische Moleküle nach dem Schema AF1-S-AF2, wobei S optional nicht vorhanden ist, sodass die Tabelle 6 gleichzeitig auch Moleküle nach dem Schema AF1-AF2 darstellt. In Klammern sind die Werte für ΔHOMO, ΔLUMO und Gap angegeben.

| **Erfindungsgemäße Moleküle, in denen die chemischen Einheiten aus Tabelle 1 als AF2 (Elektronen-donierend) wirken (nicht erfindungsgemäße Moleküle sind mit einem * markiert)** | | | |
|---|---|---|---|
| 154 - S - 152 (1.09 1.33 2.93) | 154 - S - 342 (0.94 0.93 3.33) | 154 - S - 343 (1.47 1.42 2.84) | 154 - S - 429* (1.03 2.12 2.14) |
| 184 - S - 152 (0.80 0.97 3.22) | 184 - S - 343 (1.18 1.06 3.13) | 189 - S - 150 (0.93 1.41 3.10) | 189 - S - 151 (0.84 1.67 2.84) |
| 189 - S - 152 (1.48 1.97 2.54) | 189 - S - 153 (0.86 1.51 3.00) | 189 - S - 342 (1.33 1.57 2.94) | 189 - S - 343 (1.86 2.06 2.45) |
| 189 - S - 371 (1.02 1.65 2.85) | 189 - S - 429* (1.42 2.76 1.75) | 192* - S - 429* (1.14 1.24 2.03) | 193* - S - 57 (1.07 1.62 1.41) |
| 193* - S - 70 (0.86 1.29 1.74) | 193* - S - 125 (1.31 2.06 0.96) | 193* - S - 133* (0.91 0.85 2.17) | 193* - S - 151 (1.64 0.98 2.04) |
| 193* - S - 152 (2.28 1.28 1.74) | 193* - S - 153 (1.66 0.83 2.20) | 193* - S - 192* (1.08 0.84 2.19) | 193* - S - 215 (0.89 1.411.62) |
| 193* - S - 231* (1.13 0.95 2.07) | 193* - S - 255 (1.04 0.93 2.10) | 193* - S - 259 (0.90 1.74 1.28) | 193* - S - 271* (1.31 1.02 2.01) |
| 193* - S - 291 (1.39 0.86 2.16) | 193* - S - 320 (0.98 1.34 1.68) | 193* - S - 324 (0.96 1.84 1.19) | 193* - S - 342 (2.13 0.88 2.14) |
| 193* - S - 343 (2.66 1.37 1.65) | 193* - S - 364 (0.84 1.50 1.52) | 193* - S - 371 (1.82 0.97 2.05) | 193* - S - 429* (2.22 2.07 0.95) |
| 193* - S - 472* (1.21 1.65 1.37) | 206 - S - 125 (1.17 0.87 1.09) | 206 - S - 250 (0.94 1.00 0.96) | 206 - S - 429* (2.09 0.88 1.08) |
| 207 - S - 429* (1.57 0.93 1.60) | 244* - S - 125 (0.91 1.70 1.36) | 244* - S - 152 (1.88 0.91 2.14) | 244* - S - 343 (2.26 1.01 2.05) |
| 244* - S - 429* (1.82 1.71 1.35) | 244* - S - 472* (0.82 1.28 1.77) | 287* - S - 43 (0.82 1.02 1.92) | 287* - S - 57 (1.09 1.56 1.38) |
| 287* - S - 70 (0.89 1.23 1.71) | 287* - S - 125 (1.33 2.01 0.93) | 287* - S - 151 (1.66 0.93 2.01) | 287* - S - 152 (2.31 1.23 1.71) |
| 287* - S - 215 (0.91 1.35 1.59) | 287* - S - 231* (1.15 0.90 2.05) | 287* - S - 247 (0.81 1.86 1.08) | 287* - S - 255 (1.07 0.87 2.07) |
| 287* - S - 259 (0.92 1.69 1.25) | 287* - S - 271* (1.33 0.96 1.98) | 287* - S - 291 (1.41 0.81 2.13) | 287* - S - 320 (1.01 1.29 1.65) |
| 287* - S - 324 (0.98 1.78 1.16) | 287* - S - 342 (2.16 0.83 2.11) | 287* - S - 343 (2.68 1.32 1.62) | 287* - S - 364 (0.86 1.45 1.49) |
| 287* - S - 371 (1.84 0.92 2.03) | 287* - S - 429* (2.25 2.02 0.92) | 287* - S - 472* (1.24 1.60 1.35) | 291 - S - 429* (0.83 1.21 2.34) |
| 292 - S - 343 (1.94 0.85 2.37) | 292 - S - 429* (1.50 1.55 1.67) | 294 - S - 152 (1.60 0.92 2.42) | 294 - S - 343 (1.98 1.01 2.33) |
| 294 - S - 429* (1.54 1.71 1.63) | 295 - S - 125 (0.87 1.46 1.39) | 295 - S - 429* (1.79 1.47 1.38) | 296 - S - 429* (1.51 1.47 1.66) |
| 300 - S - 46 (1.11 0.99 2.71) | 300 - S - 125 (1.02 2.46 1.24) | 300 - S - 150 (1.44 1.12 2.58) | 300 - S - 151 (1.35 1.38 2.33) |
| 300 - S - 152 (1.99 1.67 2.03) | 300 - S - 153 (1.37 1.22 2.48) | 300 - S - 231* (0.84 1.34 2.36) | 300 - S - 271* (1.02 1.41 2.29) |
| 300 - S - 291 (1.10 1.26 2.45) | 300 - S - 342 (1.84 1.27 2.43) | 300 - S - 343 (2.37 1.77 1.93) | 300 - S - 371 (1.53 1.36 2.34) |
| 300 - S - 429* (1.93 2.47 1.24) | 300 - S - 472* (0.93 2.04 1.66) | 301 - S - 46 (1.06 1.28 2.76) | 301 - S - 125 (0.97 2.75 1.30) |
| 301 - S - 150 (1.39 1.41 2.64) | 301 - S - 151 (1.30 1.66 2.38) | 301 - S - 152 (1.94 1.96 2.08) | 301 - S - 153 (1.32 1.51 2.53) |

(fortgesetzt)

| | | | |
|---|---|---|---|
| **Erfindungsgemäße Moleküle, in denen die chemischen Einheiten aus Tabelle 1 als AF2 (Elektronen-donierend) wirken (nicht erfindungsgemäße Moleküle sind mit einem * markiert)** | | | |
| 301 - S - 184 (1.14 1.00 3.04) | 301 - S - 271* (0.97 1.70 2.34) | 301 - S - 291 (1.05 1.54 2.50) | 301 - S - 342 (1.79 1.56 2.48) |
| 301 - S - 343 (2.32 2.06 1.99) | 301 - S - 371 (1.48 1.65 2.39) | 301 - S - 429* (1.88 2.75 1.29) | 301 - S - 472* (0.88 2.33 1.71) |
| 302 - S - 46 (0.94 1.43 2.88) | 302 - S - 125 (0.85 2.90 1.42) | 302 - S - 150 (1.27 1.56 2.76) | 302 - S - 151 (1.18 1.82 2.50) |
| 302 - S - 152 (1.82 2.12 2.20) | 302 - S - 153 (1.20 1.66 2.66) | 302 - S - 184 (1.02 1.15 3.16) | 302 - S - 271* (0.85 1.85 2.46) |
| 302 - S - 291 (0.93 1.70 2.62) | 302 - S - 342 (1.67 1.72 2.60) | 302 - S - 343 (2.20 2.21 2.11) | 302 - S - 371 (1.36 1.80 2.51) |
| 302 - S - 429* (1.76 2.91 1.41) | 305 - S - 152 (1.68 1.01 2.34) | 305 - S - 343 (2.06 1.10 2.25) | 305 - S - 429* (1.62 1.80 1.55) |
| 331 - S - 57 (0.83 1.77 1.64) | 331 - S - 125 (1.07 2.21 1.19) | 331 - S - 150 (1.49 0.88 2.53) | 331 - S - 151 (1.40 1.13 2.27) |
| 331 - S - 152 (2.05 1.43 1.97) | 331 - S - 153 (1.43 0.98 2.43) | 331 - S - 192* (0.85 0.99 2.42) | 331 - S - 231* (0.89 1.10 2.31) |
| 331 - S - 250 (0.84 2.34 1.07) | 331 - S - 255 (0.81 1.08 2.33) | 331 - S - 271* (1.07 1.17 2.24) | 331 - S - 291 (1.15 1.01 2.39) |
| 331 - S - 342 (1.90 1.03 2.37) | 331 - S - 343 (2.43 1.52 1.88) | 331 - S - 371 (1.58 1.12 2.29) | 331 - S - 429* (1.99 2.22 1.18) |
| 331 - S - 472* (0.98 1.80 1.61) | 468* - S - 57 (1.13 1.14 1.35) | 468* - S - 70 (0.92 0.81 1.68) | 468* - S - 125 (1.36 1.58 0.90) |
| 468* - S - 152 (2.34 0.80 1.68) | 468* - S - 215 (0.94 0.93 1.56) | 468* - S - 247 (0.84 1.43 1.05) | 468* - S - 259 (0.96 1.26 1.22) |
| 468* - S - 320 (1.04 0.86 1.62) | 468* - S - 324 (1.01 1.36 1.13) | 468* - S - 343 (2.72 0.89 1.59) | 468* - S - 364 (0.90 1.02 1.46) |
| 468* - S - 368 (0.83 0.89 1.60) | 468* - S - 472* (1.27 1.17 1.31) | | |
| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
| 1* - S - 150 (2.45 1.42 1.57) | 1* - S - 151 (2.36 1.68 1.31) | 1* - S - 152 (3.01 1.98 1.01) | 1* - S - 153 (2.39 1.52 1.47) |
| 1* - S - 184 (2.20 1.01 1.98) | 1* - S - 192* (1.81 1.53 1.46) | 1* - S - 206 (0.86 1.89 1.10) | 1* - S - 207 (1.38 1.84 1.15) |
| 1* - S - 244* (1.12 1.06 1.93) | 1* - S - 291 (2.11 1.56 1.43) | 1* - S - 292 (1.45 1.22 1.77) | 1* - S - 294 (1.41 1.06 1.93) |
| 1* - S - 295 (1.16 1.30 1.69) | 1* - S - 296 (1.44 1.30 1.69) | 1* - S - 305 (1.33 0.96 2.03) | 1* - S - 342 (2.86 1.58 1.41) |
| 1* - S - 343 (3.39 2.07 0.92) | 2* - S - 150 (2.44 1.34 1.58) | 2* - S - 151 (2.35 1.60 1.32) | 2* - S - 152 (3.00 1.90 1.02) |
| 2* - S - 153 (2.38 1.44 1.48) | 2* - S - 184 (2.19 0.93 1.99) | 2* - S - 192* (1.80 1.45 1.47) | 2* - S - 206 (0.85 1.81 1.11) |
| 2* - S - 207 (1.37 1.76 1.16) | 2* - S - 244* (1.11 0.98 1.94) | 2* - S - 291 (2.11 1.48 1.44) | 2* - S - 292 (1.44 1.14 1.78) |
| 2* - S - 294 (1.40 0.98 1.94) | 2* - S - 295 (1.15 1.22 1.70) | 2* - S - 296 (1.43 1.22 1.70) | 2* - S - 305 (1.32 0.88 2.03) |
| 2* - S - 342 (2.85 1.50 1.42) | 2* - S - 343 (3.38 1.99 0.93) | 3* - S - 150 (2.70 1.79 1.32) | 3* - S - 151 (2.61 2.05 1.06) |
| 3* - S - 153 (2.64 1.89 1.22) | 3* - S - 154 (2.17 1.02 2.09) | 3* - S - 184 (2.45 1.39 1.73) | 3* - S - 192* (2.06 1.91 1.21) |
| 3* - S - 193* (0.98 1.07 2.05) | 3* - S - 207 (1.63 2.21 0.90) | 3* - S - 244* (1.37 1.44 1.68) | 3* - S - 287* (0.95 1.12 1.99) |
| 3* - S - 291 (2.36 1.93 1.18) | 3* - S - 292 (1.70 1.59 1.52) | 3* - S - 294 (1.65 1.43 1.68) | 3* - S - 295 (1.41 1.68 1.44) |
| 3* - S - 296 (1.69 1.67 1.44) | 3* - S - 305 (1.58 1.34 1.78) | 3* - S - 331 (1.21 0.92 2.20) | 3* - S - 342 (3.11 1.95 1.16) |
| 3* - S - 468* (0.92 1.55 1.57) | 4* - S - 150 (2.33 1.26 1.70) | 4* - S - 151 (2.23 1.52 1.44) | 4* - S - 152 (2.88 1.82 1.14) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 4* - S - 153 (2.26 1.36 1.60) | 4* - S - 184 (2.08 0.85 2.11) | 4* - S - 192* (1.68 1.37 1.59) | 4* - S - 207 (1.25 1.68 1.28) |
| 4* - S - 244* (1.00 0.90 2.06) | 4* - S - 291 (1.99 1.40 1.56) | 4* - S - 292 (1.32 1.06 1.90) | 4* - S - 294 (1.28 0.90 2.06) |
| 4* - S - 295 (1.03 1.14 1.82) | 4* - S - 296 (1.31 1.14 1.82) | 4* - S - 305 (1.20 0.80 2.15) | 4* - S - 342 (2.73 1.42 1.54) |
| 4* - S - 343 (3.26 1.91 1.05) | 5* - S - 150 (2.46 1.03 1.56) | 5* - S - 151 (2.37 1.29 1.30) | 5* - S - 152 (3.01 1.59 1.01) |
| 5* - S - 153 (2.39 1.13 1.46) | 5* - S - 192* (1.82 1.15 1.45) | 5* - S - 206 (0.87 1.50 1.09) | 5* - S - 207 (1.39 1.45 1.15) |
| 5* - S - 291 (2.12 1.17 1.42) | 5* - S - 292 (1.46 0.83 1.77) | 5* - S - 295 (1.17 0.92 1.68) | 5* - S - 296 (1.45 0.91 1.68) |
| 5* - S - 342 (2.86 1.19 1.41) | 5* - S - 343 (3.39 1.68 0.91) | 6 - S - 150 (2.70 1.33 1.33) | 6 - S - 151 (2.61 1.58 1.07) |
| 6 - S - 153 (2.63 1.43 1.23) | 6 - S - 184 (2.45 0.92 1.74) | 6 - S - 192* (2.05 1.44 1.22) | 6 - S - 207 (1.62 1.74 0.91) |
| 6 - S - 244* (1.37 0.97 1.69) | 6 - S - 291 (2.36 1.46 1.19) | 6 - S - 292 (1.69 1.12 1.53) | 6 - S - 294 (1.65 0.97 1.69) |
| 6 - S - 295 (1.40 1.21 1.45) | 6 - S - 296 (1.68 1.20 1.45) | 6 - S - 305 (1.57 0.87 1.78) | 6 - S - 342 (3.10 1.48 1.17) |
| 6 - S - 468* (0.91 1.08 1.57) | 7 - S - 150 (2.69 1.34 1.34) | 7 - S - 151 (2.60 1.60 1.08) | 7 - S - 153 (2.62 1.44 1.23) |
| 7 - S - 184 (2.44 0.93 1.74) | 7 - S - 192* (2.05 1.45 1.22) | 7 - S - 207 (1.61 1.76 0.92) | 7 - S - 244* (1.36 0.98 1.69) |
| 7 - S - 291 (2.35 1.48 1.20) | 7 - S - 292 (1.68 1.14 1.54) | 7 - S - 294 (1.64 0.98 1.70) | 7 - S - 295 (1.40 1.22 1.45) |
| 7 - S - 296 (1.67 1.22 1.46) | 7 - S - 305 (1.56 0.88 1.79) | 7 - S - 342 (3.09 1.50 1.18) | 7 - S - 468* (0.90 1.09 1.58) |
| 8 - S - 150 (2.55 1.46 1.48) | 8 - S - 151 (2.46 1.72 1.22) | 8 - S - 152 (3.10 2.02 0.92) | 8 - S - 153 (2.48 1.56 1.38) |
| 8 - S - 184 (2.30 1.06 1.88) | 8 - S - 192* (1.90 1.58 1.36) | 8 - S - 206 (0.95 1.93 1.01) | 8 - S - 207 (1.47 1.88 1.06) |
| 8 - S - 244* (1.22 1.11 1.83) | 8 - S - 291 (2.21 1.60 1.34) | 8 - S - 292 (1.54 1.26 1.68) | 8 - S - 294 (1.50 1.10 1.84) |
| 8 - S - 295 (1.25 1.35 1.59) | 8 - S - 296 (1.53 1.34 1.60) | 8 - S - 305 (1.42 1.01 1.93) | 8 - S - 342 (2.95 1.62 1.32) |
| 9* - S - 150 (1.58 1.00 2.44) | 9* - S - 151 (1.49 1.25 2.18) | 9* - S - 152 (2.14 1.55 1.88) | 9* - S - 153 (1.52 1.10 2.34) |
| 9* - S - 192* (0.94 1.11 2.33) | 9* - S - 291 (1.24 1.13 2.30) | 9* - S - 342 (1.99 1.15 2.28) | 9* - S - 343 (2.52 1.65 1.79) |
| 10 - S - 150 (2.72 1.27 1.30) | 10 - S - 151 (2.63 1.53 1.05) | 10 - S - 153 (2.65 1.37 1.20) | 10 - S - 184 (2.47 0.86 1.71) |
| 10 - S - 192* (2.08 1.38 1.19) | 10 - S - 244* (1.39 0.91 1.66) | 10 - S - 291 (2.38 1.41 1.17) | 10 - S - 292 (1.71 1.07 1.51) |
| 10 - S - 294 (1.67 0.91 1.67) | 10 - S - 295 (1.43 1.15 1.42) | 10 - S - 296 (1.70 1.15 1.43) | 10 - S - 305 (1.59 0.82 1.76) |
| 10 - S - 342 (3.12 1.43 1.15) | 10 - S - 468* (0.94 1.02 1.55) | 11* - S - 151 (2.38 1.01 1.29) | 11* - S - 152 (3.03 1.31 0.99) |
| 11* - S - 153 (2.41 0.85 1.45) | 11* - S - 192* (1.83 0.87 1.44) | 11* - S - 206 (0.88 1.22 1.08) | 11* - S - 207 (1.40 1.17 1.13) |
| 11* - S - 291 (2.14 0.89 1.41) | 11* - S - 342 (2.88 0.91 1.39) | 12* - S - 150 (2.72 1.02 1.30) | 12* - S - 151 (2.63 1.28 1.05) |
| 12* - S - 153 (2.65 1.12 1.20) | 12* - S - 192* (2.08 1.14 1.19) | 12* - S - 291 (2.38 1.16 1.17) | 12* - S - 292 (1.72 0.82 1.51) |
| 12* - S - 295 (1.43 0.91 1.42) | 12* - S - 296 (1.70 0.90 1.43) | 12* - S - 342 (3.12 1.18 1.15) | 13* - S - 150 (2.76 1.72 1.26) |
| 13* - S - 151 (2.67 1.98 1.00) | 13* - S - 153 (2.70 1.82 1.16) | 13* - S - 154 (2.23 0.95 2.03) | 13* - S - 184 (2.511.31 1.67) |
| 13* - S - 192* (2.12 1.83 1.15) | 13* - S - 193* (1.04 0.99 1.99) | 13* - S - 244* (1.43 1.36 1.62) | 13* - S - 287* (1.01 1.05 1.93) |
| 13* - S - 291 (2.42 1.86 1.12) | 13* - S - 292 (1.76 1.52 1.46) | 13* - S - 294 (1.72 1.36 1.62) | 13* - S - 295 (1.47 1.60 1.38) |
| 13* - S - 296 (1.75 1.60 1.38) | 13* - S - 305 (1.64 1.26 1.72) | 13* - S - 331 (1.27 0.84 2.14) | 13* - S - 342 (3.17 1.88 1.10) |
| 13* - S - 468* (0.98 1.47 1.51) | 14* - S - 150 (2.17 1.36 1.86) | 14* - S - 151 (2.08 1.61 1.60) | 14* - S - 152 (2.72 1.91 1.30) |
| 14* - S - 153 (2.10 1.46 1.76) | 14* - S - 184 (1.92 0.95 2.27) | 14* - S - 192* (1.52 1.47 1.74) | 14* - S - 207 (1.09 1.77 1.44) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 14* - S - 244* (0.84 1.00 2.21) | 14* - S - 291 (1.83 1.49 1.72) | 14* - S - 292 (1.16 1.15 2.06) | 14* - S - 294 (1.12 1.00 2.22) |
| 14* - S - 295 (0.87 1.24 1.97) | 14* - S - 296 (1.15 1.23 1.98) | 14* - S - 305 (1.04 0.90 2.31) | 14* - S - 342 (2.57 1.51 1.70) |
| 14* - S - 343 (3.10 2.01 1.21) | 15* - S - 287* (1.96 0.84 0.99) | 16* - S - 150 (2.69 1.33 1.34) | 16* - S - 151 (2.60 1.58 1.08) |
| 16* - S - 153 (2.62 1.43 1.24) | 16* - S - 184 (2.44 0.92 1.74) | 16* - S - 192* (2.04 1.44 1.22) | 16* - S - 207 (1.61 1.74 0.92) |
| 16* - S - 244* (1.36 0.97 1.69) | 16* - S - 291 (2.35 1.46 1.20) | 16* - S - 292 (1.68 1.12 1.54) | 16* - S - 294 (1.64 0.97 1.70) |
| 16* - S - 295 (1.39 1.21 1.45) | 16* - S - 296 (1.67 1.21 1.46) | 16* - S - 305 (1.56 0.87 1.79) | 16* - S - 342 (3.09 1.48 1.18) |
| 16* - S - 468* (0.90 1.08 1.58) | 17* - S - 150 (3.01 1.44 1.02) | 17* - S - 153 (2.94 1.54 0.91) | 17* - S - 184 (2.76 1.03 1.42) |
| 17* - S - 192* (2.37 1.55 0.90) | 17* - S - 244* (1.68 1.08 1.37) | 17* - S - 292 (2.00 1.24 1.22) | 17* - S - 294 (1.96 1.08 1.38) |
| 17* - S - 295 (1.72 1.32 1.13) | 17* - S - 296 (1.99 1.32 1.14) | 17* - S - 305 (1.88 0.98 1.47) | 17* - S - 468* (1.22 1.19 1.26) |
| 18* - S - 150 (2.96 1.16 1.06) | 18* - S - 153 (2.90 1.26 0.96) | 18* - S - 192* (2.32 1.28 0.95) | 18* - S - 244* (1.63 0.81 1.42) |
| 18* - S - 291 (2.62 1.30 0.92) | 18* - S - 292 (1.96 0.96 1.26) | 18* - S - 294 (1.91 0.80 1.42) | 18* - S - 295 (1.67 1.05 1.18) |
| 18* - S - 296 (1.95 1.04 1.18) | 18* - S - 342 (3.37 1.32 0.90) | 18* - S - 468* (1.18 0.92 1.31) | 19* - S - 150 (2.01 1.10 2.01) |
| 19* - S - 151 (1.92 1.36 1.75) | 19* - S - 152 (2.57 1.66 1.45) | 19* - S - 153 (1.95 1.20 1.91) | 19* - S - 192* (1.37 1.21 1.90) |
| 19* - S - 207 (0.94 1.52 1.59) | 19* - S - 291 (1.67 1.24 1.87) | 19* - S - 292 (1.01 0.90 2.21) | 19* - S - 296 (1.00 0.98 2.13) |
| 19* - S - 342 (2.42 1.26 1.85) | 19* - S - 343 (2.94 1.75 1.36) | 20* - S - 152 (2.03 1.01 1.99) | 20* - S - 343 (2.41 1.10 1.90) |
| 21 - S - 150 (2.57 1.36 1.45) | 21 - S - 151 (2.48 1.62 1.19) | 21 - S - 153 (2.51 1.46 1.35) | 21 - S - 184 (2.32 0.95 1.86) |
| 21 - S - 192* (1.93 1.47 1.34) | 21- S - 206 (0.98 1.83 0.98) | 21- S - 207 (1.50 1.78 1.03) | 21- S - 244* (1.24 1.00 1.81) |
| 21 - S - 291 (2.23 1.50 1.31) | 21 - S - 292 (1.57 1.16 1.65) | 21 - S - 294 (1.52 1.00 1.81) | 21 - S - 295 (1.28 1.24 1.57) |
| 21 - S - 296 (1.56 1.24 1.57) | 21 - S - 305 (1.45 0.91 1.91) | 21 - S - 342 (2.98 1.52 1.29) | 22 - S - 150 (3.10 1.70 0.93) |
| 22 - S - 154 (2.56 0.93 1.70) | 22 - S - 184 (2.85 1.29 1.33) | 22 - S - 193* (1.37 0.97 1.65) | 22 - S - 244* (1.77 1.34 1.28) |
| 22 - S - 287* (1.35 1.03 1.60) | 22 - S - 292 (2.09 1.50 1.13) | 22 - S - 294 (2.05 1.34 1.29) | 22 - S - 295 (1.80 1.58 1.04) |
| 22 - S - 296 (2.08 1.58 1.05) | 22 - S - 305 (1.97 1.24 1.38) | 22 - S - 331 (1.61 0.82 1.80) | 22 - S - 468* (1.31 1.45 1.17) |
| 23* - S - 150 (2.99 1.85 1.04) | 23* - S - 153 (2.92 1.95 0.93) | 23* - S - 154 (2.45 1.08 1.81) | 23* - S - 184 (2.74 1.44 1.44) |
| 23* - S - 192* (2.35 1.96 0.92) | 23* - S - 193* (1.26 1.12 1.76) | 23* - S - 244* (1.66 1.49 1.39) | 23* - S - 287* (1.24 1.18 1.71) |
| 23* - S - 292 (1.98 1.65 1.24) | 23* - S - 294 (1.94 1.49 1.40) | 23* - S - 295 (1.70 1.73 1.15) | 23* - S - 296 (1.97 1.73 1.16) |
| 23* - S - 305 (1.86 1.40 1.49) | 23* - S - 331 (1.50 0.97 1.91) | 23* - S - 468* (1.20 1.60 1.28) | 24 - S - 150 (2.73 1.25 1.30) |
| 24 - S - 151 (2.64 1.51 1.04) | 24 - S - 153 (2.66 1.35 1.20) | 24 - S - 184 (2.48 0.84 1.70) | 24 - S - 192* (2.08 1.36 1.18) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 24 - S - 244* (1.40 0.89 1.65) | 24 - S - 291 (2.39 1.39 1.16) | 24 - S - 292 (1.72 1.05 1.50) | 24 - S - 294 (1.68 0.89 1.66) |
| 24 - S - 295 (1.43 1.13 1.41) | 24 - S - 296 (1.71 1.13 1.42) | 24 - S - 342 (3.13 1.41 1.14) | 24 - S - 468* (0.94 1.01 1.54) |
| 26* - S - 150 (3.09 2.16 0.93) | 26* - S - 154 (2.56 1.39 1.70) | 26* - S - 184 (2.84 1.75 1.34) | 26* - S - 193* (1.36 1.43 1.66) |
| 26* - S - 244* (1.76 1.80 1.29) | 26* - S - 287* (1.34 1.49 1.61) | 26* - S - 292 (2.09 1.96 1.14) | 26* - S - 294 (2.04 1.80 1.29) |
| 26* - S - 295 (1.80 2.04 1.05) | 26* - S - 296 (2.08 2.04 1.06) | 26* - S - 300 (1.65 1.04 2.05) | 26* - S - 305 (1.97 1.70 1.39) |
| 26* - S - 331 (1.60 1.28 1.81) | 26* - S - 468* (1.31 1.91 1.18) | 27* - S - 152 (2.12 0.84 1.90) | 27* - S - 343 (2.50 0.93 1.81) |
| 29* - S - 152 (2.29 0.85 1.73) | 29* - S - 343 (2.67 0.94 1.63) | 30 - S - 150 (2.33 1.07 1.69) | 30 - S - 151 (2.24 1.33 1.44) |
| 30 - S - 152 (2.88 1.63 1.14) | 30 - S - 153 (2.26 1.18 1.59) | 30 - S - 192* (1.69 1.19 1.58) | 30 - S - 207 (1.26 1.49 1.28) |
| 30 - S - 291 (1.99 1.21 1.56) | 30 - S - 292 (1.32 0.87 1.90) | 30 - S - 295 (1.04 0.96 1.81) | 30 - S - 296 (1.31 0.95 1.82) |
| 30 - S - 342 (2.73 1.23 1.54) | 30 - S - 343 (3.26 1.72 1.05) | 31 - S - 150 (2.31 1.12 1.71) | 31 - S - 151 (2.22 1.38 1.45) |
| 31 - S - 152 (2.87 1.68 1.15) | 31 - S - 153 (2.25 1.22 1.61) | 31 - S - 192* (1.67 1.23 1.60) | 31 - S - 207 (1.24 1.54 1.29) |
| 31 - S - 291 (1.97 1.26 1.57) | 31 - S - 292 (1.31 0.92 1.91) | 31 - S - 295 (1.02 1.00 1.83) | 31 - S - 296 (1.30 1.00 1.83) |
| 31 - S - 342 (2.72 1.28 1.55) | 31 - S - 343 (3.25 1.77 1.06) | 32 - S - 150 (2.23 1.27 1.80) | 32 - S - 151 (2.13 1.53 1.54) |
| 32 - S - 152 (2.78 1.83 1.24) | 32 - S - 153 (2.16 1.37 1.70) | 32 - S - 184 (1.97 0.86 2.21) | 32 - S - 192* (1.58 1.38 1.69) |
| 32 - S - 207 (1.15 1.69 1.38) | 32 - S - 244* (0.90 0.91 2.16) | 32 - S - 291 (1.89 1.41 1.66) | 32 - S - 292 (1.22 1.07 2.00) |
| 32 - S - 294 (1.18 0.91 2.16) | 32 - S - 295 (0.93 1.15 1.92) | 32 - S - 296 (1.21 1.15 1.92) | 32 - S - 305 (1.10 0.82 2.25) |
| 32 - S - 342 (2.63 1.43 1.64) | 32 - S - 343 (3.16 1.92 1.15) | 33 - S - 150 (2.28 1.24 1.74) | 33 - S - 151 (2.19 1.49 1.49) |
| 33 - S - 152 (2.83 1.79 1.19) | 33 - S - 153 (2.21 1.34 1.64) | 33 - S - 184 (2.03 0.83 2.15) | 33 - S - 192* (1.64 1.35 1.63) |
| 33 - S - 207 (1.21 1.65 1.33) | 33 - S - 244* (0.95 0.88 2.10) | 33 - S - 291 (1.94 1.37 1.61) | 33 - S - 292 (1.27 1.03 1.95) |
| 33 - S - 294 (1.23 0.87 2.11) | 33 - S - 295 (0.99 1.12 1.86) | 33 - S - 296 (1.26 1.11 1.87) | 33 - S - 342 (2.68 1.39 1.59) |
| 33 - S - 343 (3.21 1.88 1.10) | 34 - S - 150 (2.40 1.24 1.62) | 34 - S - 151 (2.31 1.50 1.36) | 34 - S - 152 (2.96 1.80 1.06) |
| 34 - S - 153 (2.34 1.34 1.52) | 34 - S - 184 (2.15 0.84 2.03) | 34 - S - 192* (1.76 1.36 1.51) | 34 - S - 206 (0.81 1.71 1.15) |
| 34 - S - 207 (1.33 1.66 1.20) | 34 - S - 244* (1.07 0.89 1.98) | 34 - S - 291 (2.06 1.38 1.48) | 34 - S - 292 (1.40 1.04 1.82) |
| 34 - S - 294 (1.36 0.88 1.98) | 34 - S - 295 (1.11 1.13 1.74) | 34 - S - 296 (1.39 1.12 1.74) | 34 - S - 342 (2.81 1.40 1.46) |
| 34 - S - 343 (3.34 1.89 0.97) | 35 - S - 150 (2.27 1.29 1.76) | 35 - S - 151 (2.17 1.55 1.50) | 35 - S - 152 (2.82 1.85 1.20) |
| 35 - S - 153 (2.20 1.39 1.66) | 35 - S - 184 (2.02 0.88 2.17) | 35 - S - 192* (1.62 1.40 1.65) | 35 - S - 207 (1.19 1.71 1.34) |
| 35 - S - 244* (0.94 0.93 2.12) | 35 - S - 291 (1.93 1.43 1.62) | 35 - S - 292 (1.26 1.09 1.96) | 35 - S - 294 (1.22 0.93 2.12) |
| 35 - S - 295 (0.97 1.17 1.88) | 35 - S - 296 (1.25 1.17 1.88) | 35 - S - 305 (1.14 0.84 2.21) | 35 - S - 342 (2.67 1.45 1.60) |
| 35 - S - 343 (3.20 1.94 1.11) | 38 - S - 150 (2.12 0.86 1.90) | 38 - S - 151 (2.03 1.12 1.64) | 38 - S - 152 (2.68 1.41 1.34) |
| 38 - S - 153 (2.06 0.96 1.80) | 38 - S - 192* (1.48 0.97 1.79) | 38 - S - 207 (1.05 1.27 1.48) | 38 - S - 291 (1.78 0.99 1.76) |
| 38 - S - 342 (2.53 1.01 1.74) | 38 - S - 343 (3.06 1.51 1.25) | 39 - S - 150 (2.70 2.03 1.32) | 39 - S - 151 (2.61 2.29 1.07) |
| 39 - S - 153 (2.63 2.13 1.22) | 39 - S - 154 (2.17 1.26 2.10) | 39 - S - 184 (2.45 1.62 1.73) | 39 - S - 192* (2.06 2.14 1.21) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 39 - S - 193* (0.97 1.31 2.05) | 39 - S - 207 (1.63 2.45 0.91) | 39 - S - 244* (1.37 1.67 1.68) | 39 - S - 287* (0.95 1.36 2.00) |
| 39 - S - 291 (2.36 2.17 1.19) | 39 - S - 292 (1.69 1.83 1.53) | 39 - S - 294 (1.65 1.67 1.69) | 39 - S - 295 (1.41 1.91 1.44) |
| 39 - S - 296 (1.68 1.91 1.45) | 39 - S - 300 (1.26 0.91 2.44) | 39 - S - 305 (1.57 1.58 1.78) | 39 - S - 331 (1.21 1.16 2.20) |
| 39 - S - 342 (3.10 2.19 1.17) | 39 - S - 468* (0.91 1.79 1.57) | 40* - S - 152 (2.47 0.85 1.55) | 40* - S - 343 (2.85 0.94 1.46) |
| 41 - S - 150 (3.01 1.88 1.01) | 41 - S - 153 (2.95 1.98 0.91) | 41 - S - 154 (2.48 1.11 1.78) | 41 - S - 184 (2.76 1.47 1.42) |
| 41 - S - 193* (1.29 1.16 1.74) | 41 - S - 244* (1.68 1.52 1.37) | 41 - S - 287* (1.26 1.21 1.68) | 41 - S - 292 (2.01 1.68 1.21) |
| 41 - S - 294 (1.96 1.52 1.37) | 41 - S - 295 (1.72 1.76 1.13) | 41 - S - 296 (2.00 1.76 1.13) | 41 - S - 305 (1.89 1.43 1.47) |
| 41 - S - 331 (1.52 1.01 1.89) | 41 - S - 468* (1.23 1.64 1.26) | 44 - S - 150 (1.85 1.13 2.17) | 44 - S - 151 (1.76 1.39 1.92) |
| 44 - S - 152 (2.40 1.69 1.62) | 44 - S - 153 (1.78 1.23 2.07) | 44 - S - 192* (1.21 1.25 2.06) | 44 - S - 291 (1.51 1.27 2.04) |
| 44 - S - 292 (0.84 0.93 2.38) | 44 - S - 296 (0.83 1.01 2.30) | 44 - S - 342 (2.25 1.29 2.02) | 44 - S - 343 (2.78 1.78 1.53) |
| 45* - S - 151 (2.36 0.90 1.31) | 45* - S - 152 (3.00 1.20 1.02) | 45* - S - 206 (0.86 1.11 1.10) | 45* - S - 207 (1.38 1.06 1.16) |
| 45* - S - 342 (2.86 0.80 1.42) | 45* - S - 343 (3.38 1.29 0.92) | 47* - S - 150 (2.59 0.82 1.43) | 47* - S - 151 (2.50 1.08 1.17) |
| 47* - S - 153 (2.53 0.92 1.33) | 47* - S - 192* (1.95 0.93 1.32) | 47* - S - 206 (1.00 1.29 0.96) | 47* - S - 207 (1.52 1.24 1.01) |
| 47* - S - 291 (2.25 0.96 1.29) | 47* - S - 342 (3.00 0.98 1.27) | 48* - S - 151 (2.43 1.05 1.24) | 48* - S - 152 (3.08 1.35 0.94) |
| 48* - S - 153 (2.46 0.89 1.40) | 48* - S - 192* (1.88 0.90 1.39) | 48* - S - 206 (0.93 1.26 1.03) | 48* - S - 207 (1.45 1.21 1.08) |
| 48* - S - 291 (2.18 0.93 1.36) | 48* - S - 342 (2.93 0.95 1.34) | 49 - S - 154 (2.96 1.27 1.30) | 49 - S - 184 (3.24 1.63 0.94) |
| 49 - S - 193* (1.77 1.31 1.26) | 49 - S - 287* (1.74 1.37 1.20) | 49 - S - 300 (2.05 0.92 1.65) | 49 - S - 305 (2.37 1.58 0.99) |
| 49 - S - 331 (2.00 1.16 1.41) | 53* - S - 150 (2.47 1.60 1.56) | 53* - S - 151 (2.37 1.85 1.30) | 53* - S - 152 (3.02 2.15 1.00) |
| 53* - S - 153 (2.40 1.70 1.46) | 53* - S - 154 (1.93 0.83 2.33) | 53* - S - 184 (2.21 1.19 1.97) | 53* - S - 192* (1.82 1.71 1.45) |
| 53* - S - 206 (0.87 2.07 1.09) | 53* - S - 207 (1.39 2.01 1.14) | 53* - S - 244* (1.14 1.24 1.92) | 53* - S - 291 (2.13 1.73 1.42) |
| 53* - S - 292 (1.46 1.39 1.76) | 53* - S - 294 (1.42 1.24 1.92) | 53* - S - 295 (1.17 1.48 1.68) | 53* - S - 296 (1.45 1.47 1.68) |
| 53* - S - 305 (1.34 1.14 2.01) | 53* - S - 342 (2.87 1.75 1.40) | 53* - S - 343 (3.40 2.25 0.91) | 54 - S - 150 (2.37 1.34 1.65) |
| 54 - S - 151 (2.28 1.60 1.39) | 54 - S - 152 (2.92 1.90 1.10) | 54 - S - 153 (2.30 1.44 1.55) | 54 - S - 184 (2.12 0.93 2.06) |
| 54 - S - 192* (1.73 1.45 1.54) | 54 - S - 207 (1.30 1.76 1.24) | 54 - S - 244* (1.04 0.98 2.01) | 54 - S - 291 (2.03 1.48 1.51) |
| 54 - S - 292 (1.37 1.14 1.86) | 54 - S - 294 (1.32 0.98 2.01) | 54 - S - 295 (1.08 1.22 1.77) | 54 - S - 296 (1.36 1.22 1.77) |
| 54 - S - 305 (1.25 0.89 2.11) | 54 - S - 342 (2.77 1.50 1.50) | 54 - S - 343 (3.30 1.99 1.00) | 55 - S - 150 (2.29 1.53 1.73) |
| 55 - S - 151 (2.20 1.79 1.47) | 55 - S - 152 (2.85 2.09 1.17) | 55 - S - 153 (2.23 1.63 1.63) | 55 - S - 184 (2.04 1.13 2.14) |
| 55 - S - 192* (1.65 1.65 1.62) | 55 - S - 207 (1.22 1.95 1.31) | 55 - S - 244* (0.96 1.18 2.09) | 55 - S - 291 (1.95 1.67 1.59) |
| 55 - S - 292 (1.29 1.33 1.93) | 55 - S - 294 (1.25 1.17 2.09) | 55 - S - 295 (1.00 1.42 1.85) | 55 - S - 296 (1.28 1.41 1.85) |
| 55 - S - 305 (1.17 1.08 2.18) | 55 - S - 342 (2.70 1.69 1.57) | 55 - S - 343 (3.23 2.18 1.08) | 56 - S - 150 (1.58 0.82 2.45) |
| 56* - S - 151 (1.49 1.08 2.19) | 56* - S - 152 (2.13 1.38 1.89) | 56* - S - 153 (1.51 0.92 2.35) | 56* - S - 192* (0.93 0.93 2.34) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 56* - S - 291 (1.24 0.96 2.31) | 56* - S - 342 (1.98 0.98 2.29) | 56* - S - 343 (2.51 1.47 1.80) | 58 - S - 150 (2.43 1.06 1.59) |
| 58 - S - 151 (2.34 1.32 1.33) | 58 - S - 152 (2.99 1.62 1.03) | 58 - S - 153 (2.37 1.16 1.49) | 58 - S - 192* (1.79 1.17 1.48) |
| 58 - S - 206 (0.84 1.53 1.12) | 58 - S - 207 (1.36 1.48 1.17) | 58 - S - 291 (2.09 1.20 1.45) | 58 - S - 292 (1.43 0.86 1.79) |
| 58 - S - 295 (1.14 0.94 1.71) | 58 - S - 296 (1.42 0.94 1.71) | 58 - S - 342 (2.84 1.22 1.43) | 58 - S - 343 (3.37 1.71 0.94) |
| 59 - S - 150 (2.39 1.09 1.63) | 59 - S - 151 (2.30 1.35 1.37) | 59 - S - 152 (2.95 1.65 1.07) | 59 - S - 153 (2.33 1.19 1.53) |
| 59 - S - 192* (1.75 1.20 1.52) | 59 - S - 207 (1.32 1.51 1.21) | 59 - S - 291 (2.05 1.23 1.49) | 59 - S - 292 (1.39 0.89 1.83) |
| 59 - S - 295 (1.10 0.97 1.75) | 59 - S - 296 (1.38 0.97 1.75) | 59 - S - 342 (2.80 1.25 1.47) | 59 - S - 343 (3.33 1.74 0.98) |
| 60 - S - 150 (2.29 1.21 1.74) | 60 - S - 151 (2.20 1.46 1.48) | 60 - S - 152 (2.84 1.76 1.18) | 60 - S - 153 (2.22 1.31 1.64) |
| 60 - S - 192* (1.64 1.32 1.62) | 60 - S - 207 (1.21 1.62 1.32) | 60 - S - 244* (0.96 0.85 2.09) | 60 - S - 291 (1.95 1.34 1.60) |
| 60 - S - 292 (1.28 1.00 1.94) | 60 - S - 294 (1.24 0.85 2.10) | 60 - S - 295 (0.99 1.09 1.85) | 60 - S - 296 (1.27 1.08 1.86) |
| 60 - S - 342 (2.69 1.36 1.58) | 60 - S - 343 (3.22 1.86 1.09) | 61* - S - 152 (2.14 0.87 1.88) | 61* - S - 343 (2.52 0.96 1.78) |
| 63 - S - 150 (2.43 1.79 1.59) | 63 - S - 151 (2.34 2.05 1.34) | 63 - S - 152 (2.98 2.35 1.04) | 63 - S - 153 (2.36 1.89 1.49) |
| 63 - S - 154 (1.90 1.02 2.36) | 63 - S - 184 (2.18 1.38 2.00) | 63 - S - 192* (1.79 1.90 1.48) | 63 - S - 206 (0.84 2.26 1.12) |
| 63 - S - 207 (1.36 2.21 1.18) | 63 - S - 244* (1.10 1.43 1.95) | 63 - S - 291 (2.09 1.93 1.46) | 63 - S - 292 (1.43 1.59 1.80) |
| 63 - S - 294 (1.38 1.43 1.96) | 63 - S - 295 (1.14 1.67 1.71) | 63 - S - 296 (1.41 1.67 1.72) | 63 - S - 305 (1.31 1.33 2.05) |
| 63 - S - 331 (0.94 0.91 2.47) | 63 - S - 342 (2.83 1.95 1.44) | 63 - S - 343 (3.36 2.44 0.94) | 64 - S - 150 (2.36 1.30 1.66) |
| 64 - S - 151 (2.27 1.56 1.40) | 64 - S - 152 (2.92 1.86 1.10) | 64 - S - 153 (2.30 1.40 1.56) | 64 - S - 184 (2.11 0.89 2.07) |
| 64 - S - 192* (1.72 1.41 1.55) | 64 - S - 207 (1.29 1.72 1.24) | 64 - S - 244* (1.03 0.94 2.02) | 64 - S - 291 (2.02 1.44 1.52) |
| 64 - S - 292 (1.36 1.10 1.86) | 64 - S - 294 (1.32 0.94 2.02) | 64 - S - 295 (1.07 1.18 1.78) | 64 - S - 296 (1.35 1.18 1.78) |
| 64 - S - 305 (1.24 0.85 2.12) | 64 - S - 342 (2.77 1.46 1.50) | 64 - S - 343 (3.30 1.95 1.01) | 65* - S - 152 (2.13 1.07 1.89) |
| 65* - S - 343 (2.51 1.16 1.79) | 66 - S - 154 (2.84 1.08 1.42) | 66 - S - 184 (3.12 1.45 1.06) | 66 - S - 193* (1.64 1.13 1.38) |
| 66 - S - 244* (2.04 1.50 1.01) | 66 - S - 287* (1.62 1.18 1.33) | 66 - S - 294 (2.32 1.49 1.01) | 66 - S - 305 (2.25 1.40 1.11) |
| 66 - S - 331 (1.88 0.98 1.53) | 68* - S - 151 (1.78 0.81 1.90) | 68* - S - 152 (2.42 1.11 1.60) | 68* - S - 343 (2.80 1.20 1.51) |
| 71 - S - 150 (2.44 1.31 1.59) | 71 - S - 151 (2.35 1.57 1.33) | 71 - S - 152 (2.99 1.87 1.03) | 71 - S - 153 (2.37 1.42 1.49) |
| 71 - S - 184 (2.19 0.91 1.99) | 71 - S - 192* (1.79 1.43 1.47) | 71 - S - 206 (0.84 1.78 1.12) | 71 - S - 207 (1.36 1.73 1.17) |
| 71 - S - 244* (1.11 0.96 1.94) | 71 - S - 291 (2.10 1.45 1.45) | 71 - S - 292 (1.43 1.11 1.79) | 71 - S - 294 (1.39 0.95 1.95) |
| 71 - S - 295 (1.14 1.20 1.70) | 71 - S - 296 (1.42 1.19 1.71) | 71 - S - 305 (1.31 0.86 2.04) | 71 - S - 342 (2.84 1.47 1.43) |
| 71 - S - 343 (3.37 1.96 0.94) | 72 - S - 150 (2.70 2.07 1.32) | 72 - S - 151 (2.61 2.32 1.06) | 72 - S - 153 (2.64 2.17 1.22) |
| 72 - S - 154 (2.17 1.30 2.09) | 72 - S - 184 (2.45 1.66 1.73) | 72 - S - 192* (2.06 2.18 1.21) | 72 - S - 193* (0.98 1.34 2.05) |
| 72 - S - 207 (1.63 2.48 0.90) | 72 - S - 244* (1.37 1.71 1.68) | 72 - S - 287* (0.95 1.39 1.99) | 72 - S - 291 (2.37 2.20 1.18) |
| 72 - S - 292 (1.70 1.86 1.52) | 72 - S - 294 (1.66 1.71 1.68) | 72 - S - 295 (1.41 1.95 1.44) | 72 - S - 296 (1.69 1.95 1.44) |
| 72 - S - 300 (1.26 0.95 2.44) | 72 - S - 305 (1.58 1.61 1.77) | 72 - S - 331 (1.21 1.19 2.20) | 72 - S - 342 (3.11 2.22 1.16) |
| 72 - S - 468* (0.92 1.82 1.56) | 73 - S - 150 (2.60 2.10 1.42) | 73 - S - 151 (2.51 2.36 1.17) | 73 - S - 153 (2.53 2.20 1.32) |

(fortgesetzt)

| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
|---|---|---|---|
| 73 - S - 154 (2.07 1.33 2.19) | 73 - S - 184 (2.35 1.69 1.83) | 73 - S - 192* (1.96 2.21 1.31) | 73 - S - 193* (0.87 1.37 2.15) |
| 73 - S - 206 (1.01 2.57 0.95) | 73 - S - 207 (1.53 2.52 1.01) | 73 - S - 244* (1.27 1.74 1.78) | 73 - S - 287* (0.85 1.43 2.10) |
| 73 - S - 291 (2.26 2.24 1.29) | 73 - S - 292 (1.60 1.90 1.63) | 73 - S - 294 (1.55 1.74 1.78) | 73 - S - 295 (1.31 1.98 1.54) |
| 73 - S - 296 (1.59 1.98 1.54) | 73 - S - 300 (1.16 0.98 2.54) | 73 - S - 305 (1.48 1.64 1.88) | 73 - S - 331 (1.11 1.22 2.30) |
| 73 - S - 342 (3.00 2.26 1.27) | 73 - S - 468* (0.82 1.85 1.67) | 74 - S - 150 (2.43 0.98 1.60) | 74 - S - 151 (2.33 1.24 1.34) |
| 74 - S - 152 (2.98 1.54 1.04) | 74 - S - 153 (2.36 1.08 1.50) | 74 - S - 192* (1.78 1.09 1.49) | 74 - S - 206 (0.83 1.45 1.13) |
| 74 - S - 207 (1.35 1.40 1.18) | 74 - S - 291 (2.09 1.12 1.46) | 74 - S - 295 (1.13 0.86 1.72) | 74 - S - 296 (1.41 0.86 1.72) |
| 74 - S - 342 (2.83 1.14 1.44) | 74 - S - 343 (3.36 1.63 0.95) | 75 - S - 150 (3.05 1.19 0.98) | 75 - S - 244* (1.72 0.83 1.33) |
| 75 - S - 292 (2.04 0.98 1.18) | 75 - S - 294 (2.00 0.82 1.34) | 75 - S - 295 (1.76 1.07 1.09) | 75 - S - 296 (2.03 1.06 1.10) |
| 75 - S - 468* (1.26 0.94 1.22) | 76 - S - 154 (2.85 0.87 1.42) | 76 - S - 184 (3.13 1.23 1.05) | 76 - S - 193* (1.65 0.91 1.37) |
| 76 - S - 244* (2.05 1.28 1.00) | 76 - S - 287* (1.63 0.97 1.32) | 76 - S - 294 (2.33 1.28 1.01) | 76 - S - 305 (2.25 1.18 1.10) |
| 77 - S - 150 (2.89 1.52 1.13) | 77 - S - 153 (2.83 1.63 1.03) | 77 - S - 184 (2.64 1.12 1.54) | 77 - S - 192* (2.25 1.64 1.02) |
| 77 - S - 244* (1.56 1.17 1.49) | 77 - S - 287* (1.14 0.85 1.80) | 77 - S - 291 (2.56 1.66 0.99) | 77 - S - 292 (1.89 1.32 1.33) |
| 77 - S - 294 (1.85 1.16 1.49) | 77 - S - 295 (1.60 1.41 1.25) | 77 - S - 296 (1.88 1.40 1.25) | 77 - S - 305 (1.77 1.07 1.58) |
| 77 - S - 342 (3.30 1.68 0.97) | 77 - S - 468* (1.11 1.28 1.37) | 78 - S - 150 (3.07 1.58 0.95) | 78 - S - 154 (2.54 0.81 1.72) |
| 78 - S - 184 (2.82 1.17 1.36) | 78 - S - 193* (1.34 0.85 1.68) | 78 - S - 244* (1.74 1.22 1.31) | 78 - S - 287* (1.32 0.90 1.63) |
| 78 - S - 292 (2.07 1.37 1.16) | 78 - S - 294 (2.02 1.22 1.31) | 78 - S - 295 (1.78 1.46 1.07) | 78 - S - 296 (2.05 1.46 1.08) |
| 78 - S - 305 (1.95 1.12 1.41) | 78 - S - 468* (1.29 1.33 1.20) | 80* - S - 343 (2.58 0.83 1.73) | 83* - S - 150 (1.54 0.88 2.49) |
| 83* - S - 151 (1.45 1.14 2.23) | 83* - S - 152 (2.09 1.44 1.93) | 83* - S - 153 (1.47 0.98 2.39) | 83* - S - 192* (0.89 0.99 2.37) |
| 83* - S - 291 (1.20 1.02 2.35) | 83* - S - 342 (1.94 1.04 2.33) | 83* - S - 343 (2.47 1.53 1.84) | 85* - S - 152 (1.64 0.88 2.38) |
| 85* - S - 343 (2.02 0.97 2.29) | 88* - S - 150 (2.37 1.21 1.66) | 88* - S - 151 (2.28 1.47 1.40) | 88* - S - 152 (2.92 1.76 1.10) |
| 88* - S - 153 (2.30 1.31 1.56) | 88* - S - 192* (1.72 1.32 1.54) | 88* - S - 207 (1.29 1.62 1.24) | 88* - S - 244* (1.04 0.85 2.01) |
| 88* - S - 291 (2.03 1.34 1.52) | 88* - S - 292 (1.36 1.00 1.86) | 88* - S - 294 (1.32 0.85 2.02) | 88* - S - 295 (1.07 1.09 1.77) |
| 88* - S - 296 (1.35 1.09 1.78) | 88* - S - 342 (2.77 1.36 1.50) | 88* - S - 343 (3.30 1.86 1.01) | 91 - S - 150 (3.12 2.58 0.90) |
| 91 - S - 154 (2.59 1.81 1.68) | 91 - S - 184 (2.87 2.18 1.31) | 91 - S - 189 (2.19 1.18 2.31) | 91 - S - 193* (1.39 1.86 1.63) |
| 91 - S - 244* (1.79 2.23 1.26) | 91 - S - 287* (1.37 1.91 1.58) | 91 - S - 292 (2.11 2.38 1.11) | 91 - S - 294 (2.07 2.22 1.27) |
| 91 - S - 295 (1.83 2.47 1.02) | 91 - S - 296 (2.10 2.46 1.03) | 91 - S - 300 (1.68 1.47 2.02) | 91 - S - 301 (1.73 1.18 2.31) |
| 91 - S - 302 (1.85 1.03 2.46) | 91 - S - 305 (1.99 2.13 1.36) | 91 - S - 331 (1.63 1.71 1.78) | 91 - S - 468* (1.34 2.34 1.15) |
| 94* - S - 150 (2.63 1.63 1.40) | 94* - S - 151 (2.54 1.89 1.14) | 94* - S - 153 (2.56 1.73 1.30) | 94* - S - 154 (2.09 0.86 2.17) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 94* - S - 184 (2.38 1.22 1.80) | 94* - S - 192* (1.98 1.74 1.28) | 94* - S - 193* (0.90 0.91 2.12) | 94* - S - 206 (1.03 2.10 0.93) |
| 94* - S - 207 (1.55 2.05 0.98) | 94* - S - 244* (1.30 1.27 1.75) | 94* - S - 287* (0.88 0.96 2.07) | 94* - S - 291 (2.29 1.77 1.26) |
| 94* - S - 292 (1.62 1.43 1.60) | 94* - S - 294 (1.58 1.27 1.76) | 94* - S - 295 (1.33 1.51 1.51) | 94* - S - 296 (1.61 1.51 1.52) |
| 94* - S - 305 (1.50 1.18 1.85) | 94* - S - 342 (3.03 1.79 1.24) | 94* - S - 468* (0.84 1.39 1.64) | 95* - S - 150 (2.62 1.52 1.40) |
| 95* - S - 151 (2.53 1.78 1.14) | 95* - S - 153 (2.56 1.62 1.30) | 95* - S - 184 (2.37 1.11 1.81) | 95* - S - 192* (1.98 1.63 1.29) |
| 95* - S - 206 (1.03 1.99 0.93) | 95* - S - 207 (1.55 1.93 0.98) | 95* - S - 244* (1.29 1.16 1.76) | 95* - S - 287* (0.87 0.85 2.07) |
| 95* - S - 291 (2.28 1.65 1.26) | 9*5 - S - 292 (1.62 1.31 1.60) | 95* - S - 294 (1.58 1.16 1.76) | 95* - S - 295 (1.33 1.40 1.52) |
| 95* - S - 296 (1.61 1.40 1.52) | 95* - S - 305 (1.50 1.06 1.85) | 95* - S - 342 (3.03 1.67 1.24) | 95* - S - 468* (0.84 1.27 1.64) |
| 96* - S - 152 (3.05 1.08 0.97) | 96* - S - 206 (0.91 0.99 1.06) | 96* - S - 207 (1.43 0.94 1.11) | 97* - S - 150 (2.72 1.76 1.31) |
| 97* - S - 151 (2.63 2.01 1.05) | 97* - S - 153 (2.65 1.86 1.21) | 97* - S - 154 (2.18 0.99 2.08) | 97* - S - 184 (2.47 1.35 1.71) |
| 97* - S - 192* (2.07 1.87 1.19) | 97* - S - 193* (0.99 1.03 2.03) | 97* - S - 244* (1.39 1.40 1.66) | 97* - S - 287* (0.97 1.08 1.98) |
| 97* - S - 291 (2.38 1.89 1.17) | 97* - S - 292 (1.71 1.55 1.51) | 97* - S - 294 (1.67 1.40 1.67) | 97* - S - 295 (1.42 1.64 1.42) |
| 97* - S - 296 (1.70 1.63 1.43) | 97* - S - 305 (1.59 1.30 1.76) | 97* - S - 331 (1.23 0.88 2.18) | 97* - S - 342 (3.12 1.91 1.15) |
| 97* - S - 468* (0.93 1.51 1.55) | 98* - S - 150 (2.37 1.15 1.66) | 98* - S - 151 (2.28 1.41 1.40) | 98* - S - 152 (2.92 1.71 1.10) |
| 98* - S - 153 (2.30 1.25 1.55) | 98* - S - 192* (1.73 1.26 1.54) | 98* - S - 207 (1.30 1.57 1.24) | 98* - S - 291 (2.03 1.29 1.52) |
| 98* - S - 292 (1.36 0.95 1.86) | 98* - S - 295 (1.08 1.03 1.77) | 98* - S - 296 (1.35 1.03 1.78) | 98* - S - 342 (2.77 1.31 1.50) |
| 98* - S - 343 (3.30 1.80 1.01) | 102* - S - 150 (1.82 0.99 2.20) | 102* - S - 151 (1.73 1.25 1.95) | 102* - S - 152 (2.37 1.55 1.65) |
| 102* - S - 153 (1.75 1.09 2.10) | 102* - S - 192* (1.18 1.10 2.09) | 102* - S - 291 (1.48 1.13 2.07) | 102* - S - 296 (0.80 0.87 2.33) |
| 102* - S - 342 (2.22 1.15 2.05) | 102* - S - 343 (2.75 1.64 1.55) | 103* - S - 154 (2.74 0.84 1.52) | 103* - S - 184 (3.03 1.21 1.15) |
| 103* - S - 193* (1.55 0.89 1.47) | 103* - S - 244* (1.95 1.26 1.10) | 103* - S - 287* (1.53 0.94 1.42) | 103* - S - 292 (2.27 1.41 0.95) |
| 103* - S - 294 (2.23 1.25 1.11) | 103* - S - 305 (2.15 1.16 1.20) | 103* - S - 468* (1.49 1.37 0.99) | 104* - S - 150 (2.17 1.42 1.86) |
| 104* - S - 151 (2.07 1.68 1.60) | 104* - S - 152 (2.72 1.98 1.30) | 104* - S - 153 (2.10 1.52 1.76) | 104* - S - 184 (1.92 1.01 2.27) |
| 104* - S - 192* (1.52 1.53 1.75) | 104* - S - 207 (1.09 1.84 1.44) | 104* - S - 244* (0.84 1.06 2.22) | 104* - S - 291 (1.83 1.56 1.72) |
| 104* - S - 292 (1.16 1.21 2.06) | 104* - S - 294 (1.12 1.06 2.22) | 104* - S - 295 (0.87 1.30 1.98) | 104* - S - 296 (1.15 1.30 1.98) |
| 104* - S - 305 (1.04 0.96 2.31) | 104* - S - 342 (2.57 1.57 1.70) | 104* - S - 343 (3.10 2.07 1.21) | 105* - S - 150 (1.28 0.90 2.74) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 105* - S - 151 (1.19 1.16 2.48) | 105* - S - 152 (1.84 1.46 2.18) | 105* - S - 153 (1.22 1.00 2.64) | 105* - S - 291 (0.94 1.04 2.60) |
| 105* - S - 342 (1.69 1.06 2.58) | 105* - S - 343 (2.22 1.55 2.09) | 106* - S - 150 (1.87 1.46 2.15) | 106* - S - 151 (1.78 1.72 1.90) |
| 106* - S - 152 (2.42 2.02 1.60) | 106* - S - 153 (1.80 1.57 2.05) | 106* - S - 184 (1.62 1.06 2.56) | 106* - S - 192* (1.23 1.58 2.04) |
| 106* - S - 291 (1.53 1.60 2.02) | 106* - S - 292 (0.87 1.26 2.36) | 106* - S - 294 (0.82 1.10 2.51) | 106* - S - 296 (0.86 1.34 2.28) |
| 106* - S - 342 (2.27 1.62 2.00) | 106* - S - 343 (2.80 2.11 1.50) | 107* - S - 343 (1.75 0.81 2.55) | 111* - S - 150 (0.83 0.88 3.20) |
| 111* - S - 152 (1.38 1.44 2.64) | 111* - S - 342 (1.23 1.04 3.04) | 111* - S - 343 (1.76 1.53 2.55) | 112* - S - 152 (2.19 1.06 1.83) |
| 112* - S - 343 (2.57 1.15 1.74) | 114* - S - 150 (1.99 1.23 2.04) | 114* - S - 151 (1.90 1.48 1.78) | 114* - S - 152 (2.54 1.78 1.48) |
| 114* - S - 153 (1.92 1.33 1.94) | 114* - S - 184 (1.74 0.82 2.44) | 114* - S - 192* (1.34 1.34 1.92) | 114* - S - 207 (0.91 1.64 1.62) |
| 114* - S - 291 (1.65 1.36 1.90) | 114* - S - 292 (0.98 1.02 2.24) | 114* - S - 294 (0.94 0.86 2.40) | 114* - S - 296 (0.97 1.10 2.16) |
| 114* - S - 342 (2.39 1.38 1.88) | 114* - S - 343 (2.92 1.87 1.39) | 117* - S - 150 (2.46 1.68 1.57) | 117* - S - 151 (2.37 1.94 1.31) |
| 117* - S - 152 (3.01 2.24 1.01) | 117* - S - 153 (2.39 1.78 1.47) | 117* - S - 154 (1.92 0.91 2.34) | 117* - S - 184 (2.21 1.27 1.98) |
| 117* - S - 192* (1.81 1.79 1.46) | 117* - S - 206 (0.86 2.15 1.10) | 117* - S - 207 (1.38 2.10 1.15) | 117* - S - 244* (1.13 1.32 1.93) |
| 117* - S - 291 (2.12 1.82 1.43) | 117* - S - 292 (1.45 1.47 1.77) | 117* - S - 294 (1.41 1.32 1.93) | 117* - S - 295 (1.16 1.56 1.69) |
| 117* - S - 296 (1.44 1.56 1.69) | 117* - S - 305 (1.33 1.22 2.02) | 117* - S - 331 (0.96 0.80 2.44) | 117* - S - 342 (2.86 1.84 1.41) |
| 117* - S - 343 (3.39 2.33 0.92) | 118* - S - 151 (1.45 0.86 2.22) | 118* - S - 152 (2.10 1.16 1.92) | 118* - S - 343 (2.48 1.25 1.83) |
| 119* - S - 152 (2.13 0.95 1.89) | 119* - S - 343 (2.51 1.04 1.80) | 120 - S - 150 (2.29 2.49 1.74) | 120 - S - 151 (2.20 2.75 1.48) |
| 120 - S - 152 (2.84 3.05 1.18) | 120 - S - 153 (2.22 2.59 1.64) | 120 - S - 154 (1.75 1.72 2.51) | 120 - S - 184 (2.04 2.08 2.14) |
| 120 - S - 189 (1.36 1.08 3.15) | 120 - S - 192* (1.64 2.60 1.62) | 120 - S - 207 (1.21 2.91 1.32) | 120 - S - 244* (0.96 2.13 2.09) |
| 120 - S - 291 (1.95 2.63 1.60) | 120 - S - 292 (1.28 2.29 1.94) | 120 - S - 294 (1.24 2.13 2.10) | 120 - S - 295 (0.99 2.37 1.85) |
| 120 - S - 296 (1.27 2.37 1.86) | 120 - S - 300 (0.85 1.37 2.85) | 120 - S - 301 (0.90 1.08 3.14) | 120 - S - 302 (1.02 0.93 3.30) |
| 120 - S - 305 (1.16 2.04 2.19) | 120 - S - 342 (2.69 2.65 1.58) | 120 - S - 343 (3.22 3.14 1.09) | 123* - S - 152 (2.49 0.82 1.53) |
| 123* - S - 343 (2.87 0.91 1.44) | 124 - S - 150 (2.68 1.00 1.34) | 124 - S - 151 (2.59 1.25 1.08) | 124 - S - 153 (2.62 1.10 1.24) |
| 124 - S - 192* (2.04 1.11 1.23) | 124 - S - 207 (1.61 1.41 0.92) | 124 - S - 291 (2.34 1.13 1.20) | 124 - S - 295 (1.39 0.88 1.46) |
| 124 - S - 296 (1.67 0.87 1.46) | 124 - S - 342 (3.09 1.15 1.18) | 127* - S - 150 (1.92 1.58 2.11) | 127* - S - 151 (1.83 1.84 1.85) |
| 127* - S - 152 (2.47 2.14 1.55) | 127* - S - 153 (1.85 1.68 2.01) | 127* - S - 154 (1.38 0.81 2.88) | 127* - S - 184 (1.67 1.17 2.51) |

(fortgesetzt)

| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
|---|---|---|---|
| 127* - S - 192* (1.27 1.69 1.99) | 127* - S - 207 (0.84 2.00 1.69) | 127* - S - 291 (1.58 1.72 1.97) | 127* - S - 292 (0.91 1.38 2.31) |
| 127* - S - 294 (0.87 1.22 2.47) | 127* - S - 296 (0.90 1.46 2.23) | 127* - S - 342 (2.32 1.74 1.95) | 127* - S - 343 (2.85 2.23 1.46) |
| 128 - S - 150 (2.77 2.40 1.26) | 128 - S - 151 (2.67 2.66 1.00) | 128 - S - 153 (2.70 2.50 1.16) | 128 - S - 154 (2.23 1.63 2.03) |
| 128 - S - 184 (2.52 1.99 1.67) | 128 - S - 189 (1.84 0.99 2.67) | 128 - S - 192* (2.12 2.51 1.15) | 128 - S - 193* (1.04 1.67 1.98) |
| 128 - S - 244* (1.44 2.04 1.62) | 128 - S - 287* (1.01 1.73 1.93) | 128 - S - 291 (2.43 2.54 1.12) | 128 - S - 292 (1.76 2.19 1.46) |
| 128 - S - 294 (1.72 2.04 1.62) | 128 - S - 295 (1.47 2.28 1.38) | 128 - S - 296 (1.75 2.28 1.38) | 128 - S - 300 (1.33 1.28 2.38) |
| 128 - S - 301 (1.38 0.99 2.66) | 128 - S - 302 (1.50 0.84 2.82) | 128 - S - 305 (1.64 1.94 1.71) | 128 - S - 331 (1.27 1.52 2.13) |
| 128 - S - 342 (3.17 2.55 1.10) | 128 - S - 468* (0.98 2.15 1.50) | 129* - S - 150 (2.15 2.10 1.88) | 129* - S - 151 (2.06 2.36 1.62) |
| 129*- S - 152 (2.70 2.66 1.32) | 129*- S - 153 (2.08 2.20 1.78) | 129*- S - 154 (1.61 1.33 2.65) | 129*- S - 184 (1.90 1.69 2.29) |
| 129*- S - 192* (1.50 2.21 1.77) | 129*- S - 207 (1.07 2.52 1.46) | 129*- S - 244* (0.82 1.74 2.24) | 129*- S - 291 (1.81 2.24 1.74) |
| 129*- S - 292 (1.14 1.90 2.08) | 129*- S - 294 (1.10 1.74 2.24) | 129*- S - 295 (0.85 1.98 2.00) | 129*- S - 296 (1.13 1.98 2.00) |
| 129*- S - 305 (1.02 1.65 2.33) | 129*- S - 342 (2.55 2.26 1.72) | 129*- S - 343 (3.08 2.75 1.23) | 130*- S - 150 (2.84 1.80 1.19) |
| 130*- S - 151 (2.75 2.06 0.93) | 130*- S - 153 (2.77 1.90 1.09) | 130*- S - 154 (2.30 1.03 1.96) | 130*- S - 184 (2.59 1.39 1.60) |
| 130*- S - 192* (2.19 1.91 1.07) | 130*- S - 193* (1.11 1.08 1.91) | 130*- S - 244* (1.51 1.44 | 1.54) 130*- S - 287* (1.08 1.13 1.86) |
| 130*- S - 291 (2.50 1.94 1.05) | 130*- S - 292 (1.83 1.60 1.39) | 130*- S - 294 (1.79 1.44 1.55) | 130*- S - 295 (1.54 1.68 1.30) |
| 130*- S - 296 (1.82 1.68 1.31) | 130*- S - 305 (1.71 1.35 1.64) | 130*- S - 331 (1.34 0.93 2.06) | 130*- S - 342 (3.24 1.96 1.03) |
| 130*- S - 468* (1.05 1.56 1.43) | 131*- S - 150 (1.52 0.93 2.50) | 131*- S - 151 (1.43 1.18 2.25) | 131*- S - 152 (2.07 1.48 1.95) |
| 131*- S - 153 (1.45 1.03 2.40) | 131*- S - 192* (0.88 1.04 2.39) | 131*- S - 291 (1.18 1.06 2.37) | 131*- S - 342 (1.92 1.08 2.35) |
| 131*- S - 343 (2.45 1.57 1.86) | 132* - S - 151 (1.52 0.94 2.16) | 132*- S - 152 (2.16 1.24 1.86) | 132*- S - 291 (1.27 0.82 2.28) |
| 132*- S - 342 (2.01 0.84 2.26) | 132*- S - 343 (2.54 1.33 1.77) | 134* - S - 150 (2.31 3.44 1.72) | 134*- S - 151 (2.22 3.69 1.46) |
| 134*- S - 152 (2.86 3.99 1.16) | 134*- S - 153 (2.24 3.54 1.62) | 134*- S - 154 (1.77 2.66 2.49) | 134*- S - 184 (2.06 3.03 2.12) |
| 134*- S - 189 (1.38 2.03 3.12) | 134*- S - 192* (1.66 3.55 1.60) | 134*- S - 207 (1.23 3.85 1.30) | 134*- S - 244* (0.98 3.08 2.07) |
| 134*- S - 291 (1.97 3.57 1.58) | 134*- S - 292 (1.30 3.23 1.92) | 134*- S - 294 (1.26 3.07 2.08) | 134*- S - 295 (1.01 3.32 1.83) |
| 134*- S - 296 (1.29 3.31 1.84) | 134*- S - 300 (0.87 2.32 2.83) | 134*- S - 301 (0.92 2.03 3.12) | 134*- S - 302 (1.04 1.88 3.27) |
| 134*- S - 305 (1.18 2.98 2.17) | 134*- S - 331 (0.82 2.56 2.59) | 134*- S - 342 (2.71 3.59 1.56) | 134*- S - 343 (3.24 4.08 1.07) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 135* - S - 150 (1.71 2.59 2.31) | 135*- S - 151 (1.62 2.85 2.06) | 135*- S - 152 (2.26 3.15 1.76) | 135*- S - 153 (1.64 2.69 2.21) |
| 135*- S - 154 (1.18 1.82 3.09) | 135*- S - 184 (1.46 2.18 2.72) | 135*- S - 192* (1.07 2.70 2.20) | 135*- S - 291 (1.37 2.73 2.18) |
| 135*- S - 342 (2.11 2.75 2.16) | 135*- S - 343 (2.64 3.24 1.67) | 136* - S - 150 (1.50 1.99 2.52) | 136* - S - 151 (1.41 2.25 2.26) |
| 136* - S - 152 (2.06 2.54 1.96) | 136* - S - 153 (1.44 2.09 2.42) | 136* - S - 154 (0.97 1.22 3.29) | 136* - S - 184 (1.25 1.58 2.93) |
| 136* - S - 192* (0.86 2.10 2.41) | 136* - S - 291 (1.16 2.12 2.38) | 136* - S - 342 (1.91 2.14 2.36) | 136* - S - 343 (2.44 2.64 1.87) |
| 137* - S - 150 (2.97 1.56 1.06) | 137* - S - 153 (2.90 1.66 0.96) | 137* - S - 184 (2.72 1.16 1.46) | 137* - S - 192* (2.32 1.68 0.94) |
| 137* - S - 193* (1.24 0.84 1.78) | 137* - S - 244* (1.64 1.21 1.41) | 137* - S - 287* (1.22 0.89 | 1.73) 137* - S - 291 (2.63 1.70 0.92) |
| 137* - S - 292 (1.96 1.36 1.26) | 137* - S - 294 (1.92 1.20 1.42) | 137* - S - 295 (1.67 1.45 1.17) | 137* - S - 296 (1.95 1.44 1.18) |
| 137* - S - 305 (1.84 1.11 1.51) | 137* - S - 468* (1.18 1.32 1.30) | 138* - S - 150 (2.29 0.95 1.74) | 138* - S - 151 (2.20 1.21 1.48) |
| 138* - S - 152 (2.84 1.51 1.18) | 138* - S - 153 (2.22 1.05 1.64) | 138* - S - 192* (1.64 1.06 1.62) | 138* - S - 207 (1.21 1.37 1.32) |
| 138* - S - 291 (1.95 1.09 1.60) | 138* - S - 295 (0.99 0.83 1.85) | 138* - S - 296 (1.27 0.83 1.86) | 138* - S - 342 (2.69 1.11 1.58) |
| 138* - S - 343 (3.22 1.60 1.09) | 140* - S - 150 (2.19 2.23 1.84) | 140* - S - 151 (2.10 2.49 1.58) | 140* - S - 152 (2.74 2.79 1.28) |
| 140* - S - 153 (2.12 2.33 1.74) | 140* - S - 154 (1.65 1.46 2.61) | 140* - S - 184 (1.94 1.83 2.25) | 140* - S - 189 (1.26 0.82 3.25) |
| 140* - S - 192* (1.54 2.35 1.73) | 140* - S - 207 (1.11 2.65 1.42) | 140* - S - 244* (0.86 1.88 2.20) | 140* - S - 291 (1.85 2.37 1.70) |
| 140* - S - 292 (1.18 2.03 2.04) | 140* - S - 294 (1.14 1.87 2.20) | 140* - S - 295 (0.89 2.12 1.96) | 140* - S - 296 (1.17 2.11 1.96) |
| 140* - S - 305 (1.06 1.78 2.29) | 140* - S - 342 (2.59 2.39 1.68) | 140* - S - 343 (3.12 2.88 1.19) | 141* - S - 150 (1.04 2.15 2.99) |
| 141* - S - 151 (0.94 2.41 2.73) | 141* - S - 152 (1.59 2.71 2.43) | 141* - S - 153 (0.97 2.25 2.89) | 141* - S - 342 (1.44 2.31 2.83) |
| 141* - S - 343 (1.97 2.80 2.34) | 147* - S - 152 (2.63 0.94 1.39) | 147* - S - 207 (1.01 0.80 1.53) | 147* - S - 343 (3.01 1.04 1.30) |
| 148* - S - 150 (1.83 1.09 2.20) | 148* - S - 151 (1.74 1.35 1.94) | 148* - S - 152 (2.38 1.65 1.64) | 148* - S - 153 (1.76 1.19 2.10) |
| 148* - S - 192* (1.18 1.21 2.09) | 148* - S - 291 (1.49 1.23 2.06) | 148* - S - 292 (0.82 0.89 2.40) | 148* - S - 296 (0.81 0.97 2.32) |
| 148* - S - 342 (2.23 1.25 2.04) | 148* - S - 343 (2.76 1.74 1.55) | 149* - S - 150 (2.46 1.76 1.57) | 149*- S - 151 (2.36 2.01 1.31) |
| 149* - S - 152 (3.01 2.31 1.01) | 149* - S - 153 (2.39 1.86 1.47) | 149* - S - 154 (1.92 0.98 2.34) | 149* - S - 184 (2.20 1.35 1.98) |
| 149* - S - 192* (1.81 1.87 1.46) | 149* - S - 206 (0.86 2.23 1.10) | 149* - S - 207 (1.38 2.17 1.15) | 149* - S - 244* (1.13 1.40 1.93) |
| 149* - S - 291 (2.12 1.89 1.43) | 149* - S - 292 (1.45 1.55 1.77) | 149* - S - 294 (1.41 1.39 1.93) | 149* - S - 295 (1.16 1.64 1.69) |
| 149* - S - 296 (1.44 1.63 1.69) | 149* - S - 305 (1.33 1.30 2.02) | 149* - S - 331 (0.96 0.88 2.44) | 149* - S - 342 (2.86 1.91 1.41) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 149* - S - 343 (3.39 2.40 0.92) | 154 - S - 152 (1.09 1.33 2.93) | 154 - S - 342 (0.94 0.93 3.33) | 154 - S - 343 (1.47 1.42 2.84) |
| 155* - S - 150 (1.55 2.24 2.47) | 155* - S - 151 (1.46 2.50 2.22) | 155* - S - 152 (2.10 2.80 1.92) | 155* - S - 153 (1.48 2.35 2.37) |
| 155* - S - 154 (1.02 1.47 3.25) | 155* - S - 184 (1.30 1.84 2.88) | 155* - S - 192* (0.91 2.36 2.36) | 155* - S - 291 (1.21 2.38 2.34) |
| 155* - S - 342 (1.95 2.40 2.32) | 155* - S - 343 (2.48 2.89 1.83) | 156* - S - 150 (2.28 2.89 1.75) | 156* - S - 151 (2.19 3.15 1.49) |
| 156* - S - 152 (2.83 3.45 1.19) | 156* - S - 153 (2.21 2.99 1.64) | 156* - S - 154 (1.75 2.12 2.52) | 156* - S - 184 (2.03 2.48 2.15) |
| 156* - S - 189 (1.35 1.48 3.15) | 156* - S - 192* (1.64 3.00 1.63) | 156* - S - 207 (1.21 3.31 1.33) | 156* - S - 244* (0.95 2.53 2.10) |
| 156* - S - 291 (1.94 3.03 1.61) | 156* - S - 292 (1.27 2.69 1.95) | 156* - S - 294 (1.23 2.53 2.11) | 156* - S - 295 (0.99 2.77 1.86) |
| 156* - S - 296 (1.26 2.77 1.87) | 156* - S - 300 (0.84 1.77 2.86) | 156* - S - 301 (0.89 1.48 3.15) | 156* - S - 302 (1.01 1.33 3.30) |
| 156* - S - 305 (1.15 2.44 2.20) | 156* - S - 342 (2.68 3.05 1.59) | 156* - S - 343 (3.21 3.54 1.10) | 157* - S - 150 (1.52 2.48 2.50) |
| 157* - S - 151 (1.43 2.74 2.24) | 157* - S - 152 (2.08 3.04 1.94) | 157* - S - 153 (1.46 2.58 2.40) | 157* - S - 154 (0.99 1.71 3.27) |
| 157* - S - 184 (1.27 2.08 2.91) | 157* - S - 192* (0.88 2.60 2.39) | 157* - S - 291 (1.18 2.62 2.36) | 157* - S - 342 (1.93 2.64 2.34) |
| 157* - S - 343 (2.46 3.13 1.85) | 158* - S - 150 (1.46 1.56 2.56) | 158* - S - 151 (1.37 1.82 2.30) | 158* - S - 152 (2.01 2.12 2.01) |
| 158* - S - 153 (1.39 1.66 2.46) | 158* - S - 184 (1.21 1.15 2.97) | 158* - S - 192* (0.82 1.67 2.45) | 158* - S - 291 (1.12 1.70 2.42) |
| 158* - S - 342 (1.87 1.72 2.41) | 158* - S - 343 (2.39 2.21 1.91) | 159* - S - 150 (1.73 2.98 2.29) | 159* - S - 151 (1.64 3.23 2.03) |
| 159* - S - 152 (2.28 3.53 1.74) | 159* - S - 153 (1.66 3.08 2.19) | 159* - S - 154 (1.20 2.20 3.06) | 159* - S - 184 (1.48 2.57 2.70) |
| 159* - S - 189 (0.80 1.57 3.70) | 159* - S - 192* (1.09 3.09 2.18) | 159* - S - 291 (1.39 3.11 2.15) | 159* - S - 342 (2.13 3.13 2.14) |
| 159* - S - 343 (2.66 3.62 1.64) | 160* - S - 150 (1.04 2.06 2.99) | 160* - S - 151 (0.95 2.32 2.73) | 160* - S - 152 (1.59 2.62 2.43) |
| 160* - S - 153 (0.97 2.16 2.89) | 160* - S - 342 (1.44 2.22 2.83) | 160* - S - 343 (1.97 2.71 2.34) | 161* - S - 150 (1.02 1.47 3.00) |
| 161* - S - 151 (0.93 1.73 2.74) | 161* - S - 152 (1.58 2.03 2.44) | 161* - S - 153 (0.96 1.57 2.90) | 161* - S - 342 (1.43 1.63 2.84) |
| 161* - S - 343 (1.96 2.12 2.35) | 166* - S - 150 (0.81 0.94 3.21) | 166* - S - 152 (1.37 1.50 2.65) | 166* - S - 342 (1.22 1.10 3.05) |
| 166* - S - 343 (1.75 1.59 2.56) | 167* - S - 150 (0.95 1.26 3.07) | 167* - S - 151 (0.86 1.52 2.82) | 167* - S - 152 (1.50 1.82 2.52) |
| 167* - S - 153 (0.88 1.36 2.97) | 167* - S - 342 (1.35 1.42 2.92) | 167* - S - 343 (1.88 1.91 2.42) | 168* - S - 150 (1.39 1.97 2.64) |
| 168* - S - 151 (1.30 2.23 2.38) | 168* - S - 152 (1.94 2.53 2.08) | 168* - S - 153 (1.32 2.07 2.54) | 168* - S - 154 (0.85 1.20 3.41) |
| 168* - S - 184 (1.14 1.57 3.04) | 168* - S - 291 (1.05 2.11 2.50) | 168* - S - 342 (1.79 2.13 2.48) | 168* - S - 343 (2.32 2.62 1.99) |
| 170* - S - 152 (1.30 2.09 2.72) | 170* - S - 342 (1.15 1.69 3.12) | 170* - S - 343 (1.68 2.18 2.63) | 171* - S - 150 (1.04 0.95 2.98) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 171* - S - 151 (0.95 1.21 2.72) | 171* - S - 152 (1.60 1.51 2.42) | 171* - S - 153 (0.98 1.05 2.88) | 171* - S - 342 (1.45 1.10 2.82) |
| 171* - S - 343 (1.98 1.60 2.33) | 175* - S - 150 (1.81 1.31 2.21) | 175* - S - 151 (1.72 1.57 1.95) | 175* - S - 152 (2.36 1.87 1.66) |
| 175* - S - 153 (1.74 1.41 2.11) | 175* - S - 184 (1.56 0.90 2.62) | 175* - S - 192* (1.17 1.42 2.10) | 175* - S - 291 (1.47 1.45 2.07) |
| 175* - S - 292 (0.81 1.11 2.42) | 175* - S - 342 (2.22 1.47 2.06) | 175* - S - 343 (2.74 1.96 1.56) | 176* - S - 150 (1.87 1.55 2.16) |
| 176* - S - 151 (1.78 1.81 1.90) | 176* - S - 152 (2.42 2.11 1.60) | 176* - S - 153 (1.80 1.66 2.06) | 176* - S - 184 (1.62 1.15 2.57) |
| 176* - S - 192* (1.22 1.67 2.04) | 176* - S - 291 (1.53 1.69 2.02) | 176* - S - 292 (0.86 1.35 2.36) | 176* - S - 294 (0.82 1.19 2.52) |
| 176* - S - 296 (0.85 1.43 2.28) | 176* - S - 342 (2.27 1.71 2.00) | 176* - S - 343 (2.80 2.20 1.51) | 177* - S - 343 (1.92 0.87 2.39) |
| 182* - S - 150 (1.85 1.38 2.18) | 182* - S - 151 (1.75 1.64 1.92) | 182* - S - 152 (2.40 1.94 1.62) | 182* - S - 153 (1.78 1.48 2.08) |
| 182* - S - 184 (1.60 0.97 2.59) | 182* - S - 192* (1.20 1.49 2.07) | 182* - S - 291 (1.51 1.52 2.04) | 182* - S - 292 (0.84 1.18 2.38) |
| 182* - S - 296 (0.83 1.26 2.30) | 182* - S - 342 (2.25 1.54 2.02) | 182* - S - 343 (2.78 2.03 1.53) | 184 - S - 152 (0.80 0.97 3.22) |
| 184 - S - 343 (1.18 1.06 3.13) | 187* - S - 150 (1.11 1.04 2.92) | 187* - S - 151 (1.01 1.29 2.66) | 187* - S - 152 (1.66 1.59 2.36) |
| 187* - S - 153 (1.04 1.14 2.82) | 187* - S - 342 (1.51 1.19 2.76) | 187* - S - 343 (2.04 1.68 2.27) | 188* - S - 152 (1.23 1.55 2.79) |
| 188* - S - 342 (1.08 1.15 3.19) | 188* - S - 343 (1.61 1.64 2.70) | 189* - S - 150 (0.93 1.41 3.10) | 189* - S - 151 (0.84 1.67 2.84) |
| 189* - S - 152 (1.48 1.97 2.54) | 189* - S - 153 (0.86 1.51 3.00) | 189* - S - 342 (1.33 1.57 2.94) | 189* - S - 343 (1.86 2.06 2.45) |
| 193* - S - 151 (1.64 0.98 2.04) | 193* - S - 152 (2.28 1.28 1.74) | 193* - S - 153 (1.66 0.83 2.20) | 193* - S - 192* (1.08 0.84 2.19) |
| 193* - S - 291 (1.39 0.86 2.16) | 193* - S - 342 (2.13 0.88 2.14) | 193* - S - 343 (2.66 1.37 1.65) | 194* - S - 150 (0.87 1.28 3.15) |
| 194* - S - 152 (1.43 1.84 2.59) | 194* - S - 153 (0.81 1.38 3.05) | 194* - S - 342 (1.28 1.44 2.99) | 194* - S - 343 (1.81 1.93 2.50) |
| 195* - S - 152 (1.37 1.12 2.65) | 195* - S - 343 (1.75 1.21 2.56) | 196* - S - 150 (1.78 2.18 2.25) | 196* - S - 151 (1.68 2.44 1.99) |
| 196* - S - 152 (2.33 2.74 1.69) | 196* - S - 153 (1.71 2.28 2.15) | 196* - S - 154 (1.24 1.41 3.02) | 196* - S - 184 (1.53 1.77 2.66) |
| 196* - S - 192* (1.13 2.29 2.14) | 196* - S - 291 (1.44 2.32 2.11) | 196* - S - 342 (2.18 2.34 2.09) | 196* - S - 343 (2.71 2.83 1.60) |
| 197* - S - 152 (1.23 2.06 2.79) | 197* - S - 342 (1.08 1.66 3.19) | 197* - S - 343 (1.61 2.15 2.70) | 198* - S - 152 (1.66 1.06 2.36) |
| 198* - S - 343 (2.04 1.15 2.27) | 199 - S - 150 (2.88 1.62 1.15) | 199 - S - 153 (2.81 1.72 1.04) | 199 - S - 154 (2.35 0.85 1.92) |
| 199 - S - 184 (2.63 1.21 1.55) | 199 - S - 192* (2.24 1.73 1.03) | 199 - S - 193* (1.15 0.90 1.87) | 199 - S - 244* (1.55 1.26 1.50) |
| 199 - S - 287* (1.13 0.95 1.82) | 199 - S - 291 (2.54 1.76 1.01) | 199 - S - 292 (1.87 1.42 1.35) | 199 - S - 294 (1.83 1.26 1.51) |
| 199 - S - 295 (1.59 1.50 1.26) | 199 - S - 296 (1.86 1.50 1.27) | 199 - S - 305 (1.75 1.17 1.60) | 199 - S - 342 (3.28 1.78 0.99) |

(fortgesetzt)

| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
|---|---|---|---|
| 199 - S - 468* (1.09 1.38 1.39) | 200 - S - 150 (2.42 1.66 1.61) | 200 - S - 151 (2.33 1.92 1.35) | 200 - S - 152 (2.97 2.22 1.05) |
| 200 - S - 153 (2.35 1.76 1.51) | 200 - S - 154 (1.88 0.89 2.38) | 200 - S - 184 (2.17 1.25 2.02) | 200 - S - 192* (1.77 1.77 1.50) |
| 200 - S - 206 (0.82 2.13 1.14) | 200 - S - 207 (1.34 2.08 1.19) | 200 - S - 244* (1.09 1.30 1.96) | 200 - S - 291 (2.08 1.80 1.47) |
| 200 - S - 292 (1.41 1.46 1.81) | 200 - S - 294 (1.37 1.30 1.97) | 200 - S - 295 (1.12 1.54 1.73) | 200 - S - 296 (1.40 1.54 1.73) |
| 200 - S - 305 (1.29 1.21 2.06) | 200 - S - 342 (2.82 1.82 1.45) | 200 - S - 343 (3.35 2.31 0.96) | 205* - S - 152 (2.14 1.00 1.88) |
| 205* - S - 343 (2.52 1.09 1.79) | 208* - S - 150 (1.63 0.97 2.40) | 208* - S - 151 (1.53 1.23 2.14) | 208* - S - 152 (2.18 1.53 1.84) |
| 208* - S - 153 (1.56 1.07 2.30) | 208* - S - 192* (0.98 1.08 2.29) | 208* - S - 291 (1.29 1.11 2.26) | 208* - S - 342 (2.03 1.13 2.24) |
| 208* - S - 343 (2.56 1.62 1.75) | 209* - S - 150 (1.88 0.89 2.15) | 209* - S - 151 (1.79 1.15 1.89) | 209* - S - 152 (2.43 1.45 1.59) |
| 209* - S - 153 (1.81 0.99 2.05) | 209* - S - 192* (1.23 1.01 2.03) | 209* - S - 207 (0.80 1.31 1.73) | 209* - S - 291 (1.54 1.03 2.01) |
| 209* - S - 342 (2.28 1.05 1.99) | 209* - S - 343 (2.81 1.54 1.50) | 210* - S - 150 (1.98 1.49 2.04) | 210* - S - 151 (1.89 1.75 1.78) |
| 210* - S - 152 (2.53 2.05 1.48) | 210* - S - 153 (1.92 1.59 1.94) | 210* - S - 184 (1.73 1.08 2.45) | 210* - S - 192* (1.34 1.60 1.93) |
| 210* - S - 207 (0.91 1.91 1.62) | 210* - S - 291 (1.64 1.63 1.90) | 210* - S - 292 (0.98 1.29 2.25) | 210* - S - 294 (0.93 1.13 2.40) |
| 210* - S - 296 (0.97 1.37 2.16) | 210* - S - 305 (0.86 1.04 2.50) | 210* - S - 342 (2.39 1.65 1.89) | 210* - S - 343 (2.91 2.14 1.39) |
| 211* - S - 151 (2.44 0.98 1.24) | 211* - S - 152 (3.08 1.28 0.94) | 211* - S - 153 (2.46 0.83 1.39) | 211* - S - 192* (1.89 0.84 1.38) |
| 211* - S - 206 (0.94 1.19 1.03) | 211* - S - 207 (1.46 1.14 1.08) | 211* - S - 291 (2.19 0.86 1.36) | 211* - S - 342 (2.93 0.88 1.34) |
| 212* - S - 150 (2.72 1.21 1.31) | 212* - S - 151 (2.63 1.47 1.05) | 212* - S - 153 (2.65 1.32 1.21) | 212* - S - 184 (2.47 0.81 1.71) |
| 212* - S - 192* (2.07 1.33 1.19) | 212* - S - 244* (1.39 0.86 1.66) | 212* - S - 291 (2.38 1.35 1.17) | 212* - S - 292 (1.71 1.01 1.51) |
| 212* - S - 294 (1.67 0.85 1.67) | 212* - S - 295 (1.42 1.10 1.42) | 212* - S - 296 (1.70 1.09 1.43) | 212* - S - 342 (3.12 1.37 1.15) |
| 212* - S - 468* (0.93 0.97 1.55) | 213* - S - 151 (1.05 0.81 2.63) | 213* - S - 152 (1.69 1.11 2.33) | 213* - S - 343 (2.07 1.20 2.23) |
| 214* - S - 150 (1.58 1.31 2.44) | 214* - S - 151 (1.49 1.57 2.19) | 214* - S - 152 (2.13 1.87 1.89) | 214* - S - 153 (1.511.41 2.34) |
| 214* - S - 184 (1.33 0.91 2.85) | 214* - S - 192* (0.94 1.43 2.33) | 214* - S - 291 (1.24 1.45 2.31) | 214* - S - 342 (1.98 1.47 2.29) |
| 214* - S - 343 (2.51 1.96 1.79) | 217* - S - 152 (2.27 1.07 1.75) | 217* - S - 343 (2.65 1.16 1.66) | 218* - S - 152 (2.30 0.89 1.72) |
| 218* - S - 343 (2.68 0.99 1.63) | 219* - S - 151 (1.86 1.04 1.82) | 219* - S - 152 (2.50 1.34 1.52) | 219* - S - 153 (1.88 0.88 1.98) |
| 219* - S - 192* (1.30 0.89 1.96) | 219* - S - 207 (0.87 1.20 1.66) | 219* - S - 291 (1.61 0.92 1.94) | 219* - S - 342 (2.35 0.94 1.92) |
| 219* - S - 343 (2.88 1.43 1.43) | 222* - S - 150 (1.83 0.93 2.19) | 222* - S - 151 (1.74 1.19 1.93) | 222* - S - 152 (2.39 1.49 1.63) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 222* - S - 153 (1.77 1.03 2.09) | 222* - S - 192* (1.19 1.05 2.08) | 222* - S - 291 (1.50 1.07 2.05) | 222* - S - 296 (0.82 0.81 2.31) |
| 222* - S - 342 (2.24 1.09 2.03) | 222* - S - 343 (2.77 1.58 1.54) | 223 - S - 150 (2.59 1.99 1.44) | 223 - S - 151 (2.50 2.25 1.18) |
| 223 - S - 153 (2.52 2.09 1.34) | 223 - S - 154 (2.05 1.22 2.21) | 223 - S - 184 (2.34 1.58 1.85) | 223 - S - 192* (1.94 2.10 1.32) |
| 223 - S - 193* (0.86 1.26 2.16) | 223 - S - 206 (0.99 2.46 0.97) | 223 - S - 207 (1.51 2.41 1.02) | 223 - S - 244* (1.26 1.63 1.79) |
| 223 - S - 287* (0.83 1.32 2.11) | 223 - S - 291 (2.25 2.13 1.30) | 223 - S - 292 (1.58 1.79 1.64) | 223 - S - 294 (1.54 1.63 1.80) |
| 223 - S - 295 (1.29 1.87 1.55) | 223 - S - 296 (1.57 1.87 1.56) | 223 - S - 300 (1.15 0.87 2.55) | 223 - S - 305 (1.46 1.54 1.89) |
| 223 - S - 331 (1.09 1.11 2.31) | 223 - S - 342 (2.99 2.15 1.28) | 223 - S - 468* (0.80 1.74 1.68) | 224* - S - 150 (2.20 1.26 1.82) |
| 224* - S - 151 (2.11 1.52 1.57) | 224* - S - 152 (2.75 1.82 1.27) | 224* - S - 153 (2.13 1.36 1.72) | 224* - S - 184 (1.95 0.86 2.23) |
| 224* - S - 192* (1.56 1.38 1.71) | 224* - S - 207 (1.13 1.68 1.41) | 224* - S - 244* (0.87 0.91 2.18) | 224* - S - 291 (1.86 1.40 1.69) |
| 224* - S - 292 (1.19 1.06 2.03) | 224* - S - 294 (1.15 0.90 2.19) | 224* - S - 295 (0.91 1.15 1.94) | 224* - S - 296 (1.18 1.14 1.95) |
| 224* - S - 305 (1.07 0.81 2.28) | 224* - S - 342 (2.60 1.42 1.67) | 224* - S - 343 (3.13 1.91 1.18) | 225* - S - 150 (2.34 0.93 1.68) |
| 225* - S - 151 (2.25 1.19 1.42) | 225* - S - 152 (2.89 1.49 1.13) | 225* - S - 153 (2.27 1.03 1.58) | 225* - S - 192* (1.70 1.04 1.57) |
| 225* - S - 207 (1.27 1.35 1.27) | 225* - S - 291 (2.00 1.07 1.55) | 225* - S - 295 (1.05 0.81 1.80) | 225* - S - 296 (1.33 0.81 1.80) |
| 225* - S - 342 (2.74 1.09 1.53) | 225* - S - 343 (3.27 1.58 1.03) | 227* - S - 152 (3.10 0.96 0.92) | 227* - S - 206 (0.95 0.88 1.01) |
| 227* - S - 207 (1.47 0.82 1.06) | 228* - S - 151 (2.12 1.04 1.56) | 228* - S - 152 (2.76 1.34 1.26) | 228* - S - 153 (2.14 0.88 1.72) |
| 228* - S - 192* (1.56 0.89 1.70) | 228* - S - 207 (1.13 1.20 1.40) | 228* - S - 291 (1.87 0.92 1.68) | 228* - S - 342 (2.61 0.94 1.66) |
| 228* - S - 343 (3.14 1.43 1.17) | 230* - S - 150 (1.91 1.11 2.12) | 230* - S - 151 (1.81 1.36 1.86) | 230* - S - 152 (2.46 1.66 1.56) |
| 230* - S - 153 (1.84 1.21 2.02) | 230* - S - 192* (1.26 1.22 2.01) | 230* - S - 207 (0.83 1.52 1.70) | 230* - S - 291 (1.57 1.24 1.98) |
| 230* - S - 292 (0.90 0.90 2.32) | 230* - S - 296 (0.89 0.98 2.24) | 230* - S - 342 (2.31 1.26 1.96) | 230* - S - 343 (2.84 1.76 1.47) |
| 232* - S - 150 (2.90 1.16 1.12) | 232* - S - 153 (2.83 1.26 1.02) | 232* - S - 192* (2.26 1.27 1.01) | 232* - S - 291 (2.56 1.30 0.99) |
| 232* - S - 292 (1.89 0.95 1.33) | 232* - S - 295 (1.61 1.04 1.24) | 232* - S - 296 (1.88 1.04 1.25) | 232* - S - 342 (3.30 1.31 0.97) |
| 232* - S - 468* (1.12 0.91 1.37) | 233* - S - 152 (1.77 0.91 2.25) | 233* - S - 343 (2.15 1.00 2.16) | 234* - S - 152 (1.95 0.83 2.07) |
| 234* - S - 343 (2.33 0.92 1.98) | 235* - S - 150 (2.48 3.20 1.54) | 235* - S - 151 (2.39 3.46 1.28) | 235* - S - 152 (3.04 3.76 0.98) |
| 235* - S - 153 (2.42 3.30 1.44) | 235* - S - 154 (1.95 2.43 2.31) | 235* - S - 184 (2.23 2.79 1.95) | 235* - S - 189 (1.56 1.79 2.95) |
| 235* - S - 192* (1.84 3.32 1.43) | 235* - S - 206 (0.89 3.67 1.07) | 235* - S - 207 (1.41 3.62 1.12) | 235* - S - 244* (1.15 2.85 1.90) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 235* - S - 291 (2.14 3.34 1.40) | 235* - S - 292 (1.48 3.00 1.74) | 235* - S - 294 (1.44 2.84 1.90) | 235* - S - 295 (1.19 3.08 1.66) |
| 235* - S - 296 (1.47 3.08 1.66) | 235* - S - 300 (1.04 2.08 2.66) | 235* - S - 301 (1.09 1.80 2.95) | 235* - S - 302 (1.22 1.64 3.10) |
| 235* - S - 305 (1.36 2.75 2.00) | 235* - S - 331 (0.99 2.33 2.42) | 235* - S - 342 (2.89 3.36 1.38) | 236* - S - 150 (1.17 1.38 2.85) |
| 236* - S - 151 (1.08 1.64 2.59) | 236* - S - 152 (1.73 1.94 2.29) | 236* - S - 153 (1.111.48 2.75) | 236* - S - 184 (0.92 0.97 3.26) |
| 236* - S - 291 (0.83 1.52 2.71) | 236* - S - 342 (1.58 1.54 2.69) | 236* - S - 343 (2.11 2.03 2.20) | 239* - S - 150 (1.68 1.81 2.35) |
| 239* - S - 151 (1.59 2.07 2.09) | 239* - S - 152 (2.23 2.37 1.79) | 239* - S - 153 (1.61 1.92 2.24) | 239* - S - 154 (1.14 1.04 3.12) |
| 239* - S - 184 (1.43 1.41 2.75) | 239* - S - 192* (1.04 1.93 2.23) | 239* - S - 291 (1.34 1.95 2.21) | 239* - S - 342 (2.08 1.97 2.19) |
| 239* - S - 343 (2.61 2.46 1.70) | 240* - S - 150 (1.78 1.49 2.24) | 240* - S - 151 (1.69 1.74 1.98) | 240* - S - 152 (2.34 2.04 1.68) |
| 240* - S - 153 (1.72 1.59 2.14) | 240* - S - 184 (1.53 1.08 2.65) | 240* - S - 192* (1.14 1.60 2.13) | 240* - S - 291 (1.44 1.62 2.10) |
| 240* - S - 342 (2.19 1.64 2.08) | 240* - S - 343 (2.72 2.13 1.59) | 242* - S - 150 (2.40 2.01 1.62) | 242* - S - 151 (2.31 2.27 1.37) |
| 242* - S - 152 (2.95 2.57 1.07) | 242* - S - 153 (2.33 2.12 1.52) | 242* - S - 154 (1.87 1.24 2.39) | 242* - S - 184 (2.15 1.61 2.03) |
| 242* - S - 192* (1.76 2.13 1.51) | 242* - S - 206 (0.81 2.48 1.15) | 242* - S - 207 (1.33 2.43 1.21) | 242* - S - 244* (1.07 1.66 1.98) |
| 242* - S - 291 (2.06 2.15 1.49) | 242* - S - 292 (1.40 1.81 1.83) | 242* - S - 294 (1.35 1.65 1.98) | 242* - S - 295 (1.11 1.90 1.74) |
| 242* - S - 296 (1.39 1.89 1.74) | 242* - S - 300 (0.96 0.90 2.74) | 242* - S - 305 (1.28 1.56 2.08) | 242* - S - 331 (0.91 1.14 2.50) |
| 242* - S - 342 (2.80 2.17 1.47) | 242* - S - 343 (3.33 2.66 0.97) | 243* - S - 152 (1.20 1.70 2.82) | 243* - S - 342 (1.05 1.30 3.22) |
| 243* - S - 343 (1.58 1.79 2.73) | 244* - S - 152 (1.88 0.91 2.14) | 244* - S - 343 (2.26 1.01 2.05) | 245* - S - 151 (1.32 0.84 2.35) |
| 245* - S - 152 (1.96 1.14 2.06) | 245* - S - 343 (2.34 1.23 1.96) | 254* - S - 151 (1.48 0.92 2.19) | 254* - S - 152 (2.13 1.22 1.89) |
| 254* - S - 342 (1.98 0.82 2.29) | 254* - S - 343 (2.51 1.31 1.80) | 256* - S - 150 (2.83 1.97 1.19) | 256* - S - 151 (2.74 2.23 0.93) |
| 256* - S - 153 (2.76 2.07 1.09) | 256* - S - 154 (2.30 1.20 1.96) | 256* - S - 184 (2.58 1.56 1.60) | 256* - S - 192* (2.19 2.08 1.08) |
| 256* - S - 193* (1.11 1.24 1.92) | 256* - S - 244* (1.50 1.61 1.55) | 256* - S - 287* (1.08 1.30 1.86) | 256* - S - 291 (2.49 2.11 1.05) |
| 256* - S - 292 (1.83 1.77 1.40) | 256* - S - 294 (1.78 1.61 1.55) | 256* - S - 295 (1.54 1.85 1.31) | 256* - S - 296 (1.82 1.85 1.31) |
| 256* - S - 300 (1.39 0.85 2.31) | 256* - S - 305 (1.71 1.51 1.65) | 256* - S - 331 (1.34 1.09 2.07) | 256* - S - 342 (3.23 2.13 1.04) |
| 256* - S - 468* (1.05 1.72 1.44) | 261* - S - 150 (1.95 1.58 2.07) | 261* - S - 151 (1.86 1.83 1.82) | 261* - S - 152 (2.50 2.13 1.52) |
| 261* - S - 153 (1.88 1.68 1.97) | 261* - S - 154 (1.42 0.81 2.85) | 261* - S - 184 (1.70 1.17 2.48) | 261* - S - 192* (1.31 1.69 1.96) |
| 261* - S - 207 (0.88 1.99 1.66) | 261* - S - 291 (1.61 1.71 1.94) | 261* - S - 292 (0.94 1.37 2.28) | 261* - S - 294 (0.90 1.22 2.44) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 261* - S - 296 (0.93 1.45 2.20) | 261* - S - 305 (0.82 1.12 2.53) | 261* - S - 342 (2.35 1.73 1.92) | 261* - S - 343 (2.88 2.23 1.43) |
| 262* - S - 150 (1.85 1.90 2.18) | 262* - S - 151 (1.76 2.16 1.92) | 262* - S - 152 (2.40 2.45 1.62) | 262* - S - 153 (1.78 2.00 2.08) |
| 262* - S - 154 (1.31 1.13 2.95) | 262* - S - 184 (1.60 1.49 2.59) | 262* - S - 192* (1.20 2.01 2.06) | 262* - S - 291 (1.51 2.03 2.04) |
| 262* - S - 292 (0.84 1.69 2.38) | 262* - S - 296 (0.83 1.78 2.30) | 262* - S - 342 (2.25 2.05 2.02) | 262* - S - 343 (2.78 2.55 1.53) |
| 263* - S - 150 (2.02 2.49 2.00) | 263* - S - 151 (1.93 2.75 1.74) | 263* - S - 152 (2.58 3.05 1.44) | 263* - S - 153 (1.96 2.59 1.90) |
| 263* - S - 154 (1.49 1.72 2.77) | 263* - S - 184 (1.77 2.08 2.41) | 263* - S - 189 (1.10 1.08 3.41) | 263* - S - 192* (1.38 2.60 1.89) |
| 263* - S - 207 (0.95 2.91 1.58) | 263* - S - 291 (1.69 2.63 1.86) | 263* - S - 292 (1.02 2.29 2.20) | 263* - S - 294 (0.98 2.13 2.36) |
| 263* - S - 296 (1.01 2.37 2.12) | 263* - S - 305 (0.90 2.04 2.45) | 263* - S - 342 (2.43 2.65 1.84) | 263* - S - 343 (2.96 3.14 1.35) |
| 264* - S - 150 (1.29 1.94 2.73) | 264* - S - 151 (1.20 2.19 2.47) | 264* - S - 152 (1.85 2.49 2.17) | 264* - S - 153 (1.23 2.04 2.63) |
| 264* - S - 184 (1.04 1.53 3.14) | 264* - S - 291 (0.95 2.07 2.59) | 264* - S - 342 (1.70 2.09 2.57) | 264* - S - 343 (2.23 2.58 2.08) |
| 266* - S - 152 (2.03 0.86 1.99) | 266* - S - 343 (2.41 0.95 1.90) | 267* - S - 151 (1.59 0.86 2.09) | 267* - S - 152 (2.23 1.16 1.79) |
| 267* - S - 343 (2.61 1.25 1.70) | 272* - S - 150 (1.29 1.25 2.73) | 272* - S - 151 (1.20 1.51 2.47) | 272* - S - 152 (1.85 1.81 2.17) |
| 272* - S - 153 (1.23 1.35 2.63) | 272* - S - 184 (1.04 0.85 3.14) | 272* - S - 291 (0.95 1.39 2.59) | 272* - S - 342 (1.70 1.41 2.57) |
| 272* - S - 343 (2.23 1.90 2.08) | 274* - S - 151 (1.72 1.06 1.96) | 274* - S - 152 (2.36 1.36 1.66) | 274* - S - 153 (1.74 0.90 2.11) |
| 274* - S - 192* (1.17 0.91 2.10) | 274* - S - 291 (1.47 0.94 2.08) | 274* - S - 342 (2.21 0.96 2.06) | 274* - S - 343 (2.74 1.45 1.57) |
| 275* - S - 152 (2.56 0.94 1.46) | 275* - S - 343 (2.94 1.03 1.37) | 276* - S - 150 (1.89 1.39 2.14) | 276* - S - 151 (1.80 1.65 1.88) |
| 276* - S - 152 (2.44 1.95 1.58) | 276* - S - 153 (1.82 1.49 2.03) | 276* - S - 184 (1.64 0.98 2.54) | 276* - S - 192* (1.25 1.50 2.02) |
| 276* - S - 207 (0.81 1.81 1.72) | 276* - S - 291 (1.55 1.53 2.00) | 276* - S - 292 (0.88 1.19 2.34) | 276* - S - 294 (0.84 1.03 2.50) |
| 276* - S - 296 (0.87 1.27 2.26) | 276* - S - 342 (2.29 1.55 1.98) | 276* - S - 343 (2.82 2.04 1.49) | 277* - S - 152 (2.14 1.09 1.88) |
| 277* - S - 343 (2.52 1.18 1.79) | 278* - S - 150 (2.89 1.36 1.14) | 278* - S - 153 (2.82 1.46 1.04) | 278* - S - 184 (2.64 0.95 1.55) |
| 278* - S - 192* (2.24 1.47 1.03) | 278* - S - 244* (1.56 1.00 1.50) | 278* - S - 291 (2.55 1.50 1.00) | 278* - S - 292 (1.88 1.16 1.34) |
| 278* - S - 294 (1.84 1.00 1.50) | 278* - S - 295 (1.59 1.24 1.26) | 278* - S - 296 (1.87 1.24 1.26) | 278* - S - 305 (1.76 0.91 1.59) |
| 278* - S - 342 (3.29 1.52 0.98) | 278* - S - 468* (1.10 1.12 1.38) | 279* - S - 152 (2.09 1.03 1.93) | 279* - S - 343 (2.47 1.12 1.84) |
| 280* - S - 151 (1.77 1.05 1.91) | 280* - S - 152 (2.41 1.35 1.61) | 280* - S - 153 (1.79 0.89 2.07) | 280* - S - 192* (1.21 0.90 2.05) |
| 280* - S - 291 (1.52 0.93 2.03) | 280* - S - 342 (2.26 0.95 2.01) | 280* - S - 343 (2.79 1.44 1.52) | 281* - S - 154 (2.67 0.82 1.59) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 281* - S - 184 (2.96 1.18 1.23) | 281* - S - 193* (1.48 0.86 1.54) | 281* - S - 244* (1.88 1.23 1.17) | 281* - S - 287* (1.45 0.92 1.49) |
| 281* - S - 292 (2.20 1.39 1.02) | 281* - S - 294 (2.16 1.23 1.18) | 281* - S - 295 (1.91 1.47 0.93) | 281* - S - 296 (2.19 1.47 0.94) |
| 281* - S - 305 (2.08 1.13 1.27) | 281* - S - 468* (1.42 1.34 1.06) | 282* - S - 150 (1.74 0.96 2.29) | 282* - S - 151 (1.65 1.22 2.03) |
| 282* - S - 152 (2.29 1.52 1.73) | 282* - S - 153 (1.67 1.06 2.18) | 282* - S - 192* (1.10 1.08 2.17) | 282* - S - 291 (1.40 1.10 2.15) |
| 282* - S - 342 (2.14 1.12 2.13) | 282* - S - 343 (2.67 1.61 1.64) | 283* - S - 150 (3.09 1.36 0.94) | 283* - S - 184 (2.84 0.96 1.34) |
| 283* - S - 244* (1.76 1.01 1.29) | 283* - S - 292 (2.08 1.16 1.14) | 283* - S - 294 (2.04 1.00 1.30) | 283* - S - 295 (1.79 1.25 1.05) |
| 283* - S - 296 (2.07 1.24 1.06) | 283* - S - 305 (1.96 0.91 1.39) | 283* - S - 468* (1.30 1.12 1.18) | 284* - S - 150 (1.96 0.96 2.07) |
| 284* - S - 151 (1.87 1.21 1.81) | 284* - S - 152 (2.51 1.51 1.51) | 284* - S - 153 (1.89 1.06 1.96) | 284* - S - 192* (1.32 1.07 1.95) |
| 284* - S - 207 (0.89 1.37 1.65) | 284* - S - 291 (1.62 1.09 1.93) | 284* - S - 296 (0.94 0.83 2.19) | 284* - S - 342 (2.36 1.11 1.91) |
| 284* - S - 343 (2.89 1.61 1.42) | 285* - S - 151 (1.72 0.85 1.95) | 285* - S - 152 (2.37 1.14 1.65) | 285* - S - 343 (2.75 1.24 1.56) |
| 286* - S - 151 (1.47 0.94 2.21) | 286* - S - 152 (2.11 1.24 1.91) | 286* - S - 291 (1.22 0.82 2.33) | 286* - S - 342 (1.96 0.84 2.31) |
| 286* - S - 343 (2.49 1.33 1.82) | 287*- S - 151 (1.66 0.93 2.01) | 287*- S - 152 (2.31 1.23 1.71) | 287*- S - 291 (1.41 0.81 2.13) |
| 287*- S - 342 (2.16 0.83 2.11) | 287*- S - 343 (2.68 1.32 1.62) | 288* - S - 151 (1.68 0.99 2.00) | 288* - S - 152 (2.32 1.29 1.70) |
| 288* - S - 153 (1.70 0.83 2.16) | 288* - S - 192* (1.12 0.85 2.15) | 288* - S - 291 (1.43 0.87 2.12) | 288* - S - 342 (2.17 0.89 2.10) |
| 288* - S - 343 (2.70 1.38 1.61) | 289* - S - 150 (1.51 0.89 2.52) | 289* - S - 151 (1.42 1.15 2.26) | 289* - S - 152 (2.06 1.45 1.96) |
| 289* - S - 153 (1.44 0.99 2.42) | 289* - S - 192* (0.86 1.01 2.40) | 289* - S - 291 (1.17 1.03 2.38) | 289* - S - 342 (1.91 1.05 2.36) |
| 289* - S - 343 (2.44 1.54 1.87) | 290* - S - 152 (2.03 0.83 1.99) | 290* - S - 343 (2.41 0.92 1.90) | 292* - S - 343 (1.94 0.85 2.37) |
| 293* - S - 152 (2.05 0.95 1.97) | 293* - S - 343 (2.43 1.04 1.87) | 294* - S - 152 (1.60 0.92 2.42) | 294* - S - 343 (1.98 1.01 2.33) |
| 297* - S - 150 (1.70 0.99 2.32) | 297* - S - 151 (1.61 1.25 2.06) | 297* - S - 152 (2.26 1.55 1.76) | 297* - S - 153 (1.64 1.09 2.22) |
| 297* - S - 192* (1.06 1.10 2.21) | 297* - S - 291 (1.36 1.13 2.18) | 297* - S - 342 (2.11 1.15 2.16) | 297* - S - 343 (2.64 1.64 1.67) |
| 298* - S - 152 (2.00 1.07 2.02) | 298* - S - 343 (2.38 1.17 1.93) | 299* - S - 343 (3.12 0.83 1.19) | 300* - S - 150 (1.44 1.12 2.58) |
| 300* - S - 151 (1.35 1.38 2.33) | 300* - S - 152 (1.99 1.67 2.03) | 300* - S - 153 (1.37 1.22 2.48) | 300* - S - 291 (1.10 1.26 2.45) |
| 300* - S - 342 (1.84 1.27 2.43) | 300* - S - 343 (2.37 1.77 1.93) | 301* - S - 150 (1.39 1.41 2.64) | 301* - S - 151 (1.30 1.66 2.38) |
| 301* - S - 152 (1.94 1.96 2.08) | 301* - S - 153 (1.32 1.51 2.53) | 301* - S - 184 (1.14 1.00 3.04) | 301* - S - 291 (1.05 1.54 2.50) |
| 301* - S - 342 (1.79 1.56 2.48) | 301* - S - 343 (2.32 2.06 1.99) | 302* - S - 150 (1.27 1.56 2.76) | 302* - S - 151 (1.18 1.82 2.50) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 302* - S - 152 (1.82 2.12 2.20) | 302* - S - 153 (1.20 1.66 2.66) | 302* - S - 184 (1.02 1.15 3.16) | 302* - S - 291 (0.93 1.70 2.62) |
| 302* - S - 342 (1.67 1.72 2.60) | 302* - S - 343 (2.20 2.21 2.11) | 303* - S - 150 (1.68 1.14 2.34) | 303* - S - 151 (1.59 1.40 2.08) |
| 303* - S - 152 (2.23 1.70 1.78) | 303* - S - 153 (1.62 1.25 2.24) | 303* - S - 192* (1.04 1.26 2.23) | 303* - S - 291 (1.34 1.28 2.20) |
| 303* - S - 342 (2.09 1.30 2.19) | 303* - S - 343 (2.61 1.79 1.69) | 304* - S - 151 (1.55 0.93 2.13) | 304* - S - 152 (2.19 1.23 1.83) |
| 304* - S - 291 (1.30 0.81 2.25) | 304* - S - 342 (2.04 0.83 2.23) | 304* - S - 343 (2.57 1.32 1.73) | 305* - S - 152 (1.68 1.01 2.34) |
| 305* - S - 343 (2.06 1.10 2.25) | 308* - S - 150 (1.65 1.51 2.38) | 308* - S - 151 (1.56 1.77 2.12) | 308* - S - 152 (2.20 2.07 1.82) |
| 308* - S - 153 (1.58 1.61 2.28 | 308* - S - 184 (1.40 1.10 2.78) | 308* - S - 192* (1.00 1.62 2.26) | 308* - S - 291 (1.31 1.65 2.24) |
| 308* - S - 342 (2.05 1.67 2.22) | 308* - S - 343 (2.58 2.16 1.73) | 310* - S - 150 (1.53 1.06 2.50) | 310* - S - 151 (1.44 1.32 2.24) |
| 310* - S - 152 (2.08 1.62 1.94) | 310* - S - 153 (1.46 1.16 2.40) | 310* - S - 192* (0.88 1.17 2.38) | 310* - S - 291 (1.19 1.20 2.36) |
| 310* - S - 342 (1.93 1.22 2.34) | 310* - S - 343 (2.46 1.71 1.85) | 311* - S - 151 (1.71 1.04 1.96) | 311* - S - 152 (2.36 1.34 1.66) |
| 311* - S - 153 (1.74 0.88 2.12) | 311* - S - 192* (1.16 0.90 2.11) | 311* - S - 291 (1.46 0.92 2.08) | 311* - S - 342 (2.21 0.94 2.06) |
| 311* - S - 343 (2.74 1.43 1.57) | 312* - S - 150 (1.71 0.91 2.32) | 312* - S - 151 (1.61 1.17 2.06) | 312* - S - 152 (2.26 1.47 1.76) |
| 312* - S - 153 (1.64 1.01 2.22) | 312* - S - 192* (1.06 1.02 2.21) | 312* - S - 291 (1.37 1.05 2.18) | 312* - S - 342 (2.11 1.07 2.16) |
| 312* - S - 343 (2.64 1.56 1.67) | 313* - S - 151 (1.84 1.00 1.84) | 313* - S - 152 (2.48 1.30 1.54) | 313* - S - 153 (1.86 0.84 2.00) |
| 313* - S - 192* (1.28 0.86 1.98) | 313* - S - 207 (0.85 1.16 1.68) | 313* - S - 291 (1.59 0.88 1.96) | 313* - S - 342 (2.33 0.90 1.94) |
| 313* - S - 343 (2.86 1.39 1.45) | 314* - S - 150 (3.12 2.08 0.90) | 314* - S - 154 (2.59 1.31 1.68) | 314* - S - 184 (2.87 1.67 1.31) |
| 314* - S - 193* (1.39 1.36 1.63) | 314* - S - 244* (1.79 1.72 1.26) | 314* - S - 287* (1.37 1.41 1.58) | 314* - S - 292 (2.11 1.88 1.11) |
| 314* - S - 294 (2.07 1.72 1.27) | 314* - S - 295 (1.83 1.96 1.02) | 314* - S - 296 (2.10 1.96 1.03) | 314* - S - 300 (1.68 0.96 2.02) |
| 314* - S - 305 (1.99 1.63 1.36) | 314* - S - 331 (1.63 1.21 1.78) | 314* - S - 468* (1.34 1.84 1.15) | 315* - S - 154 (3.04 1.58 1.22) |
| 315* - S - 189 (2.65 0.94 1.86) | 315* - S - 193* (1.85 1.62 1.18) | 315* - S - 287* (1.82 1.67 1.12) | 315* - S - 300 (2.13 1.23 1.57) |
| 315* - S - 301 (2.18 0.94 1.86) | 315* - S - 305 (2.45 1.89 0.91) | 315* - S - 331 (2.08 1.47 1.33) | 316* - S - 152 (2.19 1.01 1.83) |
| 316* - S - 343 (2.57 1.10 1.73) | 317* - S - 150 (2.70 1.80 1.33) | 317* - S - 151 (2.61 2.06 1.07) | 317* - S - 153 (2.63 1.90 1.22) |
| 317* - S - 154 (2.16 1.03 2.10) | 317* - S - 184 (2.45 1.39 1.73) | 317* - S - 192* (2.06 1.91 1.21) | 317* - S - 193* (0.97 1.07 2.05) |
| 317* - S - 207 (1.62 2.22 0.91) | 317* - S - 244* (1.37 1.44 1.68) | 317* - S - 287* (0.95 1.13 2.00) | 317* - S - 291 (2.36 1.94 1.19) |
| 317* - S - 292 (1.69 1.60 1.53) | 317* - S - 294 (1.65 1.44 1.69) | 317* - S - 295 (1.40 1.68 1.44) | 317* - S - 296 (1.68 1.68 1.45) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 317* - S - 305 (1.57 1.35 1.78) | 317* - S - 331 (1.21 0.92 2.20) | 317* - S - 342 (3.10 1.96 1.17) | 317* - S - 468* (0.91 1.55 1.57) |
| 318* - S - 150 (1.35 1.41 2.68) | 318* - S - 151 (1.26 1.67 2.42) | 318* - S - 152 (1.90 1.97 2.12) | 318* - S - 153 (1.28 1.51 2.58) |
| 318* - S - 184 (1.10 1.01 3.09) | 318* - S - 291 (1.01 1.55 2.54) | 318* - S - 342 (1.75 1.57 2.52) | 318* - S - 343 (2.28 2.06 2.03) |
| 319* - S - 151 (2.35 0.85 1.33) | 319* - S - 152 (2.99 1.15 1.03) | 319* - S - 206 (0.85 1.07 1.11) | 319* - S - 207 (1.37 1.01 1.17) |
| 319* - S - 343 (3.37 1.25 0.94) | 321* - S - 150 (2.94 1.58 1.09) | 321* - S - 153 (2.87 1.69 0.99) | 321* - S - 154 (2.40 0.81 1.86) |
| 321* - S - 184 (2.69 1.18 1.50) | 321* - S - 192* (2.29 1.70 0.98) | 321* - S - 193* (1.21 0.86 1.81) | 321* - S - 244* (1.61 1.23 1.45) |
| 321* - S - 287* (1.18 0.91 1.76) | 321* - S - 291 (2.60 1.72 0.95) | 321* - S - 292 (1.93 1.38 1.29) | 321* - S - 294 (1.89 1.22 1.45) |
| 321* - S - 295 (1.64 1.47 1.21) | 321* - S - 296 (1.92 1.46 1.21) | 321* - S - 305 (1.81 1.13 1.54) | 321* - S - 342 (3.34 1.74 0.93) |
| 321* - S - 468* (1.15 1.34 1.33) | 322* - S - 154 (2.99 1.12 1.28) | 322* - S - 184 (3.27 1.48 0.91) | 322* - S - 193* (1.79 1.17 1.23) |
| 322* - S - 287* (1.77 1.22 1.18) | 322* - S - 305 (2.39 1.44 0.96) | 322* - S - 331 (2.03 1.02 1.38) | 323* - S - 150 (2.71 1.80 1.32) |
| 323 - S - 151 (2.62 2.06 1.06) | 323 - S - 153 (2.64 1.90 1.22) | 323* - S - 154 (2.17 1.03 2.09) | 323* - S - 184 (2.46 1.39 1.72) |
| 323 - S - 192* (2.06 1.91 1.20) | 323 - S - 193* (0.98 1.07 2.04) | 323* - S - 244* (1.38 1.44 1.67) | 323* - S - 287* (0.96 1.13 1.99) |
| 323 - S - 291 (2.37 1.94 1.18) | 323 - S - 292 (1.70 1.60 1.52) | 323* - S - 294 (1.66 1.44 1.68) | 323* - S - 295 (1.41 1.68 1.43) |
| 323 - S - 296 (1.69 1.68 1.44) | 323 - S - 305 (1.58 1.34 1.77) | 323* - S - 331 (1.22 0.92 2.19) | 323* - S - 342 (3.11 1.96 1.16) |
| 323 - S - 468* (0.92 1.55 1.56) | 325 - S - 151 (2.50 0.92 1.17) | 325* - S - 206 (1.00 1.13 0.96) | 325* - S - 207 (1.52 1.08 1.01) |
| 325 - S - 342 (3.00 0.82 1.27) | 328 - S - 150 (2.23 1.55 1.80) | 328 - S - 151 (2.14 1.81 1.54) | 328 - S - 152 (2.78 2.11 1.24) |
| 328 - S - 153 (2.16 1.65 1.70) | 328 - S - 184 (1.98 1.14 2.21) | 328 - S - 192* (1.58 1.66 1.69) | 328 - S - 207 (1.15 1.97 1.38) |
| 328 - S - 244* (0.90 1.19 2.15) | 328 - S - 291 (1.89 1.69 1.66) | 328 - S - 292 (1.22 1.35 2.00) | 328 - S - 294 (1.18 1.19 2.16) |
| 328 - S - 295 (0.93 1.43 1.92) | 328 - S - 296 (1.21 1.43 1.92) | 328 - S - 305 (1.10 1.10 2.25) | 328 - S - 342 (2.63 1.71 1.64) |
| 328 - S - 343 (3.16 2.20 1.15) | 330* - S - 150 (2.02 1.05 2.00) | 330* - S - 151 (1.93 1.31 1.74) | 330* - S - 152 (2.58 1.60 1.44) |
| 330* - S - 153 (1.96 1.15 1.90) | 330* - S - 192* (1.38 1.16 1.89) | 330* - S - 207 (0.95 1.46 1.58) | 330* - S - 291 (1.68 1.18 1.86) |
| 330* - S - 292 (1.02 0.84 2.20) | 330* - S - 296 (1.01 0.93 2.12) | 330* - S - 342 (2.43 1.20 1.84) | 330* - S - 343 (2.96 1.70 1.35) |
| 331* - S - 150 (1.49 0.88 2.53) | 331* - S - 151 (1.40 1.13 2.27) | 331* - S - 152 (2.05 1.43 1.97) | 331* - S - 153 (1.43 0.98 2.43) |
| 331* - S - 192* (0.85 0.99 2.42) | 331* - S - 291 (1.15 1.01 2.39) | 331* - S - 342 (1.90 1.03 2.37) | 331* - S - 343 (2.43 1.52 1.88) |
| 332* - S - 151 (1.55 0.96 2.13) | 332* - S - 152 (2.19 1.26 1.83) | 332* - S - 153 (1.57 0.80 2.28) | 332* - S - 192* (1.00 0.81 2.27) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 332* - S - 291 (1.30 0.84 2.25) | 332* - S - 342 (2.04 0.86 2.23) | 332* - S - 343 (2.57 1.35 1.74) | 336* - S - 150 (1.54 0.81 2.49) |
| 336* - S - 151 (1.45 1.07 2.23) | 336* - S - 152 (2.09 1.37 1.93) | 336* - S - 153 (1.47 0.91 2.39) | 336* - S - 192* (0.89 0.92 2.37) |
| 336* - S - 291 (1.20 0.95 2.35) | 336* - S - 342 (1.94 0.97 2.33) | 336* - S - 343 (2.47 1.46 1.84) | 337 - S - 150 (2.78 2.07 1.24) |
| 337 - S - 151 (2.69 2.32 0.98) | 337 - S - 153 (2.71 2.17 1.14) | 337 - S - 154 (2.25 1.29 2.01) | 337 - S - 184 (2.53 1.66 1.65) |
| 337 - S - 192* (2.14 2.18 1.13) | 337 - S - 193* (1.06 1.34 1.97) | 337 - S - 244* (1.45 1.71 1.60) | 337 - S - 287* (1.03 1.39 1.91) |
| 337 - S - 291 (2.44 2.20 1.11) | 337 - S - 292 (1.78 1.86 1.45) | 337 - S - 294 (1.73 1.70 1.60) | 337 - S - 295 (1.49 1.95 1.36) |
| 337 - S - 296 (1.77 1.94 1.36) | 337 - S - 300 (1.34 0.95 2.36) | 337 - S - 305 (1.66 1.61 1.70) | 337 - S - 331 (1.29 1.19 2.12) |
| 337 - S - 342 (3.18 2.22 1.09) | 337 - S - 468* (1.00 1.82 1.49) | 338* - S - 150 (1.24 2.04 2.79) | 338* - S - 151 (1.15 2.30 2.53) |
| 338* - S - 152 (1.79 2.60 2.23) | 338* - S - 153 (1.17 2.14 2.69) | 338* - S - 184 (0.99 1.64 3.20) | 338* - S - 291 (0.90 2.18 2.65) |
| 338* - S - 342 (1.64 2.20 2.63) | 338* - S - 343 (2.17 2.69 2.14) | 339* - S - 150 (2.84 2.43 1.18) | 339* - S - 151 (2.75 2.69 0.93) |
| 339* - S - 153 (2.77 2.53 1.08) | 339* - S - 154 (2.31 1.66 1.96) | 339* - S - 184 (2.59 2.02 1.59) | 339* - S - 189 (1.91 1.02 2.59) |
| 339* - S - 192* (2.20 2.54 | 1.07)339* - S - 193* (1.11 1.71 1.91) | 339* - S - 244*(1.51 2.07 1.54) | 339* - S - 287* (1.09 1.76 1.86) |
| 339* - S - 291 (2.50 2.57 1.05) | 339* - S - 292 (1.83 2.23 1.39) | 339* - S - 294 (1.79 2.07 1.55) | 339* - S - 295 (1.55 2.31 1.30) |
| 339* - S - 296 (1.82 2.31 1.31) | 339* - S - 300 (1.40 1.31 2.30) | 339* - S - 301 (1.45 1.02 2.59) | 339* - S - 302 (1.57 0.87 2.74) |
| 339* - S - 305 (1.71 1.98 1.64) | 339* - S - 331 (1.35 1.56 2.06) | 339* - S - 342 (3.24 2.59 1.03) | 339* - S - 468* (1.06 2.19 1.43) |
| 341* - S - 343 (2.97 0.83 1.33) | 344 - S - 150 (2.49 2.54 1.54) | 344 - S - 151 (2.39 2.80 1.28) | 344 - S - 152 (3.04 3.10 0.98) |
| 344 - S - 153 (2.42 2.64 1.44) | 344 - S - 154 (1.95 1.77 2.31) | 344 - S - 184 (2.23 2.14 1.95) | 344 - S - 189 (1.56 1.14 2.95) |
| 344 - S - 192* (1.84 2.66 1.43) | 344 - S - 206 (0.89 3.01 1.07) | 344 - S - 207 (1.41 2.96 1.12) | 344 - S - 244* (1.16 2.19 1.90) |
| 344 - S - 291 (2.15 2.68 1.40) | 344 - S - 292 (1.48 2.34 1.74) | 344 - S - 294 (1.44 2.18 1.90) | 344 - S - 295 (1.19 2.43 1.66) |
| 344 - S - 296 (1.47 2.42 1.66) | 344 - S - 300 (1.04 1.43 2.66) | 344 - S - 301 (1.10 1.14 2.95) | 344 - S - 302 (1.22 0.99 3.10) |
| 344 - S - 305 (1.36 2.09 1.99) | 344 - S - 331 (0.99 1.67 2.41) | 344 - S - 342 (2.89 2.70 1.38) | 345 - S - 150 (2.36 2.39 1.67) |
| 345 - S - 151 (2.27 2.65 1.41) | 345 - S - 152 (2.91 2.95 1.11) | 345 - S - 153 (2.29 2.49 1.56) | 345 - S - 154 (1.83 1.62 2.44) |
| 345 - S - 184 (2.11 1.98 2.07) | 345 - S - 189 (1.43 0.98 3.07) | 345 - S - 192* (1.72 2.50 1.55) | 345 - S - 207 (1.29 2.81 1.25) |
| 345 - S - 244* (1.03 2.03 2.02) | 345 - S - 291 (2.02 2.53 1.53) | 345 - S - 292 (1.35 2.19 1.87) | 345 - S - 294 (1.31 2.03 2.03) |
| 345 - S - 295 (1.07 2.27 1.78) | 345 - S - 296 (1.34 2.27 1.79) | 345 - S - 300 (0.92 1.27 2.78) | 345 - S - 301 (0.97 0.99 3.07) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 345 - S - 302 (1.09 0.83 3.22) | 345 - S - 305 (1.23 1.94 2.12) | 345 - S - 331 (0.87 1.52 2.54) | 345 - S - 342 (2.76 2.55 1.51) |
| 345 - S - 343 (3.29 3.04 1.02) | 346 - S - 150 (2.18 2.38 1.85) | 346 - S - 151 (2.09 2.63 1.59) | 346 - S - 152 (2.73 2.93 1.29) |
| 346 - S - 153 (2.11 2.48 1.75) | 346 - S - 154 (1.64 1.60 2.62) | 346 - S - 184 (1.93 1.97 2.26) | 346 - S - 189 (1.25 0.97 3.26) |
| 346 - S - 192* (1.53 2.49 1.73) | 346 - S - 207 (1.10 2.79 1.43) | 346 - S - 244* (0.85 2.02 2.20) | 346 - S - 291 (1.84 2.51 1.71) |
| 346 - S - 292 (1.17 2.17 2.05) | 346 - S - 294 (1.13 2.01 2.21) | 346 - S - 295 (0.88 2.26 1.97) | 346 - S - 296 (1.16 2.25 1.97) |
| 346 - S - 302 (0.91 0.82 3.41) | 346 - S - 305 (1.05 1.92 2.30) | 346 - S - 342 (2.58 2.53 1.69) | 346 - S - 343 (3.11 3.02 1.20) |
| 347 - S - 150 (2.23 2.46 1.80) | 347 - S - 151 (2.14 2.72 1.54) | 347 - S - 152 (2.78 3.02 1.24) | 347 - S - 153 (2.16 2.56 1.70) |
| 347 - S - 154 (1.69 1.69 2.57) | 347 - S - 184 (1.98 2.06 2.20) | 347 - S - 189 (1.30 1.06 3.20) | 347 - S - 192* (1.58 2.58 1.68) |
| 347 - S - 207 (1.15 2.88 1.38) | 347 - S - 244* (0.90 2.11 2.15) | 347 - S - 291 (1.89 2.60 1.66) | 347 - S - 292 (1.22 2.26 2.00) |
| 347 - S - 294 (1.18 2.10 2.16) | 347 - S - 295 (0.93 2.35 1.91) | 347 - S - 296 (1.21 2.34 1.92) | 347 - S - 301 (0.84 1.06 3.20) |
| 347 - S - 302 (0.96 0.91 3.35) | 347 - S - 305 (1.10 2.01 2.25) | 347 - S - 342 (2.63 2.62 1.64) | 347 - S - 343 (3.16 3.11 1.15) |
| 348* - S - 150 (2.58 2.23 1.45) | 348* - S - 151 (2.48 2.48 1.19) | 348* - S - 153 (2.51 2.33 1.35) | 348* - S - 154 (2.04 1.45 2.22) |
| 348 - S - 184 (2.33 1.82 1.86) | 348* - S - 189 (1.65 0.82 2.86) | 348* - S - 192* (1.93 2.34 1.34) | 348* - S - 193* (0.85 1.50 2.17) |
| 348* - S - 206 (0.98 2.69 0.98) | 348* - S - 207 (1.50 2.64 1.03) | 348* - S - 244* (1.25 1.87 1.81) | 348* - S - 287* (0.82 1.55 2.12) |
| 348* - S - 291 (2.24 2.36 1.31) | 348* - S - 292 (1.57 2.02 1.65) | 348* - S - 294 (1.53 1.86 1.81) | 348* - S - 295 (1.28 2.11 1.57) |
| 348* - S - 296 (1.56 2.10 1.57) | 348* - S - 300 (1.14 1.11 2.57) | 348* - S - 301 (1.19 0.82 2.86) | 348* - S - 305 (1.45 1.77 1.90) |
| 348* - S - 331 (1.08 1.35 2.32) | 348* - S - 342 (2.98 2.38 1.29) | 349 - S - 150 (2.09 2.36 1.93) | 349 - S - 151 (2.00 2.62 1.68) |
| 349 - S - 152 (2.64 2.92 1.38) | 349 - S - 153 (2.02 2.46 1.83) | 349 - S - 154 (1.56 1.59 2.70) | 349 - S - 184 (1.84 1.95 2.34) |
| 349 - S - 189 (1.16 0.95 3.34) | 349 - S - 192* (1.45 2.47 1.82) | 349 - S - 207 (1.02 2.78 1.52) | 349 - S - 291 (1.75 2.50 1.80) |
| 349 - S - 292 (1.09 2.16 2.14) | 349 - S - 294 (1.04 2.00 2.29) | 349 - S - 296 (1.07 2.24 2.06) | 349 - S - 302 (0.82 0.80 3.49) |
| 349 - S - 305 (0.97 1.91 2.39) | 349 - S - 342 (2.49 2.52 1.78) | 349 - S - 343 (3.02 3.01 1.28) | 350 - S - 150 (2.07 2.27 1.96) |
| 350 - S - 151 (1.98 2.53 1.70) | 350 - S - 152 (2.62 2.83 1.40) | 350 - S - 153 (2.00 2.37 1.86) | 350 - S - 154 (1.53 1.50 2.73) |
| 350 - S - 184 (1.82 1.87 2.37) | 350 - S - 189 (1.14 0.87 3.37) | 350 - S - 192* (1.42 2.39 1.85) | 350 - S - 207 (0.99 2.69 1.54) |
| 350 - S - 291 (1.73 2.41 1.82) | 350 - S - 292 (1.06 2.07 2.16) | 350 - S - 294 (1.02 1.91 2.32) | 350 - S - 296 (1.05 2.15 2.08) |
| 350 - S - 305 (0.94 1.82 2.41) | 350 - S - 342 (2.47 2.43 1.80) | 350 - S - 343 (3.00 2.92 1.31) | 351 - S - 150 (2.17 2.27 1.86) |

(fortgesetzt)

| | | | |
|---|---|---|---|
| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
| 351 - S - 151 (2.08 2.53 1.60) | 351 - S - 152 (2.72 2.83 1.30) | 351 - S - 153 (2.10 2.37 1.76) | 351 - S - 154 (1.63 1.50 2.63) |
| 351 - S - 184 (1.92 1.87 2.27) | 351 - S - 189 (1.24 0.87 3.27) | 351 - S - 192* (1.52 2.39 1.75) | 351 - S - 207 (1.09 2.69 1.44) |
| 351 - S - 244* (0.84 1.92 2.22) | 351 - S - 291 (1.83 2.41 1.72) | 351 - S - 292 (1.16 2.07 2.06) | 351 - S - 294 (1.12 1.91 2.22) |
| 351 - S - 295 (0.87 2.16 1.98) | 351 - S - 296 (1.15 2.15 1.98) | 351 - S - 305 (1.04 1.82 2.31) | 351 - S - 342 (2.57 2.43 1.70) |
| 351 - S - 343 (3.10 2.92 1.21) | 352* - S - 150 (1.93 1.57 2.09) | 352* - S - 151 (1.84 1.83 1.84) | 352 - S - 152 (2.48 2.13 1.54) |
| 352* - S - 153 (1.86 1.67 1.99) | 352* - S - 154 (1.40 0.80 2.86) | 352* - S - 184 (1.68 1.17 2.50) | 352* - S - 192* (1.29 1.69 1.98) |
| 352* - S - 207 (0.86 1.99 1.68) | 352* - S - 291 (1.59 1.71 1.96) | 352* - S - 292 (0.93 1.37 2.30) | 352* - S - 294 (0.88 1.21 2.46) |
| 352* - S - 296 (0.91 1.45 2.22) | 352* - S - 305 (0.81 1.12 2.55) | 352* - S - 342 (2.33 1.73 1.94) | 352* - S - 343 (2.86 2.22 1.44) |
| 353* - S - 150 (2.00 1.53 2.02) | 353* - S - 151 (1.91 1.79 1.76) | 353* - S - 152 (2.56 2.09 1.46) | 353* - S - 153 (1.94 1.64 1.92) |
| 353* - S - 184 (1.75 1.13 2.43) | 353* - S - 192* (1.36 1.65 1.91) | 353* - S - 207 (0.93 1.95 1.60) | 353* - S - 291 (1.66 1.67 1.88) |
| 353* - S - 292 (1.00 1.33 2.22) | 353* - S - 294 (0.96 1.17 2.38) | 353* - S - 296 (0.99 1.41 2.14) | 353* - S - 305 (0.88 1.08 2.48) |
| 353* - S - 342 (2.41 1.69 1.86) | 353* - S - 343 (2.94 2.18 1.37) | 354* - S - 150 (2.51 1.71 1.52) | 354* - S - 151 (2.41 1.97 1.26) |
| 354* - S - 152 (3.06 2.26 0.96) | 354* - S - 153 (2.44 1.81 1.42) | 354* - S - 154 (1.97 0.94 2.29) | 354* - S - 184 (2.26 1.30 1.93) |
| 354* - S - 192* (1.86 1.82 1.41) | 354* - S - 206 (0.91 2.18 1.05) | 354* - S - 207 (1.43 2.12 1.10) | 354* - S - 244* (1.18 1.35 1.88) |
| 354* - S - 291 (2.17 1.84 1.38) | 354* - S - 292 (1.50 1.50 1.72) | 354* - S - 294 (1.46 1.35 1.88) | 354* - S - 295 (1.21 1.59 1.64) |
| 354* - S - 296 (1.49 1.59 1.64) | 354* - S - 305 (1.38 1.25 1.97) | 354* - S - 331 (1.01 0.83 2.39) | 354* - S - 342 (2.91 1.86 1.36) |
| 355* - S - 184 (3.25 1.02 0.93) | 355* - S - 305 (2.38 0.97 0.98) | 356* - S - 150 (1.87 0.99 2.15) | 356* - S - 151 (1.78 1.25 1.89) |
| 356* - S - 152 (2.43 1.55 1.59) | 356* - S - 153 (1.81 1.10 2.05) | 356* - S - 192* (1.23 1.11 2.04) | 356* - S - 207 (0.80 1.41 1.73) |
| 356* - S - 291 (1.53 1.13 2.01) | 356* - S - 296 (0.86 0.87 2.27) | 356* - S - 342 (2.28 1.15 1.99) | 356* - S - 343 (2.81 1.64 1.50) |
| 357* - S - 150 (1.18 1.25 2.85) | 357* - S - 151 (1.09 1.51 2.59) | 357* - S - 152 (1.73 1.80 2.29) | 357* - S - 153 (1.11 1.35 2.75) |
| 357* - S - 184 (0.93 0.84 3.25) | 357* - S - 291 (0.84 1.38 2.71) | 357* - S - 342 (1.58 1.40 2.69) | 357* - S - 343 (2.11 1.90 2.20) |
| 358* - S - 152 (2.25 1.09 1.77) | 358* - S - 343 (2.63 1.18 1.68) | 359* - S - 150 (2.29 1.44 1.74) | 359* - S - 151 (2.20 1.70 1.48) |
| 359* - S - 152 (2.84 2.00 1.18) | 359* - S - 153 (2.22 1.54 1.64) | 359* - S - 184 (2.04 1.03 2.14) | 359* - S - 192* (1.64 1.55 1.62) |
| 359* - S - 207 (1.21 1.86 1.32) | 359* - S - 244* (0.96 1.08 2.09) | 359 - S - 291 (1.95 1.58 1.60) | 359* - S - 292 (1.28 1.24 1.94) |
| 359* - S - 294 (1.24 1.08 2.10) | 359* - S - 295 (0.99 1.32 1.85) | 359* - S - 296 (1.27 1.32 1.86) | 359* - S - 305 (1.16 0.98 2.19) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 359* - S - 342 (2.69 1.60 1.58) | 359* - S - 343 (3.22 2.09 1.09) | 360* - S - 150 (2.25 1.14 1.78) | 360* - S - 151 (2.16 1.40 1.52) |
| 360* - S - 152 (2.80 1.70 1.22) | 360* - S - 153 (2.18 1.24 1.68) | 360* - S - 192* (1.60 1.25 1.66) | 360* - S - 207 (1.17 1.56 1.36) |
| 360* - S - 291 (1.91 1.28 1.64) | 360* - S - 292 (1.24 0.94 1.98) | 360* - S - 295 (0.95 1.02 1.89) | 360* - S - 296 (1.23 1.02 1.90) |
| 360* - S - 342 (2.65 1.30 1.62) | 360* - S - 343 (3.18 1.79 1.13) | 361* - S - 150 (2.53 1.83 1.49) | 361* - S - 151 (2.44 2.09 1.24) |
| 361* - S - 152 (3.08 2.39 0.94) | 361* - S - 153 (2.46 1.93 1.39) | 361* - S - 154 (2.00 1.06 2.26) | 361* - S - 184 (2.28 1.42 1.90) |
| 361* - S - 192* (1.89 1.94 1.38) | 361* - S - 193* (0.80 1.11 2.22) | 361* - S - 206 (0.94 2.30 1.02) | 361* - S - 207 (1.46 2.25 1.08) |
| 361* - S - 244* (1.20 1.47 1.85) | 361* - S - 291 (2.19 1.97 1.36) | 361* - S - 292 (1.53 1.63 1.70) | 361* - S - 294 (1.48 1.47 1.85) |
| 361* - S - 295 (1.24 1.71 1.61) | 361* - S - 296 (1.52 1.71 1.62) | 361* - S - 305 (1.41 1.38 1.95) | 361* - S - 331 (1.04 0.96 2.37) |
| 361* - S - 342 (2.93 1.99 1.34) | 366 - S - 150 (2.15 1.88 1.87) | 366 - S - 151 (2.06 2.14 1.62) | 366 - S - 152 (2.70 2.44 1.32) |
| 366 - S - 153 (2.08 1.98 1.77) | 366 - S - 154 (1.62 1.11 2.65) | 366 - S - 184 (1.90 1.48 2.28) | 366 - S - 192* (1.51 2.00 1.76) |
| 366 - S - 207 (1.08 2.30 1.46) | 366 - S - 244* (0.82 1.53 2.23) | 366 - S - 291 (1.81 2.02 1.74) | 366 - S - 292 (1.14 1.68 2.08) |
| 366 - S - 294 (1.10 1.52 2.24) | 366 - S - 295 (0.86 1.77 1.99) | 366 - S - 296 (1.13 1.76 2.00) | 366 - S - 305 (1.02 1.43 2.33) |
| 366 - S - 342 (2.55 2.04 1.72) | 366 - S - 343 (3.08 2.53 1.23) | 369* - S - 150 (2.64 1.72 1.38) | 369* - S - 151 (2.55 1.98 1.13) |
| 369* - S - 153 (2.57 1.82 1.28) | 369* - S - 154 (2.11 0.95 2.15) | 369* - S - 184 (2.39 1.31 1.79) | 369* - S - 192* (2.00 1.83 1.27) |
| 369* - S - 193* (0.91 0.99 2.11) | 369* - S - 206 (1.05 2.19 0.91) | 369* - S - 207 (1.57 2.13 0.97) | 369* - S - 244* (1.31 1.36 1.74) |
| 369* - S - 287* (0.89 1.05 2.06) | 369* - S - 291 (2.30 1.86 1.25) | 369* - S - 292 (1.64 1.51 1.59) | 369* - S - 294 (1.59 1.36 1.74) |
| 369* - S - 295 (1.35 1.60 1.50) | 369* - S - 296 (1.62 1.60 1.51) | 369* - S - 305 (1.52 1.26 1.84) | 369* - S - 331 (1.15 0.84 2.26) |
| 369* - S - 342 (3.04 1.87 1.23) | 369* - S - 468* (0.86 1.47 1.63) | 370* - S - 152 (2.28 1.03 1.74) | 370* - S - 343 (2.66 1.12 1.65) |
| 372* - S - 151 (1.41 1.03 2.27) | 372* - S - 152 (2.05 1.33 1.97) | 372* - S - 153 (1.43 0.87 2.43) | 372* - S - 192* (0.85 0.88 2.41) |
| 372* - S - 291 (1.16 0.91 2.39) | 372* - S - 342 (1.90 0.93 2.37) | 372* - S - 343 (2.43 1.42 1.88) | 373* - S - 151 (2.02 1.02 1.66) |
| 373* - S - 152 (2.66 1.32 1.36) | 373* - S - 153 (2.04 0.86 1.82) | 373* - S - 192* (1.46 0.88 1.80) | 373* - S - 207 (1.03 1.18 1.50) |
| 373* - S - 291 (1.77 0.90 1.78) | 373* - S - 342 (2.51 0.92 1.76) | 373* - S - 343 (3.04 1.41 1.27) | 374* - S - 152 (2.44 0.83 1.58) |
| 374* - S - 343 (2.82 0.93 1.49) | 375* - S - 150 (2.63 1.40 1.40) | 375* - S - 151 (2.54 1.66 1.14) | 375* - S - 153 (2.56 1.50 1.29) |
| 375* - S - 184 (2.38 0.99 1.80) | 375* - S - 192* (1.99 1.51 1.28) | 375* - S - 206 (1.03 1.87 0.93) | 375* - S - 207 (1.55 1.82 0.98) |
| 375* - S - 244* (1.30 1.04 1.75) | 375* - S - 291 (2.29 1.54 1.26) | 375* - S - 292 (1.62 1.20 1.60) | 375* - S - 294 (1.58 1.04 1.76) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 375* - S - 295 (1.34 1.28 1.51) | 375* - S - 296 (1.61 1.28 1.52) | 375* - S - 305 (1.50 0.95 1.85) | 375* - S - 342 (3.03 1.56 1.24) |
| 375* - S - 468* (0.84 1.16 1.64) | 377* - S - 150 (2.68 1.44 1.34) | 377* - S - 151 (2.59 1.70 1.08) | 377* - S - 153 (2.62 1.54 1.24) |
| 377* - S - 184 (2.43 1.03 1.75) | 377* - S - 192* (2.04 1.55 1.23) | 377* - S - 207 (1.61 1.86 0.92) | 377* - S - 244* (1.35 1.08 1.70) |
| 377* - S - 291 (2.34 1.58 1.20) | 377* - S - 292 (1.68 1.24 1.54) | 377* - S - 294 (1.64 1.08 1.70) | 377* - S - 295 (1.39 1.32 1.46) |
| 377* - S - 296 (1.67 1.32 1.46) | 377* - S - 305 (1.56 0.99 1.80) | 377* - S - 342 (3.09 1.60 1.18) | 377* - S - 468* (0.90 1.20 1.59) |
| 378* - S - 150 (2.37 0.92 1.65) | 378* - S - 151 (2.28 1.18 1.39) | 378* - S - 152 (2.92 1.48 1.10) | 378* - S - 153 (2.30 1.02 1.55) |
| 378* - S - 192* (1.73 1.04 1.54) | 378* - S - 207 (1.30 1.34 1.24) | 378* - S - 291 (2.03 1.06 1.52) | 378* - S - 295 (1.08 0.81 1.77) |
| 378* - S - 296 (1.36 0.80 1.77) | 378* - S - 342 (2.77 1.08 1.50) | 378* - S - 343 (3.30 1.57 1.00) | 379* - S - 151 (1.72 1.03 1.95) |
| 379* - S - 152 (2.37 1.33 1.65) | 379* - S - 153 (1.75 0.87 2.11) | 379* - S - 192* (1.17 0.88 2.10) | 379* - S - 291 (1.48 0.91 2.07) |
| 379* - S - 342 (2.22 0.93 2.05) | 379* - S - 343 (2.75 1.42 1.56) | 380* - S - 150 (1.97 1.36 2.05) | 380* - S - 151 (1.88 1.61 1.79) |
| 380* - S - 152 (2.53 1.91 1.49) | 380* - S - 153 (1.91 1.46 1.95) | 380* - S - 184 (1.72 0.95 2.46) | 380* - S - 192* (1.33 1.47 1.94) |
| 380* - S - 207 (0.90 1.77 1.63) | 380* - S - 291 (1.63 1.49 1.91) | 380* - S - 292 (0.97 1.15 2.25) | 380* - S - 294 (0.93 0.99 2.41) |
| 380* - S - 296 (0.96 1.23 2.17) | 380* - S - 305 (0.85 0.90 2.51) | 380* - S - 342 (2.38 1.51 1.89) | 380* - S - 343 (2.91 2.00 1.40) |
| 381* - S - 151 (2.15 0.86 1.52) | 381* - S - 152 (2.80 1.16 1.22) | 381* - S - 207 (1.17 1.02 1.36) | 381* - S - 343 (3.18 1.25 1.13) |
| 382* - S - 151 (1.65 0.85 2.02) | 382* - S - 152 (2.30 1.15 1.72) | 382* - S - 343 (2.68 1.24 1.63) | 383 - S - 150 (2.86 2.48 1.16) |
| 383 - S - 151 (2.77 2.74 0.91) | 383 - S - 153 (2.79 2.58 1.06) | 383 - S - 154 (2.33 1.71 1.94) | 383 - S - 184 (2.61 2.08 1.57) |
| 383 - S - 189 (1.93 1.08 2.57) | 383 - S - 192* (2.22 2.60 1.05) | 383 - S - 193* (1.13 1.76 1.89) | 383 - S - 244* (1.53 2.13 1.52) |
| 383 - S - 287* (1.11 1.81 1.84) | 383 - S - 291 (2.52 2.62 1.03) | 383 - S - 292 (1.85 2.28 1.37) | 383 - S - 294 (1.81 2.12 1.53) |
| 383 - S - 295 (1.57 2.37 1.28) | 383 - S - 296 (1.84 2.36 1.29) | 383 - S - 300 (1.42 1.37 2.28) | 383 - S - 301 (1.47 1.08 2.57) |
| 383 - S - 302 (1.59 0.93 2.72) | 383 - S - 305 (1.73 2.03 1.62) | 383 - S - 331 (1.37 1.61 2.04) | 383 - S - 342 (3.26 2.64 1.01) |
| 383 - S - 468* (1.08 2.24 1.41) | 384* - S - 150 (2.32 2.23 1.70) | 384* - S - 151 (2.23 2.49 1.44) | 384* - S - 152 (2.88 2.79 1.14) |
| 384* - S - 153 (2.26 2.33 1.60) | 384* - S - 154 (1.79 1.46 2.47) | 384* - S - 184 (2.07 1.82 2.11) | 384* - S - 189 (1.39 0.82 3.11) |
| 384* - S - 192* (1.68 2.34 1.59) | 384* - S - 207 (1.25 2.65 1.28) | 384* - S - 244* (0.99 1.87 2.06) | 384* - S - 291 (1.98 2.37 1.56) |
| 384* - S - 292 (1.32 2.03 1.90) | 384* - S - 294 (1.28 1.87 2.06) | 384* - S - 295 (1.03 2.11 1.82) | 384* - S - 296 (1.31 2.11 1.82) |
| 384* - S - 300 (0.88 1.11 2.82) | 384* - S - 301 (0.93 0.82 3.11) | 384* - S - 305 (1.20 1.78 2.16) | 384* - S - 331 (0.83 1.35 2.58) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 384* - S - 342 (2.73 2.39 1.54) | 384* - S - 343 (3.26 2.88 1.05) | 385* - S - 150 (3.03 2.27 1.00) | 385* - S - 154 (2.49 1.49 1.77) |
| 385* - S - 184 (2.77 1.86 1.41) | 385* - S - 189 (2.10 0.86 2.41) | 385* - S - 193* (1.30 1.54 1.72) | 385* - S - 244* (1.70 1.91 1.36) |
| 385* - S - 287* (1.27 1.59 1.67) | 385* - S - 292 (2.02 2.06 1.20) | 385* - S - 294 (1.98 1.90 1.36) | 385* - S - 295 (1.73 2.15 1.12) |
| 385* - S - 296 (2.01 2.14 1.12) | 385* - S - 300 (1.58 1.15 2.12) | 385* - S - 301 (1.64 0.86 2.41) | 385* - S - 305 (1.90 1.81 1.45) |
| 385* - S - 331 (1.53 1.39 1.87) | 385* - S - 468* (1.24 2.02 1.24) | 386* - S - 150 (3.01 1.94 1.02) | 386* - S - 153 (2.94 2.05 0.92) |
| 386* - S - 154 (2.47 1.17 1.79) | 386* - S - 184 (2.76 1.54 1.42) | 386* - S - 192* (2.36 2.06 0.90) | 386* - S - 193* (1.28 1.22 1.74) |
| 386* - S - 244* (1.68 1.59 1.37) | 386* - S - 287* (1.26 1.27 1.69) | 386* - S - 292 (2.00 1.74 1.22) | 386* - S - 294 (1.96 1.58 1.38) |
| 386* - S - 295 (1.71 1.83 1.13) | 386* - S - 296 (1.99 1.82 1.14) | 386* - S - 300 (1.57 0.83 2.13) | 386* - S - 305 (1.88 1.49 1.47) |
| 386* - S - 331 (1.52 1.07 1.89) | 386* - S - 468* (1.22 1.70 1.26) | 387* - S - 150 (2.92 2.50 1.10) | 387* - S - 153 (2.85 2.60 1.00) |
| 387* - S - 154 (2.39 1.72 1.87) | 387* - S - 184 (2.67 2.09 1.51) | 387* - S - 189 (1.99 1.09 2.51) | 387* - S - 192* (2.28 2.61 0.99) |
| 387* - S - 193* (1.20 1.77 1.83) | 387* - S - 244* (1.59 2.14 | 1.46) 387* - S - 287* (1.17 1.82 1.77) | 387* - S - 291 (2.58 2.63 0.96) |
| 387* - S - 292 (1.92 2.29 1.31) | 387* - S - 294 (1.87 2.13 1.46) | 387* - S - 295 (1.63 2.38 1.22) | 387* - S - 296 (1.91 2.37 1.22) |
| 387* - S - 300 (1.48 1.38 2.22) | 387* - S - 301 (1.53 1.09 2.51) | 387* - S - 302 (1.65 0.94 2.66) | 387* - S - 305 (1.80 2.04 1.56) |
| 387* - S - 331 (1.43 1.62 1.98) | 387* - S - 342 (3.33 2.65 0.95) | 387* - S - 468* (1.14 2.25 1.35) | 388* - S - 150 (2.45 2.18 1.57) |
| 388* - S - 151 (2.36 2.44 1.31) | 388* - S - 152 (3.00 2.74 1.01) | 388* - S - 153 (2.39 2.29 1.47) | 388* - S - 154 (1.92 1.41 2.34) |
| 388* - S - 184 (2.20 1.78 1.98) | 388* - S - 192* (1.81 2.30 1.46) | 388* - S - 206 (0.86 2.65 1.10) | 388* - S - 207 (1.38 2.60 1.15) |
| 388* - S - 244* (1.12 1.83 1.93) | 388* - S - 291 (2.11 2.32 1.43) | 388* - S - 292 (1.45 1.98 1.78) | 388* - S - 294 (1.40 1.82 1.93) |
| 388* - S - 295 (1.16 2.07 1.69) | 388* - S - 296 (1.44 2.06 1.69) | 388* - S - 300 (1.01 1.07 2.69) | 388* - S - 305 (1.33 1.73 2.03) |
| 388* - S - 331 (0.96 1.31 2.45) | 388* - S - 342 (2.86 2.34 1.42) | 388* - S - 343 (3.38 2.83 0.92) | 389* - S - 150 (3.08 2.29 0.94) |
| 389* - S - 154 (2.55 1.51 1.72) | 389* - S - 184 (2.83 1.88 1.35) | 389* - S - 189 (2.15 0.88 2.35) | 389* - S - 193* (1.35 1.56 1.67) |
| 389* - S - 244* (1.75 1.93 1.30) | 389* - S - 287* (1.33 1.61 1.62) | 389* - S - 292 (2.07 2.08 1.15) | 389* - S - 294 (2.03 1.92 1.31) |
| 389* - S - 295 (1.79 2.17 1.06) | 389* - S - 296 (2.06 2.16 1.07) | 389* - S - 300 (1.64 1.17 2.06) | 389* - S - 301 (1.69 0.88 2.35) |
| 389* - S - 305 (1.95 1.83 1.40) | 389* - S - 331 (1.59 1.41 1.82) | 389* - S - 468* (1.30 2.04 1.19) | 390* - S - 150 (3.08 1.94 0.95) |
| 390* - S - 154 (2.54 1.16 1.72) | 390* - S - 184 (2.83 1.53 1.36) | 390* - S - 193* (1.35 1.21 1.67) | 390* - S - 244* (1.75 1.58 1.31) |
| 390* - S - 287* (1.32 1.26 1.62) | 390* - S - 292 (2.07 1.73 1.15) | 390* - S - 294 (2.03 1.57 1.31) | 390* - S - 295 (1.78 1.82 1.07) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 390* - S - 296 (2.06 1.81 1.07) | 390* - S - 300 (1.64 0.82 2.07) | 390* - S - 305 (1.95 1.48 1.40) | 390* - S - 331 (1.58 1.06 1.82) |
| 390* - S - 468* (1.29 1.69 1.19) | 391* - S - 151 (1.72 1.04 1.96) | 391* - S - 152 (2.36 1.34 1.66) | 391* - S - 153 (1.74 0.89 2.12) |
| 391* - S - 192* (1.16 0.90 2.11) | 391* - S - 291 (1.47 0.92 2.08) | 391* - S - 342 (2.21 0.94 2.06) | 391* - S - 343 (2.74 1.43 1.57) |
| 392* - S - 150 (2.35 1.62 1.67) | 392* - S - 151 (2.26 1.88 1.41) | 392* - S - 152 (2.90 2.18 1.12) | 392* - S - 153 (2.28 1.72 1.57) |
| 392* - S - 154 (1.82 0.85 2.44) | 392* - S - 184 (2.10 1.21 2.08) | 392* - S - 192* (1.71 1.73 1.56) | 392* - S - 207 (1.28 2.04 1.26) |
| 392* - S - 244* (1.02 1.26 2.03) | 392* - S - 291 (2.01 1.76 1.53) | 392* - S - 292 (1.35 1.42 1.88) | 392* - S - 294 (1.30 1.26 2.03) |
| 392* - S - 295 (1.06 1.50 1.79) | 392* - S - 296 (1.34 1.50 1.79) | 392* - S - 305 (1.23 1.16 2.13) | 392* - S - 342 (2.76 1.78 1.52) |
| 392* - S - 343 (3.28 2.27 1.02) | 393* - S - 150 (2.11 3.92 1.91) | 393* - S - 151 (2.02 4.18 1.66) | 393* - S - 152 (2.66 4.48 1.36) |
| 393* - S - 153 (2.04 4.03 1.81) | 393* - S - 154 (1.58 3.15 2.69) | 393* - S - 184 (1.86 3.52 2.32) | 393* - S - 189 (1.18 2.52 3.32) |
| 393* - S - 192* (1.47 4.04 1.80) | 393* - S - 207 (1.04 4.34 1.50) | 393* - S - 291 (1.77 4.06 1.78) | 393* - S - 292 (1.10 3.72 2.12) |
| 393* - S - 294 (1.06 3.56 2.28) | 393* - S - 295 (0.82 3.81 2.03) | 393* - S - 296 (1.09 3.80 2.04) | 393* - S - 302 (0.84 2.37 3.47) |
| 393* - S - 305 (0.98 3.47 2.37) | 393* - S - 342 (2.51 4.08 1.76) | 393* - S - 343 (3.04 4.57 1.27) | 394* - S - 150 (1.91 1.49 2.12) |
| 394* - S - 151 (1.82 1.75 1.86) | 394* - S - 152 (2.46 2.05 1.56) | 394* - S - 153 (1.84 1.59 2.01) | 394* - S - 184 (1.66 1.08 2.52) |
| 394* - S - 192* (1.27 1.60 2.00) | 394* - S - 207 (0.84 1.91 1.70) | 394* - S - 291 (1.57 1.63 1.98 | 394* - S - 292 (0.90 1.29 2.32) |
| 394* - S - 294 (0.86 1.13 2.48) | 394* - S - 296 (0.89 1.37 2.24) | 394* - S - 342 (2.31 1.65 1.96) | 394* - S - 343 (2.84 2.14 1.47) |
| 396* - S - 151 (1.36 0.87 2.31) | 396* - S - 152 (2.01 1.17 2.01) | 396* - S - 343 (2.39 1.27 1.92) | 397* - S - 151 (1.56 1.03 2.12) |
| 397* - S - 152 (2.20 1.33 1.82) | 397* - S - 153 (1.58 0.87 2.27) | 397* - S - 192* (1.01 0.88 2.26) | 397* - S - 291 (1.31 0.91 2.24) |
| 397* - S - 342 (2.05 0.93 2.22) | 397* - S - 343 (2.58 1.42 1.73) | 398* - S - 150 (1.74 1.64 2.28) | 398* - S - 151 (1.65 1.90 2.02) |
| 398* - S - 152 (2.30 2.20 1.72) | 398* - S - 153 (1.68 1.74 2.18) | 398* - S - 154 (1.21 0.87 3.05) | 398* - S - 184 (1.49 1.24 2.69) |
| 398* - S - 192* (1.10 1.76 2.17) | 398* - S - 291 (1.40 1.78 2.14) | 398* - S - 342 (2.15 1.80 2.12) | 398* - S - 343 (2.68 2.29 1.63) |
| 399* - S - 150 (2.98 1.85 1.04) | 399* - S - 153 (2.91 1.95 0.94) | 399* - S - 154 (2.45 1.08 1.81) | 399* - S - 184 (2.73 1.44 1.45) |
| 399* - S - 192* (2.34 1.96 | 0.93)399* - S - 193* (1.26 1.12 | 1.77)399* - S - 244* (1.65 1.49 1.40) | 399* - S - 287* (1.23 1.18 1.71) |
| 399* - S - 291 (2.64 1.99 0.90) | 399* - S - 292 (1.98 1.65 1.25) | 399* - S - 294 (1.93 1.49 1.40) | 399* - S - 295 (1.69 1.73 1.16) |
| 399* - S - 296 (1.97 1.73 1.16) | 399* - S - 305 (1.86 1.39 1.50) | 399* - S - 331 (1.49 0.97 1.92) | 399* - S - 468* (1.20 1.60 1.29) |
| 400 - S - 150 (2.82 0.91 1.21) | 400 - S - 151 (2.72 1.17 0.95) | 400 - S - 153 (2.75 1.01 1.11) | 400 - S - 192* (2.17 1.03 1.10) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 400 - S - 291 (2.48 1.05 1.07) | 400 - S - 342 (3.22 1.07 1.05) | 401* - S - 150 (1.81 1.74 2.22) | 401* - S - 151 (1.72 1.99 1.96) |
| 401* - S - 152 (2.36 2.29 1.66) | 401* - S - 153 (1.74 1.84 2.12) | 401* - S - 154 (1.27 0.97 2.99) | 401* - S - 184 (1.56 1.33 2.63) |
| 401* - S - 192* (1.16 1.85 2.11) | 401* - S - 291 (1.47 1.87 2.08) | 401* - S - 292 (0.80 1.53 2.42) | 401* - S - 342 (2.21 1.89 2.06) |
| 401* - S - 343 (2.74 2.39 1.57) | 402* - S - 150 (2.69 1.55 1.34) | 402* - S - 151 (2.60 1.81 1.08) | 402* - S - 153 (2.62 1.65 1.23) |
| 402* - S - 184 (2.44 1.14 1.74) | 402* - S - 192* (2.05 1.66 1.22) | 402* - S - 193* (0.96 0.82 2.06) | 402* - S - 207 (1.61 1.97 0.92) |
| 402* - S - 244* (1.36 1.19 1.69) | 402* - S - 287* (0.94 0.88 2.01) | 402* - S - 291 (2.35 1.69 1.20) | 402* - S - 292 (1.68 1.35 1.54) |
| 402* - S - 294 (1.64 1.19 1.70) | 402* - S - 295 (1.40 1.43 1.45) | 402* - S - 296 (1.67 1.43 1.46) | 402* - S - 305 (1.56 1.09 1.79) |
| 402* - S - 342 (3.09 1.71 1.18) | 402* - S - 468* (0.90 1.30 1.58) | 403* - S - 150 (1.86 1.52 2.16) | 403* - S - 151 (1.77 1.78 1.91) |
| 403* - S - 152 (2.41 2.08 1.61) | 403* - S - 153 (1.79 1.62 2.06) | 403* - S - 184 (1.61 1.11 2.57) | 403* - S - 192* (1.22 1.63 2.05) |
| 403* - S - 291 (1.52 1.66 2.03) | 403* - S - 292 (0.85 1.32 2.37) | 403* - S - 294 (0.81 1.16 2.53) | 403* - S - 296 (0.84 1.40 2.29) |
| 403* - S - 342 (2.26 1.68 2.01) | 403* - S - 343 (2.79 2.17 1.52) | 404 - S - 150 (2.82 2.50 1.21) | 404 - S - 151 (2.73 2.76 0.95) |
| 404 - S - 153 (2.75 2.60 1.10) | 404 - S - 154 (2.28 1.73 1.98) | 404 - S - 184 (2.57 2.09 1.61) | 404 - S - 189 (1.89 1.09 2.61) |
| 404 - S - 192* (2.18 2.62 1.09) | 404 - S - 193* (1.09 1.78 1.93) | 404 - S - 244* (1.49 2.15 1.56) | 404 - S - 287* (1.07 1.83 1.88) |
| 404 - S - 291 (2.48 2.64 1.07) | 404 - S - 292 (1.81 2.30 1.41) | 404 - S - 294 (1.77 2.14 1.57) | 404 - S - 295 (1.53 2.39 1.32) |
| 404 - S - 296 (1.80 2.38 1.33) | 404 - S - 300 (1.38 1.39 2.32) | 404 - S - 301 (1.43 1.10 2.61) | 404 - S - 302 (1.55 0.94 2.76) |
| 404 - S - 305 (1.69 2.05 1.66) | 404 - S - 331 (1.33 1.63 2.08) | 404 - S - 342 (3.22 2.66 1.05) | 404 - S - 468* (1.03 2.26 1.45) |
| 405* - S - 150 (2.47 1.83 1.55) | 405* - S - 151 (2.38 2.09 1.29) | 405* - S - 152 (3.03 2.38 0.99) | 405* - S - 153 (2.41 1.93 1.45) |
| 405* - S - 154 (1.94 1.06 2.32) | 405* - S - 184 (2.22 1.42 1.96) | 405* - S - 192* (1.83 1.94 1.44) | 405* - S - 206 (0.88 2.30 1.08) |
| 405* - S - 207 (1.40 2.24 1.13) | 405* - S - 244* (1.14 1.47 1.91) | 405* - S - 291 (2.13 1.96 1.41) | 405* - S - 292 (1.47 1.62 1.75) |
| 405* - S - 294 (1.42 1.47 1.91) | 405* - S - 295 (1.18 1.71 1.67) | 405* - S - 296 (1.46 1.71 1.67) | 405* - S - 305 (1.35 1.37 2.01) |
| 405* - S - 331 (0.98 0.95 2.43) | 405* - S - 342 (2.88 1.98 1.39) | 405* - S - 343 (3.41 2.48 0.90) | 406 - S - 150 (2.76 2.41 1.27) |
| 406 - S - 151 (2.66 2.67 1.01) | 406 - S - 153 (2.69 2.51 1.17) | 406 - S - 154 (2.22 1.64 2.04) | 406 - S - 184 (2.51 2.00 1.68) |
| 406 - S - 189 (1.83 1.00 2.68) | 406 - S - 192* (2.11 2.52 1.16) | 406 - S - 193* (1.03 1.68 1.99) | 406 - S - 244* (1.43 2.05 1.63) |
| 406 - S - 287* (1.00 1.74 1.94) | 406 - S - 291 (2.42 2.55 1.13) | 406 - S - 292 (1.75 2.21 1.47) | 406 - S - 294 (1.71 2.05 1.63) |
| 406 - S - 295 (1.46 2.29 1.39) | 406 - S - 296 (1.74 2.29 1.39) | 406 - S - 300 (1.31 1.29 2.39) | 406 - S - 301 (1.37 1.00 2.68) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 406 - S - 302 (1.49 0.85 2.83) | 406 - S - 305 (1.63 1.96 1.72) | 406 - S - 331 (1.26 1.53 2.14) | 406 - S - 342 (3.16 2.57 1.11) |
| 406 - S - 468* (0.97 2.17 1.51) | 407 - S - 150 (2.89 2.45 1.14) | 407 - S - 153 (2.82 2.55 1.04) | 407 - S - 154 (2.35 1.68 1.91) |
| 407 - S - 184 (2.64 2.04 1.54) | 407 - S - 189 (1.96 1.04 2.54) | 407 - S - 192* (2.24 2.56 1.02) | 407 - S - 193* (1.16 1.72 1.86) |
| 407 - S - 244* (1.56 2.09 1.49) | 407 - S - 287* (1.14 1.77 1.81) | 407 - S - 291 (2.55 2.58 1.00) | 407 - S - 292 (1.88 2.24 1.34) |
| 407 - S - 294 (1.84 2.09 1.50) | 407 - S - 295 (1.59 2.33 1.25) | 407 - S - 296 (1.87 2.33 1.26) | 407 - S - 300 (1.45 1.33 2.25) |
| 407 - S - 301 (1.50 1.04 2.54) | 407 - S - 302 (1.62 0.89 2.69) | 407 - S - 305 (1.76 1.99 1.59) | 407 - S - 331 (1.40 1.57 2.01) |
| 407 - S - 342 (3.29 2.60 0.98) | 407 - S - 468* (1.10 2.20 1.38) | 408* - S - 150 (2.57 2.29 1.46) | 408* - S - 151 (2.48 2.54 1.20) |
| 408* - S - 153 (2.50 2.39 1.36) | 408* - S - 154 (2.03 1.52 2.23) | 408* - S - 184 (2.32 1.88 1.86) | 408* - S - 189 (1.64 0.88 2.86) |
| 408* - S - 192* (1.93 2.40 1.34) | 408* - S - 193* (0.84 1.56 2.18) | 408* - S - 206 (0.97 2.76 0.99) | 408* - S - 207 (1.49 2.70 1.04) |
| 408* - S - 244* (1.24 1.93 1.81) | 408* - S - 287* (0.82 1.61 2.13) | 408* - S - 291 (2.23 2.42 1.32) | 408* - S - 292 (1.56 2.08 1.66) |
| 408* - S - 294 (1.52 1.93 1.82) | 408* - S - 295 (1.27 2.17 1.57) | 408* - S - 296 (1.55 2.16 1.58) | 408* - S - 300 (1.13 1.17 2.57) |
| 408* - S - 301 (1.18 0.88 2.86) | 408* - S - 305 (1.44 1.83 1.91) | 408* - S - 331 (1.08 1.41 2.33) | 408* - S - 342 (2.97 2.44 1.30) |
| 409* - S - 150 (2.72 2.27 1.31) | 409* - S - 151 (2.62 2.53 1.05) | 409* - S - 153 (2.65 2.37 1.21) | 409* - S - 154 (2.18 1.50 2.08) |
| 409* - S - 184 (2.47 1.86 1.72) | 409* - S - 189 (1.79 0.86 2.72) | 409* - S - 192* (2.07 2.38 1.20) | 409* - S - 193* (0.99 1.55 2.03) |
| 409* - S - 244* (1.39 1.91 1.67) | 409* - S - 287* (0.96 1.60 1.98) | 409* - S - 291 (2.38 2.41 1.17) | 409* - S - 292 (1.71 2.07 1.51) |
| 409* - S - 294 (1.67 1.91 1.67) | 409* - S - 295 (1.42 2.15 1.43) | 409* - S - 296 (1.70 2.15 1.43) | 409* - S - 300 (1.28 1.15 2.43) |
| 409* - S - 301 (1.33 0.86 2.71) | 409* - S - 305 (1.59 1.82 1.76) | 409* - S - 331 (1.22 1.40 2.18) | 409* - S - 342 (3.12 2.43 1.15) |
| 409* - S - 468* (0.93 2.03 1.55) | 410* - S - 150 (2.82 2.58 1.20) | 410* - S - 151 (2.73 2.84 0.94) | 410* - S - 153 (2.75 2.68 1.10) |
| 410* - S - 154 (2.29 1.81 1.97) | 410* - S - 184 (2.57 2.17 1.61) | 410* - S - 189 (1.89 1.17 2.61) | 410* - S - 192* (2.18 2.69 1.09) |
| 410* - S - 193* (1.10 1.85 1.93) | 410* - S - 244* (1.49 2.22 1.56) | 410* - S - 287* (1.07 1.91 1.87) | 410* - S - 291 (2.48 2.72 1.06) |
| 410* - S - 292 (1.82 2.38 1.41) | 410* - S - 294 (1.77 2.22 1.56) | 410* - S - 295 (1.53 2.46 1.32) | 410* - S - 296 (1.81 2.46 1.32) |
| 410* - S - 300 (1.38 1.46 2.32) | 410* - S - 301 (1.43 1.17 2.61) | 410* - S - 302 (1.55 1.02 2.76) | 410* - S - 305 (1.70 2.12 1.66) |
| 410* - S - 331 (1.33 1.70 2.08) | 410* - S - 342 (3.23 2.74 1.05) | 410* - S - 468* (1.04 2.33 1.45) | 411* - S - 150 (2.89 2.64 1.13) |
| 411* - S - 153 (2.83 2.74 1.03) | 411* - S - 154 (2.36 1.87 1.90) | 411* - S - 184 (2.64 2.24 1.54) | 411* - S - 189 (1.97 1.24 2.54) |
| 411* - S - 192* (2.25 2.76 1.02) | 411* - S - 193* (1.17 1.92 1.86) | 411* - S - 244* (1.56 2.29 1.49) | 411* - S - 287* (1.14 1.97 1.80) |

(fortgesetzt)

| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
|---|---|---|---|
| 411* - S - 291 (2.56 2.78 0.99) | 411* - S - 292 (1.89 2.44 1.33) | 411* - S - 294 (1.85 2.28 1.49) | 411* - S - 295 (1.60 2.53 1.25) |
| 411* - S - 296 (1.88 2.52 1.25) | 411* - S - 300 (1.45 1.53 2.25) | 411* - S - 301 (1.51 1.24 2.54) | 411* - S - 302 (1.63 1.09 2.69) |
| 411* - S - 305 (1.77 2.19 1.58) | 411* - S - 331 (1.40 1.77 2.00) | 411* - S - 342 (3.30 2.80 0.97) | 411* - S - 468* (1.11 2.40 1.37) |
| 412* - S - 150 (2.51 2.41 1.52) | 412* - S - 151 (2.42 2.67 1.26) | 412* - S - 152 (3.06 2.97 0.96) | 412* - S - 153 (2.44 2.51 1.42) |
| 412* - S - 154 (1.97 1.64 2.29) | 412* - S - 184 (2.26 2.00 1.92) | 412* - S - 189 (1.58 1.00 2.92) | 412* - S - 192* (1.86 2.52 1.40) |
| 412* - S - 206 (0.912.88 1.05) | 412* - S - 207 (1.43 2.83 1.10) | 412* - S - 244* (1.18 2.05 1.87) | 412* - S - 291 (2.17 2.55 1.38) |
| 412* - S - 292 (1.50 2.21 1.72) | 412* - S - 294 (1.46 2.05 1.88) | 412* - S - 295 (1.21 2.29 1.63) | 412* - S - 296 (1.49 2.29 1.64) |
| 412* - S - 300 (1.07 1.29 2.63) | 412* - S - 301 (1.12 1.00 2.92) | 412* - S - 302 (1.24 0.85 3.07) | 412* - S - 305 (1.38 1.95 1.97) |
| 412* - S - 331 (1.02 1.53 2.39) | 412* - S - 342 (2.91 2.57 1.36) | 413* - S - 154 (2.77 2.06 1.49) | 413* - S - 184 (3.05 2.42 1.13) |
| 413* - S - 189 (2.37 1.42 2.13) | 413* - S - 193* (1.58 2.10 1.45) | 413* - S - 244* (1.97 2.47 1.08) | 413* - S - 287* (1.55 2.16 1.39) |
| 413* - S - 292 (2.30 2.63 0.92) | 413* - S - 294 (2.26 2.47 1.08) | 413* - S - 300 (1.86 1.71 1.84) | 413* - S - 301 (1.911.42 2.13) |
| 413* - S - 302 (2.04 1.27 2.28) | 413* - S - 305 (2.18 2.37 1.18) | 413* - S - 331 (1.81 1.95 1.60) | 413* - S - 468* (1.52 2.58 0.97) |
| 414* - S - 154 (2.78 2.08 1.48) | 414* - S - 184 (3.07 2.44 1.12) | 414* - S - 189 (2.39 1.44 2.12) | 414* - S - 193* (1.59 2.12 1.43) |
| 414* - S - 244* (1.99 2.49 1.07) | 414* - S - 287* (1.56 2.17 1.38) | 414* - S - 292 (2.31 2.64 0.91) | 414* - S - 294 (2.27 2.49 1.07) |
| 414* - S - 300 (1.88 1.73 1.83) | 414* - S - 301 (1.93 1.44 2.11) | 414* - S - 302 (2.05 1.29 2.27) | 414* - S - 305 (2.19 2.39 1.16) |
| 414* - S - 331 (1.82 1.97 1.58) | 414* - S - 468* (1.53 2.60 0.95) | 415* - S - 150 (1.76 0.89 2.27) | 415* - S - 151 (1.67 1.15 2.01) |
| 415* - S - 152 (2.311.45 1.71) | 415* - S - 153 (1.69 0.99 2.17) | 415* - S - 192* (1.11 1.00 2.15) | 415* - S - 291 (1.42 1.03 2.13) |
| 415* - S - 342 (2.16 1.05 2.11) | 415* - S - 343 (2.69 1.54 1.62) | 416* - S - 150 (2.60 2.00 1.42) | 416* - S - 151 (2.51 2.25 1.17) |
| 416* - S - 153 (2.53 2.10 1.32) | 416* - S - 154 (2.07 1.23 2.19) | 416* - S - 184 (2.35 1.59 1.83) | 416* - S - 192* (1.96 2.111.31) |
| 416* - S - 193* (0.87 1.27 2.15) | 416* - S - 206 (1.01 2.47 0.95) | 416* - S - 207 (1.53 2.41 1.01) | 416* - S - 244* (1.27 1.64 1.78) |
| 416* - S - 287* (0.85 1.32 2.10) | 416* - S - 291 (2.26 2.13 1.29) | 416* - S - 292 (1.60 1.79 1.63) | 416* - S - 294 (1.55 1.64 1.78) |
| 416* - S - 295 (1.31 1.88 1.54) | 416* - S - 296 (1.59 1.87 1.54) | 416* - S - 300 (1.16 0.88 2.54) | 416* - S - 305 (1.48 1.54 1.88) |
| 416* - S - 331 (1.11 1.12 2.30) | 416* - S - 342 (3.00 2.15 1.27) | 416* - S - 468* (0.82 1.75 1.67) | 417* - S - 150 (2.90 2.58 1.13) |
| 417* - S - 153 (2.83 2.68 1.03) | 417* - S - 154 (2.36 1.81 1.90) | 417* - S - 184 (2.65 2.17 1.54) | 417* - S - 189 (1.97 1.17 2.54) |
| 417* - S - 192* (2.25 2.70 1.02) | 417* - S - 193* (1.17 1.86 1.85) | 417* - S - 244* (1.57 2.23 1.49) | 417* - S - 287* (1.14 1.91 1.80) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 417* - S - 291 (2.56 2.72 0.99) | 417* - S - 292 (1.89 2.38 1.33) | 417* - S - 294 (1.85 2.22 1.49) | 417* - S - 295 (1.60 2.47 1.25) |
| 417* - S - 296 (1.88 2.46 1.25) | 417* - S - 300 (1.46 1.46 2.25) | 417* - S - 301 (1.51 1.18 2.53) | 417* - S - 302 (1.63 1.02 2.69) |
| 417* - S - 305 (1.77 2.13 1.58) | 417* - S - 331 (1.40 1.712.00) | 417* - S - 342 (3.30 2.74 0.97) | 417* - S - 468* (1.11 2.34 1.37) |
| 418* - S - 150 (2.86 1.94 1.17) | 418* - S - 151 (2.77 2.20 0.91) | 418* - S - 153 (2.79 2.04 1.07) | 418* - S - 154 (2.32 1.17 1.94) |
| 418* - S - 184 (2.61 1.53 1.57) | 418* - S - 192* (2.21 2.05 1.05) | 418* - S - 193* (1.13 1.21 1.89) | 418* - S - 244* (1.53 1.58 1.52) |
| 418* - S - 287* (1.11 1.27 1.84) | 418* - S - 291 (2.52 2.08 1.03) | 418* - S - 292 (1.85 1.74 1.37) | 418* - S - 294 (1.81 1.58 1.53) |
| 418* - S - 295 (1.56 1.82 1.28) | 418* - S - 296 (1.84 1.82 1.29) | 418* - S - 300 (1.42 0.82 2.28) | 418* - S - 305 (1.73 1.48 1.62) |
| 418* - S - 331 (1.37 1.06 2.04) | 418* - S - 342 (3.26 2.10 1.01) | 418* - S - 468* (1.07 1.69 1.41) | 419* - S - 151 (2.27 1.05 1.40) |
| 419* - S - 152 (2.92 1.35 1.10) | 419* - S - 153 (2.30 0.89 1.56) | 419* - S - 192* (1.72 0.90 1.55) | 419* - S - 207 (1.29 1.21 1.24) |
| 419* - S - 291 (2.03 0.93 1.52) | 419* - S - 342 (2.77 0.95 1.50) | 419* - S - 343 (3.30 1.44 1.01) | 421* - S - 150 (2.11 1.75 1.92) |
| 421* - S - 151 (2.02 2.01 1.66) | 421* - S - 152 (2.66 2.31 1.36) | 421* - S - 153 (2.04 1.85 1.82) | 421* - S - 154 (1.57 0.98 2.69) |
| 421* - S - 184 (1.86 1.34 2.33) | 421* - S - 192* (1.46 1.86 1.80) | 421* - S - 207 (1.03 2.17 1.50) | 421* - S - 291 (1.77 1.89 1.78) |
| 421* - S - 292 (1.10 1.55 2.12) | 421* - S - 294 (1.06 1.39 2.28) | 421* - S - 295 (0.81 1.63 2.03) | 421* - S - 296 (1.09 1.63 2.04) |
| 421* - S - 305 (0.98 1.30 2.37) | 421* - S - 342 (2.51 1.91 1.76) | 421* - S - 343 (3.04 2.40 1.27) | 422* - S - 150 (2.46 1.20 1.57) |
| 422* - S - 151 (2.37 1.46 1.31) | 422* - S - 152 (3.01 1.76 1.01) | 422* - S - 153 (2.39 1.30 1.47) | 422* - S - 192* (1.81 1.31 1.45) |
| 422* - S - 206 (0.86 1.67 1.10) | 422* - S - 207 (1.38 1.62 1.15) | 422* - S - 244* (1.13 0.84 1.92) | 422* - S - 291 (2.12 1.34 1.43) |
| 422* - S - 292 (1.45 1.00 1.77) | 422* - S - 294 (1.41 0.84 1.93) | 422* - S - 295 (1.16 1.08 1.68) | 422* - S - 296 (1.44 1.08 1.69) |
| 422* - S - 342 (2.86 1.36 1.41) | 422* - S - 343 (3.39 1.85 0.92) | 423* - S - 150 (2.48 2.12 1.55) | 423* - S - 151 (2.39 2.38 1.29) |
| 423* - S - 152 (3.03 2.68 0.99) | 423* - S - 153 (2.41 2.23 1.44) | 423* - S - 154 (1.95 1.35 2.32) | 423* - S - 184 (2.23 1.72 1.95) |
| 423* - S - 192* (1.84 2.24 1.43) | 423* - S - 206 (0.89 2.59 1.08) | 423* - S - 207 (1.41 2.54 1.13) | 423* - S - 244* (1.15 1.77 1.90) |
| 423* - S - 291 (2.14 2.26 1.41) | 423* - S - 292 (1.47 1.92 1.75) | 423* - S - 294 (1.43 1.76 1.91) | 423* - S - 295 (1.19 2.01 1.66) |
| 423* - S - 296 (1.46 2.00 1.67) | 423* - S - 300 (1.04 1.01 2.66) | 423* - S - 305 (1.35 1.67 2.00) | 423* - S - 331 (0.99 1.25 2.42) |
| 423* - S - 342 (2.88 2.28 1.39) | 424* - S - 150 (2.56 2.24 1.47) | 424* - S - 151 (2.47 2.50 1.21) | 424* - S - 152 (3.11 2.79 0.91) |
| 424* - S - 153 (2.49 2.34 1.37) | 424* - S - 154 (2.02 1.47 2.24) | 424* - S - 184 (2.31 1.83 1.88) | 424* - S - 189 (1.63 0.83 2.88) |
| 424* - S - 192* (1.91 2.35 1.35) | 424* - S - 193* (0.83 1.51 | 2.19) 424* - S - 206 (0.96 2.71 1.00) | 424* - S - 207 (1.48 2.65 1.05) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 424* - S - 244* (1.23 1.88 1.82) | 424* - S - 287* (0.81 1.57 | 2.14) 424* - S - 291 (2.22 2.38 1.33) | 424* - S - 292 (1.55 2.03 1.67) |
| 424* - S - 294 (1.51 1.88 1.83) | 424* - S - 295 (1.26 2.12 1.58) | 424* - S - 296 (1.54 2.12 1.59) | 424* - S - 300 (1.12 1.12 2.58) |
| 424* - S - 301 (1.17 0.83 2.87) | 424* - S - 305 (1.43 1.78 1.92) | 424* - S - 331 (1.06 1.36 2.34) | 424* - S - 342 (2.96 2.39 1.31) |
| 425 - S - 154 (3.02 0.95 1.24) | 425 - S - 193* (1.83 1.00 1.19) | 425 - S - 287* (1.81 1.05 1.14) | 425 - S - 305 (2.43 1.27 0.92) |
| 425 - S - 331 (2.07 0.85 1.34) | 426* - S - 150 (2.53 1.95 1.49) | 426* - S - 151 (2.44 2.21 1.23) | 426* - S - 152 (3.09 2.51 0.93) |
| 426* - S - 153 (2.47 2.05 1.39) | 426* - S - 154 (2.00 1.18 2.26) | 426* - S - 184 (2.28 1.55 1.90) | 426* - S - 192* (1.89 2.07 1.38) |
| 426* - S - 193* (0.81 1.23 2.22) | 426* - S - 206 (0.94 2.42 1.02) | 426* - S - 207 (1.46 2.37 1.07) | 426* - S - 244* (1.20 1.60 1.85) |
| 426* - S - 291 (2.19 2.09 1.35) | 426* - S - 292 (1.53 1.75 1.69) | 426* - S - 294 (1.49 1.59 1.85) | 426* - S - 295 (1.24 1.84 1.61) |
| 426* - S - 296 (1.52 1.83 1.61) | 426* - S - 300 (1.09 0.84 2.61) | 426* - S - 305 (1.41 1.50 1.95) | 426* - S - 331 (1.04 1.08 2.37) |
| 426* - S - 342 (2.94 2.11 1.33) | 427* - S - 150 (2.85 2.23 1.18) | 427* - S - 151 (2.75 2.49 0.92) | 427* - S - 153 (2.78 2.33 1.08) |
| 427* - S - 154 (2.311.46 1.95) | 427* - S - 184 (2.59 1.83 1.59) | 427* - S - 189 (1.92 0.83 2.59) | 427* - S - 192* (2.20 2.35 1.07) |
| 427* - S - 193* (1.12 1.51 1.90) | 427* - S - 244* (1.51 1.88 1.54) | 427* - S - 287* (1.09 1.56 1.85) | 427* - S - 291 (2.51 2.37 1.04) |
| 427* - S - 292 (1.84 2.03 1.38) | 427* - S - 294 (1.80 1.87 1.54) | 427* - S - 295 (1.55 2.12 1.30) | 427* - S - 296 (1.83 2.11 1.30) |
| 427* - S - 300 (1.40 1.12 2.30) | 427* - S - 301 (1.46 0.83 2.59) | 427* - S - 305 (1.72 1.78 1.63) | 427* - S - 331 (1.35 1.36 2.05) |
| 427* - S - 342 (3.25 2.39 1.02) | 427* - S - 468* (1.06 1.99 1.42) | 428* - S - 154 (2.77 0.96 1.49) | 428* - S - 184 (3.05 1.32 1.13) |
| 428* - S - 193* (1.58 1.00 1.45) | 428* - S - 244* (1.97 1.37 1.08) | 428* - S - 287* (1.55 1.06 1.39) | 428* - S - 292 (2.30 1.52 0.92) |
| 428* - S - 294 (2.26 1.37 1.08) | 428* - S - 305 (2.18 1.27 1.17) | 428* - S - 331 (1.81 0.85 1.60) | 428* - S - 468* (1.52 1.48 0.97) |
| 432 - S - 150 (1.63 1.28 2.39) | 432 - S - 151 (1.54 1.54 2.14) | 432 - S - 152 (2.18 1.84 1.84) | 432 - S - 153 (1.56 1.38 2.29) |
| 432 - S - 184 (1.38 0.87 2.80) | 432 - S - 192* (0.99 1.39 2.28) | 432 - S - 291 (1.29 1.42 2.26) | 432 - S - 342 (2.03 1.44 2.24) |
| 432 - S - 343 (2.56 1.93 1.75) | 433 - S - 150 (1.92 1.27 2.11) | 433 - S - 151 (1.82 1.53 1.85) | 433 - S - 152 (2.47 1.83 1.55) |
| 433 - S - 153 (1.85 1.37 2.01) | 433 - S - 184 (1.67 0.86 2.52) | 433 - S - 192* (1.27 1.38 2.00) | 433 - S - 207 (0.84 1.69 1.69) |
| 433 - S - 291 (1.58 1.41 1.97) | 433 - S - 292 (0.91 1.07 2.31) | 433 - S - 294 (0.87 0.91 2.47) | 433 - S - 296 (0.90 1.15 2.23) |
| 433 - S - 342 (2.32 1.43 1.95) | 433 - S - 343 (2.85 1.92 1.46) | 434* - S - 152 (2.36 1.04 1.66) | 434* - S - 343 (2.74 1.13 1.57) |
| 435* - S - 152 (2.27 0.87 1.75) | 435* - S - 343 (2.65 0.96 1.66) | 436 - S - 150 (2.62 1.59 1.40) | 436 - S - 151 (2.53 1.85 1.15) |
| 436 - S - 153 (2.55 1.69 1.30) | 436 - S - 154 (2.09 0.82 2.18) | 436 - S - 184 (2.37 1.18 1.81) | 436 - S - 192* (1.98 1.70 1.29) |

(fortgesetzt)

| **Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)** | | | |
|---|---|---|---|
| 436 - S - 193* (0.89 0.87 2.13) | 436 - S - 206 (1.03 2.06 0.94) | 436 - S - 207 (1.55 2.01 0.99) | 436 - S - 244* (1.29 1.23 1.76) |
| 436 - S - 287* (0.87 0.92 2.08) | 436 - S - 291 (2.28 1.73 1.27) | 436 - S - 292 (1.61 1.39 1.61) | 436 - S - 294 (1.57 1.23 1.77) |
| 436 - S - 295 (1.33 1.47 1.52) | 436 - S - 296 (1.60 1.47 1.53) | 436 - S - 305 (1.49 1.14 1.86) | 436 - S - 342 (3.02 1.75 1.25) |
| 436 - S - 468* (0.83 1.35 1.65) | 437* - S - 150 (1.54 0.94 2.49) | 437* - S - 151 (1.45 1.19 2.23) | 437* - S - 152 (2.09 1.49 1.93) |
| 437* - S - 153 (1.47 1.04 2.39) | 437* - S - 192* (0.89 1.05 2.38) | 437* - S - 291 (1.20 1.07 2.35) | 437* - S - 342 (1.94 1.09 2.33) |
| 437* - S - 343 (2.47 1.59 1.84) | 441* - S - 343 (2.19 0.87 2.11) | 444* - S - 150 (1.73 1.63 2.29) | 444* - S - 151 (1.64 1.88 2.04) |
| 444* - S - 152 (2.28 2.18 1.74) | 444* - S - 153 (1.66 1.73 2.19) | 444* - S - 154 (1.20 0.86 3.06) | 444* - S - 184 (1.48 1.22 2.70) |
| 444* - S - 192* (1.09 1.74 2.18) | 444* - S - 291 (1.39 1.76 2.16) | 444* - S - 342 (2.13 1.78 2.14) | 444* - S - 343 (2.66 2.28 1.64) |
| 445* - S - 150 (2.05 1.67 1.97) | 445* - S - 151 (1.96 1.92 1.71) | 445* - S - 152 (2.61 2.22 1.41) | 445* - S - 153 (1.99 1.77 1.87) |
| 445* - S - 154 (1.52 0.90 2.74) | 445* - S - 184 (1.80 1.26 2.38) | 445* - S - 192* (1.41 1.78 1.86) | 445* - S - 207 (0.98 2.08 1.55) |
| 445* - S - 291 (1.71 1.80 1.83) | 445* - S - 292 (1.05 1.46 2.17) | 445* - S - 294 (1.01 1.31 2.33) | 445* - S - 296 (1.04 1.54 2.09) |
| 445* - S - 305 (0.93 1.21 2.43) | 445* - S - 342 (2.46 1.82 1.81) | 445* - S - 343 (2.99 2.32 1.32) | 446* - S - 150 (2.05 1.67 1.97) |
| 446* - S - 151 (1.96 1.92 1.71) | 446* - S - 152 (2.61 2.22 1.41) | 446* - S - 153 (1.99 1.77 1.87) | 446* - S - 154 (1.52 0.90 2.74) |
| 446* - S - 184 (1.80 1.26 2.38) | 446* - S - 192* (1.41 1.78 1.86) | 446* - S - 207 (0.98 2.08 1.55) | 446* - S - 291 (1.71 1.80 1.83) |
| 446* - S - 292 (1.05 1.46 2.17) | 446* - S - 294 (1.01 1.31 2.33) | 446* - S - 296 (1.04 1.54 2.09) | 446* - S - 305 (0.93 1.21 2.43) |
| 446* - S - 342 (2.46 1.82 1.81) | 446* - S - 343 (2.99 2.32 1.32) | 448* - S - 150 (2.31 1.28 1.71) | 448* - S - 151 (2.22 1.54 1.46) |
| 448* - S - 152 (2.86 1.84 1.16) | 448* - S - 153 (2.24 1.38 1.61) | 448* - S - 184 (2.06 0.87 2.12) | 448* - S - 192* (1.67 1.39 1.60) |
| 448* - S - 207 (1.24 1.70 1.30) | 448* - S - 244* (0.98 0.92 2.07) | 448* - S - 291 (1.97 1.42 1.58) | 448* - S - 292 (1.31 1.08 1.92) |
| 448* - S - 294 (1.26 0.92 2.07) | 448* - S - 295 (1.02 1.16 1.83) | 448* - S - 296 (1.30 1.16 1.84) | 448* - S - 305 (1.19 0.82 2.17) |
| 448* - S - 342 (2.711.44 1.56) | 448* - S - 343 (3.24 1.93 1.06) | 450* - S - 152 (2.44 0.95 1.58) | 450* - S - 207 (0.81 0.81 1.72) |
| 450* - S - 343 (2.82 1.04 1.49) | 453* - S - 150 (1.62 1.09 2.40) | 453* - S - 151 (1.53 1.35 2.15) | 453* - S - 152 (2.17 1.65 1.85) |
| 453* - S - 153 (1.55 1.19 2.30) | 453* - S - 192* (0.98 1.20 2.29) | 453* - S - 291 (1.28 1.23 2.27) | 453* - S - 342 (2.02 1.25 2.25) |
| 453* - S - 343 (2.55 1.74 1.75) | 454* - S - 150 (1.80 0.93 2.23) | 454* - S - 151 (1.71 1.18 1.97) | 454* - S - 152 (2.35 1.48 1.67) |
| 454* - S - 153 (1.73 1.03 2.13) | 454* - S - 192* (1.15 1.04 2.12) | 454* - S - 291 (1.46 1.06 2.09) | 454* - S - 342 (2.20 1.08 2.07) |
| 454* - S - 343 (2.73 1.58 1.58) | 455* - S - 151 (1.72 0.91 1.95) | 455* - S - 152 (2.37 1.21 1.65) | 455* - S - 342 (2.22 0.81 2.05) |

(fortgesetzt)

| Erfindungsgemäße Moleküle, in denen die chemischen Einheiten aus Tabelle 1 als AF1 (Elektronen-akzeptierend) wirken (nicht erfindungsgemäße Moleküle sind mit einem * markiert) | | | |
|---|---|---|---|
| 455* - S - 343 (2.75 1.31 1.56) | 456* - S - 150 (2.12 0.94 1.91) | 456* - S - 151 (2.03 1.20 1.65) | 456* - S - 152 (2.67 1.50 1.35) |
| 456* - S - 153 (2.05 1.04 1.80) | 456* - S - 192* (1.48 1.06 1.79) | 456* - S - 207 (1.04 1.36 1.49) | 456* - S - 291 (1.78 1.08 1.77) |
| 456* - S - 295 (0.83 0.83 2.02) | 456* - S - 296 (1.10 0.82 2.03) | 456* - S - 342 (2.52 1.10 1.75) | 456* - S - 343 (3.05 1.59 1.26) |
| 458* - S - 152 (2.67 0.91 1.35) | 458* - S - 343 (3.05 1.01 1.25) | 466* - S - 150 (2.29 1.07 1.73) | 466* - S - 151 (2.20 1.33 1.47) |
| 466* - S - 152 (2.84 1.63 1.18) | 466* - S - 153 (2.22 1.17 1.63) | 466* - S - 192* (1.65 1.19 1.62) | 466* - S - 207 (1.22 1.49 1.32) |
| 466* - S - 291 (1.95 1.21 1.60) | 466* - S - 292 (1.29 0.87 1.94) | 466* - S - 295 (1.00 0.96 1.85) | 466* - S - 296 (1.28 0.95 1.85) |
| 466* - S - 342 (2.69 1.23 1.58) | 466* - S - 343 (3.22 1.72 1.08) | 467* - S - 151 (1.89 0.81 1.78) | 467* - S - 152 (2.54 1.11 1.48) |
| 467* - S - 207 (0.91 0.97 1.62) | 467* - S - 343 (2.92 1.20 1.39) | 468* - S - 152 (2.34 0.80 1.68) | 468* - S - 343 (2.72 0.89 1.59) |
| 471* - S - 152 (1.64 1.04 2.38) | 471* - S - 343 (2.02 1.14 2.29) | 475* - S - 150 (2.25 0.86 1.78) | 475* - S - 151 (2.16 1.12 1.52) |
| 475* - S - 152 (2.80 1.42 1.22) | 475* - S - 153 (2.18 0.96 1.68) | 475* - S - 192* (1.60 0.98 1.66) | 475* - S - 207 (1.17 1.28 1.36) |
| 475* - S - 291 (1.91 1.00 1.64) | 475* - S - 342 (2.65 1.02 1.62) | 475* - S - 343 (3.18 1.51 1.13) | 476* - S - 150 (2.57 0.83 1.46) |
| 476* - S - 151 (2.48 1.09 1.20) | 476* - S - 153 (2.50 0.93 1.36) | 476* - S - 192* (1.92 0.95 1.34) | 476* - S - 206 (0.97 1.30 0.99) |
| 476* - S - 207 (1.49 1.25 1.04) | 476* - S - 291 (2.23 0.97 1.32) | 476* - S - 342 (2.97 0.99 1.30) | 477* - S - 154 (2.60 0.84 1.66) |
| 477* - S - 184 (2.89 1.20 1.29) | 477* - S - 193* (1.41 0.88 1.61) | 477* - S - 244* (1.81 1.25 1.24) | 477* - S - 287* (1.39 0.94 1.56) |
| 477* - S - 292 (2.13 1.40 1.09) | 477* - S - 294 (2.09 1.25 1.25) | 477* - S - 295 (1.84 1.49 1.00) | 477* - S - 296 (2.12 1.49 1.01) |
| 477* - S - 305 (2.01 1.15 1.34) | 477* - S - 468* (1.35 1.36 1.13) | 478* - S - 154 (2.67 0.88 1.59) | 478* - S - 184 (2.95 1.24 1.23) |
| 478* - S - 193* (1.48 0.93 1.55) | 478* - S - 244* (1.87 1.29 1.18) | 478* - S - 287* (1.45 0.98 1.49) | 478* - S - 292 (2.20 1.45 1.02) |
| 478* - S - 294 (2.15 1.29 1.18) | 478* - S - 295 (1.91 1.53 0.94) | 478* - S - 296 (2.19 1.53 0.94) | 478* - S - 305 (2.08 1.20 1.28) |
| 478* - S - 468* (1.42 1.41 1.07) | 479* - S - 184 (3.08 1.10 1.10) | 479* - S - 244* (2.00 1.15 1.05) | 479* - S - 287* (1.58 0.84 1.37) |
| 479* - S - 294 (2.28 1.15 1.05) | 479* - S - 305 (2.21 1.06 1.15) | 479* - S - 468* (1.55 1.27 0.94) | 480* - S - 150 (2.17 1.51 1.86) |
| 480* - S - 151 (2.07 1.77 1.60) | 480* - S - 152 (2.72 2.07 1.30) | 480* - S - 153 (2.10 1.61 1.76) | 480* - S - 184 (1.91 1.10 2.27) |
| 480* - S - 192* (1.52 1.62 1.75) | 480* - S - 207 (1.09 1.93 1.44) | 480* - S - 244* (0.84 1.15 2.22) | 480* - S - 291 (1.83 1.65 1.72) |
| 480* - S - 292 (1.16 1.31 2.06) | 480* - S - 294 (1.12 1.15 2.22) | 480* - S - 295 (0.87 1.39 1.98) | 480* - S - 296 (1.15 1.39 1.98) |
| 480* - S - 305 (1.04 1.06 2.31) | 480* - S - 342 (2.57 1.67 1.70) | 480* - S - 343 (3.10 2.16 1.21) | 481 - S - 150 (2.62 2.22 1.41) |
| 481 - S - 151 (2.52 2.48 1.15) | 481 - S - 153 (2.55 2.32 1.31) | 481 - S - 154 (2.08 1.45 2.18) | 481 - S - 184 (2.36 1.811.82) |

(fortgesetzt)

**Erfindungsgemäße Moleküle, in denen die chemischen Einheiten aus Tabelle 1 als AF1 (Elektronen-akzeptierend) wirken (nicht erfindungsgemäße Moleküle sind mit einem * markiert)**

| | | | |
|---|---|---|---|
| 481 - S - 189 (1.69 0.81 2.82) | 481 - S - 192* (1.97 2.33 1.30) | 481 - S - 193* (0.89 1.50 2.13) | 481 - S - 206 (1.02 2.69 0.94) |
| 481 - S - 207 (1.54 2.64 0.99) | 481 - S - 244* (1.29 1.86 1.77) | 481 - S - 287* (0.86 1.55 2.08) | 481 - S - 291 (2.28 2.36 1.27) |
| 481 - S - 292 (1.61 2.02 1.61) | 481 - S - 294 (1.57 1.86 1.77) | 481 - S - 295 (1.32 2.10 1.53) | 481 - S - 296 (1.60 2.10 1.53) |
| 481 - S - 300 (1.17 1.10 2.53) | 481 - S - 301 (1.23 0.81 2.82) | 481 - S - 305 (1.49 1.77 1.86) | 481 - S - 331 (1.12 1.35 2.28) |
| 481 - S - 342 (3.02 2.38 1.25) | 481 - S - 468* (0.83 1.98 1.65) | 482* - S - 152 (2.71 0.93 1.31) | 482* - S - 343 (3.09 1.02 1.22) |
| 483* - S - 152 (2.66 0.86 1.36) | 483* - S - 343 (3.04 0.95 1.27) | 484* - S - 150 (1.85 1.21 2.18) | 484* - S - 151 (1.76 1.46 1.92) |
| 484* - S - 152 (2.40 1.76 1.62) | 484* - S - 153 (1.78 1.31 2.07) | 484* - S - 192* (1.21 1.32 2.06) | 484* - S - 291 (1.51 1.34 2.04) |
| 484* - S - 292 (0.84 1.00 2.38) | 484* - S - 294 (0.80 0.84 2.54) | 484* - S - 296 (0.83 1.08 2.30) | 484* - S - 342 (2.25 1.36 2.02) |
| 484* - S - 343 (2.78 1.85 1.53) | 485* - S - 150 (3.01 1.88 1.01) | 485* - S - 153 (2.95 1.98 0.91) | 485* - S - 154 (2.48 1.11 1.78) |
| 485* - S - 184 (2.76 1.47 1.42) | 485* - S - 193* (1.29 1.15 1.74) | 485* - S - 244* (1.68 1.52 1.37) | 485* - S - 287* (1.26 1.21 1.68) |
| 485* - S - 292 (2.01 1.68 1.21) | 485* - S - 294 (1.97 1.52 1.37) | 485* - S - 295 (1.72 1.76 1.13) | 485* - S - 296 (2.00 1.76 1.13) |
| 485* - S - 305 (1.89 1.43 1.46) | 485* - S - 331 (1.52 1.00 1.89) | 485* - S - 468* (1.23 1.64 1.25) | 486* - S - 150 (0.91 0.95 3.12) |
| 486* - S - 151 (0.82 1.21 2.86) | 486* - S - 152 (1.46 1.51 2.56) | 486* - S - 153 (0.84 1.05 3.02) | 486* - S - 342 (1.31 1.11 2.96) |
| 486* - S - 343 (1.84 1.60 2.47) | | | |

Insbesondere die Verwendung der erfindungsgemäßen chemischen Einheiten AF1 und AF2 unterscheidet die organischen Moleküle funktionell von Molekülen gemäß dem Stand der Technik. Durch die enthaltenen Anknüpfungsmuster stehen die beiden pi-Systeme von AF1 und AF2 durch Verdrillung nicht in Konjugation, sind aber dennoch in enger räumlicher Nähe fixiert.

In einer Ausführungsform unterscheidet insbesondere der Separator S die organischen Moleküle funktionell von Molekülen gemäß dem Stand der Technik, da die hier gezeigte Art der Trennung von AFs (oder Donoren und Akzeptoren) bisher noch nicht bekannt ist. Separatoren dienen der Unterbrechung der elektronischen Kommunikation zwischen den chemischen Einheiten AF1 und AF2 durch eine derartige Verknüpfung der Einheiten, dass die Grenzorbitale HOMO und LUMO auf mehrheitlich separaten Molekülteilen liegen, was ohne den Separator nicht zwingend der Fall sein muss.

Separatoren im Sinne dieser Erfindung verändern die Lage des HOMO bzw. LUMO der AFs nicht signifikant. Als nicht signifikant gilt im Rahmen dieser Erfindung eine Veränderung von nicht mehr als +/- 0,4 eV. Die Berechnung derartiger Energien ist bekannt und funktioniert nach der oben beschriebenen Weise per DFT-Rechnung.

Anhand spektroskopischer Auswahlregeln (symmetrische Moleküle) oder durch Messung des Extinktionskoeffizienten (UV/VIS-Spektroskopie) oder anhand quantenchemischer Berechnung der Oszillatorstärke kann vorhergesagt werden, ob ein quantenmechanischer Übergang erlaubt ist. Je größer die Oszillatorstärke, desto eher ist ein Übergang erlaubt und desto schneller ist der damit verbundene Prozess (Abklingdauer). Angestrebt sind Abklingdauern von < 300 μs, insbesondere < 100 μs, oder von < 50 μs. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert.

In einer Ausführungsform erfüllen die Separatoren S die zwei folgenden funktionellen Merkmale (s. Figur 2):

- die HOMO-Energie ist niedriger als die HOMO-Energie der als Donor fungierenden chemischen Einheit AF und

- die LUMO-Energie ist höher als die LUMO-Energie der als Akzeptor fungierenden chemischen Einheit AF.

In einer Ausführungsform der Beschreibung werden an die chemisch substituierbaren Positionen der so erhaltenen organischen Moleküle weitere Reste R angefügt, um die Löslichkeit der Emitter zu steigern und/oder die Polymerisierbarkeit zu ermöglichen ohne dabei die elektronischen Eigenschaften des Moleküls signifikant zu verändern, sodass auch bei Verwendung von R ein Emitter vorliegt, wobei

jedes R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$,$-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $AS(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein mono- oder polycyclisches, aliphatisches, aromatisches und/oder benzoannelliertes Ringsystem bilden. In einer Ausführungsform ist das Ringsystem, das gebildet werden kann, auf ein monozyklisches aliphatisches Ringsystem mit insgesamt fünf oder sechs Ringgliedern beschränkt.

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $AS(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S-ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden.

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden.

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $AS(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden.

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden.

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch-$Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden.

$R^7$ ist bei jedem Auftreten unabhängig voneinander ausgewählt aus der Gruppe bestehend aus Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden.

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden.

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein

können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem.

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 7 und 8 erhalten werden, die als Polymere in der lichtemittierenden Schicht des optoelektronischen Bauelements Verwendung finden können.

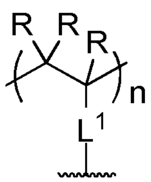
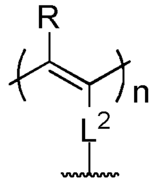

**Formel 7** **Formel 8**

In Formel 7 und 8 stehen L1 und L2 für gleiche oder verschiedene Linkergruppen, die 0 bis 20, insbesondere 1 bis 15, oder 2 bis 10 Kohlenstoffatome aufweist, und wobei die gewellte Linie die Position kennzeichnet, über die die Linkergruppe an das organische Molekül der Formel 1 angebunden ist. In einer Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, insbesondere eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form - C(=O)O- auf.

Weitere beschriebene Ausführungsformen der Wiederholungseinheiten sind Strukturen der Formeln 9 bis 14:

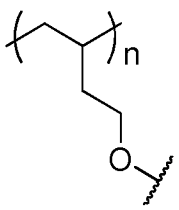
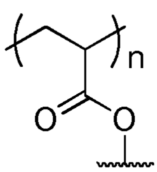
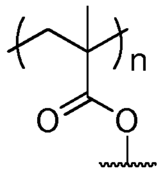
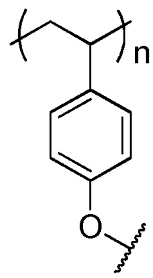
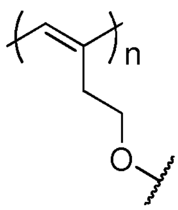
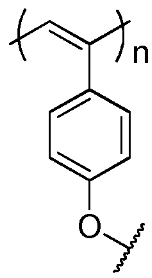

**Formel 9** **Formel 10** **Formel 11** **Formel 12** **Formel 13** **Formel 14**

Zur Herstellung der Polymere, die die Wiederholungseinheiten gemäß Formel 9 bis 14 aufweisen, werden die polymerisierbaren funktionellen Einheiten über eine Linkergruppe der Formeln 15 bis 20, die eine Hydroxyleinheit aufweisen, an ein organisches Molekül der Formel 1 angebunden und die daraus resultierenden Verbindungen mit sich selbst homopolymerisiert oder mit anderen geeigneten Monomeren copolymerisiert.

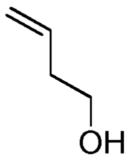 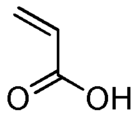 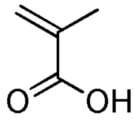 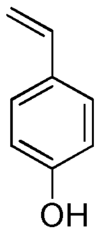 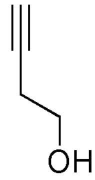 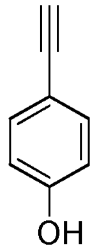

**Formel 15** **Formel 16** **Formel 17** **Formel 18** **Formel 19** **Formel 20**

Polymere, die eine Einheit gemäß Formel 7 oder Formel 8 aufweisen, können dabei entweder ausschließlich Wiederholungseinheiten mit einer Struktur der allgemeinen Formel 7 oder 8, oder Wiederholungseinheiten mit einer anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organischen Moleküle, welche

- einen $\Delta E(S_1\text{-}T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweisen und/oder
- eine Emissionslebensdauer von höchstens 50 $\mu$s aufweisen.

Die Erfindung betrifft in einem Aspekt die Verwendung eines erfindungsgemäßen organischen Moleküls als lumineszierender Emitter und/oder als Hostmaterial und/oder als Elektronentransportmaterial und/oder als Lochinjektionsmaterial und/oder als Lochblockiermaterial in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden
- organischen Solarzellen,
- organischen Transistoren,
- organischen Feldeffekttransistoren,
- organischen Lasern und
- Down-Konversions-Elementen.

Der Anteil des erfindungsgemäßen organischen Moleküls am lumineszierenden Emitter und/oder Hostmaterial und/oder Elektronentransportmaterial und/oder Lochinjektionsmaterial und/oder Lochblockiermaterial beträgt in einer Ausführungsform 1 % bis 99 % (Gew%), insbesondere beträgt der Anteil am Emitter in optischen Licht emittierenden Bauelementen, insbesondere in OLEDs, zwischen 5 % und 80 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine Ausführungsform betrifft das erfindungsgemäße optoelektronische Bauelement aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein erfindungsgemäßes organisches Molekül enthält.

In einer weiteren Ausführungsform des Bauelements wird das organische Molekül als Emissionsmaterial in einer Emissionsschicht eingesetzt, wobei sie in Kombination mit mindestens einem Hostmaterial oder insbesondere als Reinschicht eingesetzt werden kann. In einer Ausführungsform beträgt dabei der Anteil des organischen Moleküls als Emissionsmaterial in einer Emissionsschicht in optischen Licht-emittierenden Bauelementen, insbesondere in OLEDs,

zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement neben dem erfindungsgemäßen organischen Molekül mindestens ein Hostmaterial auf, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls, und wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform weist das optoelektronische Bauelement ein Substrat, eine Anode, eine Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einer weiteren Ausführungsform weist das optoelektronische Bauelement mindestens ein Hostmaterial aus einem Material gemäß Formel 1 auf.

In einer weiteren Ausführungsform des optoelektronischen Bauelements beinhaltet die lichtemittierende Schicht fluoreszente oder phosphoreszente Materialien, welche eine Struktur der Formel 1 aufweisen.

In einer weiteren Ausführungsform des optoelektronischen Bauelements bilden ein organisches Molekül gemäß Formel 1 und ein funktionelles Material, beispielsweise in Form eines weiteren Emitter-Materials, eines Host-Materials, oder eines weiteren organisches Moleküls, welches zur Bildung eines Exciplex mit dem Molekül gemäß Formel 1 befähigt ist, einen Exciplex. Funktionelle Materialien sind beispielsweise Hostmaterialien wie MCP, Elektronentransportmaterialien wie TPBI und Lochtransportmaterialien wie NPD oder MTDATA. Exciplexe sind Addukte aus elektronisch angeregten Molekülen und solchen im elektronischen Grundzustand, die zur Lichtemission fähig sind.

In einer weiteren Ausführungsform des optoelektronischen Bauelements ist die Emission durch thermisch aktivierte verzögerte Fluoreszenz (TADF) charakterisiert.

In einer weiteren Ausführungsform des optoelektronischen Bauelements werden organische Moleküle gemäß Formel 1 als Ladungstransportschicht verwendet.

Die Erfindung betrifft in einem Aspekt ein lichtemittierendes Material, aufweisend ein erfindungsgemäßes organisches Molekül und ein Hostmaterial, wobei die Triplett ($T_1$)- und Singulett ($S_1$)-Energieniveaus des Hostmaterials energetisch höher liegen als die Triplett ($T_1$)- und Singulett ($S_1$)-Energieniveaus des organischen Moleküls, und wobei das organische Molekül Fluoreszenz oder thermisch aktivierte verzögerte Fluoreszenz (TADF) emittiert, und einen $\Delta E(S_1\text{-}T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweist.

Ein Aspekt der Erfindung betrifft ein Verfahren zur Herstellung eines optoelektronischen Bauelements aufweisend ein erfindungsgemäßes organisches Molekül. In einer Ausführungsform weist das Verfahren den Schritt der Verarbeitung des organischen Moleküls mittels eines Vakuumverdampfungsverfahrens oder aus einer Lösung auf.

In einer Ausführungsform weist das Verfahren das Aufbringen des organischen Moleküls auf einen Träger auf, wobei das Aufbringen insbesondere nass-chemisch, mittels kolloidaler Suspension oder mittels Sublimation erfolgt.

In einer weiteren Ausführungsform des Verfahrens wird mindestens eine Schicht

- mit einem Sublimationsverfahren beschichtet
- mit einem OVPD (Organic Vapor Phase Deposition) Verfahren beschichtet
- mit Hilfe einer Trägergassublimation beschichtet oder

- aus Lösung oder mit einem beliebigen Druckverfahren hergestellt.

Ein Aspekt der Erfindung betrifft ein Verfahren zur Veränderung der Emissions- und/oder Absorptionseigenschaften eines elektronischen Bauelements, wobei ein erfindungsgemäßes organisches Molekül in ein Matrixmaterial zur Leitung von Elektronen oder Löchern in einem optoelektronischen Bauelement eingebracht wird.
Die Erfindung betrifft zudem in einem weiteren Aspekt die Verwendung eines erfindungsgemäßen Moleküls zur Umwandlung von UV-Strahlung oder von blauem Licht in sichtbares Licht, insbesondere in grünes, gelbes oder rotes Licht (Down-Konversion), insbesondere in einem optoelektronischen Bauelement der hier beschriebenen Art.
In einem weiteren Aspekt betrifft die Erfindung eine Anwendung, in der mindestens ein Material mit einer Struktur gemäß Formel 1, durch äußere energetische Anregung zum Leuchten angeregt wird. Die äußere Anregung kann elektronisch oder optisch oder radioaktiv sein.

**Beispiele**

**Beispiel 1: DFT-Berechnung**

Tabelle 5 enthält DFT-Berechnungen sowie beispielhafte erfindungsgemäße organische Moleküle
Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D. , J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F. ; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4-Grid zur numerischen Integration verwendet. Alle DFT-Rechnungen wurden mit dem Turbomole-Programmpaket (Version 6.5) (TURBOMOLE V6.4 2012, University of Karlsruhe and Forschungszentrum Karlsruhe GmbH, 1989-2007, TURBOMOLE GmbH, since 2007; http://www.turbomole.com) durchgeführt.
Die Grenzorbitale in der Figur 3 wurden mit dem B3LYP-Funktional für die optimierten Grundzustandsgeometrien berechnet.

**Beispiel 2: PL Messungen**

**Photophysikalische Messungen**

*Vorbehandlung von optischen Gläsern*

Alle Gläser (Küvetten und Substrate aus Quarzglas, Durchmesser: 1cm) wurden nach jeder Benutzung gereinigt: Je dreimaliges Spülen mit Dichlormethan, Aceton, Ethanol, demineralisiertem Wasser, Einlegen in 5% Hellmanex-Lösung für 24h, gründliches Ausspülen mit demineralisiertem Wasser. Zum Trocknen wurden die optischen Gläser mit Stickstoff abgeblasen.

***Probenvorbereitung: Lösungen***

1-2 mg der Probe wurden in 100 ml des jeweiligen Lösemittels gelöst, Konzentration $10^{-5}$ mol/L. Die Küvette wurde luftdicht verschlossen und 10 min. entgast.
***Probenvorbereitung, Film: Spin-Coating*** (Gerät: Spin150, SPS euro.)
Die Probenkonzentration entsprach 10mg/ml, angesetzt in Toluol oder Chlorbenzol.
Programm: 1) 3 s bei 400 U/min; 2) 20 Sek. bei 1000 U/min bei 1000 Upm/ s. 3) 10s bei 4000 U/min bei 1000 Upm/ s. Die Filme wurden nach dem Beschichten für 1 min bei 70 °C an Luft auf einer Präzisionsheizplatte von LHG getrocknet.

***Absorptionsspektroskopie***

Lösungen: UV-VIS-Spektren wurden auf einem Gerät der Firma Thermo Scientific, Modell Evolution 201 aufgenommen. (Siehe Probenvorbereitung: Lösungen)

Film: UV-VIS-Spektren wurden auf einem Gerät der Firma Thermo Scientific, Modell Evolution 201 aufgenommen. (Siehe Probenvorbereitung, Film: Spin-Coating)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen: NanoLED 370 (Wellenlänge: 371 nm, Pulsdauer: 1.1 ns), NanoLED 290 (Wellenlänge: 294 nm, Pulsdauer: <1 ns), SpectraLED 310 (Wellenlänge: 314nm), SpectraLED 355 (Wellenlänge: 355nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: Gemessenen Größe.

***Quanteneffizienzbestimmung***

Die Messung der Photolumineszenzquantenausbeute (PLQY) erfolgte mittels eines *Absolute PL Quantum Yield Measurement* C9920-03G-Systems der Firma *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 $\mu$m) verbunden ist. Die Auswertung der Quanteneffizienz und der CIE-Koordinaten erfolgte mit Hilfe der Software U6039-05 Version 3.6.0

Für G9920-OXG (PMA-12). Das Emissionsmaximum wird in nm, die Quantenausbeute $\Phi$ in % und die CIE-Farbkoordinaten als x,y-Werte angegeben.

PLQY wurde für Polymerfilme, Lösungen und Pulverproben nach folgendem Protokoll bestimmt:

1) Durchführung der Qualitätssicherung: Als Refernzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung: Es wurde von entgasten Lösungen und Filmen unter Stickstoff-Atmosphäre die absolute Quantenausbeute bestimmt. Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Beispiel 3: Synthese von erfindungsgemäßen Molekülen (Vorschrift 1)**

Zahlreiche erfindungsgemäße Beispiele können unter Buchwald-Hartwig-Amninierungsbedigungen synthetisiert werden.

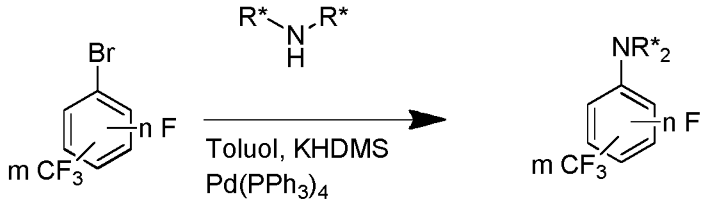


Dabei werden ein kommerzieller Bromid- oder Iodid-Vorläufer (z. B. erhältlich bei Sigma-Aldrich oder Fluorochem, 1 Äquivalent) unter Schutzgas in trockenem Toluol gelöst (50 mL pro g Edukt). Es werden 1,2 Äquivalente des kommerziellen Aminvorläufers, 1,5 Äquivalente Kaliumdisilylhexamethylamid der Natriumhydrid oder Natrium-alkoholat als Base zugegeben. Im Stickstoffgegenstrom wurde Tetrakis-triphenylpalladium (10 mol-%) oder PdCl2(dppf) zugegeben und für 24 Stunden unter Rückfluss erhitzt.

Die Reaktionsmischung wurde mit Wasser (10 mL) gequencht, über Kieselgel filtriert und anschließend mittels MPLC aufgereinigt. Das Rohprodukt wurde mittels Sublimation bei 250 °C unter 10E-5 mbar Druck aufgereinigt und das aufgereinigte Produkt danach als Feststoff erhalten.

Mit dieser Methode lassen sich auch Mehrfachsubstitutionen durchführen in diesem Fall werden entweder Diamino-Verbindungen wie Bicarbazol oder Dibromide eingesetzt. In diesem Fall werden nur 0,5 Äquivalente des difunktionalen Startmaterials verwendet.

**Beispiel 4: Synthese von erfindungsgemäßen Molekülen (Vorschrift 2)**

Weiter erfindungsgemäße Beispiele können durch nukleophile aromatische Substituion hergestellt werden. Als kommerziell verfügbare Ausgangsstoffe dienen dabei Arylfluoride, die entweder weitere Fluor oder CF3-Gruppen enthalten (obere Reihe) oder einen weiter funktionalisierbaren Rest wie I oder Br tragen, wodurch Folgeumsetzungen mögliche sind (Reihe 2). Beispielhaft soll hier die Suzuki-Kreuzkupplung genannt werden.

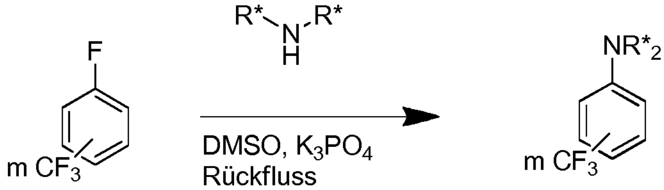


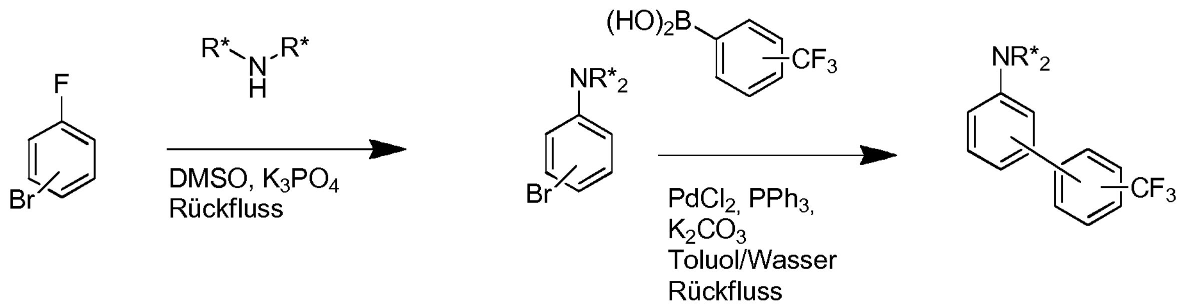


Dabei werden ein kommerzieller Fluor-Vorläufer (z.B. erhältlich bei Sigma-Aldrich oder Fluorochem, 1 Äquivalent) unter Schutzgas in trockenem DMSO gelöst (50 mL pro 2g Edukt). Es werden 1,2 Äquivalente des kommerziellen Aminvorläufers und 1,5 Äquivalente Kaliumphosphat als Base zugegeben. Es wird für 24 Stunden unter Rückfluss erhitzt.

Die Reaktionsmischung wurde in Wasser gegeben und der entstandene Niederschlag abgetrennt, mit Wasser gewaschen und getrocknet. Die Rohware wurde entweder aufgereinigt oder weiter umgesetzt (Reihe 2). Eine weitere Aufreinigung erfolgte durch MPLC mit Cyclohexan/Essigester als Laufmittel. Das Rohprodukt wurde mittels Sublimation bei 250 °C unter 10E-5 mbar Druck aufgereinigt und das aufgereinigte Produkt danach als Feststoff erhalten.

Mit dieser Methode lassen sich auch Mehrfachsubstitutionen durchführen in diesem Fall werden entweder Diamino-Verbindungen wie Bicarbazol oder Dibromide eingesetzt. In diesem Fall werden nur 0,5 Äquivalente des difunktionalen Startmaterials verwendet.

**Beispiel 5: Synthese von erfindungsgemäßen Molekülen (Vorschrift 3)**

Eine weitere Syntheseroute zu erfindungsgemäßen Molekülen stellt die BHAS-Methode dar, die dem Fachmann bekannt ist (S. Lindner et al., Journal of Fluorine Chemistry 2015, 179, 102-105)

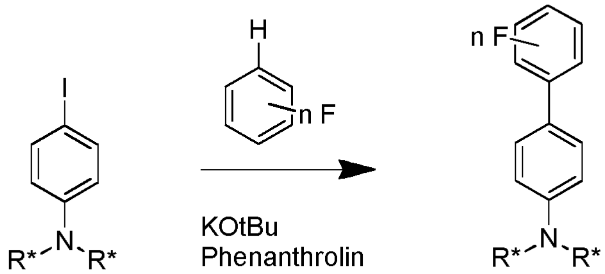


Ein Aryliodid (1 Äquivalent) wird in 20-fachem Überschuss eines Fluoraromaten gelöst. Unter Schutzgas wird 1 Äquivalent KOtBu sowie 50 mol-% Phenanthrolin zugegeben und am Rückfluss gerührt.

Nach 48 Stunden wurde das Lösungsmittel durch Destillation entfernt. Die Rohware wurde in Essigester aufgenommen, über Kieselgel filtriert und anschließend mittels MPLC aufgereinigt. Das Rohprodukt wurde mittels Sublimation bei 250 °C unter 10E-5 mbar Druck aufgereinigt und das aufgereinigte Produkt danach als Feststoff erhalten.

Mit dieser Methode lassen sich auch Mehrfachsubstitutionen durchführen in diesem Fall werden Di- oder Triodide eingesetzt. In diesem Fall werden nur 0,5 bzw. 0,33 Äquivalente des di- oder trifunktionalen Startmaterials verwendet.

**Beispiel 6 bis Beispiel 13: Synthese von erfindungsgemäßen Molekülen (Vorschrift 1)**

Die gezeigten Verbindungen wurden nach den in Beispiel 3, 4 und 5 geschilderten Methoden als weiße bis hellgelb gefärbte Feststoffe hergestellt. Die Ausbeuten der Synthesen betrugen dabei 10 bis 80% bezogen auf die letzte Synthesestufe. Die chemische Charakterisierung erfolgt dabei über NMR Spektroskopie in Deuterochloroform als Lösungsmittel (1H NMR, 13C NMR sowie 19F NMR). Ferner wurden Massenspektroskopie (LC-MC, FAB-MS) zur Identitätsbestätigung und HPLC (THF-Wasser, DAD zur Peakdetektion) verwendet. Die Reinheit der Emitterbetrug nach der Aufreinigung mindestens 97% (HPLC).

Alle gezeigten Moleküle wurden nach der in Beispiel 1 geschilderten Methode quantenchemisch berechnet (siehe Tabelle 5). Die angegebenen Photophysik-Daten wurden nach den in Beispiel 2 geschilderten Methoden gemessen. Dabei wurden Dünnfilme in Polymethylmethacrylat (PMMA) zu 10 Gewichts-% Dotierkonzentration vermessen.

| Beispiel | Struktur | $\lambda_{max}$ [nm] | $CIE_x$ | $CIE_y$ | PLQY [%] |
|---|---|---|---|---|---|
| | | 10% Emitterkonzentration in PMMA | | | |
| 7 | 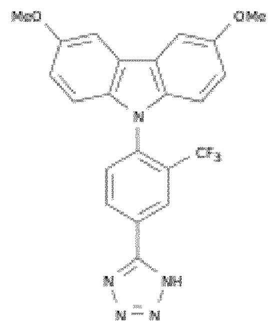 | 466 | 0.19 | 0.25 | 26 |

(fortgesetzt)

| Beispiel | Struktur | $\lambda_{max}$ [nm] | $CIE_x$ | $CIE_y$ | PLQY [%] |
|---|---|---|---|---|---|
| 8 | 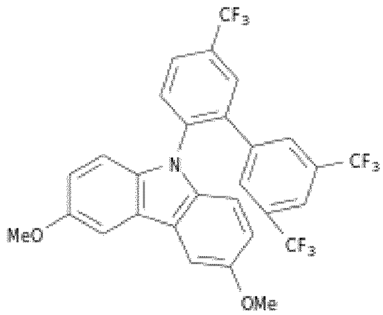 | 458 | 0.17 | 0.18 | 43 |
| 9 | 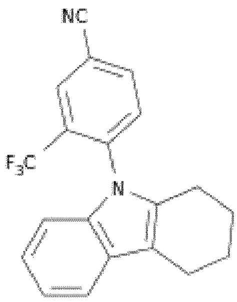 | 473 | 0.20 | 0.27 | 17 |
| 10 | 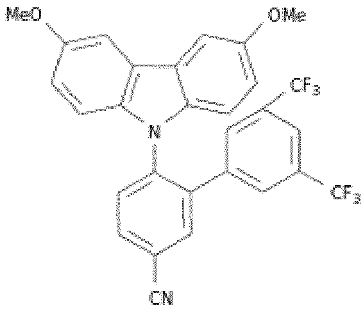 | 467 | 0.17 | 0.22 | 66 |
| 11 | 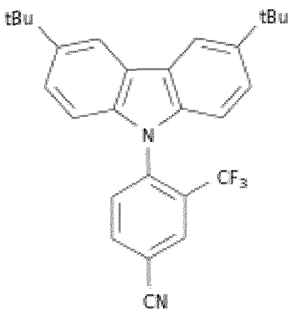 | 445 | 0.16 | 0.14 | 67 |
| 12 | 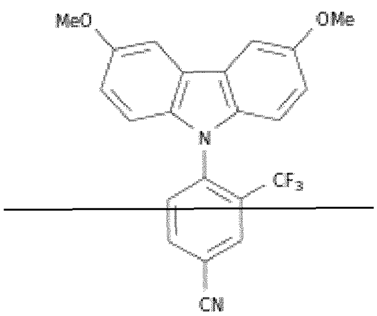 | 485 | 0.21 | 0.34 | 44 |

## Figuren

Es zeigen

Figur 1: Schematische Darstellung des Aufbaus einer organischen lichtemittierenden Diode (OLED).
Figur 2: Schematische Darstellung des Energieniveaudiagrammes (relative Energie in eV) eines erfindungsgemäßen Emittermoleküls, aufweisend einen Separator (Lichtemission resultiert aus dem Übergang LUMO AF1 hin zu HOMO AF2).
Figur 3: Schematische Darstellung des Energieniveaudiagrammes (relative Energie in eV) eines erfindungsgemäßen Emittermoleküls, aufweisend keinen Separator (Lichtemission resultiert aus dem Übergang LUMO AF1 hin zu HOMO AF2).
Figur 4: Berechnete Grenzorbitale erfindungsgemäßer Verbindungen.
Figur 5: Berechnete Grenzorbitale nicht erfindungsgemäßer Verbindungen.

## Patentansprüche

1. Organisches Molekül, aufweisend eine Struktur der Formel 1

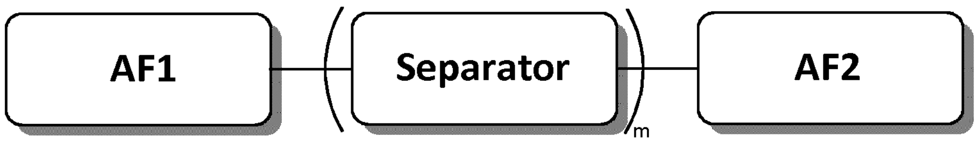


Formel 1

wobei gilt:

m ist 0 oder 1;
AF1 ist eine erste chemische Einheit, aufweisend ein konjugiertes System;
AF2 ist eine zweite chemische Einheit, aufweisend ein konjugiertes System;
AF1 ≠ AF2;
wobei AF1 eine Struktur der Formel 2-2 aufweist:

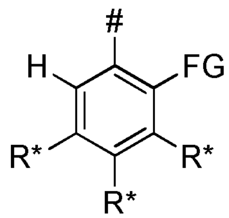

Formel 2-2

wobei

FG ausgewählt ist aus der Gruppe bestehend aus $CR^{**}{}_2F$, $CF_3$, $CF_2R^{**}$, $SF_5$, N-$(CF_3)_1$, N-$(CF_3)_2$, O-$CF_3$, S-$CF_3$ und $CF_2CO_2R^*$, und einer Gruppe E*, die eine Aryl- oder Heteroarylgruppe mit 6 bis 18 aromatischen Ringatomen ist, die mit einem oder mehreren Resten $R^8$ substituiert sein kann, und die als Bestandteile des aromatischen Rings eine oder mehrere Gruppen V* enthält, wobei die Gruppen V* bei jedem Auftreten gleich oder verschieden gewählt sind aus =C(F)- und =C($CF_3$)-;
# = Anknüpfungspunkt an den Separator oder Anknüpfungspunkt an die chemische Einheit AF2;
R** ist bei jedem Auftreten gleich oder verschieden H, eine lineare Alkyl-, Alkenyl-, Alkinylgruppe mit 3 bis 40 C-Atomen, oder eine lineare Alkenyl- oder Alkinylgruppe mit 2 bis 40 C-Atomen oder eine verzweigte oder cyclische Alkyl-, Alkenyl-, Alkinylgruppe mit 3 bis 40 C-Atomen, die optional partiell fluoriert oder perfluoriert ist;
und wobei AF2 eine Struktur der Formel 3 aufweist:

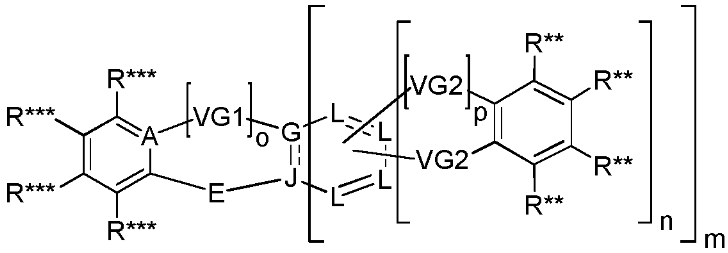

**Formel 3**

wobei gilt:

m ist 0 oder 1;
n ist 0 oder 1, wobei bei m = 0 immer auch n = 0 ist;
o ist 0 oder 1;
p ist 0 oder 1;
A ist CR*** wenn o = 0, ansonsten C;
VG1 = verbrückende Gruppe, ist ausgewählt aus der Gruppe bestehend aus

- NR**, $CR^{**}_2$, O, S und einer C-C-Einfachbindung; oder
- NR**, $CR^{**}_2$, O, S, einer C-C-Einfachbindung, BR**, AsR**, $SiR^{**}_2$, $GeR^{**}_2$,

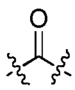, 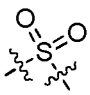, 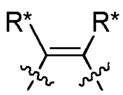, 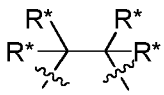, 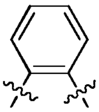, 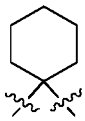, 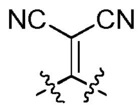, 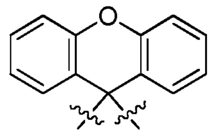,

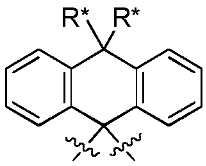, 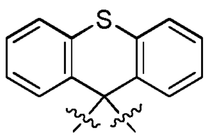, und 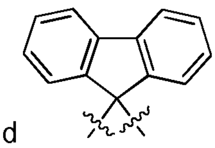,

wenn m = 1 und gleichzeitig n = 0;

VG2 = verbrückende Gruppe ist bei jedem Auftreten unabhängig voneinander ausgewählt aus der Gruppe bestehend aus $CR^{**}_2$, NR**, O, S und einer C-C-Einfachbindung, wobei nicht zwei Einheiten VG2 gleichzeitig gleich einer C-C-Einfachbindung sind;
E ist NR**,

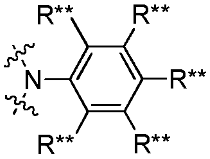,

O oder S;
G ist C wenn o = 1 und gleichzeitig m = 1; G ist CR** wenn o = 0 und gleichzeitig m = 1; G ist CR** oder $CR^{**}_2$, wenn o = 1 und gleichzeitig m = 0; G ist R* wenn o = 0 und gleichzeitig m = 0; G ist CR**, $CR^{**}_2$, N oder NR* wenn m = 0 und gleichzeitig VG1 eine C-C-Einfachbindung ist;
J ist C wenn m = 1; J ist CR**, $CR^{**}_2$ oder NR** wenn m = 0;
L ist CR*** wenn n = 0; L ist CR** oder C (bei kovalenter Bindung zu VG2 und/oder zu M) wenn n = 1;
R*** ist R** oder ist ausgewählt aus den folgenden Einheiten, wobei maximal zwei der Reste

R*** gleichzeitig gleich einer der folgenden Einheiten sind:

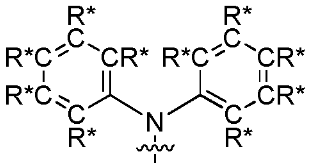
,
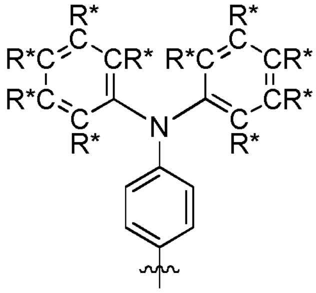
,
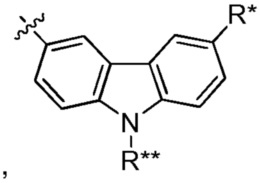
,
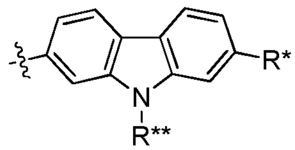

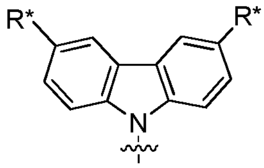
,
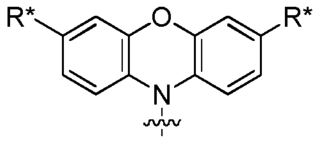
,
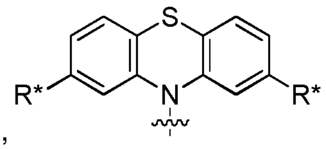
,

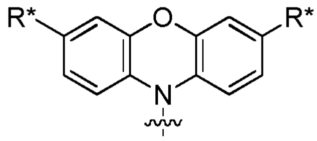
,
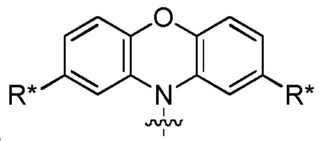
;

R** ist bei jedem Auftreten unabhängig voneinander ein Rest R* oder markiert eine Anknüpfungsstelle an einen Separator S oder die chemische Einheit AF1 wobei genau ein R** eine Anknüpfungsstelle an einen Separator S oder an die chemische Einheit AF1 ist;
und wobei der Separator S eine Struktur der Formel 4S aufweist

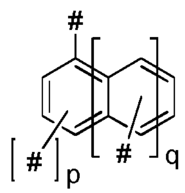

**Formel 4S**

wobei

# die Anknüpfungspositionen für die chemischen Einheiten AF1 und AF2 kennzeichnet;
p = 0 oder 1;
q = 0 oder 1;
und wobei p $\neq$ q;
Separator ist eine chemische Einheit, die AF1 und AF2 verknüpft und wobei der Separator an das konjugierte System von AF1 und an das konjugierte System von AF2 angebunden ist;
und wobei für die Formel 1, die Formel 2-2 und die Formel 3 gilt:

R* ist bei jedem Auftreten unabhängig voneinander ausgewählt aus der Gruppe bestehend aus H, Deuterium, Phenyl, Naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $AS(=O)(R^7)_2$, $P(=S)(R^7)_2$, $AS(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Grup-

pe durch -$Si(R^4)_2$-, -$Ge(R^4)_2$-, -$Sn(R^4)_2$, -C(=O)-, -C(=S)-, -C(=Se)-, -C=N-, -C(=O)O-, -C(=O)N($R^3$)-, -P(=O)($R^7$)-, -As(=O)($R^7$)-, -P(=S)($R^7$), -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R* auch miteinander ein mono- oder polycyclisches, aliphatisches, aromatisches und/oder benzoannelliertes Ringsystem bilden;

$R^2$ ist bei jedem Auftreten unabhängig voneinander ausgewählt aus der Gruppe bestehend aus H, Deuterium, Phenyl, Naphthyl, $CF_3$, C(=O)O$R^3$, C(=O)N$(R^2)_2$, $Si(R^4)_3$, C(=O)$R^3$, P(=O)$(R^7)_2$, As(=O)$(R^7)_2$ P(=S)$(R^7)_2$, AS(=S)$(R^7)_2$, S(=O)$R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, oder eine benachbarte $CH_2$-Gruppe durch -$Si(R^4)_2$-,-$Ge(R^4)_2$-, -$Sn(R^4)_2$, -C(=O)-, -C(=S)-, -C(=Se)-, -C=N-, -C(=O)O-, -C(=O)N($R^3$)-, -P(=O)($R^7$)-,-As(=O)($R^7$)-, -P(=S)($R^7$)-, -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, N$(R^2)_2$, CN, $CF_3$, OH, C(=O)O$R^3$, C(=O)N$(R^3)_2$, C(=O)$R^3$, P(=O)$(R^7)_2$, As(=O)$(R^7)_2$, P(=S)$(R^7)_2$, As(=S)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, oder eine benachbarte $CH_2$-Gruppe durch -$Si(R^4)_2$-, -$Ge(R^4)_2$-, -$Sn(R^4)_2$, -C(=O)-,-C(=S)-, -C(=Se)-, -C=N-, -C(=O)O-, -C(=O)N($R^3$)-, -P(=O)($R^7$)-, -As(=O)($R^7$)-, -P(=S)($R^7$),-As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, C(=O)$R^3$, P(=O)$(R^7)_2$, As(=O)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, oder eine benachbarte $CH_2$-Gruppe durch -$Si(R^4)_2$-, -$Ge(R^4)_2$-, -$Sn(R^4)_2$, -C(=O)-,-C(=S)-, -C(=Se)-, -C=N-, -C(=O)O-, -C(=O)N($R^3$)-, -P(=O)($R^7$)-, -As(=O)($R^7$)-, -P(=S)($R^7$)-,-As(=S)($R^7$)-, -S(=O)-, -$S(=O)_2$-, -$NR^2$-, -O-, oder -S- ersetzt sein können und wobei ein oder

mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch-$Si(R^4)_2$-, -$Ge(R^4)_2$-, -$Sn(R^4)_2$, -C(=O)-,-C(=S)-, -C(=Se)-, -C=N-, -C(=O)O-, -C(=O)N($R^3$)-, -P(=O)($R^7$)-, -As(=O)($R^7$)-, -P(=S)($R^7$),-As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch -$Si(R^4)_2$-,-$Ge(R^4)_2$-, -$Sn(R^4)_2$, -C(=O)-, -C(=S)-, -C(=Se)-, -C=N-, -C(=O)O-, -C(=O)N($R^3$)-, -P(=O)($R^7$)-,-As(=O)($R^7$)-, -P(=S)($R^7$), -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C≡C-, oder eine benachbarte $CH_2$-Gruppe durch -$Si(R^4)_2$-, -$Ge(R^4)_2$-, -$Sn(R^4)_2$, -C(=O)-, -C(=S)-, -C(=Se)-,-C=N-, -C(=O)O-, -C(=O)N($R^3$)-, -P(=O)($R^7$)-, -As(=O)($R^7$)-, -P(=S)($R^7$), -As(=S)($R^7$)-, -S(=O)-,-$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy-

oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden.

**2.** Organisches Molekül nach Anspruch 1, wobei AF1 ausgewählt ist aus einer der folgenden

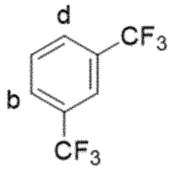
150

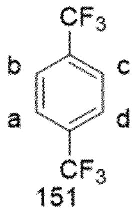
151

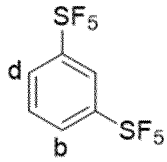
342

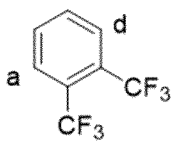
153

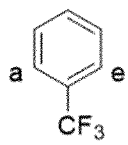
154

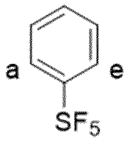
184

Strukturen: wobei die möglichen Anknüpfungspunkte an den Separator oder an die chemische Einheit AF2 mit Kleinbuchstaben a bis e bezeichnet sind.

**3.** Organisches Molekül nach Anspruch 1 bis 2, wobei AF2 eine Struktur der Formel 4 aufweist:

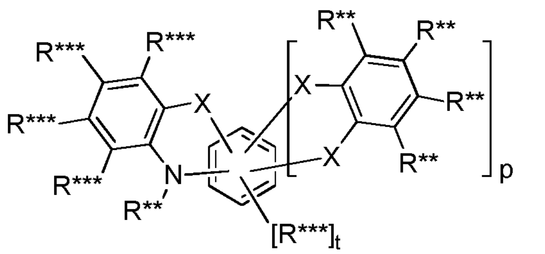

**Formel 4**

wobei gilt

p ist 0 oder 1;
t = 4 - 2p;
X ist $CR^{**}_2$, $NR^{**}$, Sauerstoff, Schwefel oder eine direkte Bindung, wobei maximal zwei Platzhalter X gleichzeitig eine direkte Bindung sind, wobei diese nicht Bestandteil desselben Rings sind;
und im Übrigen die in Anspruch 1 angegebenen Definitionen gelten.

**4.** Organisches Molekül nach Anspruch 1 bis 2, wobei AF2 ausgewählt ist einer der folgenden Strukturen;

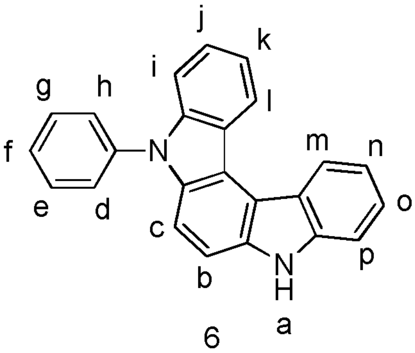

6

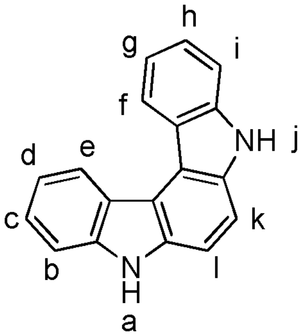

7

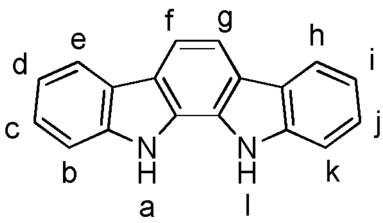

8

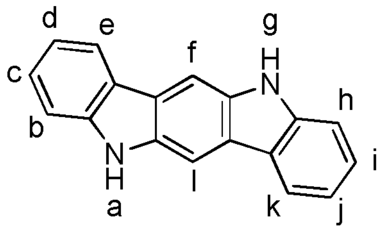

10

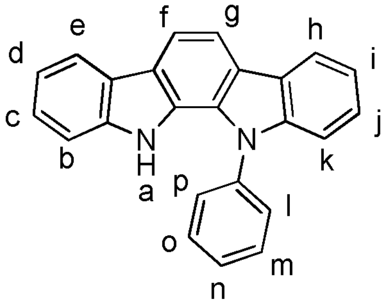

21

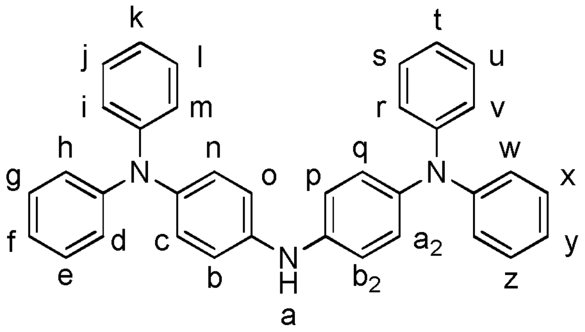

22

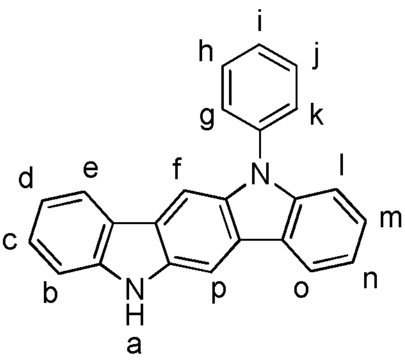

24

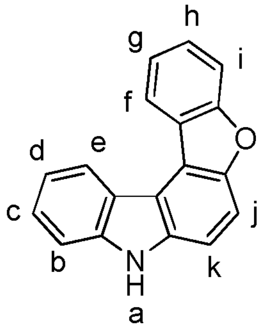

31

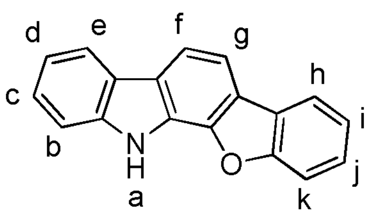

32

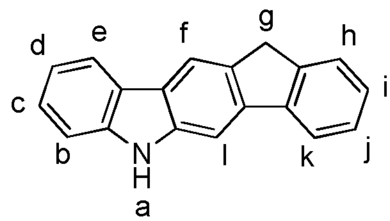

33

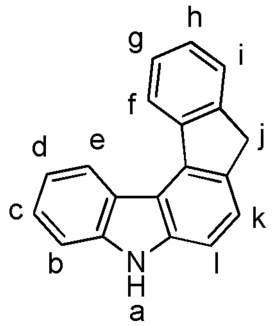

34

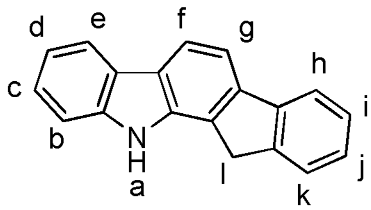

35

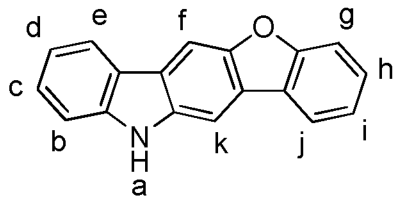

30

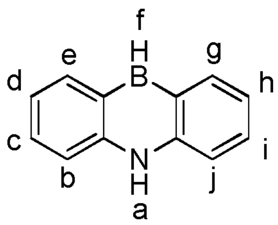

38

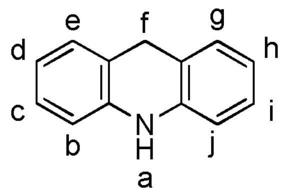

39

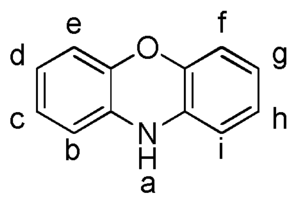

41

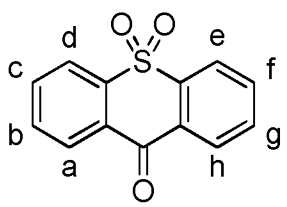

43

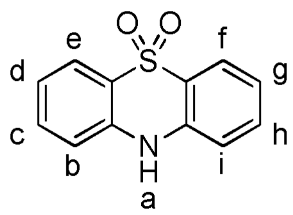

44

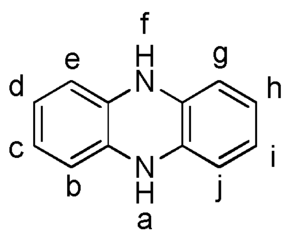

49

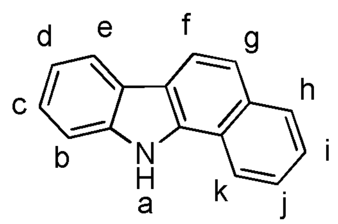

54

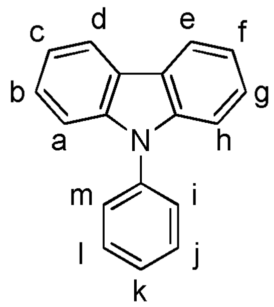

55

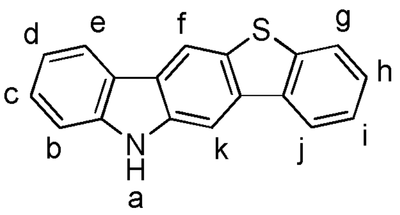

58

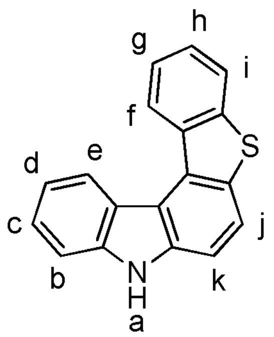

59

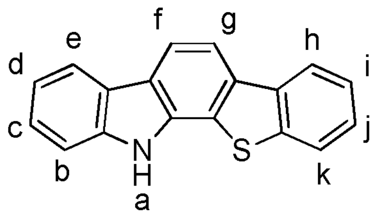

60

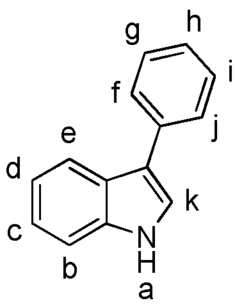

63

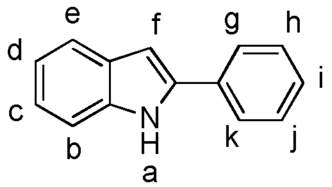

64

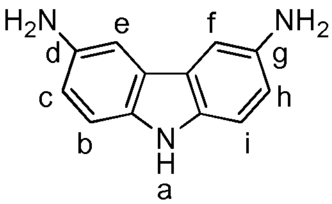

66

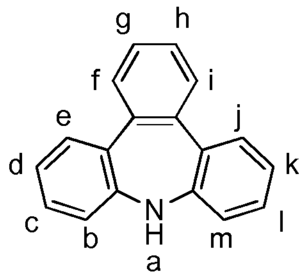

71

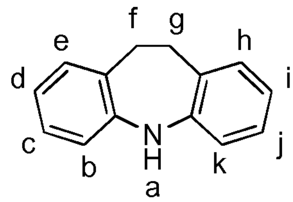

72

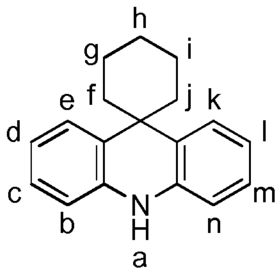

73

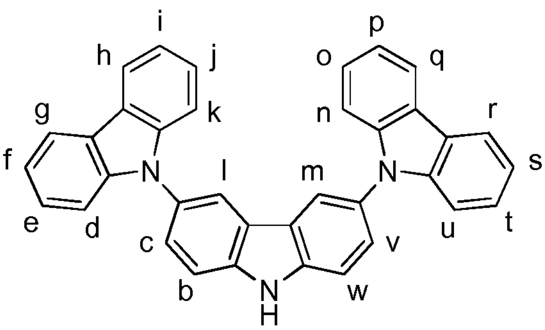

74

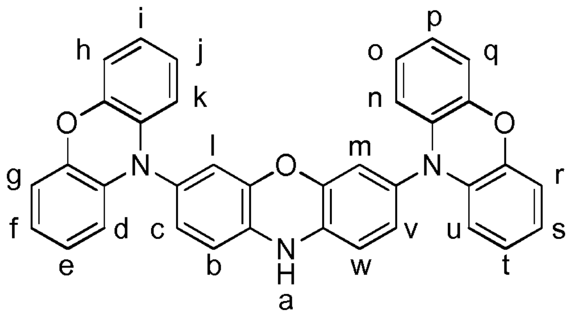

75

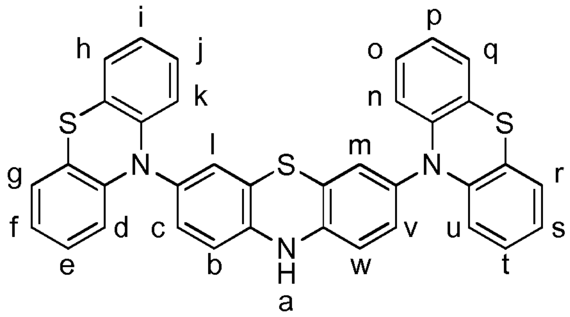

76

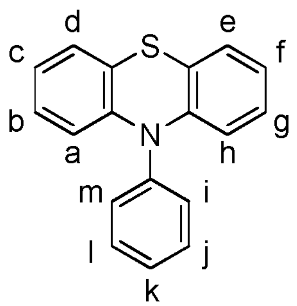

77

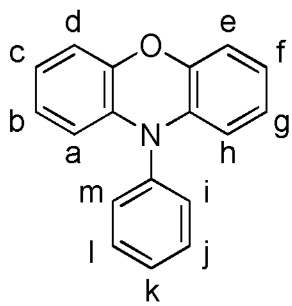

78

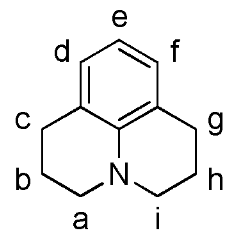

91

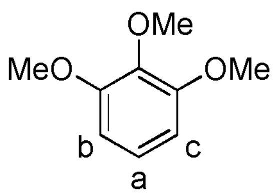

120

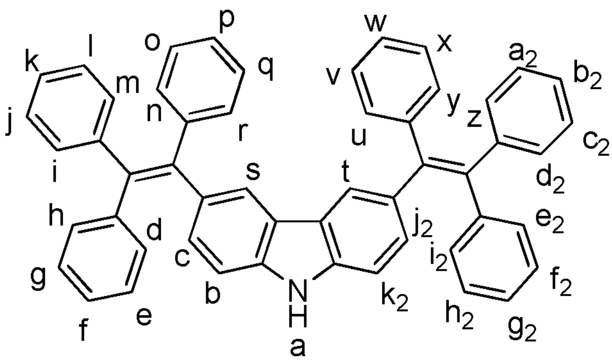

124

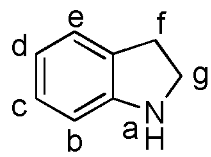

128

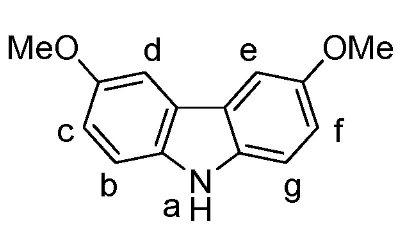

199

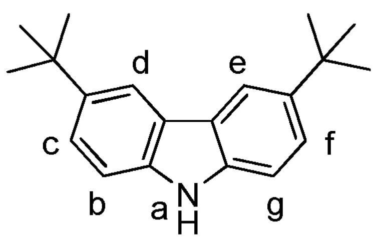

200

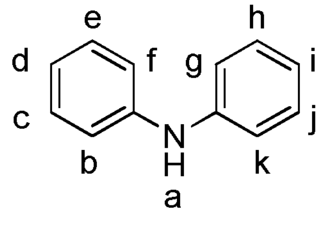

223

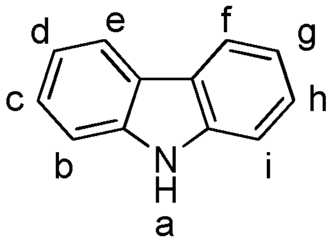

328

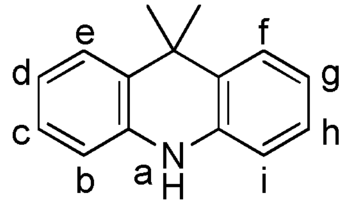

337

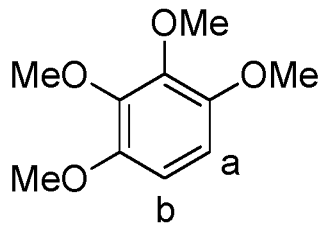

344

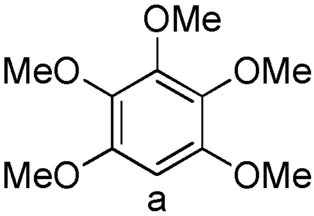

345

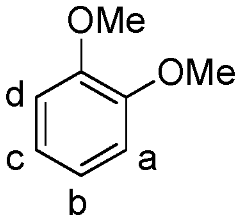

346

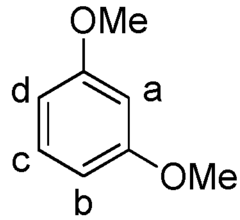

347

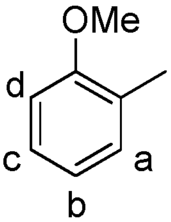

349

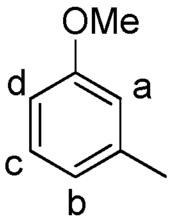

350

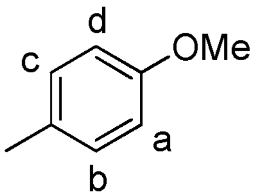

351

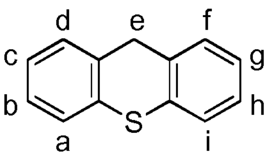

366

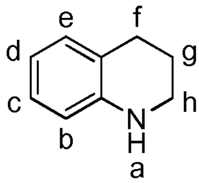

383

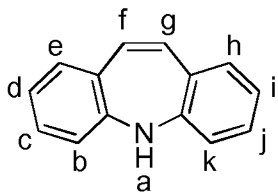

400

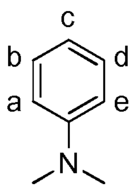
404

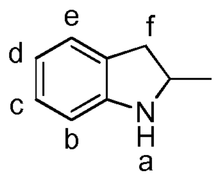
406

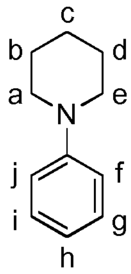
407

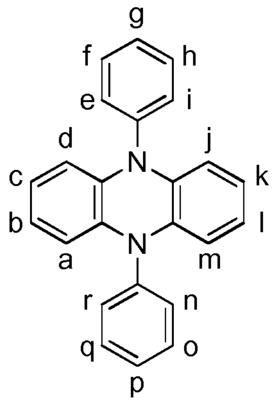
425

430

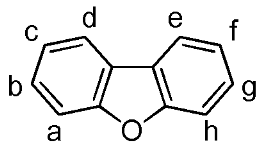
432

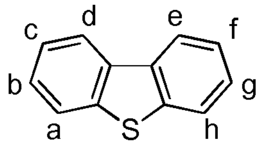
433

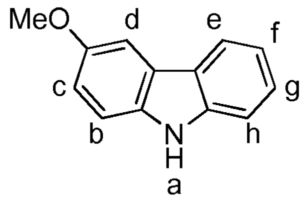
436

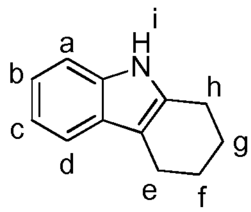
481

**5.** Organisches Molekül nach einem der Ansprüche 1 bis 4, wobei der Separator ausgewählt ist aus der Gruppe der folgenden Strukturen:

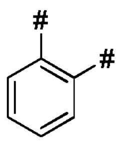
S-1

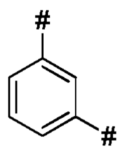
S-2

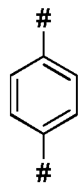
S-3

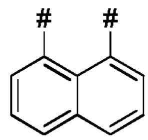
S-4

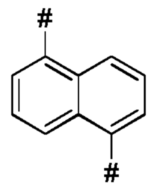
S-5

wobei # die Anknüpfungspositionen für die chemischen Einheiten AF1 und AF2 darstellen.

**6.** Verwendung eines organischen Moleküls nach einem oder mehreren der Ansprüche 1 bis 5 als lumineszierender Emitter und/oder als Hostmaterial und/oder als Elektronentransportmaterial und/oder als Lochinjektionsmaterial und/oder als Lochblockiermaterial in einem optoelektronischen Bauelement.

**7.** Verwendung nach Anspruch 6, wobei das optoelektronische Bauelement ausgewählt ist aus der Gruppe bestehend aus:

- organischen lichtemittierenden Dioden (OLEDs),
- Licht-emittierenden elektrochemischen Zellen,
- OLED-Sensoren, insbesondere in nicht hermetisch nach außen abgeschirmten Gas- und Dampf-Sensoren,
- organischen Dioden
- organischen Solarzellen,
- organischer Transistor
- organischen Feldeffekttransistoren,
- organischen Lasern und
- Down-Konversions-Elementen.

**8.** Verwendung nach Anspruch 6 oder 7, wobei der Anteil des organischen Moleküls an dem Emitter 1 % bis 99 % beträgt.

**9.** Verwendung nach Anspruch 6, 7 oder 8, wobei die Konzentration des organischen Moleküls als Emitter in optischen Licht emittierenden Bauelementen, insbesondere in OLEDs, zwischen 5 % und 80 % beträgt.

**10.** Optoelektronisches Bauelement, aufweisend ein organisches Molekül nach einem oder mehreren der Ansprüche 1 bis 5, insbesondere ausgeformt als ein Bauelement ausgewählt aus der Gruppe bestehend aus organischer lichtemittierender Diode (OLED), Lichtemittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**11.** Optoelektronisches Bauelement nach Anspruch 10, aufweisend

- ein Substrat,
- eine Anode und
- eine Kathode, wobei die Anode und die Kathode auf das Substrat aufgebracht sind, und
- mindestens eine lichtemittierende Schicht, die zwischen Anode und Kathode angeordnet ist und die ein organisches Molekül nach einem oder mehreren der Ansprüche 1 bis 5 enthält.

**12.** Optoelektronisches Bauelement nach Anspruch 10 oder 11, in dem die lichtemittierende Schicht mindestens ein Hostmaterial aufweist, wobei der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls einem oder mehreren der Ansprüche 1 bis 5.

**13.** Optoelektronisches Bauelement nach Anspruch 10 bis 12, aufweisend mindestens ein Hostmaterial bestehend aus einem Material gemäß einem oder mehreren der Ansprüche 1 bis 5.

**14.** Optoelektronisches Bauelement nach Anspruch 10 bis 13, wobei die lichtemittierende Schicht fluoreszente oder phosphoreszente Materialien beinhaltet und wobei es sich bei den Materialien der lichtemittierenden Schicht um organische Moleküle nach Anspruch 1 bis 5 handelt.

**15.** Optoelektronisches Bauelement nach Anspruch 10 bis 14, in dem ein organisches Molekül gemäß einem oder mehreren der Ansprüche 1 bis 5 zusammen mit einem funktionellen Material einen Exciplex bilden.

**16.** Optoelektronisches Bauelement nach Anspruch 10 bis 15, in dem ein organisches Molekül nach einem oder mehreren der Ansprüche 1 bis 5 als Ladungstransportschicht verwendet wird.

**17.** Verfahren zur Herstellung eines optoelektronischen Bauelements, wobei ein organisches Molekül nach einem oder mehreren der Ansprüche 1 bis 5 verwendet wird.

**18.** Verfahren nach Anspruch 17, wobei die Verarbeitung des organischen Moleküls mittels eines Vakuumverdampfungsverfahrens oder aus einer Lösung erfolgt.

## Claims

**1.** Organic molecule, comprising a structure of formula 1

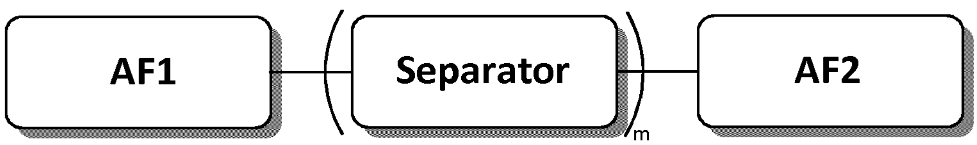


**Formula 1**

wherein:

m is 0 or 1;
AF1 is a first chemical unit, comprising a conjugated system;

AF2 is a second chemical unit, comprising a conjugated system;
AF1 ≠ AF2;
wherein AF1 comprises a structure of formula 2-2:

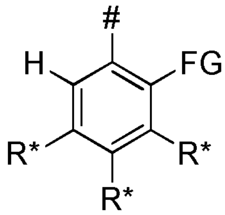

**Formula 2-2**

wherein

FG is selected from the group consisting of $CR^{**}{}_2F$, $CF_3$, $CF_2R^{**}$, $SF_5$, $N\text{-}(CF_3)_1$, $N\text{-}(CF_3)_2$, $O\text{-}CF_3$, $S\text{-}CF_3$ and $CF_2CO_2R^*$, and a group E*, which is an aryl or heteroaryl group with 6 to 18 aromatic ring atoms, which may be substituted with one or more moieties $R^8$, and which as a constituent of the aromatic ring contains one or more groups V*, wherein the groups V* are the same or different in each instance and are selected from =C(F)- and $=C(CF_3)$-;
# = attachment point to the separator or attachment point to the chemical unit AF2;
R** is the same or different at each occurrence and is H, a linear alkyl, alkenyl, alkynyl group with 3 to 40 carbon atoms, or a linear alkenyl or alkynyl group with 2 to 40 carbon atoms or a branched or cyclic alkyl, alkenyl, alkynyl group with 3 to 40 carbon atoms, which is optionally partially fluorinated or perfluorinated;
and wherein AF2 comprises a structure of formula 3:

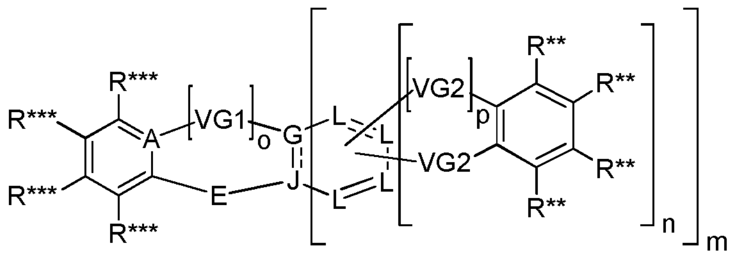

**Formula 3**

wherein:

m is 0 or 1;
n is 0 or 1, wherein n = 0 if m = 0;
o is 0 or 1;
p is 0 or 1;
A is CR*** when o = 0, otherwise C;
VG1 = bridging group selected from the group consisting of

- NR**, $CR^{**}{}_2$, O, S and a C-C single bond; or
- NR**, $CR^{**}{}_2$, O, S, a C-C single bond, BR**, AsR**, $SiR^{**}{}_2$, $GeR^{**}{}_2$,

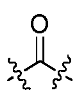 , 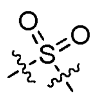 , 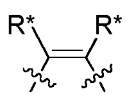 , 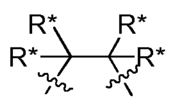 , 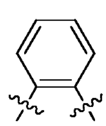 , 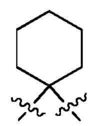 , 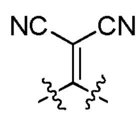 , 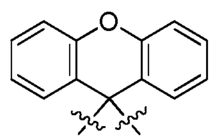 ,

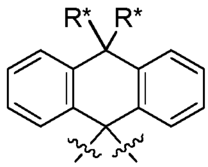, 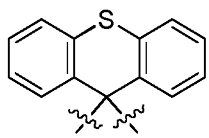, and 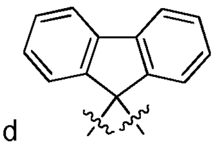,

when m = 1 and also n = 0;

VG2 = bridging group is in each instance, independently of one another, selected from the group consisting of $CR^{**}_2$, $NR^{**}$, O, S and a C-C single bond, wherein two units VG2 are not both a C-C single bond;
E is $NR^{**}$,

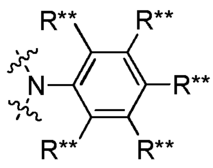,

O or S;
G is C when o = 1 and also m = 1; G is $CR^{**}$ when o = 0 and also m = 1; G is $CR^{**}$ or $CR^{**}_2$, when o = 1 and also m = 0; G is $R^*$ when o = 0 and also m = 0; G is $CR^{**}$, $CR^{**}_2$, N or $NR^*$ when m = 0 and also VG1 is a C-C single bond;
J is C when m = 1; J is $CR^{**}$, $CR^{**}_2$ or $NR^{**}$ when m = 0;
L is $CR^{***}$ when n = 0; L is $CR^{**}$ or C (in the case of covalent bonding to VG2 and/or to M) when n = 1;
$R^{***}$ is $R^{**}$ or is selected from the following units, wherein at the most two of the moieties $R^{***}$ are both equal to one of the following units:

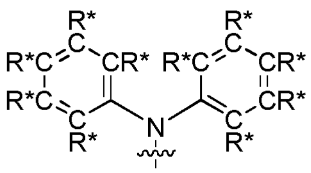, 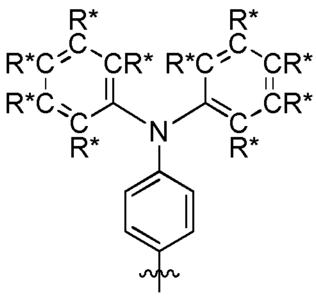, 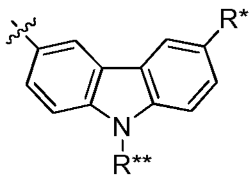, 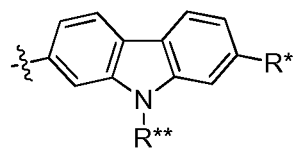

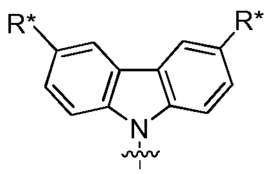, 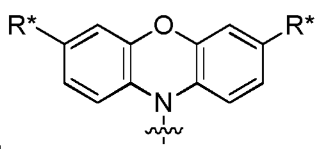, 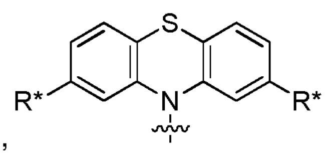,

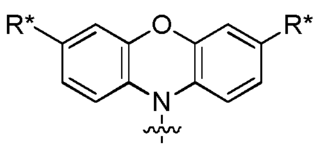, 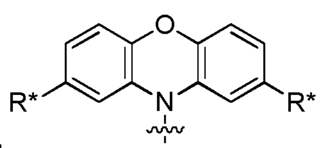;

$R^{**}$ in each instance, independently of one another, is a moiety $R^*$ or marks an attachment point to a separator S or to the chemical unit AF1 wherein exactly one $R^{**}$ is an attachment point to a separator S or to the chemical unit AF1;
and wherein the separator S comprises a structure of formula 4S

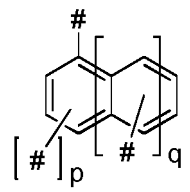

**Formula 4S**

wherein

# marks the attachment point for the chemical units AF1 and AF2;
p = 0 or 1;
q = 0 or 1;
and wherein p ≠ q;
Separator is a chemical unit that links AF1 and AF2 and wherein the separator is linked to the conjugated system of AF1 and to the conjugated system of AF2;
and wherein the following is applicable for formula 1, formula 2-2, and formula 3:

R* in each instance, independently of one another, is selected from the group consisting of H, deuterium, phenyl, naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S{=}NR^3$, $S(=O)NR^3$, $S(=O)_2NR^3$, $S(=O)_2R^3$, $O{-}S(=O)_2R^3$, $SF_5$, a linear alkyl, alkoxy or thioalkoxy group with 1 to 40 carbon atoms or a linear alkenyl or alkynyl group with 2 to 40 carbon atoms or a branched or cyclic alkyl, alkenyl, alkynyl, alkoxy or thioalkoxy group with 3 to 40 carbon atoms, which in each case may be substituted with one or more moieties $R^9$, wherein one or more adjacent $CH_2$ groups may be replaced by $-R^9C{=}CR^9-$, -C≡C-, or one adjacent $CH_2$-group may be replaced by $-Si(R^4)_2-$, $-Ge(R^4)_2-$,$-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more moieties $R^2$, or an aryloxy or heteroaryloxy group with 5 to 60 aromatic ring atoms, which may be substituted with one or more moieties $R^9$, or a diarylamino group, diheteroarylamino group or arylheteroarylamino group with 10 to 40 aromatic ring atoms, which may be substituted with one or more moieties $R^9$, or a combination of these systems; two or more of these substituents R* may also form a mono- or polycyclic, aliphatic, aromatic and/or benzoanellated ring system;

$R^2$ in each instance, independently of one another, is selected from the group consisting of H, deuterium, phenyl, naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, a linear alkyl, alkoxy or thioalkoxy group with 1 to 40 carbon atoms or a linear alkenyl or alkynyl group with 2 to 40 carbon atoms or a branched or cyclic alkyl, alkenyl, alkynyl, alkoxy or thioalkoxy group with 3 to 40 carbon atoms, each of which may be substituted with one or more radicals $R^9$, wherein one or more adjacent $CH_2$ groups may be replaced by $-R^9C{=}CR^9-$, -C≡C-, or one adjacent $CH_2$-group may be replaced by $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$,-O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more radicals $R^9$, or an aryloxy or heteroaryloxy group with 5 to 60 aromatic ring atoms, which may be substituted with one or more radicals $R^9$, or a diarylamino group, diheteroarylamino group or arylheteroarylamino group with 10 to 40 aromatic ring atoms, which may be substituted with one or more radicals $R^9$, or a combination of these systems; here two or more of these substituents $R^2$ may also form with one another a mono- or polycyclic, aliphatic, aromatic and/or benzo-fused ring system;

$R^3$ in each instance, independently of one another, is selected from the group consisting of H, deuterium, phenyl, naphthyl, $CF_3$ or an aliphatic, aromatic and/or heteroaromatic hydrocarbon moiety with 1 to 20 carbon atoms, in which also one or more H atoms may be replaced by F or $CF_3$; here also two or more substituents $R^3$ may also form a mono- or polycyclic, aliphatic ring system with one another;

$R^4$ in each instance, independently of one another, is selected from the group consisting of H, deuterium, phenyl, naphthyl, $N(R^2)_2$, CN, $CF_3$, OH, $C(=O)OR^3$, $C(=O)N(R^3)_2$, $C(=O)R^3$, $P(=O)(R^7)_2$, $AS(=O)(R^7)_2$, $P(=S)(R^7)_2$, $As(=S)(R^7)_2$, a linear alkyl, alkoxy or thioalkoxy group with 1 to 40 carbon atoms or a linear

alkenyl or alkynyl group with 2 to 40 carbon atoms or a branched or cyclic alkyl, alkenyl, alkynyl, alkoxy or thioalkoxy group with 3 to 40 carbon atoms, which in each case may be substituted with one or more moieties $R^9$, wherein one or more adjacent $CH_2$ groups may be replaced by $-R^9C=CR^9-$, -C≡C-, or one adjacent $CH_2$-group may be replaced by $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$,$-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, each of which may be substituted with one or more moieties $R^8$, or an aryloxy or heteroaryloxy group with 5 to 60 aromatic ring atoms, which may be substituted with one or more moieties $R^9$, or a diarylamino group, diheteroarylamino group or arylheteroarylamino group with 10 to 40 aromatic ring atoms, which may be substituted with one or more moieties $R^9$, or a combination of these systems; here, two or more of these substituents $R^4$ may also form a mono- or polycyclic, aliphatic, aromatic and/or benzo-fused ring system with one another;

$R^5$ in each instance, independently of one another, is selected from the group consisting of phenyl, naphthyl, $CF_3$, $C(=O)R^3$, $P(=O)(R^7)_2$, $As(=O)(R^7)_2$, a linear alkyl, alkoxy or thioalkoxy group with 1 to 40 carbon atoms or a linear alkenyl or alkynyl group with 2 to 40 carbon atoms or branched or cyclic alkyl, alkenyl, alkynyl, alkoxy or thioalkoxy group with 3 to 40 carbon atoms, which in each case may be substituted with one or more moieties $R^9$, wherein one or more adjacent $CH_2$ groups may be replaced by $-R^9C=CR^9-$, -C≡C-, or one adjacent $CH_2$-group may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more moieties $R^9$, or an aryloxy or heteroaryloxy group with 5 to 60 aromatic ring atoms, which may be substituted with one or more moieties $R^9$, or a diarylamino group, diheteroarylamino group or arylheteroarylamino group with 10 to 40 aromatic ring atoms, which may be substituted with one or more moieties $R^9$, or a combination of these systems; in this connection two or more of these substituents $R^5$ may also form a mono- or polycyclic, aliphatic, aromatic and/or benzo-fused ring system;

$R^6$ in each instance, independently of one another, is selected from the group consisting of phenyl, naphthyl, $CF_3$, $Si(R^4)^3$, $C(=O)R^3$, $P(=O)(R^7)_2$, a linear alkyl, alkoxy or thioalkoxy group with 1 to 40 carbon atoms or a linear alkenyl or alkynyl group with 2 to 40 carbon atoms or a branched or cyclic alkyl, alkenyl, alkynyl, alkoxy or thioalkoxy group with 3 to 40 carbon atoms, which may be substituted in each case with one or more moieties $R^9$, wherein one or more adjacent $CH_2$ groups may be replaced by $-R^9C=CR^9-$, -C≡C-, or one adjacent $CH_2$-group may be replaced by $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$,-O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more moieties $R^9$, or an aryloxy or heteroaryloxy group with 5 to 60 aromatic ring atoms, which may be substituted with or more moieties R*, or a diarylamino group, diheteroarylamino group or arylheteroarylamino group with 10 to 40 aromatic ring atoms, which may be substituted with one or more moieties $R^9$, or a combination of these systems; in this process two or more of these substituents $R^6$ may also form a mono- or polycyclic, aliphatic, aromatic and/or benzo-fused ring system with one another;

$R^7$ is in each instance, independently of one another, selected from the group consisting of phenyl, naphthyl, $N(R^2)_2$, CN, $CF_3$, $C(=O)OR^3$, $C(=O)N(R^3)_2$, $Si(R^4)_3$, $C(=O)R^3$, a linear alkyl, alkoxy or thioalkoxy group with 1 to 40 carbon atoms or a linear alkenyl or alkynyl group with 2 to 40 carbon atoms or a branched or cyclic alkyl, alkenyl, alkynyl, alkoxy or thioalkoxy group with 3 to 40 carbon atoms, each of which may be substituted with one or more radicals $R^9$, wherein one or more adjacent $CH_2$ groups may be replaced by $-R^9C=CR^9-$, -C≡C-, or one adjacent $CH_2$-group may be replaced by $-Si(R^4)_2-$, $-Ge(R^4)_2-$, $-Sn(R^4)_2$, -C(=O)-, -C(=S)-,-C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more radicals $R^9$, or an aryloxy or heteroaryloxy group with 5 to 60 aromatic ring atoms, which may be substituted with one or more radicals $R^9$, or a diarylamino group, diheteroarylamino group or arylheteroarylamino group with 10 to 40 aromatic ring atoms, which may be substituted with one or more radicals $R^3$, or a combination of these systems; here, two or more of these substituents $R^7$ may also form a mono- or polycyclic, aliphatic, aromatic and/or benzo-fused ring system;

$R^8$ is in each instance independently of one another selected from the group consisting of H, deuterium, phenyl, naphthyl, F, $CF_3$ or an aliphatic, aromatic and/or heteroaromatic hydrocarbon moiety with 1 to 20 carbon atoms, in which also one or more H atoms may be replaced by F or $CF_3$; here, two or more substituents $R^8$ may also form a mono- or polycyclic, aliphatic ring system;

$R^9$ is in each instance independently of one another selected from the group consisting of H, deuterium, phenyl, naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, a linear alkyl, alkoxy or thioalkoxy group with 1 to 40 carbon atoms or a linear alkenyl or alkynyl group with 2 to 40 carbon atoms or a branched or cyclic alkyl, alkenyl, alkynyl, alkoxy or thioalkoxy group with 3 to 40 carbon atoms, each of which may be substituted with one or more moieties $R^8$, wherein one or more non-adjacent $CH_2$ groups may be replaced by $-R^3C=CR^3-$, -C≡C-, or one adjacent $CH_2$-group may be replaced by $Si(R^4)_2$-, $-Ge(R^4)_2$-,$-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, or -S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more moieties $R^8$, or an aryloxy or heteroaryloxy group with 5 to 60 aromatic ring atoms, which may be substituted with one or more moieties $R^3$, or a diarylamino group, diheteroarylamino group or arylheteroarylamino group with 10 to 40 aromatic ring atoms, which may be substituted with one or more moieties $R^8$, or a combination of these systems; here, two or more of these substituents $R^9$ may also form with one another a mono- or polycyclic, aliphatic, aromatic and/or benzo-fused ring system.

2. Organic molecule according to claim 1, wherein AF1 is selected from the following structures:

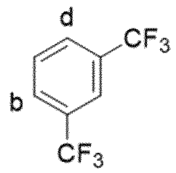
150
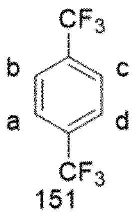
151
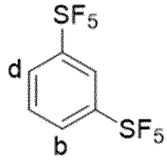
342
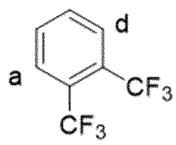
153
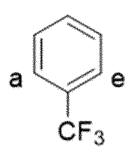
154

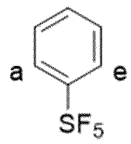

184

wherein the possible attachment points to the separator or to the chemical unit AF2 are designated with lower-case letters a to e.

3. Organic molecule according to claim 1 or 2, wherein AF2 comprises a structure of formula 4:

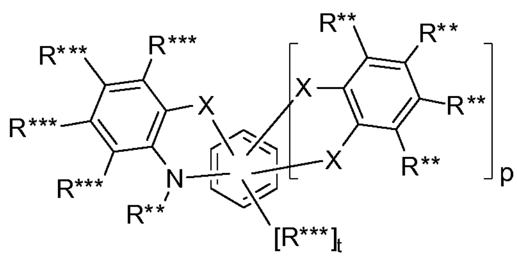

**Formula 4**

wherein

p is 0 or 1;
t = 4 - 2p;
X is $CR^{**}_2$, $NR^{**}$, oxygen, sulfur or a direct bond, wherein at the most two place holders X are both a direct

bond, wherein these are not constituents of the same ring; and otherwise the definitions given in claim 1 apply.

**4.** Organic molecule according to claim 1 or 2, wherein AF2 is selected as one of the following structures:

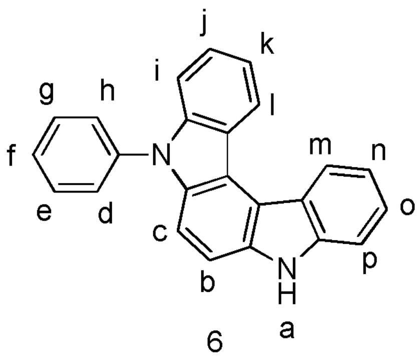

6

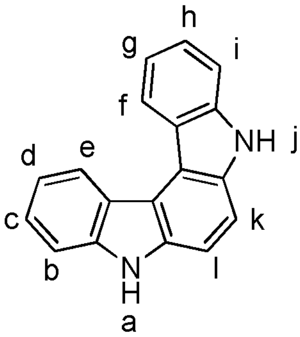

7

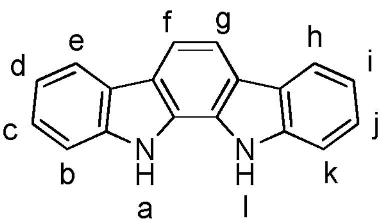

8

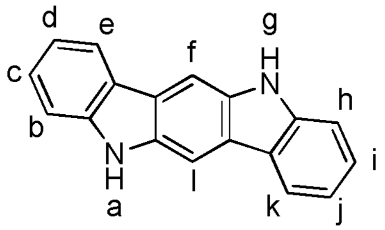

10

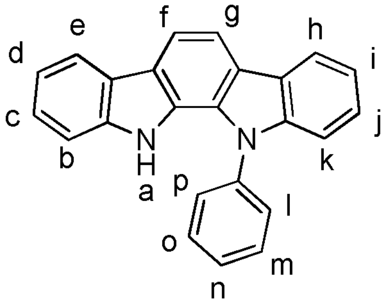

21

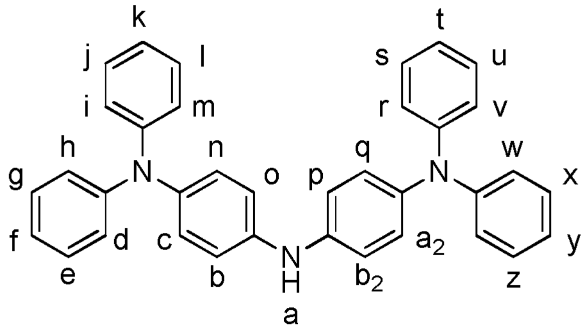

22

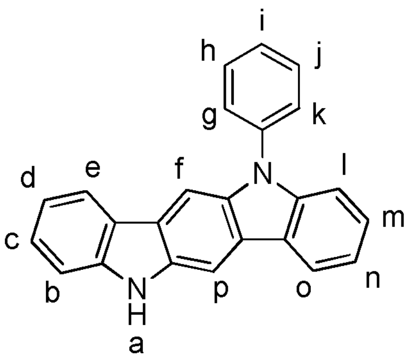

24

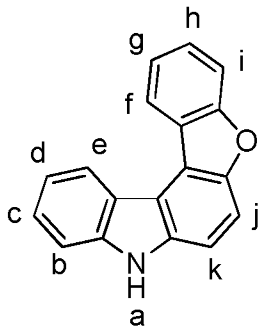

31

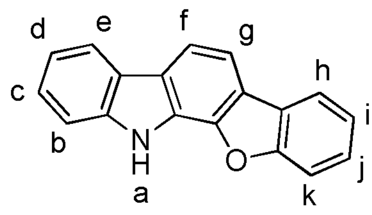

32

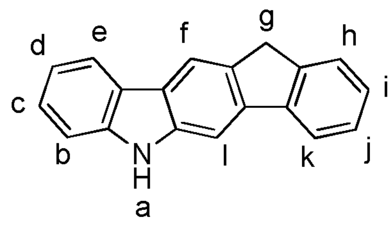

33

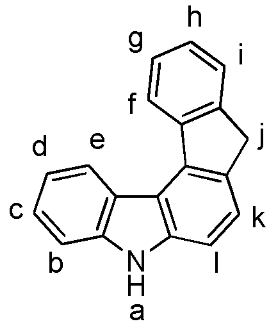

34

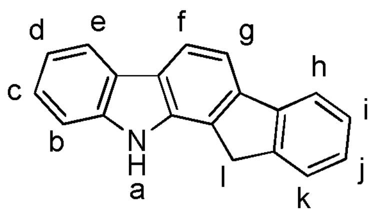

35

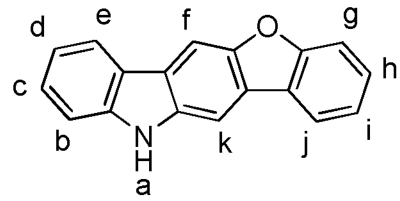

30

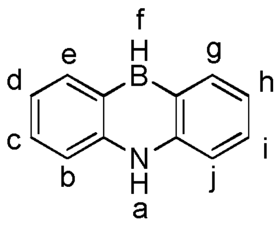

38

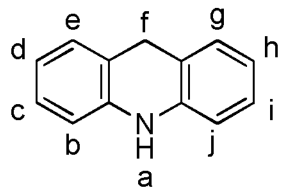

39

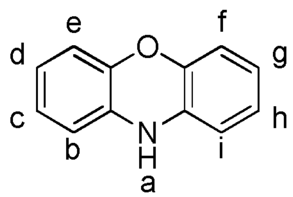

41

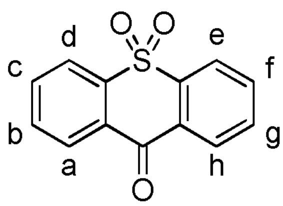

43

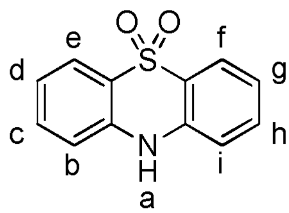

44

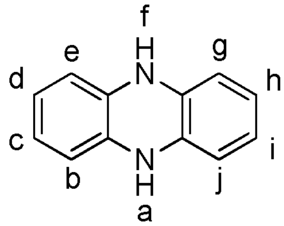

49

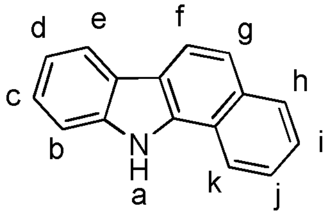

54

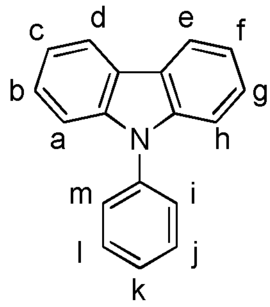

55

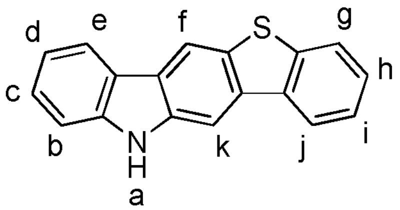

58

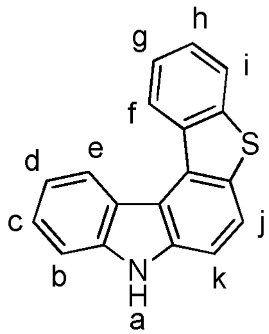

59

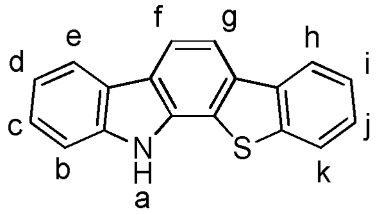

60

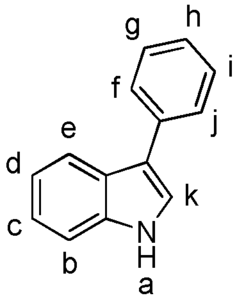

63

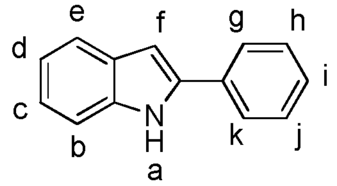

64

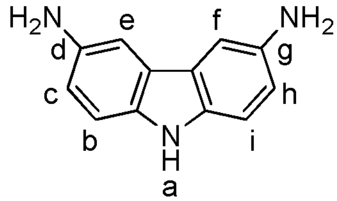

66

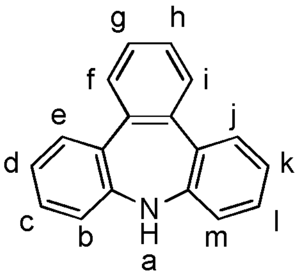

71

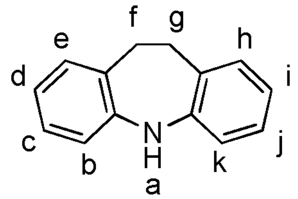

72

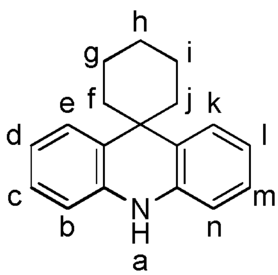

73

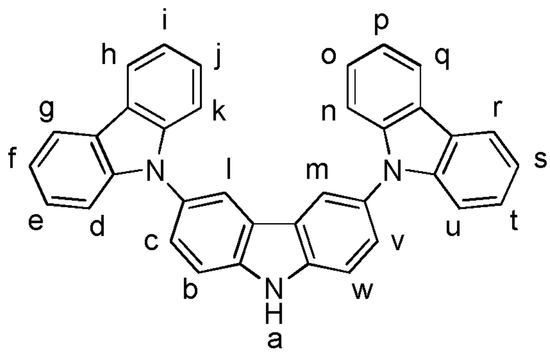

74

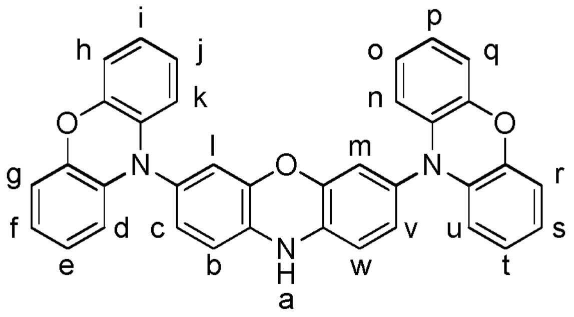

75

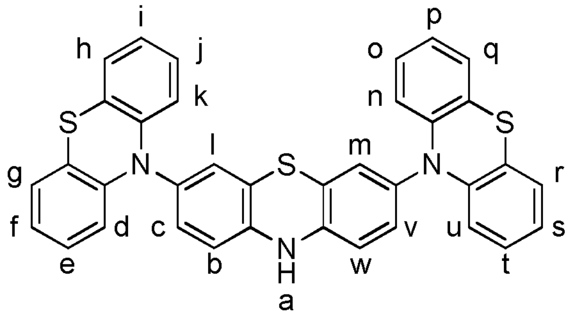

76

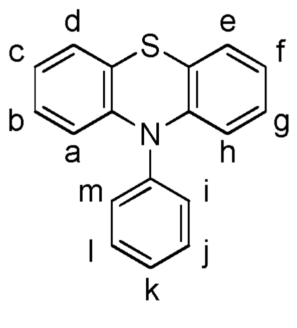

77

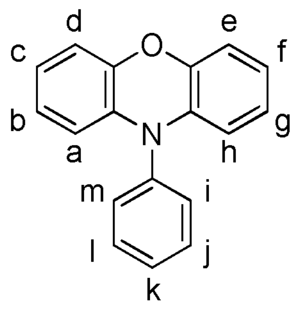

78

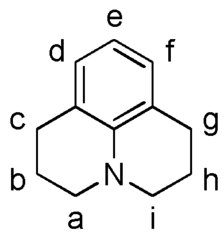

91

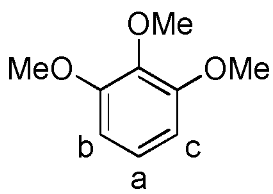

120

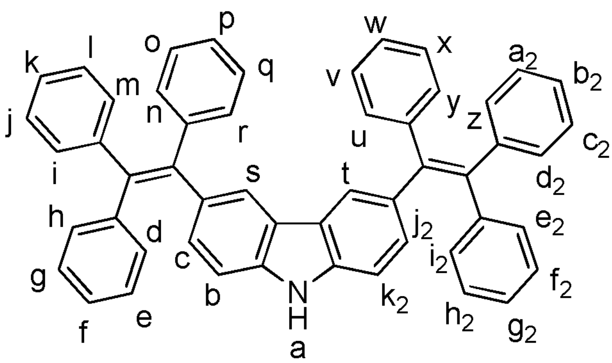

124

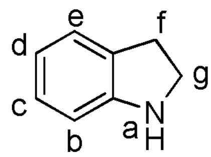

128

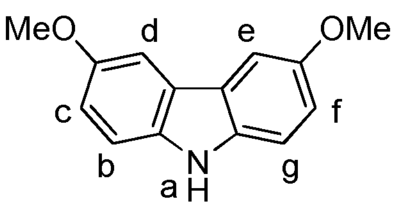

199

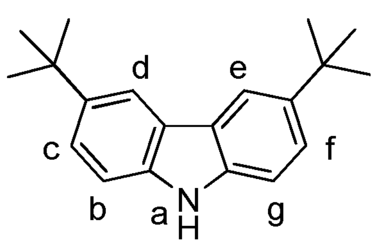

200

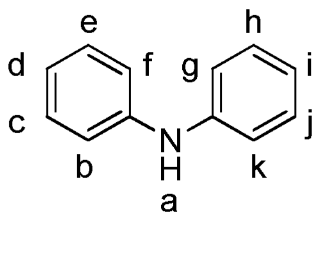

223

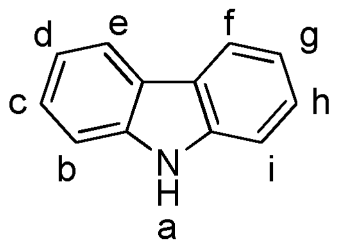

328

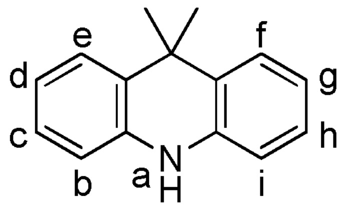

337

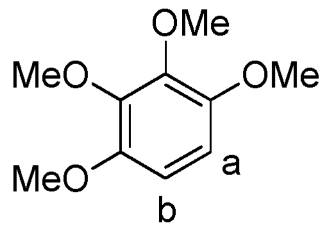

344

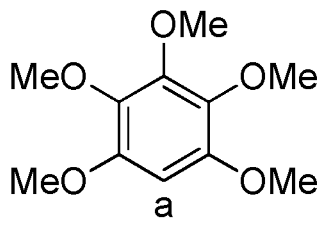

345

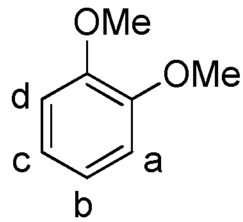

346

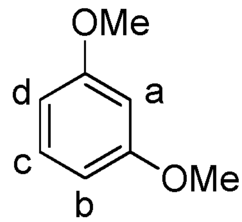

347

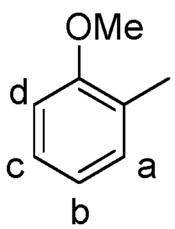

349

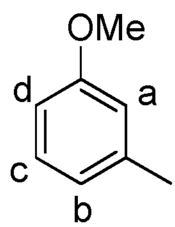

350

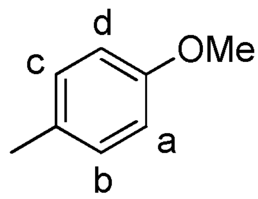

351

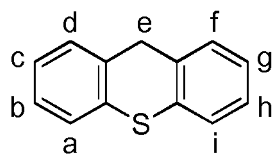

366

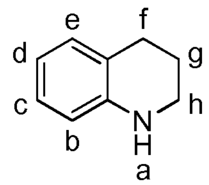

383

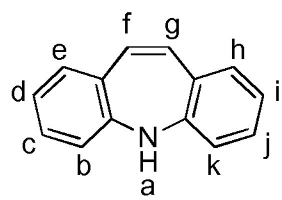

400

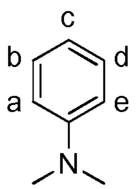

404

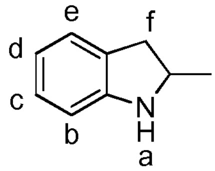

406

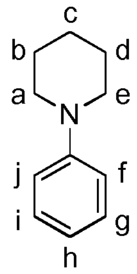

407

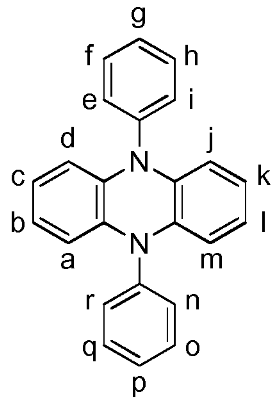

425

430

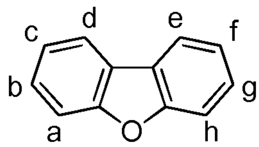

432

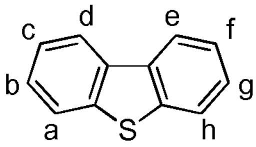

433

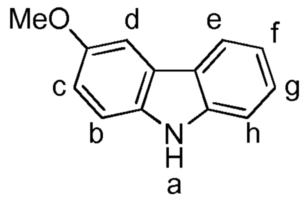

436

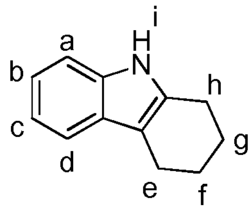

481

5. Organic molecule according to one of claims 1 to 4, wherein the separator is selected from the group of the following structures:

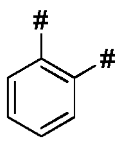

S-1

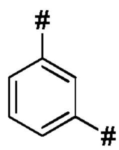

S-2

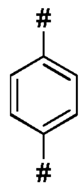

S-3

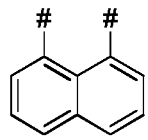

S-4

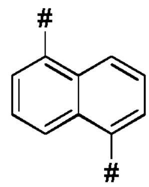

S-5

wherein # represent the attachment points for the chemical units AF1 and AF2.

6. Use of an organic molecule according to one or more of claims 1 to 5 as a luminescent emitter and/or as a host material and/or as an electron transport material and/or as a hole injection material and/or as a hole blocking material in an optoelectronic component.

7. Use according to claim 6, wherein the optoelectronic component is selected from the group consisting of:

- organic light-emitting diodes (OLEDs),
- light-emitting electrochemical cells,
- OLED sensors, especially in gas and vapor sensors not hermetically sealed off to the outside,
- organic diodes,
- organic solar cells,
- organic transistor,
- organic field effect transistors,
- organic lasers, and
- down-conversion elements.

8. Use according to claim 6 or 7, wherein the fraction of the organic molecule of the emitter is 1 % to 99 %.

**9.** Use according to claim 6, 7 or 8, wherein the concentration of the organic molecule as emitter in optical light-emitting components, in particular in OLEDs, is between 5 % and 80 %.

**10.** Optoelectronic component, comprising an organic molecule according to one or more of the claims 1 to 5, in particular formed as a component selected from the group consisting of organic light-emitting diode (OLED), light-emitting electrochemical cell, OLED sensor, in particular in gas and vapor sensors not hermetically sealed off to the outside, organic diode, organic solar cell, organic transistor, organic field effect transistor, organic laser and down-conversion element.

**11.** Optoelectronic component according to claim 10, comprising

- a substrate,
- an anode and
- a cathode, wherein the anode and the cathode are applied to the substrate, and
- at least one light-emitting layer applied between the anode and cathode and containing an organic molecule according to one or more of claims 1 to 5.

**12.** Optoelectronic component according to claim 10 or 11, in which the light-emitting layer comprises at least one host material, wherein the excited singlet state ($S_1$) and/or the excited triplet state ($T_1$) of the at least one host material is higher than that of the excited singlet state ($S_1$) and/or the excited triplet state ($T_1$) of the organic molecule of one or more of claims 1 to 5.

**13.** Optoelectronic component according to claims 10 to 12, comprising at least one host material consisting of a material according to one or more of claims 1 to 5.

**14.** Optoelectronic component according to claims 10 to 13, wherein die light-emitting layer contains fluorescent or phosphorescent materials and wherein the materials of the light-emitting layer are organic molecules according to claims 1 to 5.

**15.** Optoelectronic component according to claims 10 to 14, in which an organic molecule according to one or more of claims 1 to 5 together with a functional material form an exciplex.

**16.** Optoelectronic component according to claims 10 to 15, in which an organic molecule according to one or more of claims 1 to 5 is used as a charge transport layer.

**17.** Method for producing an optoelectronic component, wherein an organic molecule according to one or more of claims 1 to 5 is used.

**18.** Method according to claim 17, wherein the processing of the organic molecule is performed using a vacuum evaporation method or from a solution.

**Revendications**

**1.** Molécule organique, présentant une structure de formule 1

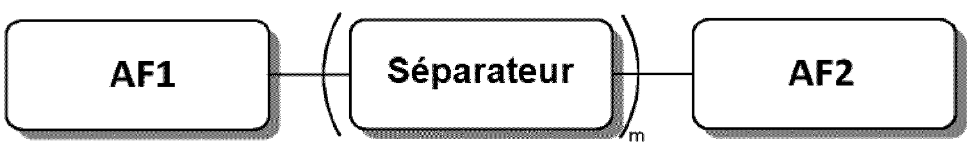


Formule 1

dans laquelle :

m représente 0 ou 1 ;
AF1 est une première unité chimique, comprenant un système conjugué ;
AF2 est une deuxième unité chimique, comprenant un système conjugué ;
AF1 ≠ AF2 ;
AF1 présentant une structure de formule 2-2 :

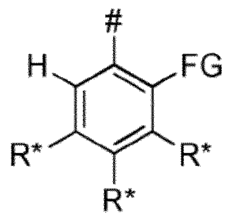

Formule 2-2

dans laquelle

FG est choisi dans le groupe constitué par $CR^{**}{}_2F$, $CF_3$ $CF_2R^{**}$, $SF_5$, N-$(CF_3)_1$, N-$(CF_3)_2$, O-$CF_3$, S-$CF_3$ et $CF_2CO_2R^*$, et un groupe E*, qui est un groupe aryle ou hétéroaryle de 6 à 18 atomes de cycle aromatique, qui peut être substitué avec un ou plusieurs radicaux $R^8$, et qui contient en tant que constituants du cycle aromatique un ou plusieurs groupes V*, les groupes V* étant choisis à chaque occurrence, de manière identique ou différente, parmi =C(F)- et =C($CF_3$)- ;
# = point de liaison au séparateur ou point de liaison à l'unité chimique AF2 ;
R** est à chaque occurrence, de manière identique ou différente, H, un groupe alkyle, alcényle, alcynyle linéaire de 3 à 40 atomes C, ou un groupe alcényle ou alcynyle linéaire de 2 à 40 atomes C, ou un groupe alkyle, alcényle, alcynyle ramifié ou cyclique de 3 à 40 atomes C, qui est éventuellement partiellement fluoré ou perfluoré ;
et AF2 présente une structure de formule 3 :

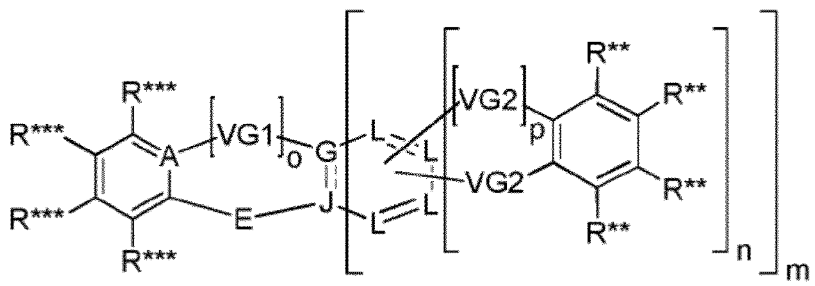

Formule 3

dans laquelle :

m représente 0 ou 1 ;
n représente 0 ou 1, avec lorsque m = 0, toujours également n = 0 ;
o représente 0 ou 1 ;
p représente 0 ou 1 ;
A représente CR*** lorsque o = 0, sinon C ;
VG1 = un groupe pontant, choisi dans le groupe constitué par :

- NR**, $CR^{**}{}_2$, O, S et une simple liaison C-C ; ou
- NR**, $CR^{**}{}_2$, O, S, une simple liaison C-C, BR**, AsR**, $SiR^{**}{}_2$, $GeR^{**}{}_2$,

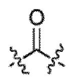, 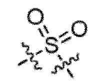, 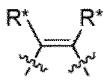, 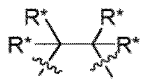, 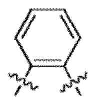, 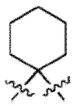, 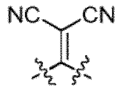, 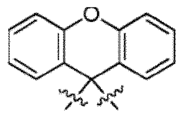,

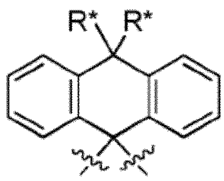, 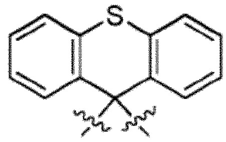 et 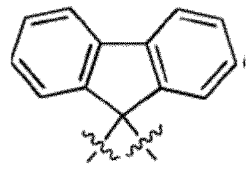,

lorsque m = 1 et simultanément n = 0;

VG2 = un groupe pontant, choisi indépendamment à chaque occurrence dans le groupe constitué par $CR^{**}_2$, NR**, O, S et une simple liaison C-C, deux unités VG2 ne représentant pas simultanément une simple liaison C-C ;
E représente NR**,

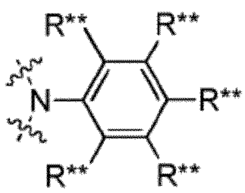,

O ou S ;
G représente C lorsque o = 1 et simultanément m = 1 ; G représente CR** lorsque o = 0 et simultanément m = 1 ; G représente CR** ou $CR^{**}_2$ lorsque o = 1 et simultanément m = 0 ; G représente R* lorsque o = 0 et simultanément m = 0 ; G représente CR**, $CR^{**}_2$, N ou NR* lorsque m = 0 et simultanément VG1 est une simple liaison C-C ;
J représente C lorsque m = 1 ; J représente CR**, $CR^{**}_2$ ou NR** lorsque m = 0 ;
L représente CR*** lorsque n = 0 ; L représente CR** ou C (en cas de liaison covalente à VG2 et/ou à M) lorsque n = 1 ;
R*** représente R** ou est choisi parmi les unités suivantes, au plus deux des radicaux R*** représentant simultanément une des unités suivantes :

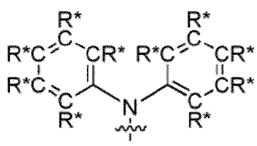, 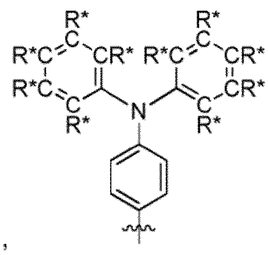, 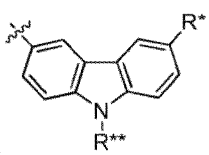, 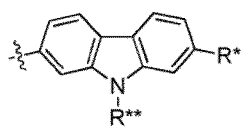,

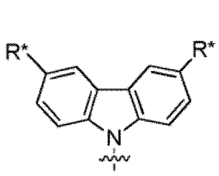, 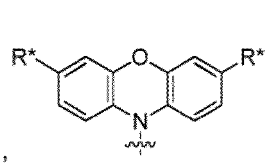, 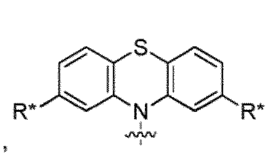,

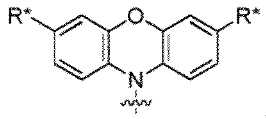,
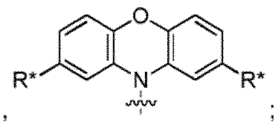;

R*** représente indépendamment à chaque occurrence un radical R* ou marque un emplacement de liaison à un séparateur S ou l'unité chimique AF1, exactement un R** étant un emplacement de liaison à un séparateur S ou à l'unité chimique AF1 ;
et le séparateur S présentant une structure de formule 4S :

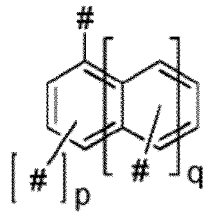

Formule 4S

dans laquelle

# caractérise les positions de liaison pour les unités chimiques AF1 et AF2 ;
p = 0 ou 1 ;
q = 0 ou 1 ;
et $p \neq q$ ;
le séparateur est une unité chimique qui relie AF1 et AF2 et le séparateur est relié au système conjugué d'AF1 et au système conjugué d'AF2 ;
et, pour la formule 1, la formule 2-2 et la formule 3 :
R* est choisi indépendamment à chaque occurrence dans le groupe constitué par H, deutérium, phényle, naphtyle, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $OS(=O)_2R^3$, $SF_5$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par-$Si(R^4)_2$-, -$Ge(R^4)_2$-, -$Sn(R^4)_2$-, -C(=O)-, -C(=S)-, -C(=Se)-, -C=N-, -C(=O)O-, -$C(=O)N(R^3)$-,-$P(=O)(R^7)$-, -$As(=O)(R^7)$-, -$P(=S)(R^7)$, -$As(=S)(R^7)$-, -S(=O)-, -$S(=O)_2$-, -$NR^2$-, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par un ou plusieurs radicaux $R^2$, ou un groupe aryloxy ou hétéroaryloxy de 5 à 60 atomes de cycle aromatique, qui peut être substitué par un ou plusieurs radicaux $R^9$, ou un groupe diarylamino, dihétéroarylamino ou arylhétéroarylamino de 10 à 40 atomes de cycle aromatique, qui peut être substitué par un ou plusieurs radicaux $R^9$, ou une combinaison de ces systèmes ; deux ou plus de ces substituants R* pouvant également former les uns avec les autres un système cyclique mono- ou polycyclique, aliphatique, aromatique et/ou benzoannelé ;
$R^2$ est choisi indépendamment à chaque occurrence dans le groupe constitué par H, deutérium, phényle, naphtyle, $CF_3$, $C(=O)OR^3$, $C(=O)N(R^2)_2$, $Si(R^4)_3$, $C(=O)R^3$, $P(=O)(R^7)_2$, $AS(=O)(R^7)_2$, $P(=S)(R^7)_2$, $As(=S)(R^7)_2$, $S(=O)R^3$, $S(=O)_2R^3$, un groupe alkyle, alcoxy ou thioalcoxy linéaire de 1 à 40 atomes C ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par -$Si(R^4)_2$-, -$Ge(R^4)_2$-, -$Sn(R^4)_2$-, -C(=O)-, -C(=S)-, -C(=Se)-, -C=N-, -C(=O)O-, -$C(=O)N(R^3)$-, -$P(=O)(R^7)$-, -$As(=O)(R^7)$-, -$P(=S)(R^7)$,-$As(=S)(R^7)$-, -S(=O)-, -$S(=O)_2$-, -$NR^2$-, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par

un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, dihétéroarylamino ou arylhétéroarylamino de 10 à 40 atomes de cycle aromatique, qui peut être substitué par un ou plusieurs radicaux $R^9$, ou une combinaison de ces systèmes ; deux ou plus de ces substituants $R^2$ pouvant également former les uns avec les autres un système cyclique mono- ou polycyclique, aliphatique, aromatique et/ou benzoannelé ;

$R^3$ est choisi indépendamment à chaque occurrence dans le groupe constitué par H, deutérium, phényle, naphtyle, $CF_3$ ou un radical hydrocarboné aliphatique, aromatique et/ou hétéroaromatique de 1 à 20 atomes C, dans lequel un ou plusieurs atomes H peuvent également être remplacés par F ou $CF_3$; deux substituants $R^3$ ou plus pouvant également former les uns avec les autres un système cyclique aliphatique mono- ou polycyclique ;

$R^4$ est choisi indépendamment à chaque occurrence dans le groupe constitué par H, deutérium, phényle, naphtyle, $N(R^2)_2$, CN, $CF_3$, OH, $C(=O)OR^3$, $C(=O)N(R^3)_2$, $C(=O)R^3$, $P(=O)(R^7)_2$, $AS(=O)(R^7)_2$, $P(=S)(R^7)_2$, $As(=S)(R^7)_2$, un groupe alkyle, alcoxy ou thioalcoxy de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par un ou plusieurs radicaux $R^8$, ou un groupe aryloxy ou hétéroaryloxy de 5 à 60 atomes de cycle aromatiques, qui peut être substitué par un ou plusieurs radicaux $R^9$, ou un groupe diarylamino, dihétéroarylamino ou arylhétéroarylamino de 10 à 40 atomes de cycle aromatique, qui peut être substitué par un ou plusieurs radicaux $R^9$, ou une combinaison de ces systèmes ; deux ou plus de ces substituants $R^4$ pouvant également former les uns avec les autres un système cyclique mono- ou polycyclique, aliphatique, aromatique et/ou benzoannelé ;

$R^5$ est choisi indépendamment à chaque occurrence dans le groupe constitué par phényle, naphtyle, $CF_3$, $C(=O)R^3$, $P(=O)(R^7)_2$, $As(=O)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, $-Sn(R^4)_2-$, -C(=O)-, -C(=S)-,-C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, dihétéroarylamino ou arylhétéroarylamino de 10 à 40 atomes de cycle aromatique, qui peut être substitué par un ou plusieurs radicaux $R^9$, ou une combinaison de ces systèmes ; deux ou plus de ces substituants $R^5$ pouvant également former les uns avec les autres un système cyclique mono- ou polycyclique, aliphatique, aromatique et/ou benzoannelé ;

$R^6$ est choisi indépendamment à chaque occurrence dans le groupe constitué par phényle, naphtyle, $CF_3$, $Si(R^4)_3$, $C(=O)R^3$, $P(=O)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par un ou plusieurs radicaux $R^9$, ou un groupe aryloxy ou hétéroaryloxy de 5 à 60 atomes de cycle aromatique, qui peut être substitué par un ou plusieurs radicaux R*, ou un groupe diarylamino, dihétéroarylamino ou arylhétéroarylamino de 10 à 40 atomes de cycle aromatique, qui peut être substitué par un ou plusieurs radicaux $R^9$, ou une combinaison de ces systèmes ; deux ou plus de ces substituants $R^6$ pouvant également former les uns avec les autres un système cyclique mono- ou polycyclique, aliphatique, aromatique et/ou benzoannelé ;

$R^7$ est choisi indépendamment à chaque occurrence dans le groupe constitué par phényle, naphtyle, $N(R^2)_2$, CN, $CF_3$, $C(=O)OR^3$, $C(=O)N(R^3)_2$, $Si(R^4)_2$, $C(=O)R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle,

alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, $-C\equiv C-$, ou un groupe $CH_2$ voisin pouvant être remplacé par $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, dihétéroarylamino ou arylhétéroarylamino de 10 à 40 atomes de cycle aromatique, qui peut être substitué par un ou plusieurs radicaux $R^3$, ou une combinaison de ces systèmes ; deux ou plus de ces substituants $R^7$ pouvant également former les uns avec les autres un système cyclique mono- ou polycyclique, aliphatique, aromatique et/ou benzoannelé ;

$R^8$ est choisi indépendamment à chaque occurrence dans le groupe constitué par H, deutérium, phényle, naphtyle, F, $CF_3$ ou un radical hydrocarboné aliphatique, aromatique et/ou hétéroaromatique de 1 à 20 atomes C, dans lequel un ou plusieurs atomes H peuvent également être remplacés par F ou $CF_3$; deux substituants $R^8$ ou plus pouvant également former les uns avec les autres un système cyclique aliphatique mono- ou polycyclique ;

$R^9$ est choisi indépendamment à chaque occurrence dans le groupe constitué par H, deutérium, phényle, naphtyle, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^8$, un ou plusieurs groupes $CH_2$ voisins pouvant être remplacés par $-R^3C=CR^{3-}$, $-C\equiv C-$, ou un groupe $CH_2$ voisin pouvant être remplacé par$-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$,$-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique contenant 5 à 60 atomes de cycle aromatique, qui peut à chaque fois être substitué par un ou plusieurs radicaux $R^8$, ou un groupe aryloxy ou hétéroaryloxy de 5 à 60 atomes de cycle aromatique, qui peut être substitué par un ou plusieurs radicaux $R^3$, ou un groupe diarylamino, dihétéroarylamino ou arylhétéroarylamino de 10 à 40 atomes de cycle aromatique, qui peut être substitué par un ou plusieurs radicaux $R^8$, ou une combinaison de ces systèmes ; deux ou plus de ces substituants $R^9$ pouvant également former les uns avec les autres un système cyclique mono- ou polycyclique, aliphatique, aromatique et/ou benzoannelé.

**2.** Molécule organique selon la revendication 1, dans laquelle AF1 est choisi parmi les structures suivantes :

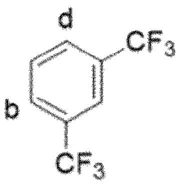
150

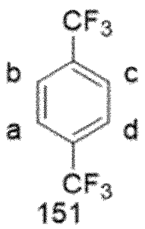
151

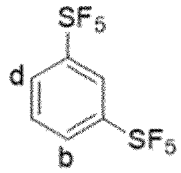
342

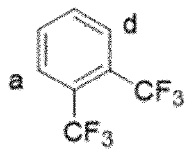
153

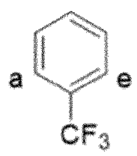
154

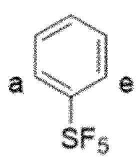
184

dans lesquelles les points de liaison possibles au séparateur ou à l'unité chimique AF2 sont désignés par les lettres minuscules a à e.

**3.** Molécule organique selon les revendications 1 à 2, dans laquelle AF2 présente une structure de formule 4 :

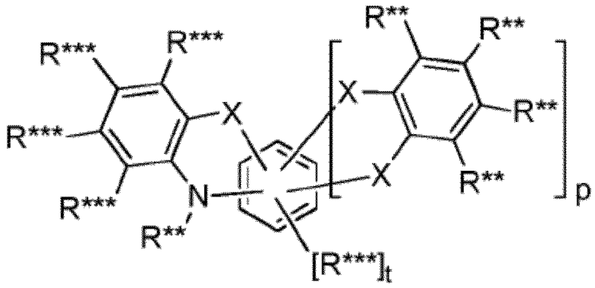

Formule 4

dans laquelle

p représente 0 ou 1 ;
t = 4-2p ;
X représente $CR^{**}_2$, $NR^{**}$, oxygène, soufre ou une liaison directe, au plus deux caractères génériques X représentant simultanément une liaison directe, ceux-ci ne faisant pas partie du même cycle ;
et les définitions indiquées dans la revendication 1 s'appliquant pour le reste.

**4.** Molécule organique selon les revendications 1 à 2, dans laquelle AF2 est choisi parmi une des structures suivantes :

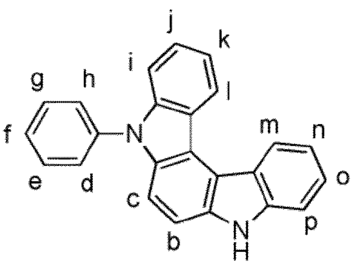

6

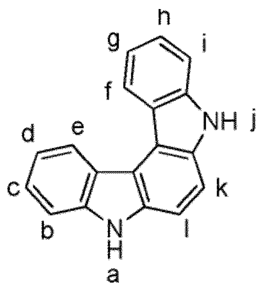

7

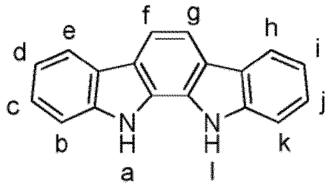

8

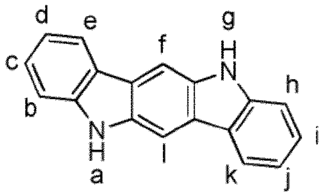

10

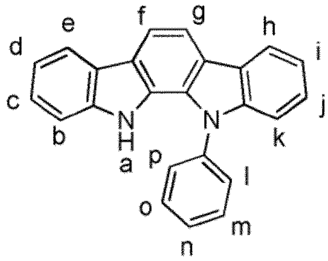

21

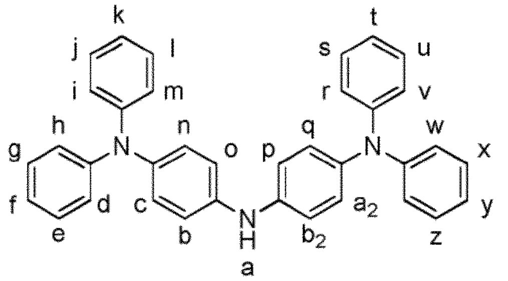

22

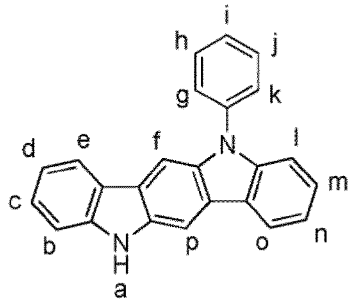

24

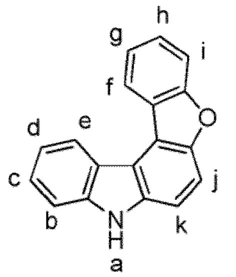

31

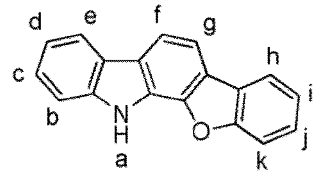

32

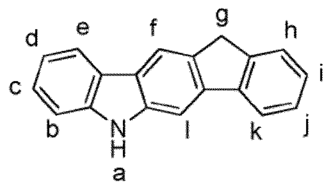

33

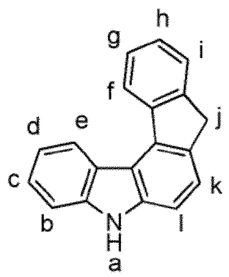

34

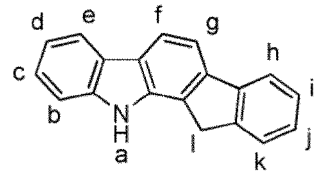

35

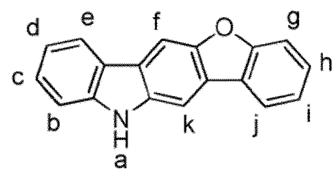

30

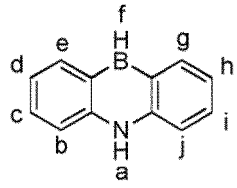

38

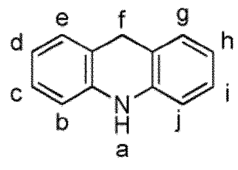

39

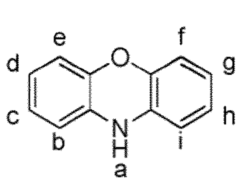

41

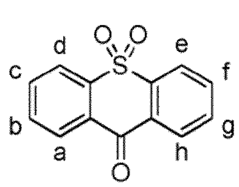

43

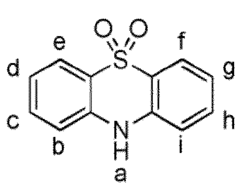

44

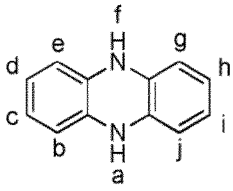

49

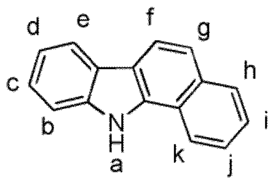

54

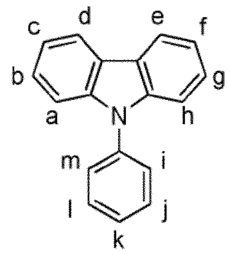

55

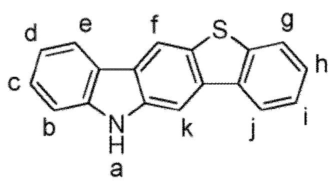

58

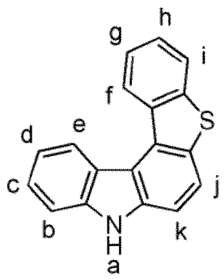

59

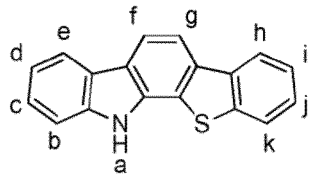

60

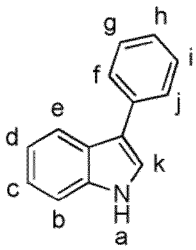

63

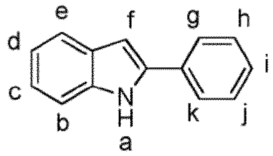

64

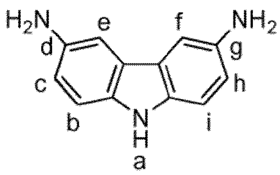

66

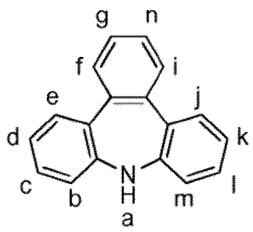

71

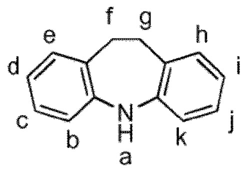

72

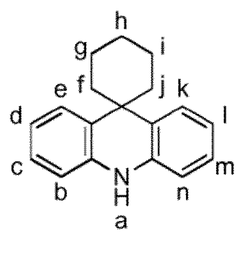

73

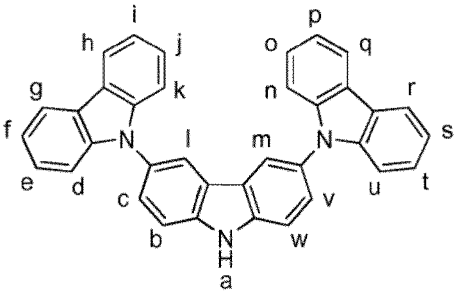

74

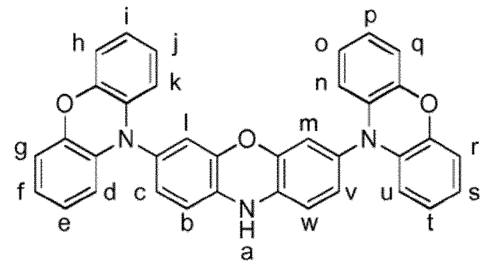

75

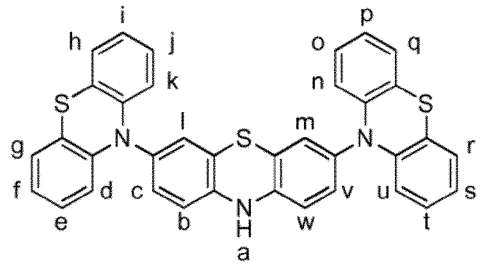

76

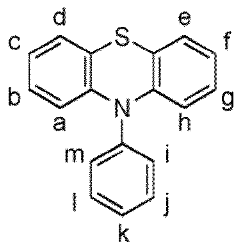

77

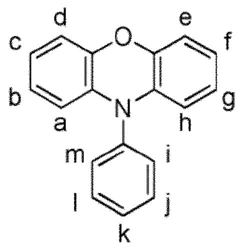

78

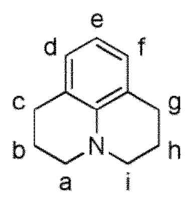

91

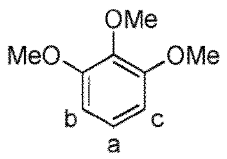

120

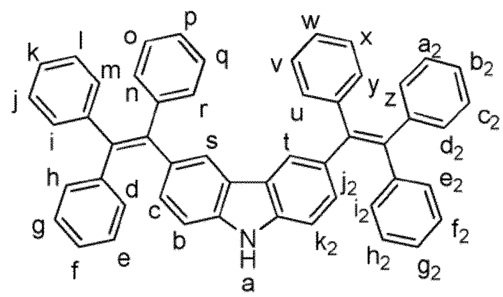

124

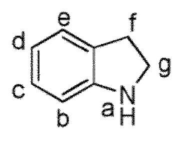

128

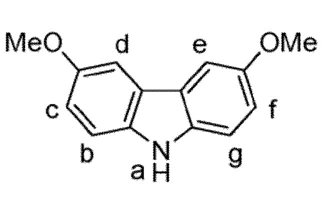

199

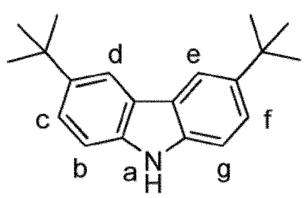

200

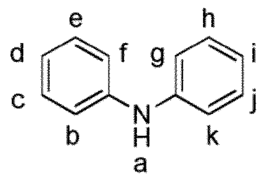

223

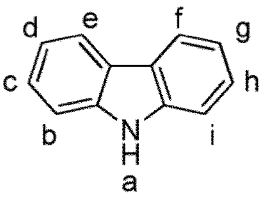

328

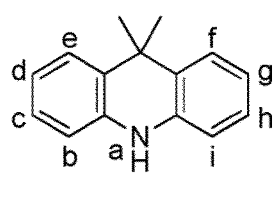

337

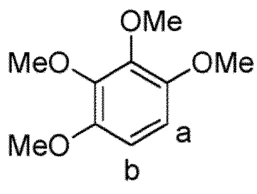

344

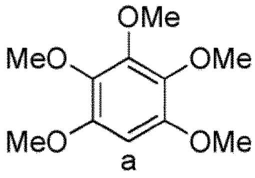

345

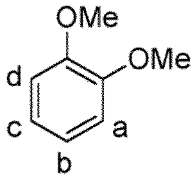

346

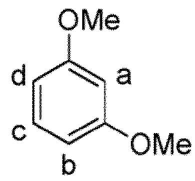

347

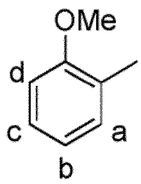

349

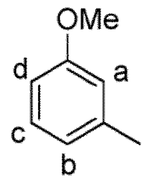

350

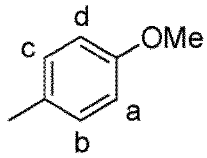

351

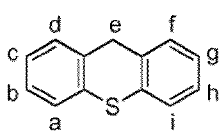

366

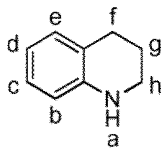

383

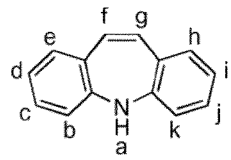

400

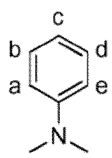

404

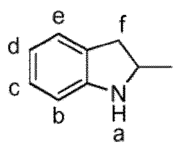

406

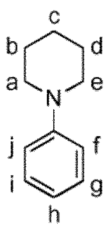

407

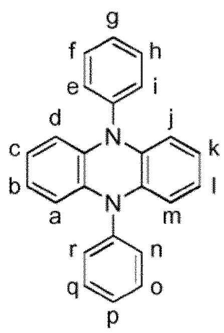

425

430

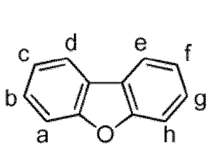

432

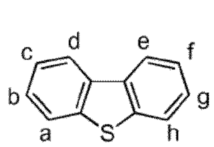

433

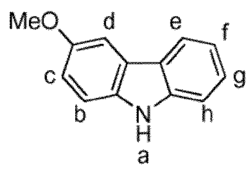

436

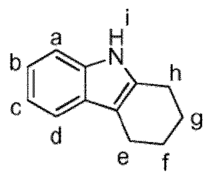

481

**5.** Molécule organique selon l'une quelconque des revendications 1 à 4, dans laquelle le séparateur est choisi dans le groupe des structures suivantes :

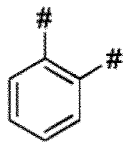
S-1

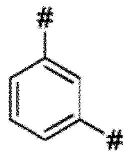
S-2

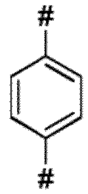
S-3

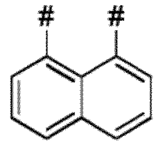
S-4

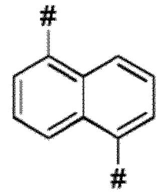
S-5

# représentant les positions de liaison pour les unités chimiques AF1 et AF2.

**6.** Utilisation d'une molécule organique selon une ou plusieurs des revendications 1 à 5 en tant qu'émetteur luminescent et/ou en tant que matériau hôte et/ou en tant que matériau de transport d'électrons et/ou en tant que matériau d'injection de trous et/ou en tant que matériau de blocage de trous dans un composant optoélectronique.

**7.** Utilisation selon la revendication 6, dans laquelle le composant optoélectronique est choisi dans le groupe constitué par :

- les diodes organiques électroluminescentes (OLED),
- les cellules électrochimiques électroluminescentes,
- les capteurs OLED, notamment les capteurs de gaz et de vapeur non blindés hermétiquement vers l'extérieur,
- les diodes organiques,
- les cellules solaires organiques,
- les transistors organiques,
- les transistors à effet de champ organiques,
- les lasers organiques et
- les éléments de conversion à la baisse.

**8.** Utilisation selon la revendication 6 ou 7, dans laquelle la proportion de la molécule organique dans l'émetteur est de 1 % à 99 %.

**9.** Utilisation selon la revendication 6, 7 ou 8, dans laquelle la concentration de la molécule organique en tant qu'émetteur dans des composants optiques électroluminescents, notamment dans des OLED, est comprise entre 5 % et 80 %.

**10.** Composant optoélectronique, comprenant une molécule organique selon une ou plusieurs des revendications 1 à 5, notamment configuré sous la forme d'un composant choisi dans le groupe constitué par une diode organique électroluminescente (OLED), une cellule électrochimique électroluminescente, un capteur OLED, notamment les capteurs de gaz et de vapeur non blindés hermétiquement vers l'extérieur, une diode organique, une cellule solaire organique, un transistor organique, un transistor à effet de champ organique, un laser organique et un éléments de conversion à la baisse.

**11.** Composant optoélectronique selon la revendication 10, comprenant :

- un substrat,
- une anode et
- une cathode, l'anode et la cathode étant appliquées sur le substrat, et
- au moins une couche électroluminescente, qui est agencée entre l'anode et la cathode, et qui contient une molécule organique selon une ou plusieurs des revendications 1 à 5.

**12.** Composant optoélectronique selon la revendication 10 ou 11, dans lequel la couche électroluminescente comprend au moins un matériau hôte, l'état singulet excité ($S_1$) et/ou l'état triplet excité ($T_1$) dudit au moins un matériau hôte étant plus élevé que l'état singulet excité ($S_1$) et/ou l'état triplet excité ($T_1$) de la molécule organique selon une ou plusieurs des revendications 1 à 5.

**13.** Composant optoélectronique selon les revendications 10 à 12, comprenant au moins un matériau hôte constitué par un matériau selon une ou plusieurs des revendications 1 à 5.

**14.** Composant optoélectronique selon les revendications 10 à 13, dans lequel la couche électroluminescente contient

des matériaux fluorescents ou phosphorescents, et les matériaux de la couche électroluminescente sont des molécules organiques selon les revendications 1 à 5.

**15.** Composant optoélectronique selon les revendications 10 à 14, dans lequel une molécule organique selon une ou plusieurs des revendications 1 à 5 forme un exciplexe conjointement avec un matériau fonctionnel.

**16.** Composant optoélectronique selon les revendications 10 à 15, dans lequel une molécule organique selon une ou plusieurs des revendications 1 à 5 est utilisée en tant que couche de transport de charges.

**17.** Procédé de fabrication d'un composant optoélectronique, selon lequel une molécule organique selon une ou plusieurs des revendications 1 à 5 est utilisée.

**18.** Procédé selon la revendication 17, dans lequel le traitement de molécule organique a lieu au moyen d'un procédé d'évaporation sous vide ou à partir d'une solution.

Figur 1

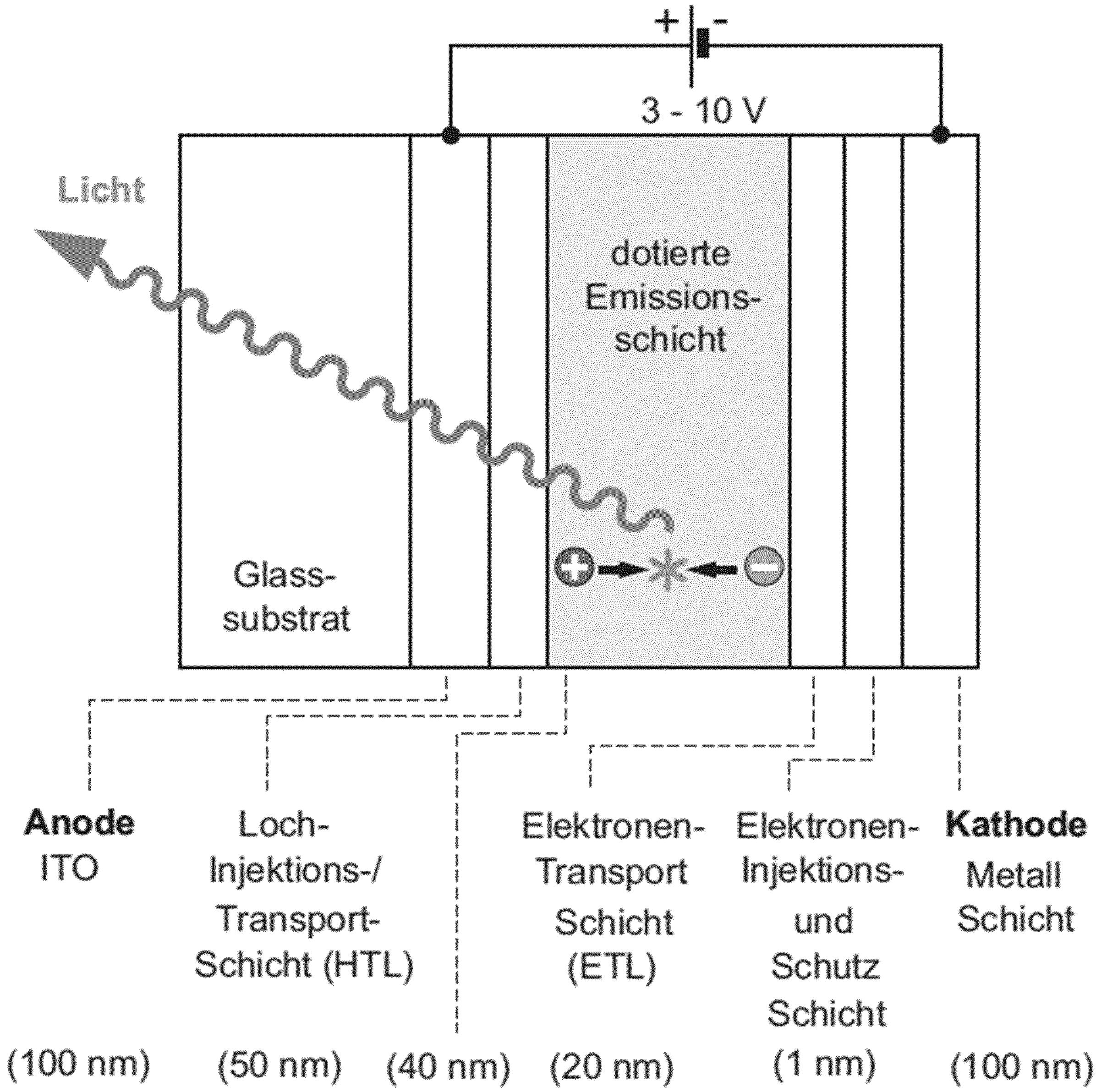
+
-
3 - 10 V
Licht
dotierte Emissions-schicht
Glass-substrat
Anode ITO
(100 nm)
Loch-Injektions-/ Transport-Schicht (HTL)
(50 nm)
(40 nm)
Elektronen-Transport Schicht (ETL)
(20 nm)
Elektronen-Injektions- und Schutz Schicht
(1 nm)
Kathode Metall Schicht
(100 nm)

Figur 2

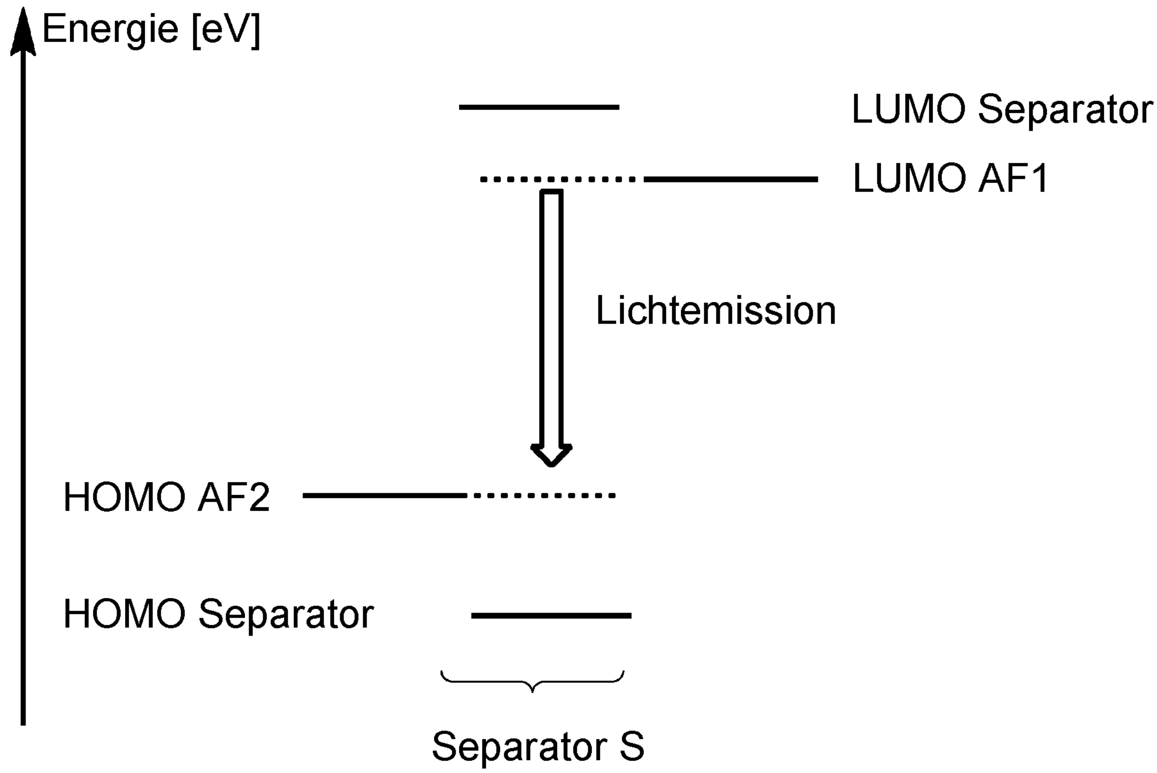
Energie [eV]
LUMO Separator
LUMO AF1
Lichtemission
HOMO AF2
HOMO Separator
Separator S

Figur 3

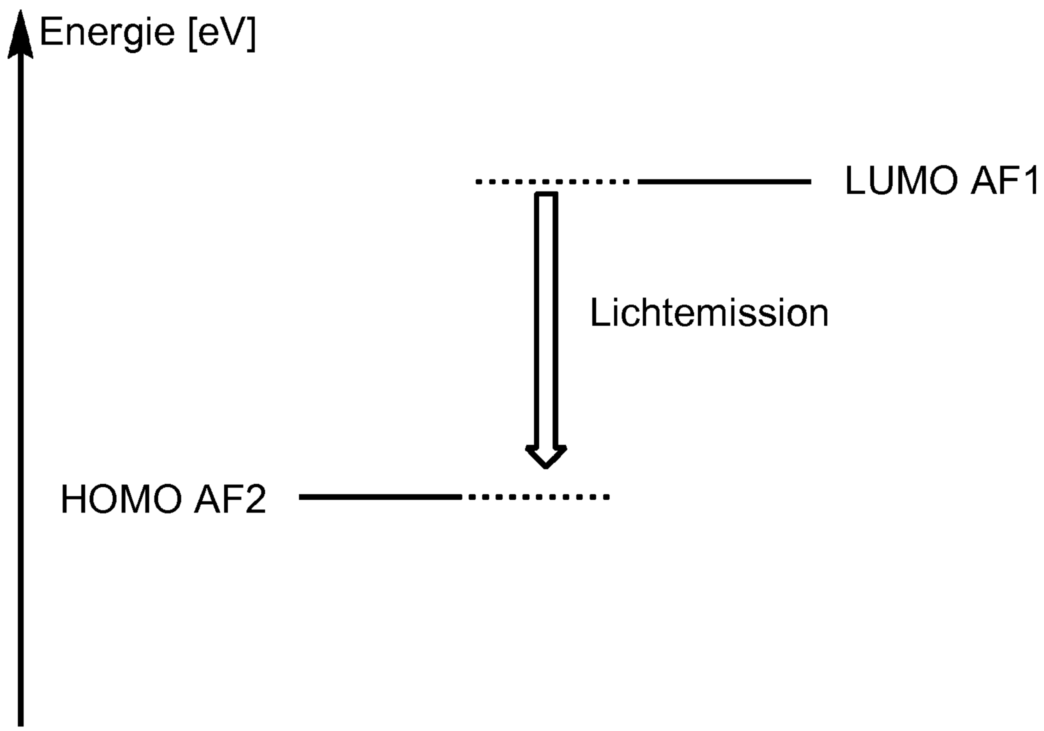

## Figur 4

| Molekülstruktur | DARSTELLUNG DES HOMO ORBITALS | DARSTELLUNG DES LUMO ORBITALS |
|---|---|---|
| 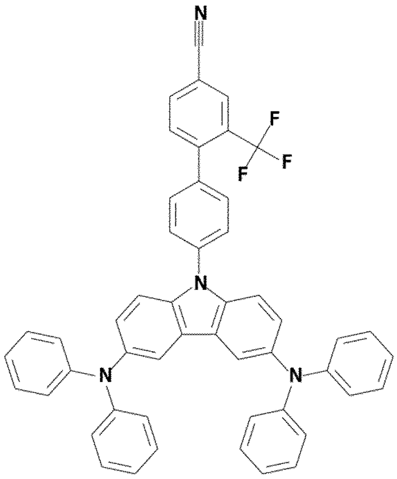 | 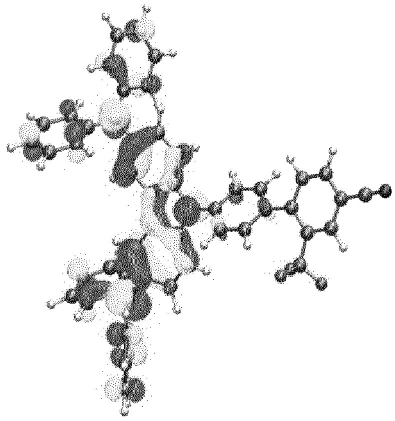 | 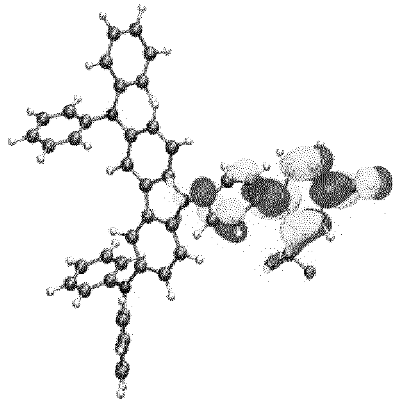 |
| 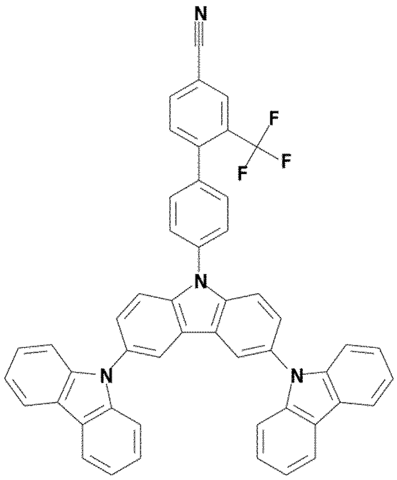 | 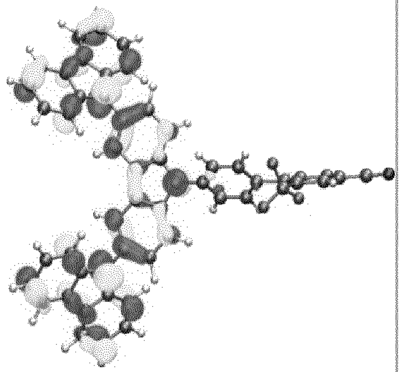 | 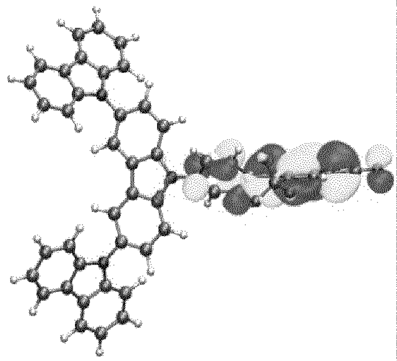 |
| 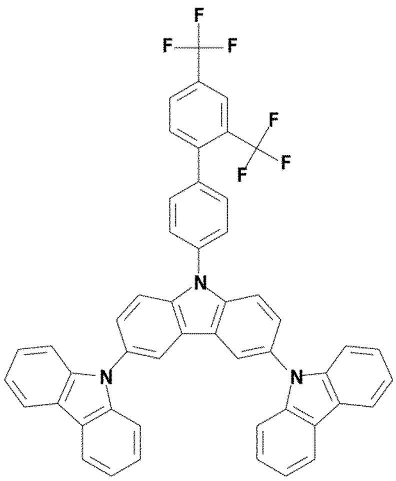 | 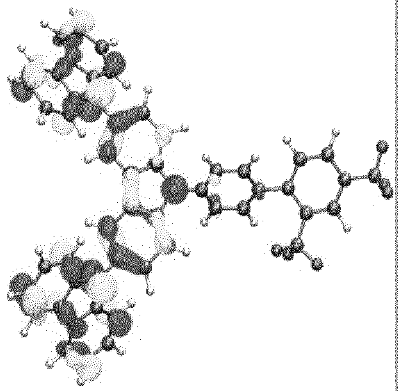 | 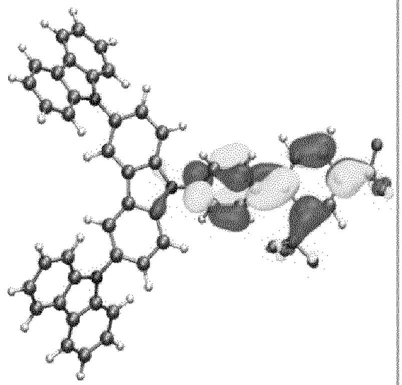 |

| | | |
|---|---|---|
| 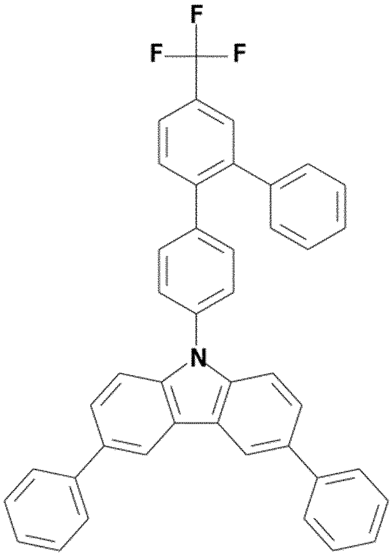 | 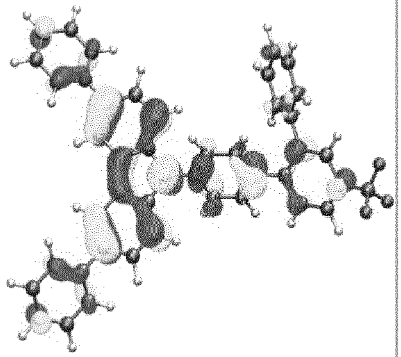 | 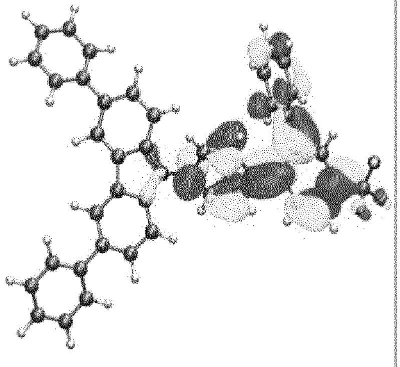 |
| 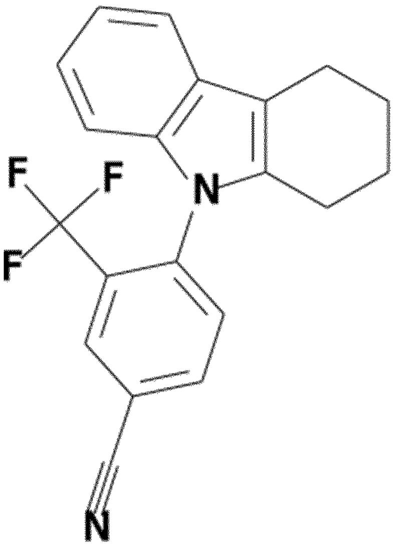 | 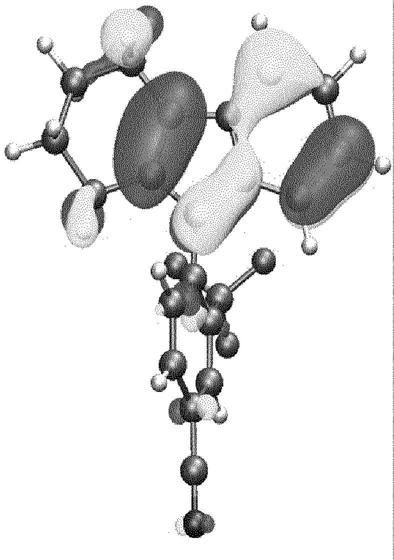 | 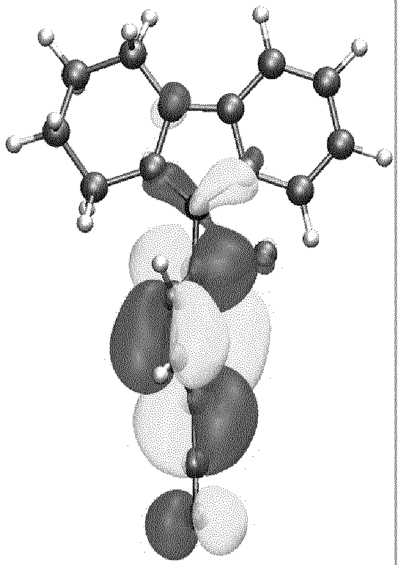 |
| 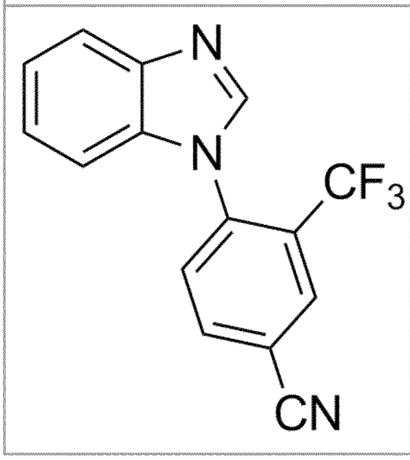 | 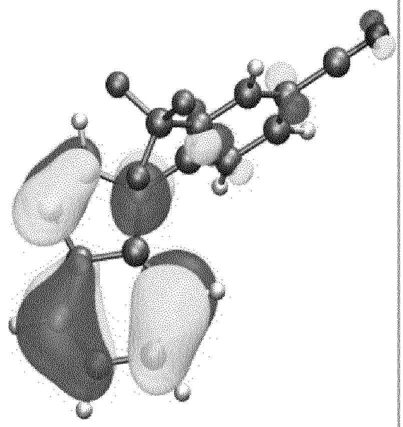 | 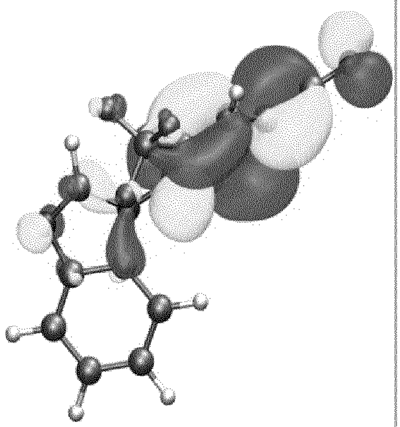 |

| | | |
|---|---|---|
| 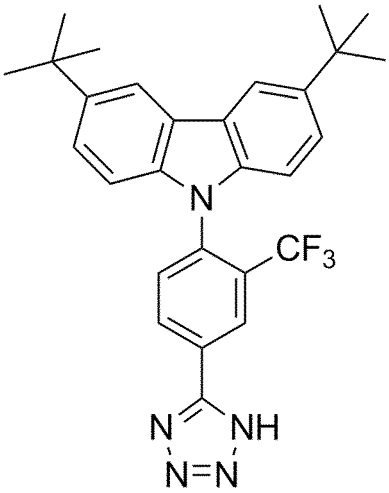 | 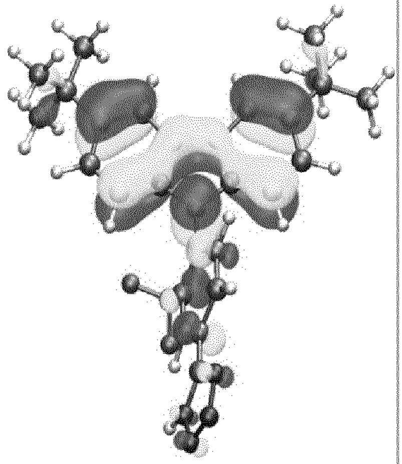 | 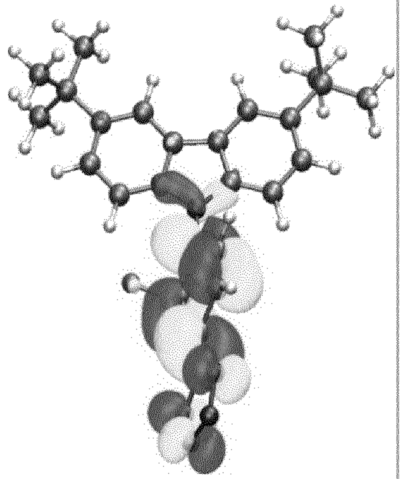 |
| 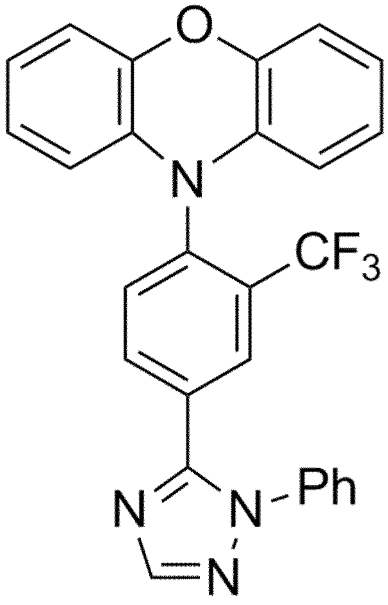 | 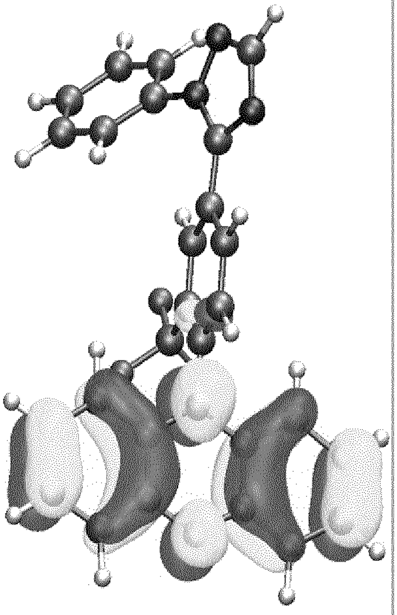 | 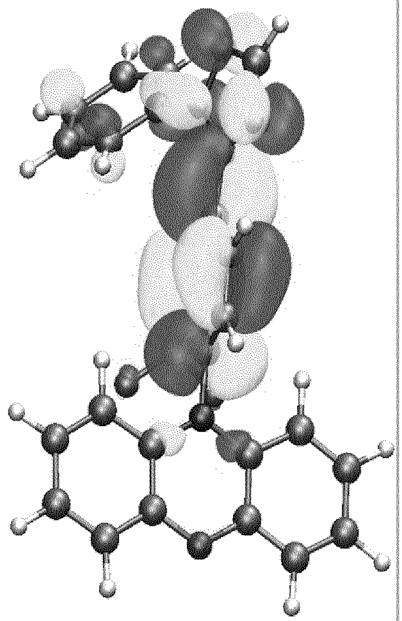 |

| | | |
|---|---|---|
| 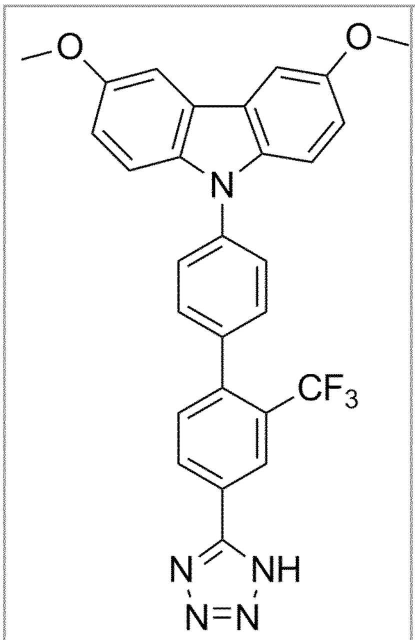 | 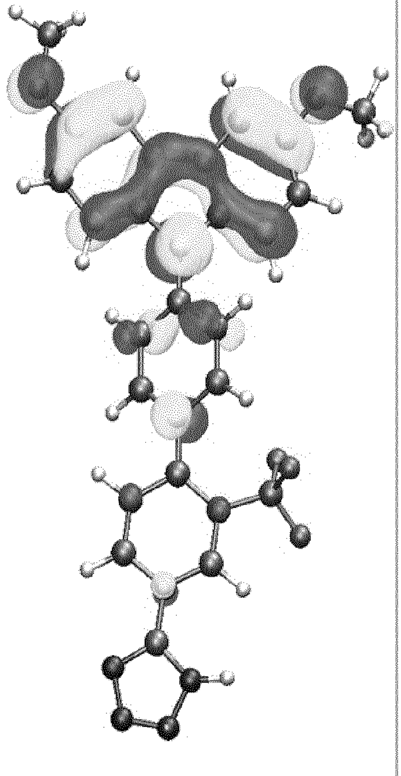 | 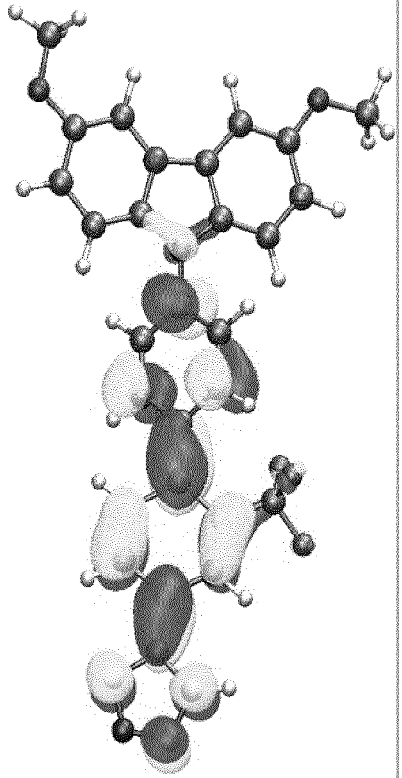 |
| 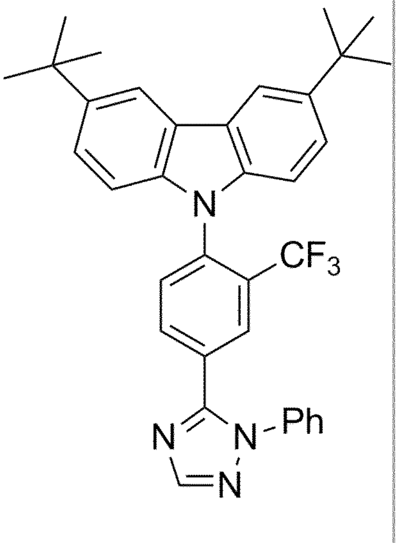 | 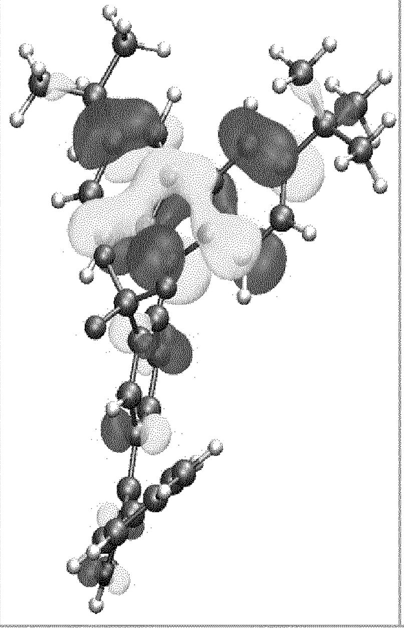 | 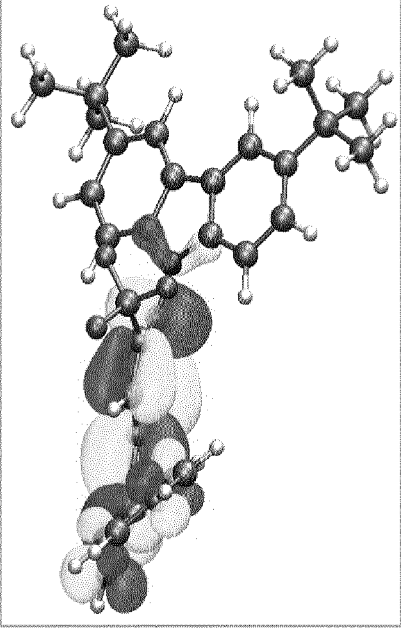 |

| | | |
|---|---|---|
| 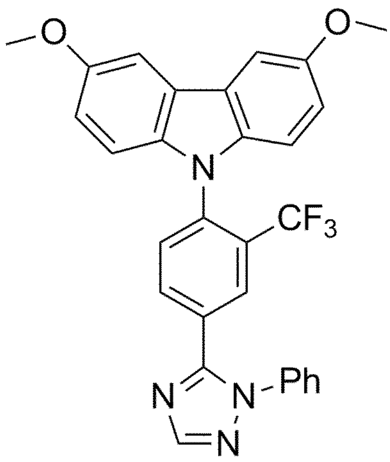 | 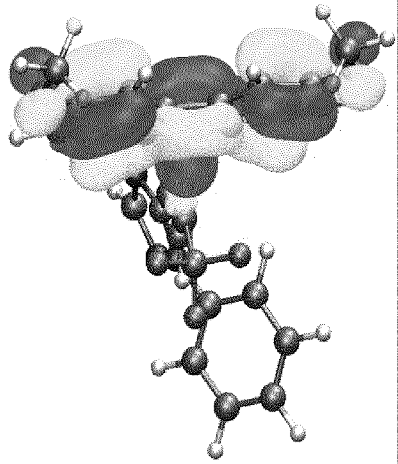 | 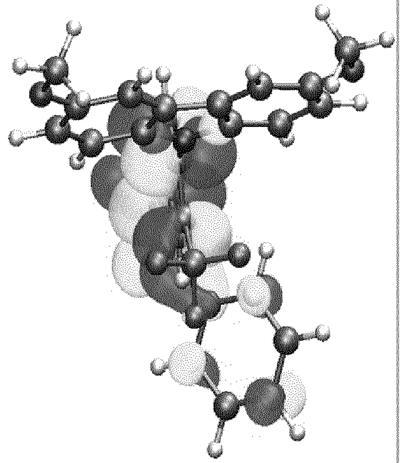 |
| 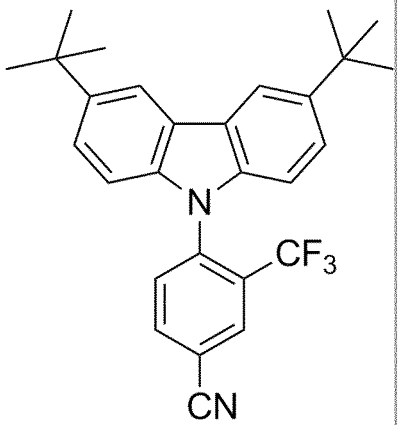 | 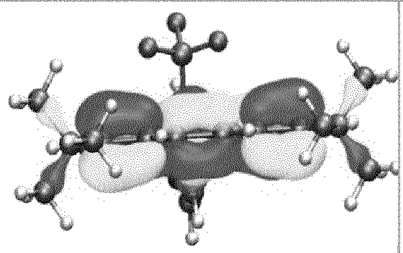 | 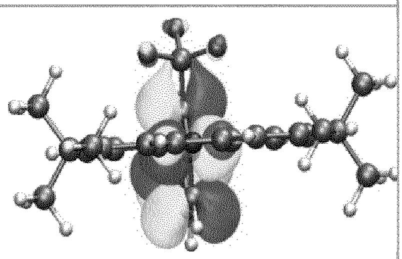 |
| 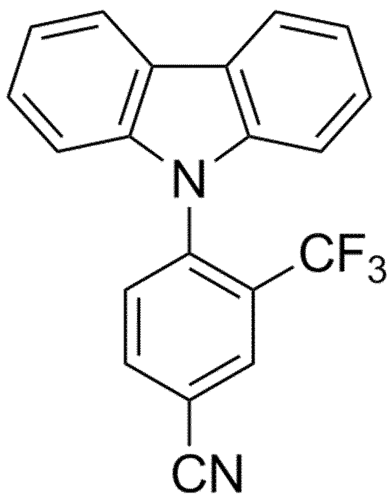 | 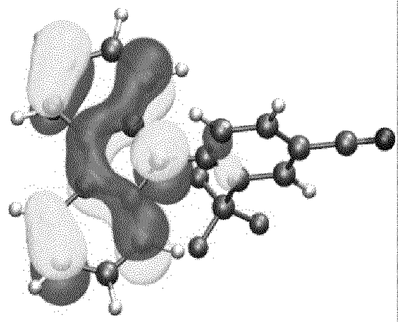 | 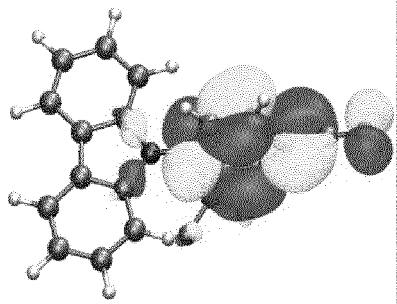 |

| | | |
|---|---|---|
| 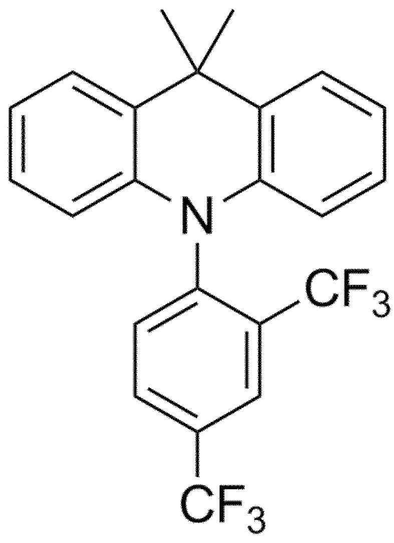 | 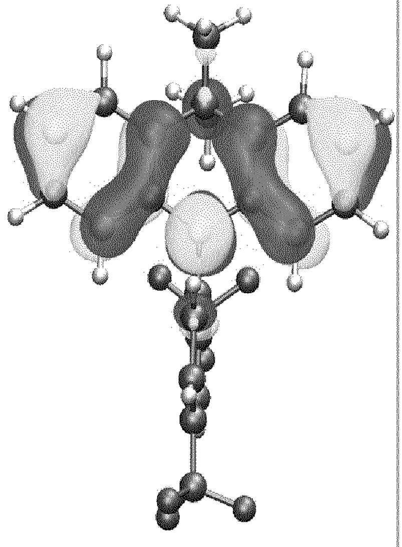 | 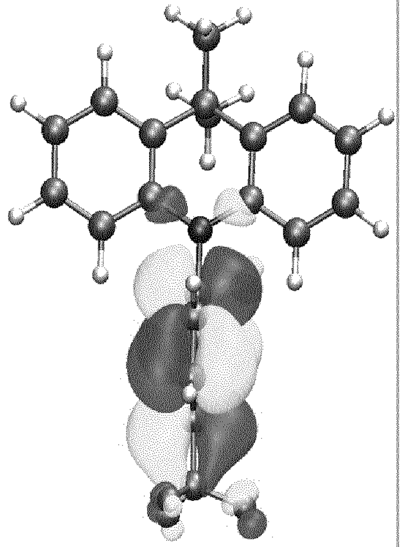 |
| 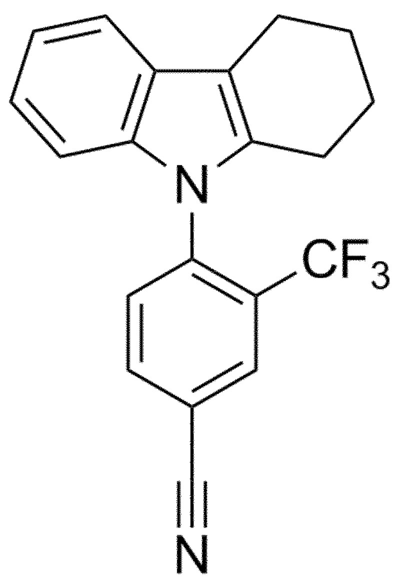 | 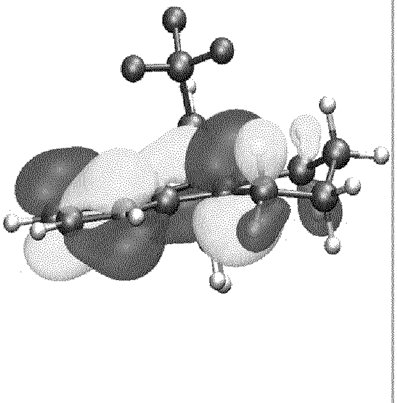 | 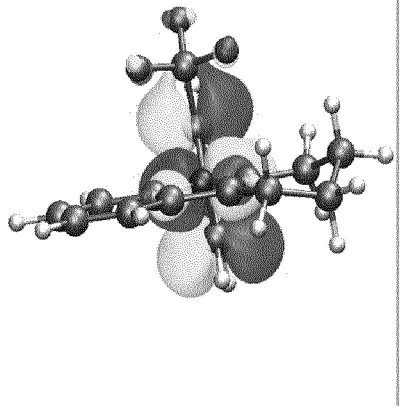 |
| 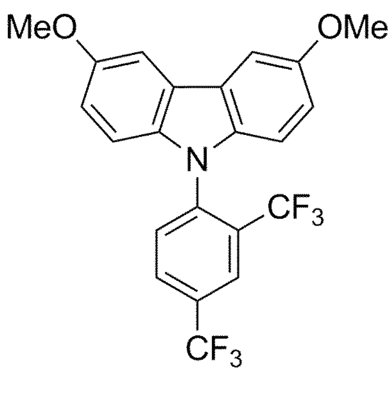 | 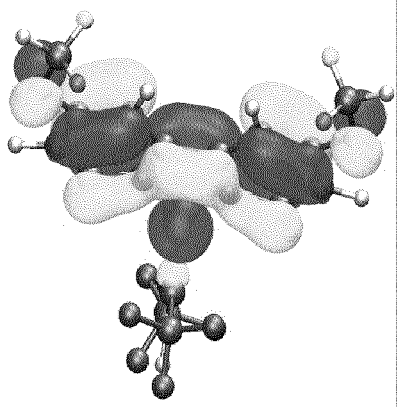 | 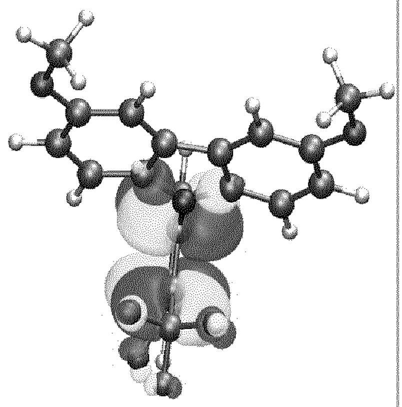 |

| | | |
|---|---|---|
| 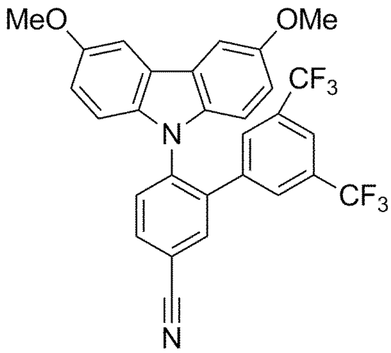 | 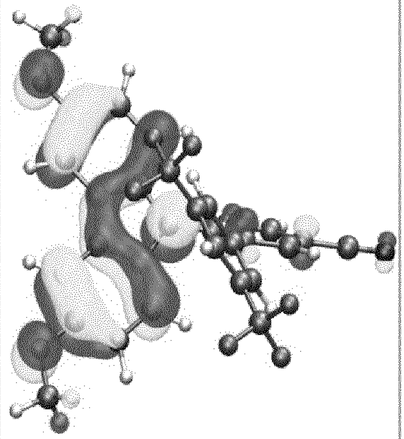 | 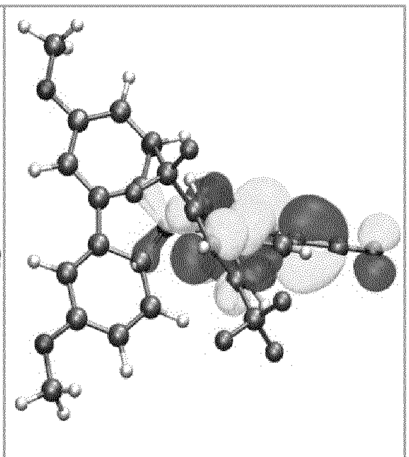 |
| 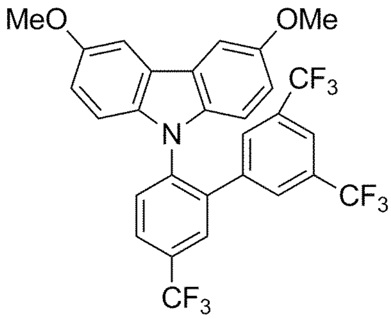 | 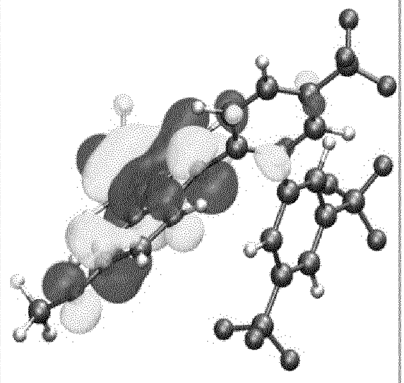 | 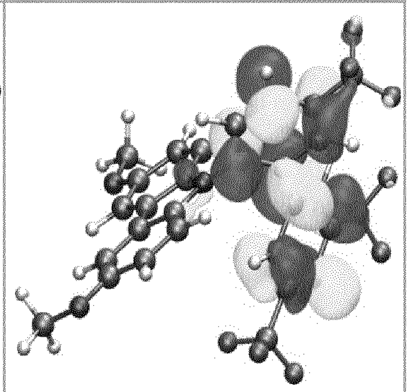 |
| 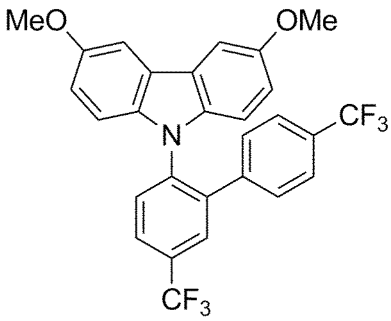 | 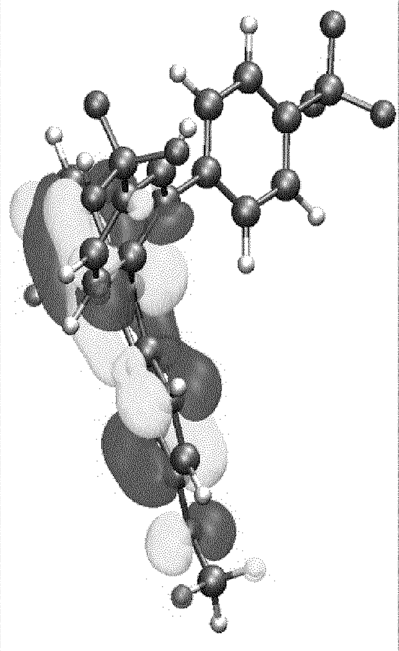 | 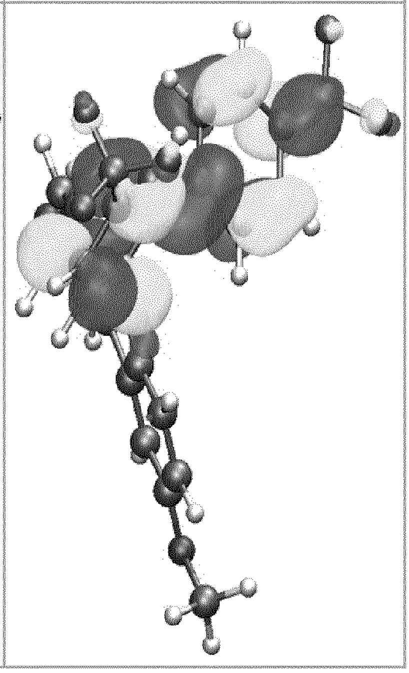 |

| | | |
|---|---|---|
| 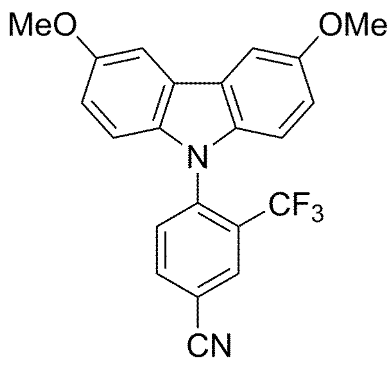 | 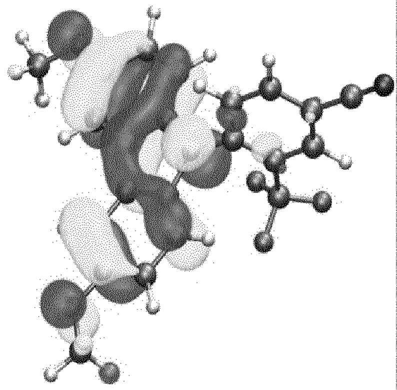 | 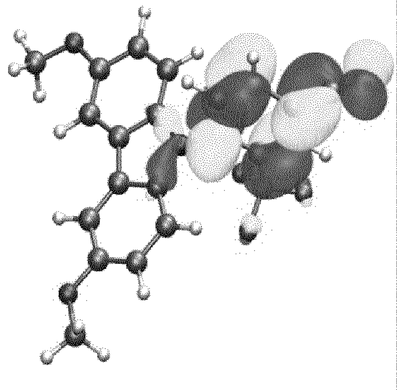 |
| 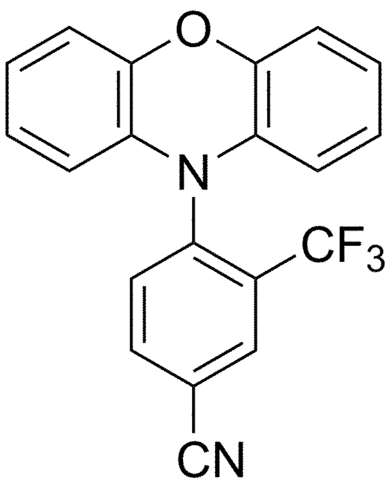 | 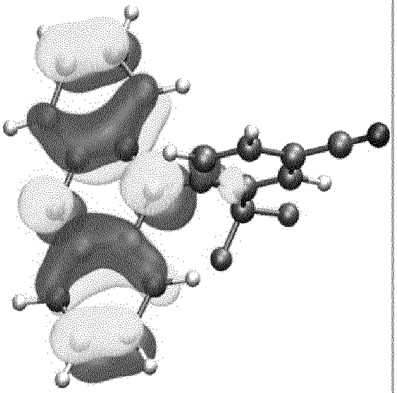 | 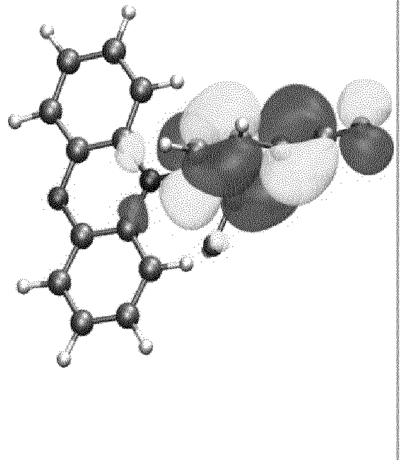 |
| 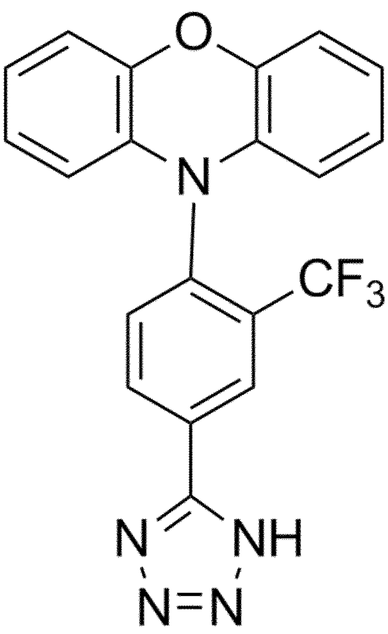 | 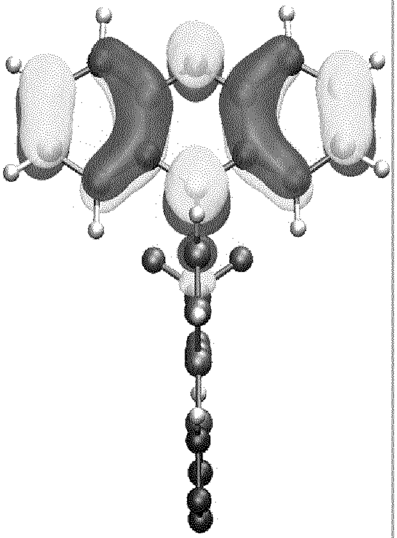 | 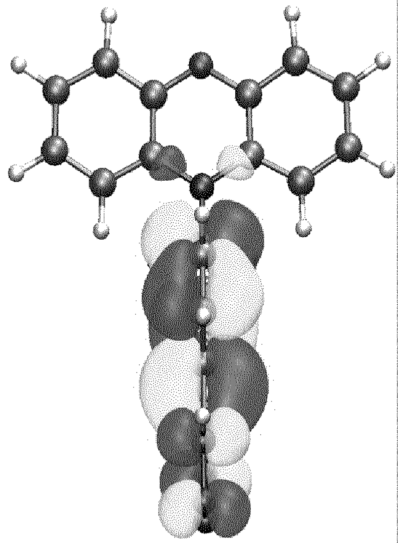 |

| | | |
|---|---|---|
| 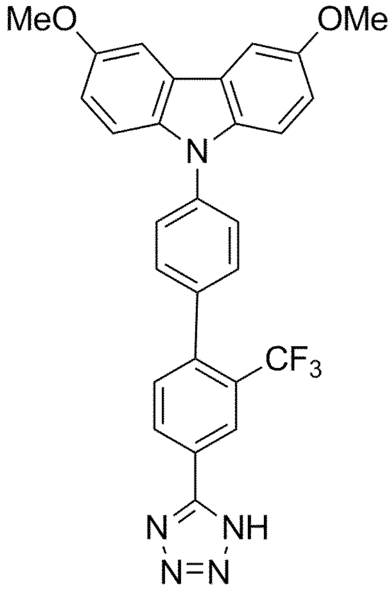 | 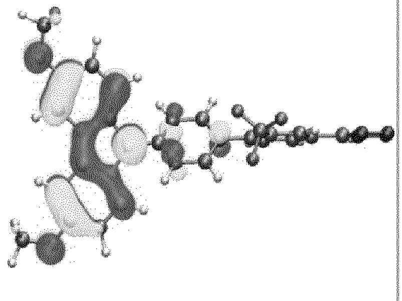 | 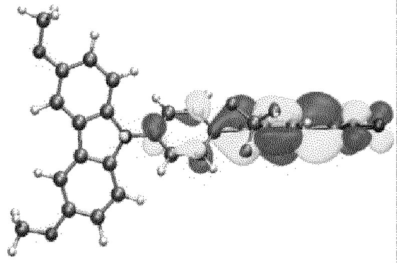 |
| 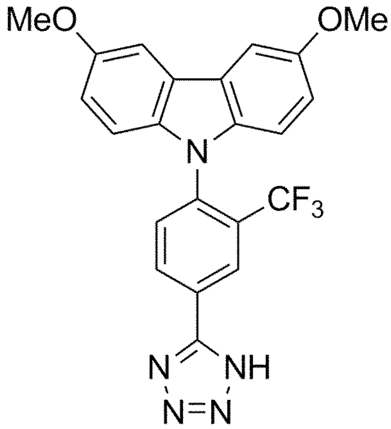 | 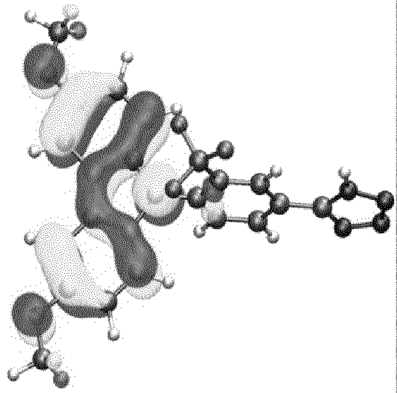 | 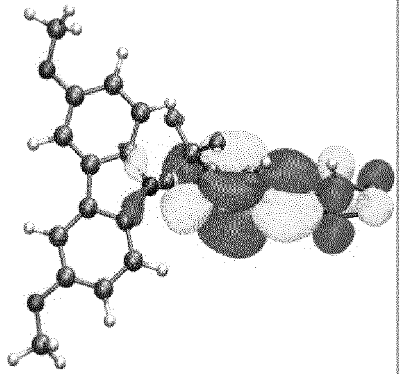 |
| 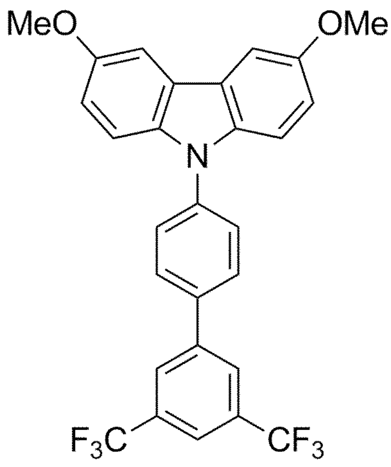 | 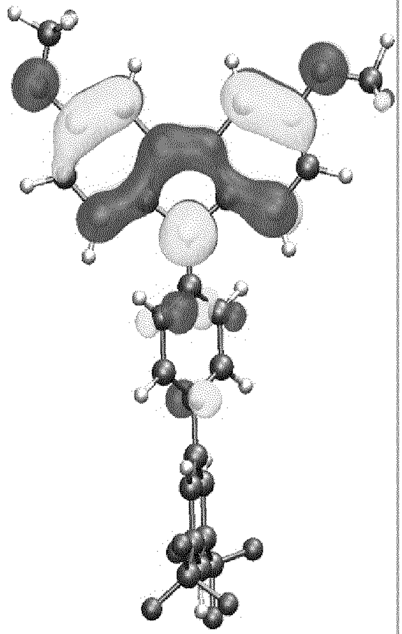 | 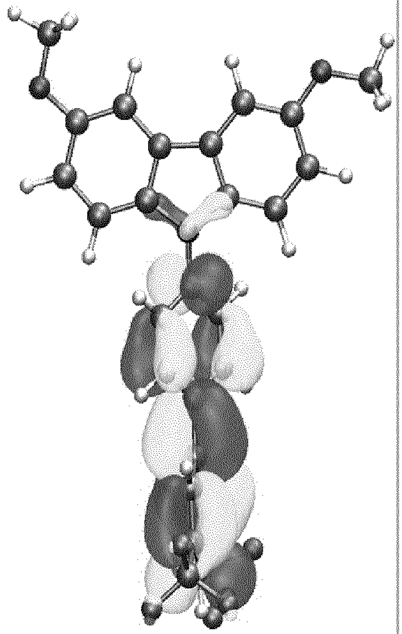 |

| | | |
|---|---|---|
| 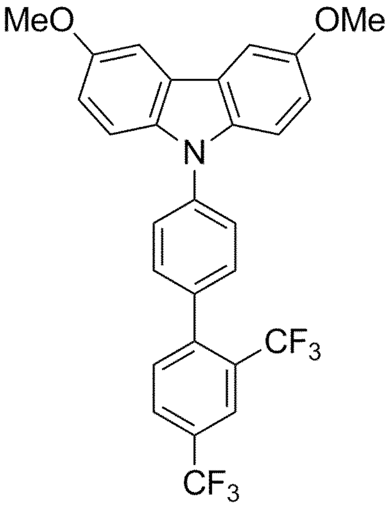 | 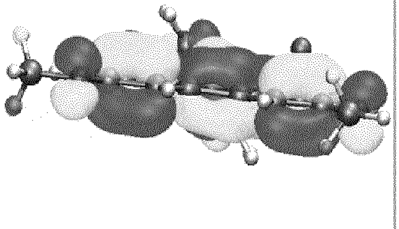 | 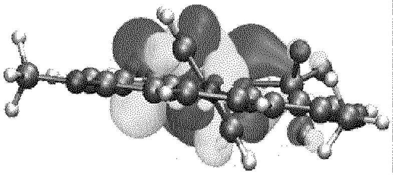 |
| 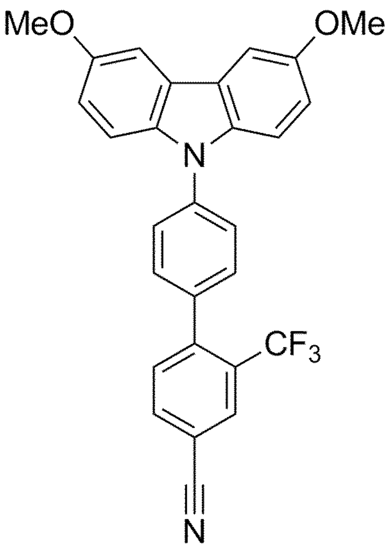 | 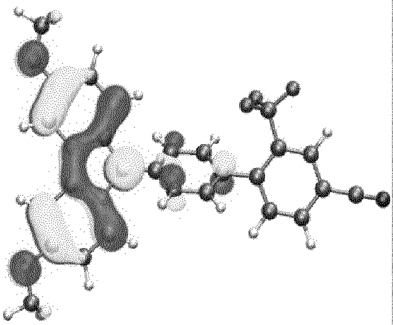 | 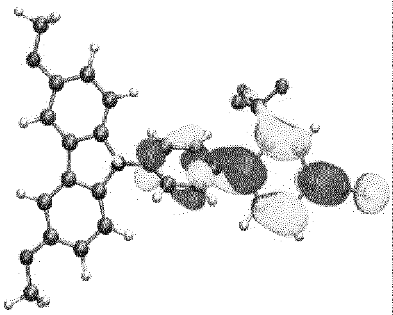 |

Figur 5

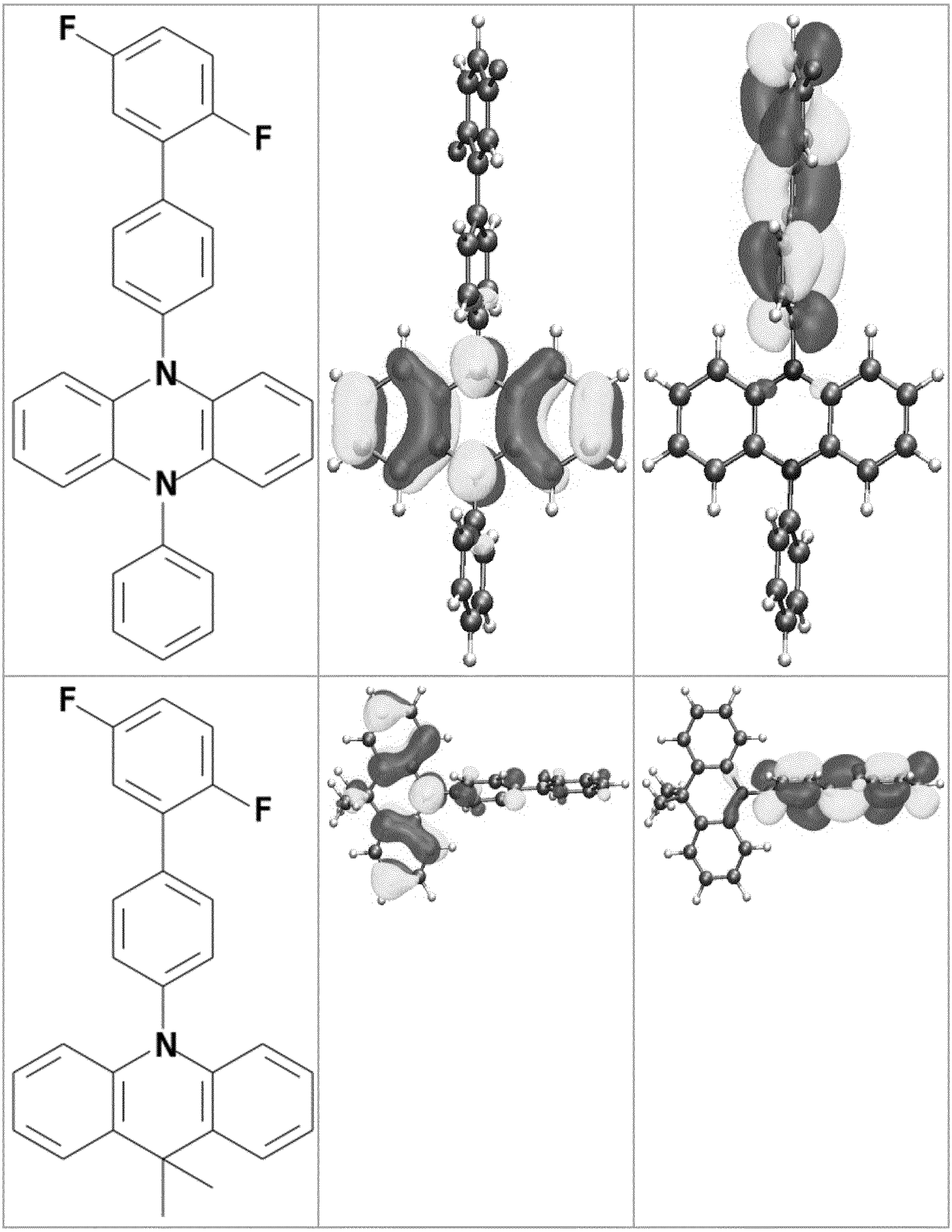

| | | |
|---|---|---|
| 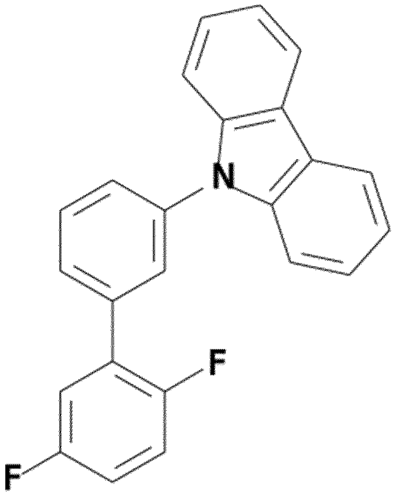 | 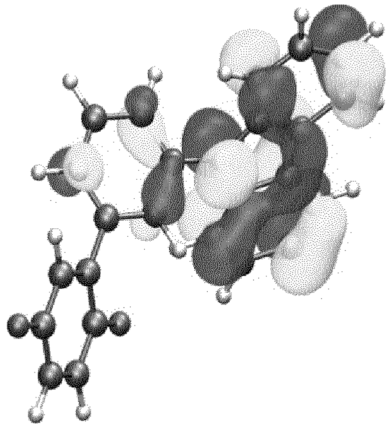 | 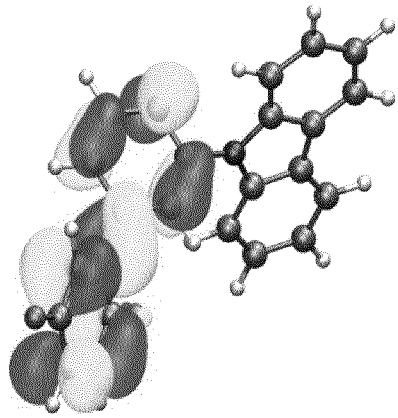 |
| 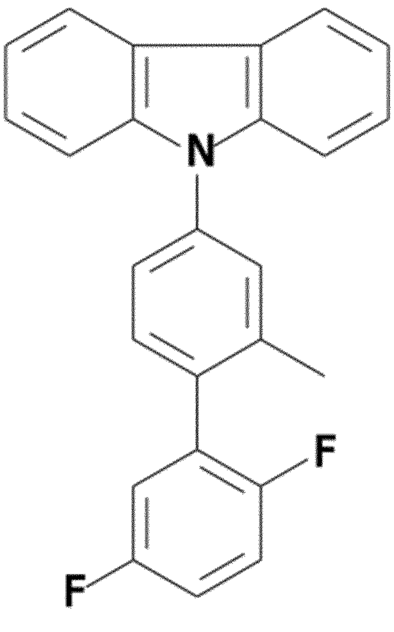 | 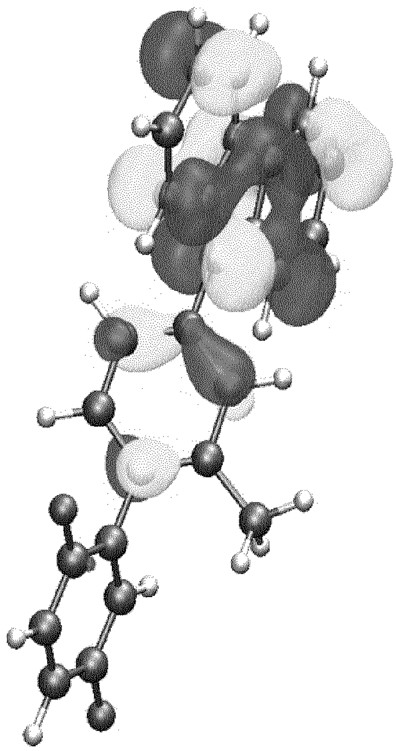 | 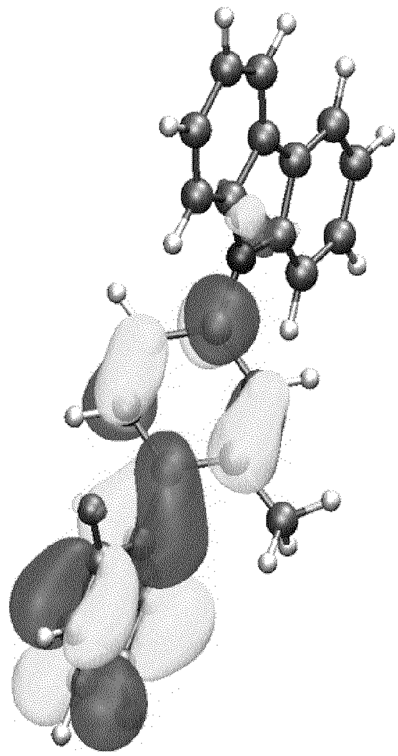 |

| | | |
|---|---|---|
| 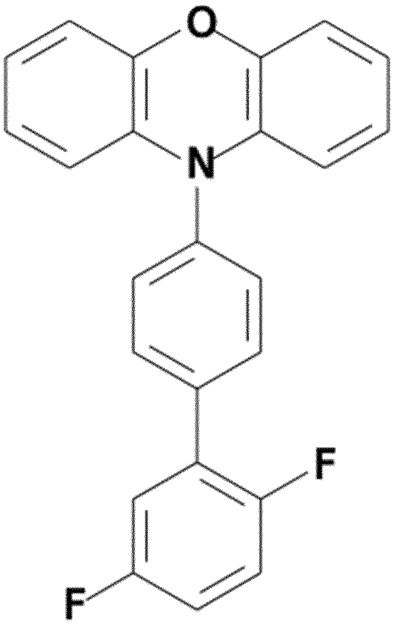 | 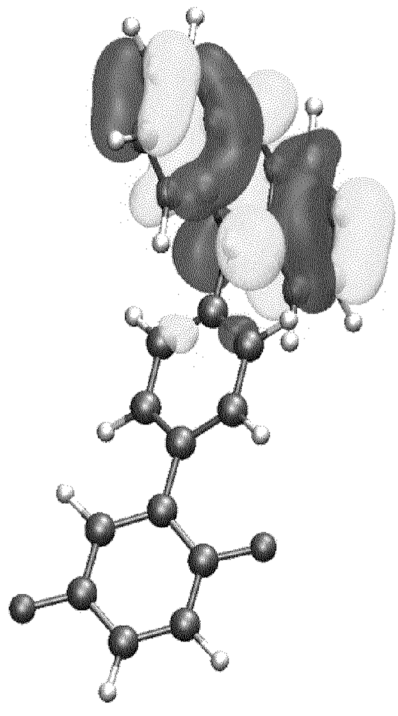 | 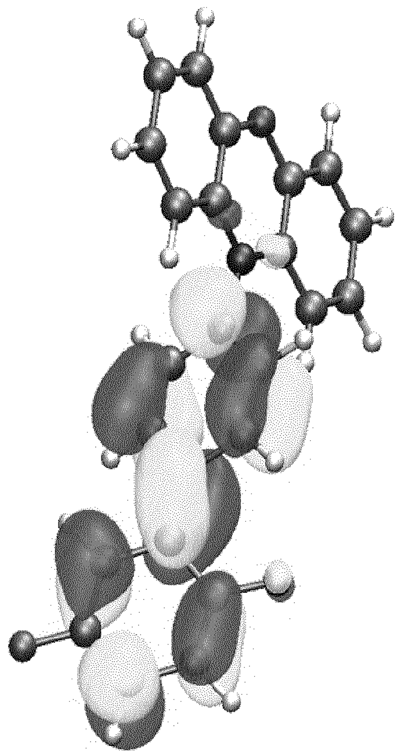 |
| 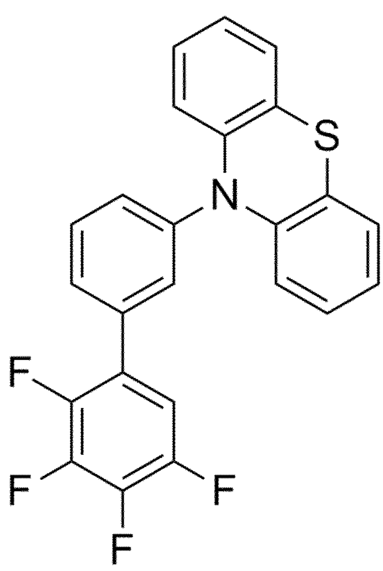 | 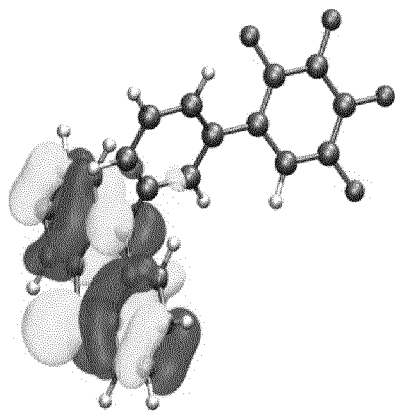 | 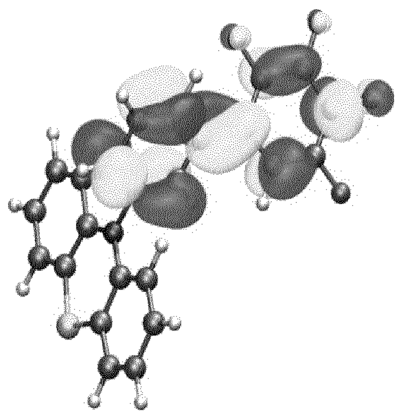 |

| | | |
|---|---|---|
| 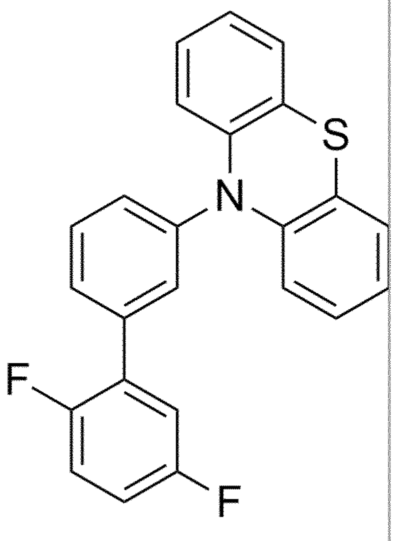 | 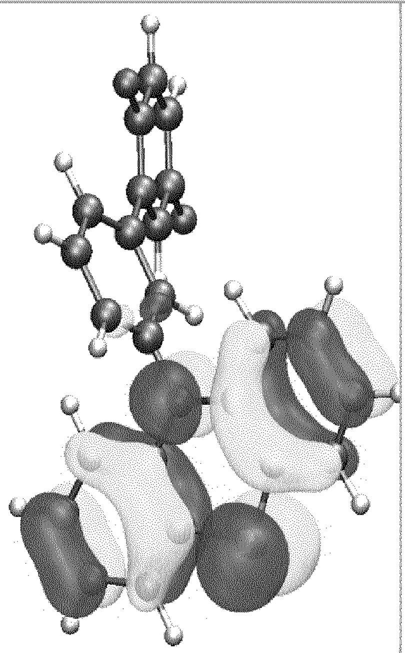 | 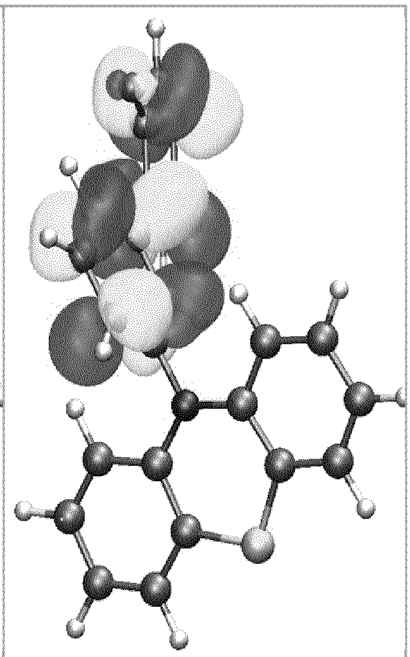 |
| 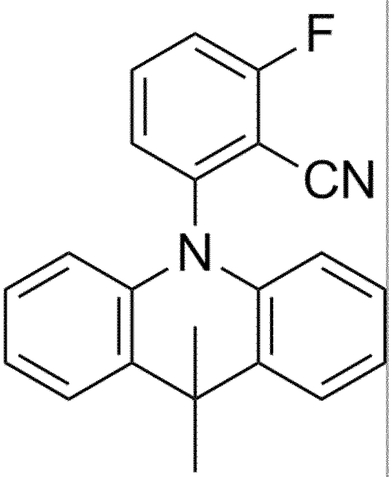 | 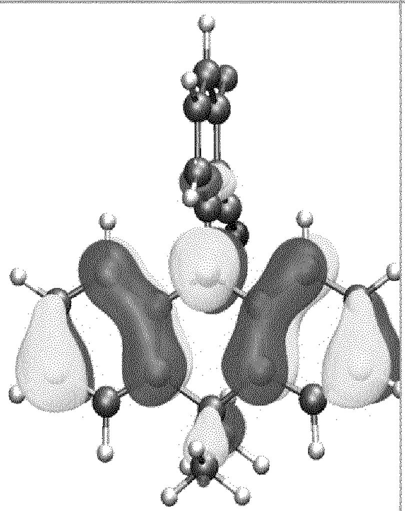 | 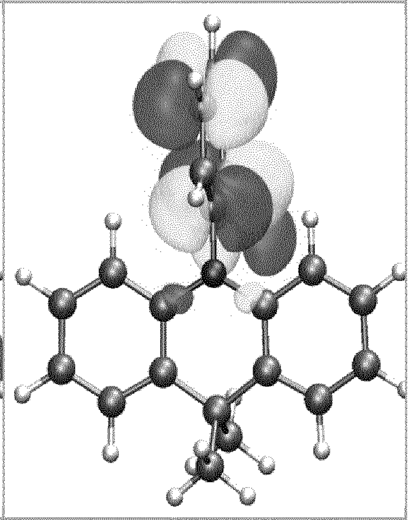 |
| 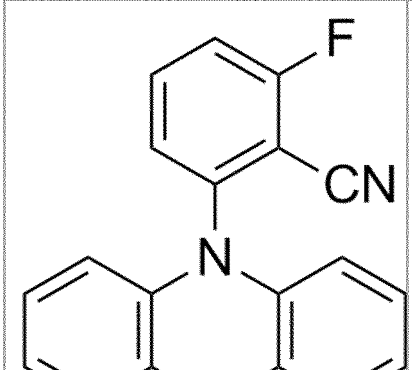 | 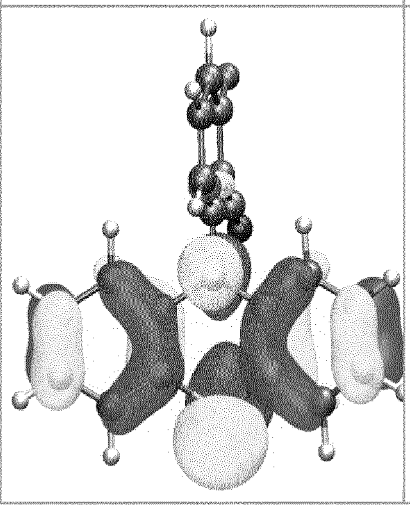 | 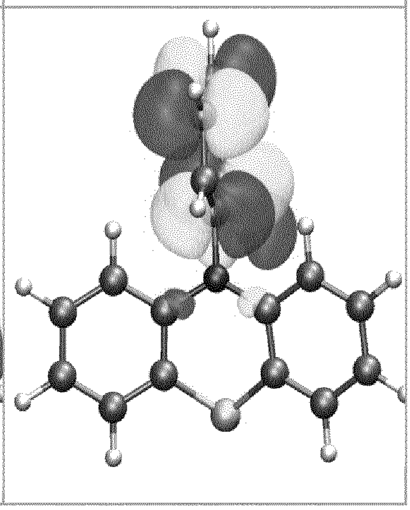 |

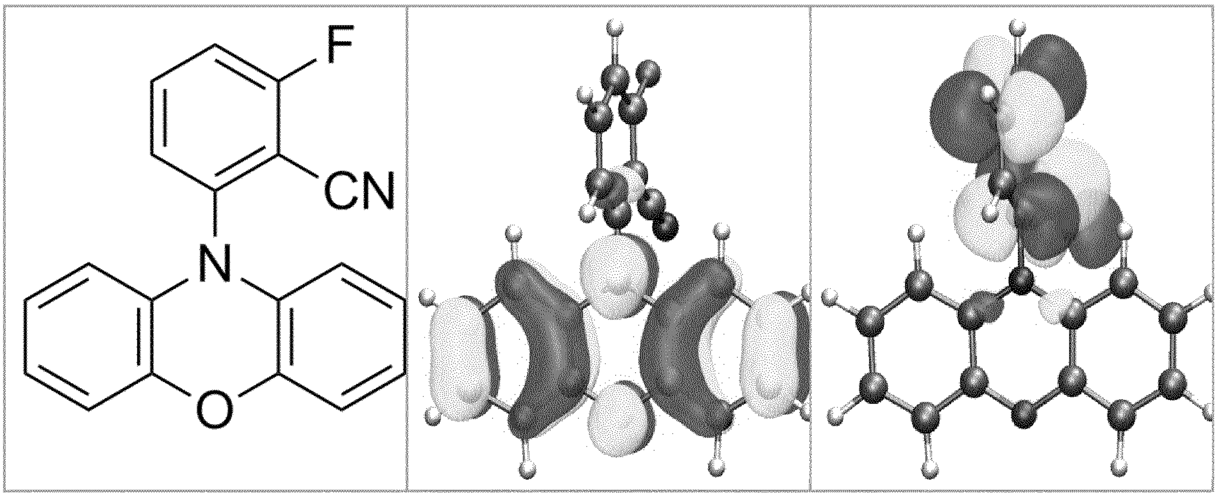

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2016003054 A1
- WO 2014146750 A1
- DE 102010045405 A1


### In der Beschreibung aufgeführte Nicht-Patentliteratur


- **H. YERSIN.** *Top. Curr. Chem.,* 2004, vol. 241, 1
- **H. YERSIN.** Highly Efficient OLEDs with Phosphorescent Materials. Wiley-VCH, 2008
- **TANG et al.** *Appl. Phys. Lett.,* 1987, vol. 51, 913
- **SIEHE DAZU AUCH H. YERSIN.** *Top. Curr. Chem.,* 2004, vol. 241, 1
- **M. A. BALDO ; D. F. O'BRIEN ; M. E. THOMPSON ; S. R. FORREST.** *Phys. Rev. B,* 1999, vol. 60, 14422
- **C. LIU et al.** *Chinese Chemical Letters,* 2016, vol. 27 (5), 631-634
- **BECKE, A. D.** *Phys. Rev. A,* 1988, vol. 38, 3098-3100
- **PERDEW, J. P.** *Phys. Rev. B,* 1986, vol. 33, 8822-8827
- **PERDEW, J. P.** *Phys. Rev. B,* 1986, vol. 33, 8822-8827
- **SIERKA, M. ; HOGEKAMP, A. ; AHLRICHS, R. J.** *Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **LEE, C ; YANG, W ; PARR, R.G.** *Phys. Rev. B,* 1988, vol. 37
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F. J.** *Chem. Phys.,* 2010, vol. 133, 134105, , 1-134105, 11
- **S. LINDNER et al.** *Journal of Fluorine Chemistry,* 2015, vol. 179, 102-105